# EUROPEAN PATENT APPLICATION

(11) **EP 3 150 626 A1**
(43) Date of publication of application: **05.04.2017**
(21) Application number: 15187968.1
(22) Date of filing: 01.10.2015
(51) Int. Cl.: C07K 14/415, C12N 15/82

(54) **MEANS AND METHODS FOR ACCELERATED BREEDING BY INDUCING TARGETED STIMULATION OF MEIOTIC RECOMBINATION**

(71) Applicant: University of Vienna, 1010 Vienna (AT)
(72) Inventor: SCHLÖGELHOFER, Peter, 1070 Vienna (AT); JANISIW, Michael, 7011 Siegendorf (AT)
(74) Representative: Vossius & Partner Patentanwälte Rechtsanwälte mbB

(57) **Abstract**

The present invention generally relates to fusion proteins, nucleic acids, vectors, host cells carrying the fusion proteins or nucleic acids or vectors of the present invention for expressing the fusion proteins of the present invention, methods and kits to increase the rate of meiotic recombination and to specifically target meiotic recombination to a specific locus in a meiotic cell such as, e.g., a meiotic plant cell. The invention relates to fusion proteins comprising a zinc finger domain, which is operably linked via its N-terminus to the C-terminus of a SPO11 polypeptide, wherein the zinc finger domain comprises six or more zinc finger modules.

## Description

The present invention generally relates to fusion proteins, nucleic acids, vectors, host cells carrying nucleic acids or vectors of the present invention for expressing the fusion proteins of the present invention, methods and kits to increase the rate of meiotic recombination and to specifically target meiotic recombination to a specific locus in a meiotic cell such as, e.g., a meiotic plant cell. The invention relates to fusion proteins comprising a zinc finger domain which is operably linked via its N-terminus to the C-terminus of a SPO11 polypeptide, wherein the zinc finger domain comprises six or more zinc finger modules.

### BACKGROUND OF THE INVENTION

In sexually reproducing organisms meiosis ensures the reduction of the genome prior to the formation of generative cells. The halving of the DNA content of a diploid germ line cell during the meiotic cell cycle allows the production of haploid gametes. During meiosis, recombination ensures proper segregation of chromosomes during the first meiotic division. Additionally, genetic information between maternal and paternal chromosomes is exchanged, leading to novel combinations of genetic traits in the following generation. The molecular basis of this process is recombination between homologous chromosomes and depends on the formation of DNA double-strand/double-stranded breaks (DSBs). These breaks are formed at non-random sites throughout the genome, called meiotic hot spots (Gerton, J.L. et al., PNAS, 2000, 97(21), pp.11383-11390). Recombination plays a dual role: it shuffles information along the lengths of homologous chromosomes, creating genetic diversity that is transmitted to progeny, and it ensures the proper segregation of homologs to opposite poles during the first of the two meiotic divisions. About a century ago, it was predicted that exchanges take place between homologous maternal and paternal chromosomes during hereditary transmission. Thereafter partial linkage between the petal color and pollen shape characters in Sweet Pea was discovered. These and successive discoveries in the emerging field of recombination led to the notion of genetic distances, as measured by the frequency of exchange (crossing-over) between linked markers, and to the development of linkage maps. Since the advent of the molecular era, this prescient insight has been extensively verified by quantitative comparisons of genetic and physical distances. For all organisms, including the yeast *Saccharomyces cerevisiae, Arabidopsis thaliana, Drosophila melanogaster, Mus musculus,* and humans, meiotic recombination rates (expressed as cM/kb) vary by several orders of magnitude along chromosomes (Gerton, J.L. et al., 2000, PNAS, 97(21), pp.11383-11390; Chen, S.Y. et al., 2008, Developmental cell, 15(3), pp.401-415; Esberg, A. et al., 2011, PLoS ONE, 6(9), p.e25211; Drouaud, J. et al., 2006, Genome research, 16(1), pp.106-114; Giraut, L. et al., 2011, PLoS Genetics, 7(11), p.e1002354; Brunschwig, H. et al., 2012, Genetics; Lee, Y.-S. et al., 2011, BMC genomics, 12, p.434; Wang, J. et al., 2012, Cell, 150(2), pp.402-412; Nishant, K.T. & Rao, M.R.S., 2006, BioEssays, 28(1), pp.45-56). Studies in budding yeast suggest that variation in recombination is related to frequencies of DSB formation (Baudat, F. & Nicolas, A., 1997, PNAS, 94(10), pp.5213-5218) but did not explain why it is relatively frequent at some loci (hotspots) and relatively infrequent at others (coldspots). Meiotic recombination results from the formation and repair of programmed DNA double-strand/double stranded breaks (DSBs). Numerous studies have shown that natural DSB sites are not evenly distributed and that cleavage frequencies vary 10-100-fold from site to site (Baudat, F. & Nicolas, A., 1997, PNAS, 94(10), pp.5213-5218; Gerton, J.L. et al., PNAS, 2000, 97(21), pp.11383-11390). The factors that determine whether a specific region or site is prone to DSB formation (and hence, recombination) are not completely understood, but they are known to act both locally and globally. Locally, gene organization and chromatin structure appear to be of paramount importance. Typically, most natural DSB sites are in promoter-containing regions (Baudat, F. & Nicolas, A., 1997, PNAS, 94(10), pp.5213-5218). More notably, all known DSB sites are located in regions that are sensitive to DNase I or micrococcal nuclease (MNase I) in both mitotic and meiotic cells, suggesting that an open chromatin configuration is necessary for cleavage (Ohta K. et al., 1994, EMBO J, 13, pp.5754-5763; Wu T.-C. and Lichten M., 1994, Science, 263, pp.515-518). However, local chromatin accessibility cannot be the sole arbiter of DSB site selectivity, because not all nuclease-hypersensitive sites are DSB sites.

In eukaryotic organisms from fungi to humans meiotic DSB formation is initiated by the conserved protein Spo11 (Bergerat, A. et al., Nature, 1997, 386(6623), pp.414-417; Romanienko, P.J. & Camerini-Otero, R.D., 1999, Genomics, 61(2), pp.156-169; Keeney, S. et al., 1999, Genomics, 61(2), pp.170-182; Sharif, W.D. et al., 2002, Cell & chromosome, 1(1), p.1; Hartung, F. & Puchta, H., 2000, Nucleic Acids Research, 28(7), pp.1548-1554; Grelon, M. et al., 2001, The EMBO Journal, 20(3), pp.589-600; Stacey, N.J. et al., 2006, The Plant journal : for cell and molecular biology, 48(2), pp.206-216; Yu, H. et al., 2010, Chromosoma, 119(6), pp.625-636). DSBs are formed by a multi-protein complex with the Spo11 protein being its catalytically active subunit. Initiation of meiotic recombination has been best studied in yeast, but is largely enigmatic in higher eukaryotes. While mammalian model systems aim at understanding meiosis to benefit human health and fertility, the model plant *Arabidopsis thaliana* is the organism of choice to elucidate meiotic recombination and its stimulation in higher plants.

Meiotic recombination depends on the controlled formation of DSBs mediated by Spo11, a homologue of the archaebacterial topoisomerase subunit Top6A (Bergerat, A. et al., Nature, 1997, 386(6623), pp.414-417; Keeney, S. et al., 1997, Cell, 88(3), pp.375-384). This process is highly regulated and to present date 12 genes essential for meiotic DSB formation (Spo11/Ski8, Rec102/Rec104, Rec114/Mei4/Mer2, Cdc7, Dbf4, and Mre11/Rad50/Xrs2) and 10 genes required for WT-levels of DSB formation (Red1, Hop1, Mek1, Rec8, Scc3, Tel1, Mec1, Set1, Spp1, Pch2) have been identified in budding yeast (Keeney, S., 2001. Current topics in developmental biology, 52, pp.1-53; Edlinger, B. & Schlögelhofer, P., 2011, Journal of Experimental Botany, 62(5), pp.1545-1563; de Massy, B., 2013, Annual review of genetics, 47, pp.563-599).

As an intermediate of the DNA cleavage process, Spo11 proteins remain covalently linked to the 5' termini of single-stranded DNA (ssDNA) at the incision sites and have to be removed (Bergerat, A. et al., 1997, Nature, 386(6623), pp.414-417; Keeney, S. et al., 1997, Cell, 88(3), pp.375-384; Neale, M.J. et al., 2005, Nature, 436(7053), pp.1053-1057). To release Spo11 from the DNA ends, DNA is nicked at a distance from the incision site by the MRX complex [Mre11-Rad50-Nbs1/Xrs2] in conjunction with Com1/Sae2 (Alani, E. et al., 1990, Cell, 61(3), pp.419-436; Cao, L. et al., 1990, Cell, 61(6), pp.1089-1101; McKee, A.H. & Kleckner, N., 1997, Genetics, 146(3), pp.797-816; Nairz, K. & Klein, F., 1997. Genes & Development, 11(17), pp.2272-2290; Prinz, S. et al., 1997, Genetics, 146(3), pp.781-795; Longhese, M.P. et al., 2009, DNA Repair, 8(9), pp.1127-1138; Mimitou, E.P. & Symington, L.S., 2009, DNA Repair, 8(9), pp.983-995; Ivanov, D. & Nasmyth, K., 2005, Cell, 122(6), pp.849-860). It is the endonuclease activity of Mre11 that mediates ssDNA nick formation and the 3'-5' exonuclease activity of Mre11 that resects ssDNA towards the Spo11 protein. Spo11 is then released from the nascent cleavage site with a short DNA oligonucleotide remaining attached to the Spo11 protein (Neale, M.J. & Keeney, S., 2006, Nature, 442(7099), pp.153-158).

To yield long stretches of ssDNA that can probe for matching strands on homologous chromosomes (or in some cases on sister chromatids) three proteins have gained attention. It seems that the exonucleases Exo1 and Dna2 together with the helicase Sgs1 are instrumental in 5'-3' strand resection, starting at the Mre11-mediated ssDNA lesions to yield long single-stranded overhangs (Cromie, G. & Smith, G.R., 2008, Genome dynamics and stability, 3, p.195; Mimitou, E.P. & Symington, L.S., 2008, Nature, 455(7214), pp.770-774; Zhu, Z. et al., 2008, Cell, 134(6), pp.981-994; Longhese, M.P. et al., 2010, The EMBO Journal, 29(17), pp.2864-2874.; Manfrini, N. et al., 2010, The Journal of biological chemistry, 285(15), pp.11628-11637). The ssDNA is bound with high affinity by replication protein A (RPA) (Fanning, E., 2006, Nucleic Acids Research, 34(15), pp.4126-4137; Broderick, S. et al., 2010, Sub-cellular biochemistry, 50, pp.143-163), a prerequisite for the loading of the strand exchange proteins Rad51 and Dmc1. In yeast, this loading is mediated by Rad52 and accessory proteins, among them Rad54, Rad55, Rad57, Rad59, and Rdh54/Tid1 (Krogh, B.O. & Symington, L.S., 2004, Annual review of genetics, 38, pp.233-271). In higher eukaryotes, the BRCA2 protein has been found to be essential for this step (a detailed description of these post-DSB steps has been reviewed in, for example, Longhese, M.P. et al., 2010, The EMBO Journal, 29(17), pp.2864-2874). Specialized meiotic DNA repair proteins, together with other DNA repair factors, mediate strand invasion, strand elongation by DNA synthesis, capture of the second DNA end, and subsequent repair and ligation of two different DNA strands to yield novel allelic combinations (Hunter, N., 2007, Meiotic recombination. In Topics in Current Genetics. Berlin, Heidelberg: Springer Berlin Heidelberg, pp. 381-442).

Even though many proteins involved in meiotic recombination are highly conserved, only six genes (SPO11-1, SPO11-2, PRD1, PRD2, PRD3 and DFO) have been identified to be essential for DSB formation in *Arabidopsis thaliana* and possibly are also present in other higher plants (Grelon, M. et al., 2001, The EMBO Journal, 20(3), pp.589-600; Stacey, N.J. et al., 2006, The Plant journal: 48(2), pp.206-216; Hartung, F. et al., 2007, The Plant cell, 19(10), pp.3090-3099; De Muyt, A. et al., 2007, The EMBO Journal, 26(18), pp.4126-4137; De Muyt, A. et al., 2009, PLoS Genetics, 5(9), p.e1000654; Zhang, C. et al., 2012, The Plant Journal, 72(2), pp.271-281). AtPRD1 has low similarity to mammalian Mei1. The mouse Mei1 was isolated and characterized in a mutant screen for infertility (Libby, B.J. et al., 2002, Developmental biology, 242(2), pp.174-187). The N-terminus of AtPRD1 interacts with AtSPO11-1 in a yeast two-hybrid assay, thereby identifying for the first time an interaction partner of a SPO11 protein in plants. The functional relevance of this interaction is unknown (De Muyt, A. et al., 2007, The EMBO Journal, 26(18), pp.4126-4137). AtPRD2 was later recognized as a homologue of Mei4 (Kumar, R. et al., 2010, Genes & Development, 24(12), pp.1266-1280). AtPRD3 is a protein of unknown function, similar to the previously identified rice PAIR1 gene, but with no homologues outside the plant kingdom (Nonomura, K.-I. et al., 2004, Molecular genetics and genomics, 271(2), pp.121-129; De Muyt, A. et al., 2009, PLoS Genetics, 5(9), p.e1000654). DFO is a protein without any known conserved domain and no homolog outside the plant kingdom is identified so far (Zhang, C. et al., 2012, The Plant Journal, 72(2), pp.271-281). In different independent screens, the plant SWI1 gene has been found to affect plant fertility. The data suggest that SWI1 is required for meiotic chromatin remodelling, sister chromatid cohesion, chromosome pairing, synapsis, and recombination. No meiotic DSBs are formed in swi1 mutants (Siddiqi, I. et al., 2000, Development (Cambridge, England), 127(1), pp.197-207; Mercier, R. et al., 2001, Genes & Development, 15(14), pp.1859-1871; Mercier, R. et al., 2003, Development (Cambridge, England), 130(14), pp.3309-3318. The meiotic protein SWI1 is required for axial element formation and recombination initiation in *Arabidopsis* (Agashe, 2002, Development (Cambridge, England), 130(14), pp.3309-3318; Boateng, K.A. et al., 2008, Molecular plant, 1(4), pp.620-633).

Global determinants likewise control the distribution of DSBs. Both the fine mapping of DSB sites on yeast chromosome III and the genome-wide mapping of DSB sites have confirmed the existence of large subchromosomal domains hot or cold for DSB formation (Baudat, F. & Nicolas, A., 1997, PNAS, 94(10), pp.5213-5218; Gerton, J.L. et al., 2000, PNAS, 97(21), pp.11383-11390). The molecular basis of these DSB-proficient or refractory domains has not been elucidated, but the finding that a recombination proficient reporter inserted at various sites along chromosome III adopts local properties with respect to DNasel sensitivity and frequencies of DSB formation and recombination demonstrates that domain-level controls are superimposed on local determinants (Borde, V., Wu, T.C. & Lichten, M., 1999, Molecular and Cellular Biology, 19(7), pp.4832-4842). That is, a hot region can be made cold, but thus far the converse has not been observed: cold regions typically remain cold.

Zinc fingers (ZFs) domains, the most abundant DNA-binding motifs encoded in eukaryotic genomes, offer a well understood protein-DNA binding mechanism (Wolfe, S.A. et al., 2000, Annu. Rev. Biophys. Biomol. Struct., 29, pp.183-212; Pabo, C.O. et al., 2001, Annu. Rev. Biochem., 70, pp.313-340; Moore, M. et al., 2003, Brief Funct. Genomic. Proteomic., 1, pp.342-355; and Klug, A. et al., 2005, FEBS Lett., 579, pp.892-894). Zinc finger proteins (ZFP) play an important role in a variety of cellular functions including transcriptional regulation, DNA and RNA binding, regulation of apoptosis, and protein-protein interactions. ZFPs can be classified into several subtypes, depending on the number and order of cysteine and histidine residues within the motif (Klug, A. and J. W. Schwabe, 1995, FASEB J, 9(8), pp.597-604; Mackay, J. P. and M. Crossley, 1998, Trends Biochem Sci, 23(1), pp.1-4). The ZFs bind to the major groove of B-DNA and wrap way around the double helix. Each ZF domain is consisting of about only 30 amino acids, including two conserved cysteine and two conserved histidine residues, which are tetrahydrally bound to a single zinc ion.

WO-A1 2004/016795 describes a method for recruitment of Spo11 to selected DNA sequences of *Saccharomyces cerevisiae* by designing an artificial fusion protein consisting of a Gal4 DNA-binding domain and the ScSpo11 protein under control of the ADH1 promoter (Gal4BD-Spo11). However, Gal4BD-Spo11 binding alone was not sufficient to induce DSB formation in DSB cold centromeric regions (Robine, N. et al., 2007, Molecular and Cellular Biology, 27(5), pp.1868-1880) or at ectopic UAS sites integrated at naturally cold loci (Fukuda, T. et al., 2008, Nucleic Acids Research, 36(3), pp.984-997).

Janisiw (Janisiw, M. P., Targeted Meiotic Recombination in Arabidopsis thaliana, Diploma thesis, University of Vienna, Center for Molecular Biology (2009); Document ID: 5477, online catalogue of the University Vienna: http://ubdata.univie.ac.at/AC08156960) describes the construction of fusion proteins consisting of a SPO11 polypeptide and a zinc finger domain, which comprises four zinc finger modules.

In view of the potential of modifying plants by meiotic recombination, there is still a need to provide reliable and improved means and methods suitable for a targeted meiotic homologous recombination in plants.

### Summary of the invention

This need is addressed by the present invention by providing the embodiments as defined in the claims.

In particular, the present invention relates to a fusion protein comprising a SPO11 polypeptide and a zinc finger domain, which comprises six or more zinc finger modules.

Also encompassed by the present invention are methods based on the fusion protein of the present invention for increasing recombination between two defined loci of two homologous chromosomes in a meiotic plant cell.

The herein described fusion protein is characterized in that the zinc finger domain is operably linked via its N-terminus to the C-terminus of the SPO11 polypeptide and that the zinc finger domain comprises six or more zinc finger modules. By using six or more zinc finger modules operably linked to a plant SPO11 polypeptide, which is encoded by a genomic DNA construct, SPO11 could be targeted to a certain genomic locus. By using a fusion to the C-terminus (instead of the N-terminus) of the SPO11 polypeptide, meiotic recombination at this site could be increased.

The invention further relates to nucleic acids, host cells carrying the nucleic acid or the fusion protein of the invention and kits and the use thereof in the methods of the invention.

The invention also relates to a method for generating variants of a plant by using a nucleic acid or fusion protein of the invention.

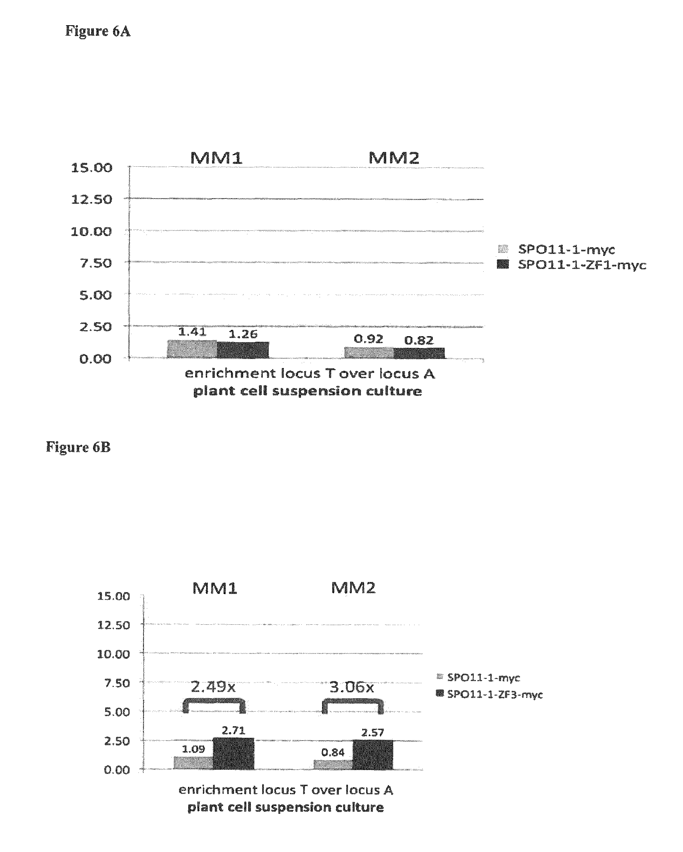

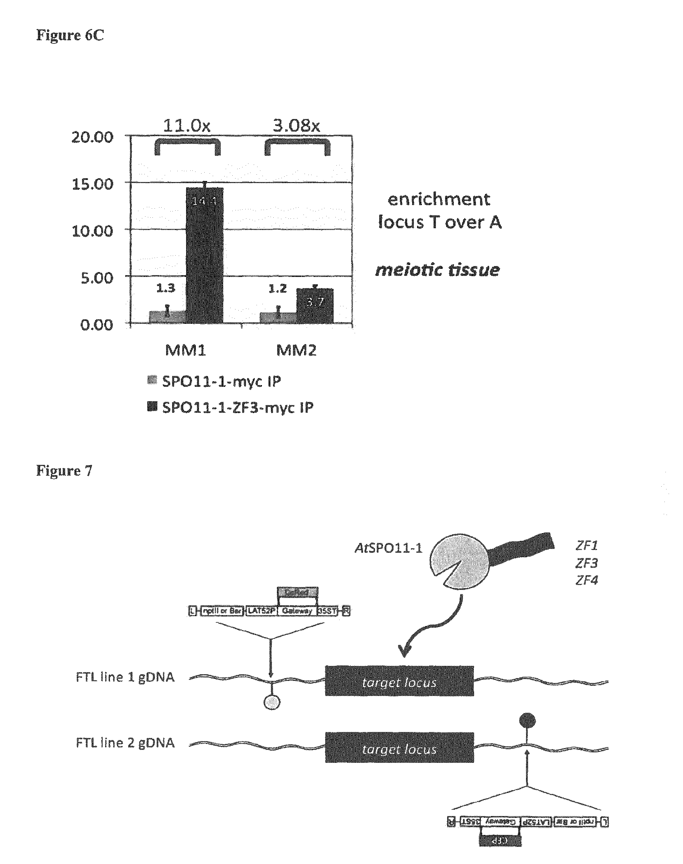

As shown in the appended examples, as a proof of the inventive concept, a fusion protein between the *Arabidopsis thaliana* SPO11-1 and a six-finger zinc array displayed enhanced target binding activity *in planta* as well as in meiotic tissue compared to a fusion protein with only four zinc finger modules (cf. Figure 6). Accordingly, it was found that the fusion proteins of the present invention are suitable to stimulate meiotic recombination in the target DNA regions to which the fusion protein was targeted. Since the fusion proteins of the present invention can be removed from plants post exchange of genetic traits, i.e. the fusion protein of the present invention is present in the parental genomes only transiently and will be eliminated after successful recombination, the strategy described herein outlines a novel procedure to improve the genome of a chosen organism without the permanent introduction of heterologous ("transgenic") sequences.

The invention relates to a fusion protein comprising a zinc finger domain which is operably linked via its N-terminus to the C-terminus of a SPO11 polypeptide, wherein the zinc finger domain comprises six or more zinc finger modules, wherein "more" is understood to mean 7, 8, 9, 10, 11, 12, 13, 14, 15. In the context of the present invention, if the fusion protein comprises a zinc finger domain which is operably linked via its N-terminus to the C-terminus of a *Arabidopsis thaliana* SPO11 polypeptide, the zinc finger domain preferably comprises six, seven or eight zing finger modules.

As used herein, the terms "polypeptide", "peptide", and "protein" are used interchangeably to refer to a polymer of amino acid residues. The term also applies to amino acid polymers in which one or more amino acid residues is an artificial chemical mimetic of a corresponding naturally occurring amino acid, as well as to naturally occurring amino acid polymers and non-naturally occurring amino acid polymers.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., an α carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that function in a manner similar to a naturally occurring amino acid. Amino acids may be referred to herein by either their commonly known three letter symbols or by the one-letter symbols recommended by the IUPAC-IUB Biochemical Nomenclature Commission.

In the context of the present invention, the term "fusion protein" relates to a protein, comprising at least two moieties originating from different sources. Accordingly, it may be also understood as a "chimeric protein". It is usually the product of a fusion gene, which is operably linked, comprising coding sequences originating from different sources, wherein said coding sequences are in frame.

In the context of the present invention, the fusion protein may comprise one or more linkers. In particular, one or more linker(s) may be located between the SPO11 polypeptide and the zinc finger domain. The one or more linker(s) may also be located downstream of the zinc finger domain or upstream the SPO11 polypeptide so as to attach further moieties, e.g. tags for the purification and the like. Also encompassed by the present invention are linkers which are located between single zinc finger modules, forming a zinc finger domain. The linker(s) may be a peptide linker, consisting of at least 1, 2, 3, 4, 5, 6, 7, 8, 9, 10, 11, 12, 13, 14, 15 or 16 amino acid residues. Exemplarily, but not limiting, linkers are depicted in SEQ ID NOs: 11, 12 or 27 to 42. The linkers may also be linkers as described by Neutebboom and coworkers (Neuteboom, L.W. et al., 2006, Biochemical and biophysical research communications, 339(1), pp. 263-270). For example, the linkers may comprise or consist of the canonical linker sequence TGEKP or longer modified linker sequences with up to 12 amino acids in length. Thus, in the context of the present invention, linkers consist at least of 5, 6, 7, 8, 9, 10, 11 or 12 amino acid residues.

The linker(s) as used herein may also be a tag. A tag can be a protein or peptide operably fused to the fusion protein of the invention. Examples of an epitope tag include, but are not limited to: glutathione-S-transferase (GST); polyhistidine comprising 2 to 12, preferably 4 or more, more preferably 4 to 7, and further preferably 5 or 6 histidines; FLAG tag (amino acid sequence DYKDDDDK; SEQ ID NO:27); Myc tag (amino acid sequence EQKLISEEDL; SEQ ID NO:28); V5 tag (amino acid sequence GKPIPNPLLGLDST; SEQ ID NO:29); Xpress tag; HQ tag (amino acid sequence HQHQHQ; SEQ ID NO:30); HA tag (amino acid sequence YPYDVPDYA; SEQ ID NO:31); AU1 tag (amino acid sequence DTYRYI; SEQ ID NO:32); T7 tag (amino acid sequence MASMTGGQQMG; SEQ ID NO:33); VSV-G tag (amino acid sequence YTDIEMNRLGK; SEQ ID NO:34); DDDDK tag (amino acid sequence DDDDK; SEQ ID NO:35); S tag (amino acid sequence KETAAAKFERQHIDSC; SEQ ID NO:36); CruzTag09 (amino acid sequence MKAEFRRQESDR; SEQ ID NO:37); CruzTag22 (amino acid sequence MRDALDRLDRLA; SEQ ID NO:38); CruzTag41 (amino acid sequence MKDGEEYSRAFR; SEQ ID NO:39); Glu-Glu tag (amino acid sequence EEEEYMPME; SEQ ID NO:40); Ha.11 tag (amino acid sequence CTPTDVPDYASL; SEQ ID NO:41); KT3 tag (amino acid sequence PPEPET; SEQ ID NO:42); thioredoxin; a maltose binding protein (MBP); an immunoglobulin Fc region; and β-galactosidase.

In the context of the present invention, the term "zinc finger domain" means a domain, consisting of zinc finger modules. Preferably, the zinc finger domain of the fusion protein of the invention comprises six or more zinc finger modules, wherein "more" is understood to mean 7, 8, 9, 10, 11, 12, 13, 14, 15, or 16. In the context of the present invention, if the fusion protein comprises a zinc finger domain which is operably linked via its N-terminus to the C-terminus of an *Arabidopsis thaliana* SPO11 polypeptide, the zinc finger domain preferably comprises six, seven or eight zing finger modules. Preferably, the zinc finger domain is a zinc finger DNA binding domain. Hence, the DNA binding domain, or DBD, may be seen as a domain which has the property to bind to DNA. That is, the zinc finger domain used in the present invention has the ability to bind to a nucleotide-sequence in a sequence-specific manner through the zinc finger modules.

As shown in the appended Examples, as a proof of concept, a "zinc finger domain" can be designed to recognize any suitable portion in a target DNA region of a plant or plant cell. This means that in the context of the present invention the use of the zinc finger domains for inducing meiotic double strand breaks in formerly cold regions in a plant or plant cell via transferring the SPO11 polypeptide to this target region.

The term "zinc finger domain" may be used interchangeably with "zinc finger protein" or "ZFP" or "polydactylic zinc finger" or "PZF". The zinc finger modules used in the present invention are regions of amino acid sequence within the binding domain whose structure is stabilized through coordination of a zinc ion. The zinc finger domains used in the present invention consist of six or more zinc finger modules, wherein "more" is understood to mean 7, 8, 9, 10, 11, 12, 13, 14, 15, or 16, arrayed zinc finger modules, each of which recognizes about 3 bp (base-pair) sub-sites. Accordingly, the zinc finger domains described herein may also be termed "zinc finger array domain" or "polydactyl zinc finger array" or "PZF (array)". The zinc finger modules may be linked via linkers as described herein to form a zinc finger domain.

The zinc finger modules used in the present invention have sequences being identical to those of the naturally-occurring, wild-type zinc finger modules or sequences that are modified by substitution of other amino acids for any amino acids in the wild-type sequence. The wild-type zinc finger module may be derived from any eukaryotic cells, for example, fungal cells (e.g., yeast), plant or animal cells, (e.g., mammalian cells such as human or mouse).

Preferably, the zinc finger modules, forming the zinc finger domain of the fusion protein of the present invention, may be designed synthetically. Zinc finger arrays in the context of the present invention may be designed by the modular assembly technique, which means that individual single finger modules are pre-validated for binding to a respective 3 bp target sequence and subsequently assembled to a zinc finger array (detailed description in Wright, D.A. et al., 2006. Nature protocols, 1(3), pp.1637-1652). Hence, the zinc finger domains used herein can be engineered to bind to a predetermined nucleotide sequence. Non-limiting examples of methods for engineering zinc finger domains are design and selection. A designed zinc finger protein is a protein not occurring in nature whose design/composition results principally from rational criteria. A selected zinc finger protein is a protein not found in nature whose production results primarily from an empirical process such as phage display, interaction trap or hybrid selection.

The zinc finger domain being part of the fusion protein of the invention recognizes preferably triplets selected from the group consisting of GNN (as mentioned in Segal DJ, et al., 1999, PNAS, 96(6), pp.2758-2763; Dreier B, et al., 2000, J Mol Biol., 303(4), pp.489-502), ANN (as mentioned in Dreier B, et al., 2001, J Biol Chem., 276(31), pp.29466-29478), CNN (as mentioned in Dreier B, et al., 2005, J Biol Chem., 280(42), pp.35588-35597) or TNN (as mentioned in Dreier B, et al., 2001, J Biol Chem., 276(31), pp.29466-29478), wherein "G", "A", "C", "T" and "N" refer to nucleotides. Nucleotides, likewise, may be referred to by their commonly accepted single-letter codes. "G" means guanine, "A" means adenine, "C" means cytosin, "T" means thymine and "N" means any nucleotide.

For recognizing "GNN" triplets, the zinc finger modules described herein and being part of the fusion protein of the present invention may consist of or comprise an amino acid sequence selected from the group consisting of SEQ ID NO:19 (TSGNLVR), SEQ ID NO:20 (DCRDLAR), SEQ ID NO:21 (DPGHLVR), SEQ ID NO:22 (DPGALVR), SEQ ID NO:23 (TSGNLVR), SEQ ID NO:24 (TSGELVR), SEQ ID NO:25 (DPGNLVR) and SEQ ID NO:26 (QRAHLER).

Alternatively, the GNN recognizing zinc finger domain used in the present invention may be encoded by a nucleotide sequence as comprised in or consisting of SEQ ID NO:1 (nucleotide sequence encoding PTF1-6F) or SEQ ID NO:3 (nucleotide sequence encoding HPT-6F). Also encompassed by the present invention is a GNN recognizing zinc finger domain being part of the fusion protein of the present invention comprising an amino acid sequence as depicted by SEQ ID NO:2 (amino acid sequence of PTF1-6F) or SEQ ID NO:4 (amino acid sequence of HPT-6F). The genetic code consists of 64 different nucleotide triplets of which three provide a translational stop codon and the remaining 61 encode for the 20 amino acids. Of these 61 amino acid encoding nucleotide triplets, 49 triplets are targetable by validated zing fine modules. Modular assembly schemes for generating zinc finger modules binding to 49 of the 61 amino acid nucleotide encoding triplets are known to the skilled person in the art (www. zincfingertools.org). In the context of the present invention any of these 49 zinc finger domains might be linked to each other to theoretically recognize any combination of the validated 49 triplets. The zinc finger domain of the fusion protein of the present invention may recognize a sequence as comprised in SEQ ID NO:5 (recognition sequence of PTF1-6F) or SEQ ID NO:6 (recognition sequence of HPT-6F). Preferably, a sequence corresponding to nucleotides 472 to 489 of SEQ ID NO:5 (preferred recognition sequence of PTF1-6F (SEQ ID NO:5) within the genomic sequence around PINOID locus, AT2G34650.1, TAIR ID 4515108265, nucleotides 241 - 258 relative to translational start codon ATG) or a sequence corresponding to nucleotides 1377 to 1394 of SEQ ID NO:6 (preferred recognition sequence of HPT-6F within aphIV, hygromycin phospotransferase plant selectable marker on pGreenll-0179 T-DNA) is recognized.

For recognizing "ANN" triplets, the zinc finger modules described herein and being part of the fusion protein of the present invention may consist of or comprise an amino acid sequence initially described by Dreier and coworkers (Dreier, B. et al., 2001, The Journal of biological chemistry, 276(31), pp. 29466-29478; and www.zincfingertools.org).

As used herein, the terms "recognizes" and "targets" may be used interchangeable. A "target site" is the nucleic acid sequence recognized by a zinc finger array. Typically, a zinc finger protein of six zinc finger modules recognizes an adjacent approximately 18 base-pair long target site, a zinc finger protein having 7 zinc finger modules recognizes an adjacent approximately 21 base-pair long target site, a zinc finger protein having 8 zinc finger modules recognizes an adjacent approximately 24 base-pair long target site. The term "adjacent target site" means a non-overlapping target site separated by 0 to about 5 base-pairs.

As used herein, the term "binding" refers to a sequence-specific, non-covalent interaction between macromolecules (e.g., between a protein and a nucleic acid). Not all components of a binding interaction need be sequence-specific (e.g., contacts with phosphate residues in a DNA backbone), as long as the interaction as a whole is sequence-specific. The term "affinity" refers to the strength of binding: increased binding affinity being correlated with a lower Kd (dissociation constant).

As used herein, the term "sequence" to which a zinc finger domain binds refers to a nucleotide sequence of any length, which can be double stranded DNA or RNA, and can be linear or circular.

If necessary, a DNA sequence recognized by said zinc finger domain operably linked to a SPO11 polypeptide can be introduced into the cell genome by standard techniques.

However, the exemplarily detailed zinc finger domains as described above are not limiting for the fusion protein of the present invention. Depending on the recognition site to be targeted, it is within the scope of a person skilled in the art to modify and adapt the zinc finger domains mentioned above to any recognition site of a genome to be targeted.

In the context of the present invention, the term "operably linked" refers to functional linkage between at least two protein sequences, i.e. between the zinc finger domain and the SPO11 polypeptide. In the context of the present invention, the zinc finger domain and the SPO11 polypeptide as comprised in the fusion proteins of the present invention may be covalently linked. The covalent linking of a zinc finger domain with a SPO11 polypeptide results in fusion proteins in which said zinc finger protein and SPO11 polypeptide are connected by (a) covalent bond(s). A covalent bond is a chemical bonding that is characterized by the sharing of pairs of electrons between atoms, as, inter alia, obtained by the herein exemplified cross-binding via chemical compounds. However, also the recombinant production of constructs with, e.g. a linker and/or a tag as disclosed herein, i.e. zing finger domain and a covalently bound SPO11 polypeptide is envisaged. Alternatively, the zinc finger domain and the SPO11 polypeptide of the present invention may be non-covalently linked. Non-covalent bonds are known in the art and include, but are not limited to the association of protein molecules as a result of protein-protein interaction. Non-limiting examples of non-covalent bonds that may be useful for linking a zinc finger domain and SPO11 polypeptide in the context of the present invention include the rapamycin system (Banaszynski, L.A. et al., 2005, Journal of the American Chemical Society, 127(13), pp. 4715-4721.). In the presence of rapamycin, tight association between FK506-binding protein 12 (FKBP) and FKBP rapamycin-binding protein (FRB) is observed. In particular, FRB could be fused with one of the binding partners (e.g. with the zinc finger domain) and FKBP could be fused with the other binding partner (e.g. with the SPO11 polypeptide). In this szenario, only upon addition of rapamycin FKBP and FRB would dimerize and bring the finc finger domain and the SPO11 polypeptide in close proximity. Yet, the skilled person is readily capable of identifying non-covalent bonds/complexes, which are useful for non-covalently linking a zinc finger domain and a SPO11 polypeptide for generating fusion proteins as described herein.

In one embodiment, the zinc finger domain and the SPO11 polypeptide are comprised in a single-chain multi-functional polypeptide. A single-chain fusion protein e.g. may consist of a polypeptide comprising several (at least six) zinc finger domains and a SPO11 domain, i.e. a SPO11 polypeptide. Within the zinc finger domain the zinc finger modules may be connected via the linkers as described herein, preferably via canonical TGEKP linkers. The SPO11 polypeptide and the zinc finger domain may be connected via the linkers as described herein, preferably via short linear linkers (e.g. canonical, (GGS)6 or XTEN) or long linear linkers (e.g. 18xMyc).

In the context of the present invention, "operably linked" also refers to a functional linkage between at least two polypeptides, which means that both polypeptides retain their abilities.

In the context of the present invention, the term "N-terminus" may be used interchangeably with the amino terminus of a polypeptide, the NH₂-terminus, the N-terminal end or amine-terminus. The term means the natural start of a protein or polypeptide.

In the context of the present invention, the term "C-terminus" may be used interchangeably with the carboxy terminus of a polypeptide, the carboxyl-terminus, the carboxy-terminus, the C-terminal tail, the C-terminal end or COOH-terminus. The term means the natural end of a protein or polypeptide.

As already mentioned, the invention relates to a fusion protein comprising a zinc finger domain which is operably linked via its N-terminus to the C-terminus of a SPO11 polypeptide, wherein the zinc finger domain comprises six or more zinc finger modules, wherein "more" is understood to mean 7, 8, 9, 10, 11, 12, 13, 14, 15, or 16.

In the context of the present invention, the term "SPO11 polypeptide" means a polypeptide encoded by a Spo11 gene or encoded by a Spo11 gene ortholog. The terms "SPO11" and "sporulation-deficient 11" may be used interchangeably. Preferred in the invention are plant derived SPO11 polypeptides. The SPO11 polypeptide used in context of the present invention may be AtSPO11-1 or AtSPO11-2 or an ortholog of AtSPO11-1 or AtSPO11-2 that is involved in meiotic DSB formation.

Accordingly, the present invention further relates to a fusion protein comprising a zinc finger domain which is operably linked via its N-terminus to the C-terminus of a SPO11 polypeptide, wherein the zinc finger domain comprises six or more zinc finger modules, wherein "more" is understood to mean 7, 8, 9, 10, 11, 12, 13, 14, 15, or 16 wherein the SPO11 polypeptide may be derived from a plant, preferably from any higher eukaryotic plant that is susceptible to *Agrobacterium* mediated transgene delivery. In the context of the present invention, the SPO11 polypeptide may be derived from a plant such as Oryza sativa, or more preferably from *Arabidopsis thaliana.*

As mentioned, in context of the present invention, the SPO11 polypeptide might be an AtSPO11-1 protein, an AtSPO11-2 protein or an ortholog thereof (i.e. a protein or gene of another species that has the same cellular function but that may have limited homology in terms of sequence identity). It is envisaged that a SPO11 protein is used that fits to the plant in question (e.g. a rice SPO11 protein for work in rice, a maize SPO11 protein for work in maize, etc.).

However, if the SPO11 polypeptide is from *Arabidopsis thaliana,* it may be either an AtSPO11-1 (SEQ ID NO:7) or an AtSPO11-2 (SEQ ID NO:9) protein or a polypeptide comprising an amino acid sequence that is at least 20%, preferably at least 30%, more preferably at least 40%, even more preferably at least 50%, even more preferably at least 60%, even more preferably at least 70%, even more preferably at least 80%, even more preferably at least 90% or even more preferably at least 95% identical to the amino acid sequence as depicted in SEQ ID NOs: 7 or 9 which is capable of initiating meiotic recombination. In the context of the present invention, the SPO11 polypeptide may be encoded by a nucleotide sequence comprised in SEQ ID NOs: 8 or 10.

In accordance with the present invention, the term "identical" in the context of two amino acid sequences, refers to two sequences or subsequences that are the same, or that have a specified percentage of amino acid residues that are the same (e.g., 60% or 65% identity, preferably, 70-95% identity, more preferably at least 95% identity), when compared and aligned for maximum correspondence over a window of comparison, or over a designated region as measured using a sequence comparison algorithm as known in the art, or by manual alignment and visual inspection. Sequences having, for example, 60% to 95% or greater sequence identity are considered to be substantially identical. Such a definition also applies to the complement of a test sequence. Preferably the described identity exists over a region that is at least about 15 to 25 amino acids, more preferably, over a region that is about 50 to 100 amino acids or nucleotides in length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Also encompassed by the present invention are nucleic acid molecules encoding the fusion proteins of the invention.

The term "nucleic acid" refers to deoxyribonucleotides or ribonucleotides and polymers thereof in double-stranded or single-stranded form, respectively. The term encompasses nucleic acids containing known nucleotide analogs or modified backbone residues or linkages, which are synthetic, naturally occurring, and non-naturally occurring, which have similar binding properties as the reference nucleic acid. Examples of such analogs include, without limitation, phosphorothioates, phosphoramidates, methyl phos- phonates, chiral-methyl phosphonates, 2-O-methyl ribonucleotides, peptide-nucleic acids (PNAs).

The nucleic acids of the invention may be under the control of a promoter, i.e. operably linked thereto. Preferably, the promoter is a meiosis specific promoter. For example, the promoter may be the endogenous AtSPO11-1 or AtSPO11-2 promoter or the promoter of the meiosis-specific cohesin subunit REC8. The latter one has been successfully used to express transgenes at the onset of meiosis (WO-A1 2004/016795).

In the context of the present invention the nucleic acid molecule encoding a fusion protein of the invention preferably comprises a meiosis specific promoter. In the context of the present invention, the term "meiosis specific promoter" means a promoter, which is especially active during meiosis. Also encompassed by this term are promoters, which are especially active in meiotically dividing cells. In plants, meiotically dividing cells are the microspore and macrospore mother cells in the male and female gamete precursors that actually undergo meiosis. These cells reside within male and female generative organs (anthers and gynoetia within flower primordia in plants). In all organisms these meiotically dividing cells are surrounded by non-meiotic cells. Meiotically active promoters are active in meiotically dividing cells in flower organs at early or late meiotic stages.

Preferably, the present invention also relates to a nucleic acid molecule encoding a fusion protein of the present invention, which comprises a meiosis specific promoter, wherein the meiosis specific promoter is an endogenous meiosis specific promoter like a SPO11 promoter. Preferably, the SPO11 promoter comprises a sequence as depicted in SEQ ID NO:13, i.e. 1127 nt upstream of the translational start codon ATG of AtSPO11-1, or a sequence as depicted in SEQ ID NO:14, i.e. 4254 nt upstream of the translational start codon ATG of AtSPO11-2.

The term "endogenous" as used herein with reference to nucleic acids or genes and a particular cell refers to any nucleic acid or gene that does occur in (and can be obtained from) that particular cell as found in nature.

Further, the invention also relates to nucleic acid molecules encoding a fusion protein of the present invention, wherein the SPO11 polypeptide is encoded by a genomic DNA.

In the context of the present invention, the term "genomic DNA" means a DNA containing introns and exons. Preferably, the 3' UTR of the endogenous SP011 gene may be comprised. More preferably, the genomic DNA contains the 5' and 3' UTR regions of the endogenous SPO11 gene, harboring the endogenous promoter region as well as regulatory downstream sequences.

Accordingly, a genomic DNA used herein differs from a cDNA, i.e. a complementary DNA. In the context of the present invention, the terms "5' UTR" and "3' UTR", respectively, refer to the 5' and respectively 3' prime untranslated region of a DNA or RNA molecule encoding a gene of interest. Accordingly, these terms encompass sequences on the 5' end (i.e. upstream) and 3' end (i.e. downstream), respectively, of the coding sequence in DNA or RNA, which are not translated into the protein of interest. The invention also relates to a fusion protein of the present invention, wherein the SPO11 polypeptide is encoded by a sequence as depicted in SEQ ID NO:13 (SPO11-1 genomic) or SEQ ID NO:14 (SPO11-2 genomic) or by a sequence having at least 20%, preferably at least 30%, more preferably at least 40%, even more preferably at least 50%, even more preferably at least 60%, even more preferably at least 70%, even more preferably at least 80%, even more preferably at least 90% and particularly preferred at least 95 % sequence identity to SEQ ID NO:13 or SEQ ID NO:14.

The invention also relates to a vector containing any of the nucleic acid molecules of the present invention, capable of expressing said nucleic acid molecule in a eukaryotic host cell, preferably in a plant host cell.

In the present invention, the nucleic acid molecules encoding a fusion protein of the present invention may be typically cloned into vectors for transformation into prokaryotic or eukaryotic cells for replication and expression. Vectors are typically prokaryotic or eukaryotic vectors, e.g., plasmids, shuttle vectors, or insect vectors. The nucleic acid molecule encoding a fusion protein of the present invention is also typically cloned into an expression vector, for administration to an animal cell, fungal cell, bacterial cell, or protozoal cell, preferably a plant cell. An expression vector is a nucleic acid construct, generated recombinantly or synthetically, with a series of specified nucleic acid elements that permit transcription of a particular nucleic acid in a host cell, and optionally integration or replication of the expression vector in a host cell. The expression vector can be part of a plasmid, virus, or nucleic acid fragment, of viral or non-viral origin. Typically, the expression vector includes an "expression cassette", which comprises a nucleic acid to be transcribed operably linked to a promoter. The term expression vector also encompasses naked DNA operably linked to a promoter. Typical vectors useful for expression of recombinant nucleic acids in higher plants are well known in the art and include, without limitation, vectors derived from the tumor-inducing (Ti) plasmid of *Agrobacterium tumefaciens* (e.g., see Rogers et al., Meth. in Enzymol. 153 (1987), 253-277). These vectors are plant integrating vectors in that on transformation, the vectors integrate a portion of vector DNA into the genome of the host plant. In principle any binary vector derived from academic or commercial sources can be used for this task. For example, in context of the present invention, the binary vectors pCB302 (BASTA Herbicide Resistance Marker; Xiang, 1999, Plant molecular biology, 40(4), pp.711-717), pBIB-HYG (Hygromycin Resistance Marker; Becker, 1990, Nucleic Acids Research, 18(1), p.203) and pGreenII 0179 (Hygromycin Resistance Marker; Hellens, 2000, Plant molecular biology, 42(6), pp.819-832) may be used. Further exemplary *Agrobacterium tumefaciens* vectors useful herein are the plasmids pKYLX6 and pKYLX7 (e.g., see of Schardl et al., 1987, Gene, 61, pp.1-11; and Berger et al., 1989, Proc. Natl. Acad. Sci. USA, 86, pp.8402-8406); and plasmid pBI 101.2 that is available from Clontech Laboratories, Inc. (Palo Alto, CA).

The present invention further provides expression vectors containing a nucleic acid molecule encoding a fusion protein of the present invention. A nucleic acid molecule expressing the fusion protein of the present invention can be used to construct a recombinant expression vector, which can be introduced into the desired host cell. A recombinant expression vector will typically contain a nucleic acid molecule encoding a fusion protein of the present invention, operably linked to transcriptional initiation regulatory sequences, which will direct the transcription of the nucleic acid molecule in the intended host cell, such as cells of a transformed plant.

For example, a plant expression vector may include a nucleic acid encoding a fusion protein of the present invention under the transcriptional control of 5' and 3' regulatory sequences. Such plant expression vectors may also contain, if desired, a promoter regulatory region (e.g., one conferring inducible or constitutive, environmentally- or developmentally-regulated, or cell- or tissue-specific/selective expression), a transcription initiation start site, a ribosome binding site, an RNA processing signal, a transcription termination site, and/or a polyadenylation signal.

In a particular preferred embodiment, the vector of the invention contains a nucleic acid molecule encoding a fusion protein of the present invention under the transcriptional control of 5' and 3' regulatory sequences of the SPO11 gene.

The invention also relates to a host cell which comprises a nucleic acid molecule or a vector of the present invention or which expresses a fusion protein of the present invention.

In the present invention, "host cell" or "cell" means a cell that is capable of expressing the fusion protein of the present invention. Host cells can be prokaryotic cells such as E. coli, or eukaryotic cells such as yeast, fungal, protozoal, plant, higher plant and insect cells, or amphibian cells, or mammalian cells such as CHO, HeLa 293, COS-I, and HEK, e.g., cultured cells (in vitro), explants and primary cultures (in vitro and ex vivo), and cells in vivo. As discussed below, the host cell is preferably a plant cell. This plant cell is preferably a plant meiocyte. The host cells used herein may carry artifical chromosomes carrying one or more sequence(s) recognized by the zinc finger domain operably linked to the SPO11 protein.

In the context of the present invention, the term "eukaryotic host cell", "host cell" or "cell" preferably means a plant cell or a plant. The term "plant" refers to any various photosynthetic, eukaryotic multicellular organisms of the kingdom Plantae, characteristically producing embryos, containing chloroplasts, having cellulose cell walls and lacking locomotion. As used herein, a "plant" includes any plant or part of a plant at any stage of development, including seeds, suspension cultures, plant cells, embryos, meristematic regions, callus tissue, leaves, roots, shoots, gametophytes, sporophytes, pollen, microspores, and progeny thereof. Also included are cuttings, and cell or tissue cultures. As used in conjunction with the present disclosure, plant tissue includes, without limitation, whole plants, plant cells, plant organs, e.g., leafs, stems, roots, meristems, plant seeds, protoplasts, callus, cell cultures, and any groups of plant cells organized into structural and/or functional units. Accordingly, the fusion proteins of the present invention may be introduced into plant cells via any suitable methods known in the art. Most preferably, the host cell is a plant meiocyte.

In the context of the invention, the nucleic acid molecule of the invention or the vector of the invention may be introduced into a host cell, preferably a plant. Plant transformation protocols as well as protocols for introducing recombinant nucleic acids into plants may vary depending on the type of plant or plant cell, e.g., monocot or dicot, targeted for transformation. Suitable methods of introducing recombinant nucleic acids into plant cells include, without limitation, microinjection (Crossway et al., 1986, Biotechniques, 4, pp.320-334), electroporation (Riggs et al., 1986, Proc. Natl. Acad Sci. USA, 83, pp.5602-5606), *Agrobacterium-mediated* transformation (U.S. Pat. No. 5,563,055), direct gene transfer (Paszkowski et al., 1984, EMBO J, 3, pp.2717-2722), and ballistic particle acceleration (U.S. Pat. No. 4,945,050 and McCabe et al., 1988, Biotechnology, 6, pp.923-926).

The present invention also relates to methods for producing the fusion protein of the invention, comprising the steps of expressing a nucleic acid molecule of the invention in a host cell and recovering the fusion protein, from said cell or the cell culture supernatant. If the host cells are cultured in a medium, the fusion protein may also be recovered from the culture medium. Said fusion protein, said cell or said cell culture supernatant may be recovered by methods known in the art. Exemplarily, but not limiting, host cells and cell culture supernatant may be separated by centrifugation or filtration procedures. Host cells carrying the fusion protein of the present invention may be disrupted by methods known in the art, e.g. by mechanical or enzymatic methods. Said fusion protein may be recovered by standard protein purification methods like ammonium sulfate precipitation, affinity chromatography, ion exchange chromatography and the like.

In the context of the present invention the method for producing the fusion protein of the present invention is carried out in a plant cell as host cell, producing the fusion protein of the present invention. A method which employs a plant cell for carrying out a method can be an engineered plant cell which expresses the fusion protein described herein, i.e. which has in its genome a nucleotide acid molecule encoding a zinc finger domain and a SPO11 polypeptide and which has been modified to over-express it. The expression may occur constitutively or in an induced or regulated manner. The nucleic acid molecule can be stably integrated into the genome of the plant cell.

However, the zinc finger-SPO11-fusion protein of the present invention is preferably produced in meiocytes of a plant *in vivo.* Accordingly, for producing the zinc finger-SPO11-fusion protein a whole plant is preferably grown and the zinc-finger-SPO11-fusion protein is produced in the meiocytes of said plant.

Also encompassed by the invention is a fusion protein, which is obtained by a method of the present invention.

The present invention further relates to a method for increasing recombination between two defined loci of two homologous chromosomes in a meiotic plant cell comprising the steps of introducing into said cell the nucleic acid molecule of the present invention or the vector of the present invention encoding the fusion protein as defined in the invention, which induces double strand/double-stranded breaks in DNA during meiosis, in which the recombination between two defined loci of two homologous chromosomes in said plant cell is increased.

The term meiosis describes a specialized cell division, which main purpose is to reduce the cellular chromosome content by half. In unicellular organisms like yeasts this process can be triggered by depletion of the nitrogen source (i.e. starvation). A diploid yeast cell thereby forms four spores, which are more likely to endure challenging environmental conditions. Surviving cells display altered genetic content and may perform better in changing environmental conditions. Surviving cells may mate and reconstitute a diploid organism with novel combinations of the genetic content. Within higher eukaryotes/metazoans meiosis takes place in germ cell precursors situated within the generative organs of said organism. Meiosis in higher eukaryotes is subject to complex regulation during development of that organism (reviewed in Roig, I., et al., 2011, Genes, 2(4), pp.152-168).

In the context of the present invention, the term "increase" encompasses a stimulation of meiotic recombination. Meiotic recombination frequency can be measured, e.g., by analysis of ecotype specific SNPs, or by analysis of fluorescent pollen marker signals (as described in more detail below). Meiotic recombination frequence is typically measured in centrimorgan (cM). One cM equal to a change that one recombination event at one genetic locus is expected in 100 tested plants. In the context of the present invention the term "increase" encompasses a recombination rate of at least 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, or 100 cM.

The invention further relates to a method for generating variants of a plant comprising the steps of introducing into a cell of a plant the nucleic acid molecule of the invention, or the vector of the invention encoding the fusion protein as defined in the invention, which induces meiotic double-stranded breaks in DNA; having said cell perform a meiosis, so that meiotic recombination occurs between homologous chromosomes of said cell, at a higher rate and/or at different loci than in natural meiosis of said plant; and generating variants of said plant containing said cells.

In the context of the present invention, the term "variant" should be understood broadly, and designates an organism, which presents at least one phenotypic or genotypic difference having regard to its parent(s). A variant here designates an organism, the haplotype of which is different from the haplotype(s) of its parent(s).

The method for generating variants of a plant encompassed by the invention may further comprise a step of performing a genetic and/or phenotypic analysis for the presence of the meiotic recombination in said variants. In the context of the present invention, the term "genetic analysis" means detection of recombination through PCR mediated readout of polymorphic markers that are flanking the zinc finger target locus (next paragraph). The term "phenotypic analysis" means detection of recombination through visual analysis of pollen marker configurations that are flanking the zinc finger target locus (next paragraph).

The readout of recombination rate stimulation is dependent on the availability of target locus flanking markers that can be analyzed. Genetic analysis of recombination rates can be performed by analyzing crosses of different *Arabidopsis thaliana* ecotypes (geographically isolated inbred populations with a unique and distinct pattern of single nucleotide polymorphisms (SNPs) and small insertions/deletions (INDELs)). To obtain recombination readout, two *Arabidopsis* ecotypes can be crossed, giving rise to a F1 generation harboring all SNPs and INDELs genome-wide in a heterozygous configuration. F1 plants are then grown until flowering occurs. Prior to flowering male and female gamete precursor cells undergo meiosis. Meiotic recombination events will eventually lead to a *trans* to cis exchange of flanking markers and thereby to combination of both of ecotype A and ecotype B specific SNP/INDEL markers on the same chromosome, flanking the zinc finger target locus. The configuration of SNPs and INDELs flanking the zinc finger target locus will be analyzed in the subsequent F2 generation. Therefore F1 heterozygous plants have to be back-crossed to one of the parental lines. Genomic DNA can then be isolated from several F2 individuals (pool sizes are dependent on the genetic distance of zinc finger target-flanking INDELS and the recombination stimulus conferred by the SPO11-zinc finger fusion). Configuration of target flanking INDELS will be analyzed by PCR. Plants harboring chromosomes that did not participate in recombination events at the target locus will have markers of one parent in *cis* configuration. Plants harboring chromosomes that did participate in recombination events at the target locus will have markers of both parents in *cis* configuration.

Alternatively, locus-specific recombination rates in *Arabidopsis thaliana* can be assessed using artificially introduced markers at either side of the target locus that allow convenient visual read-out. The test system of "fluorescent pollen tetrad analysis" has been established by the Copenhaver lab (Francis, K.E. et al., PNAS, 2007, 104(10), pp.3913-3918). A series of transgenic sequences encoding auto-fluorescent proteins (AFPs) of different color under control of a pollen-specific LAT52 promoter were randomly inserted into the *Arabidopsis* genome. Plant lines can be generated in the *quartet* mutant background. As a consequence the Fluorescence-Tagged Lines (FTL) give rise to plants that form fluorescent pollen grain encapsulated by a callose cell wall so that all four meiotic products stay together as a "tetrad" which can be analyzed by microscopy. Appropriate lines harboring single FTL insertions of different color in close vicinity to the zinc finger target locus can be selected to generate the tester lines. FTL lines of different color flanking the zinc finger target locus can be crossed to plant lines expressing AtSPO11 zinc finger fusion polypeptides. Isogenic plant lines can be generated by propagation and selection. Those parental lines crossed to obtain the actual tester line harbored the AtSPO11-zinc finger transgene, the endogenous *AtSpo11* mutation, the *quartet* mutation and one of the two FTL transgenes flanking the target locus, all in homozygous configuration and therefore only differed by the position and nature of the FTL marker. Parental lines were crossed to generate the heteroallelic F1 tester line, harboring both FTL insertions in trans in a heterozygous state (while all other afore mentioned genetic features remain homozygous). Plants were grown until flowering occurred. Within the flowers of these plants, gamete (spore) precursors undergo meiosis. Male gametes/spores of different fluorescence configuration are formed dependent on absence or occurrence of a meiotic recombination event between the FTL markers. Recombination events result in *trans* to *cis* pollen marker exchange and the formation of dual colored and non-colored pollen grains. Recombination readout can be performed by analysis of fluorescent marker exchange. In parallel, baseline recombination rates (in plants with the wild-type AtSPO11 protein but not the AtSPO11-zinc finger protein present) at the zinc finger target locus interval can be established. Frequencies of parental ditype (PD), tetratype (TT) and non-parental ditype (NPD) pollen tetrads can be used to calculate genetic distances (Perkins. D.D., 1949, Genetics, 34(5), pp.607-626; Ma, C. & Mortimer, R.K., 1983, Molecular and Cellular Biology, 3(10), pp.1886-1887) and thereby recombination rates in absence or prescence of the AtSPO11-zinc finger fusion protein.

In the context of the present invention, "genetic distances" may be expressed in terms of "centimorgan" (cM). Physical distance may be measured in units of one million base pairs (Mb). Accordingly, a common way to depict variation in meiotic recombination activity is to calculate recombination intensity (cM/Mb).

The method for increasing recombination between two defined loci of two homologous chromosomes in a meiotic plant cell or for generating variants of a plant encompassed by the invention may further comprise a step of removing the SPO11-zinc finger fusion protein. If the SPO11-zinc finger fusion protein is transiently expressed from a non-binary vector, the genetic information encoding said protein will be lost due to degradation of the introduced plasmid vector. If a SPO11-zinc finger fusion encoding DNA sequence is integrated into the plant genome and present in heterozygous state, this genetic information might be removed by genetic segregation during propagation of the plant. If a SPO11-zinc finger fusion encoding DNA sequence is integrated into the plant genome and present in homozygous state, crossing with a wild-type plant is preferably performed to allow subsequent genetic segregation in the next generation. In all cases mentioned, plants are selected in which the nucleic acid molecule of the invention or the vector of the invention is absent in the cells of said variants. Within these methods, after stimulation of recombination at a given locus, the gene encoding the SPO11 zinc finger fusion protein of the present invention can be eliminated by genetic segregation. To this end, offspring of the plant with stimulated recombination that has been tested to contain the desired marker configuration in *cis* (e.g.: desired genetic traits now residing on the same chromosome after successfully recombining them during meiosis utilizing the AtSPO11-ZF protein) can be crossed with wild type plants. Offspring of this cross can be tested for absence of the gene encoding the SPO11 zinc finger fusion protein and for presence of the desired marker configuration in *cis.*

In the context of the present invention, the term "wild-type plants" means a plant, which is not expressing the fusion protein of the present invention. Therefore, in the context of the present invention wild-type plants also encompass those, which *are* transgenic, but do *not* express the fusion protein of the present inverntion. The term "transgenic" when used with reference to a plant indicates that the plant and/or plants cells have been modified by the introduction of a heterologous nucleic acid or protein or the alteration of a native nucleic acid or protein, or that the plant and/or plant cell are derived from a cell so modified. Thus, for example, transgenic plant cells express genes that are not found within the native

(naturally occurring) form of the plant cell or express a second copy of a native gene that is otherwise normally or abnormally expressed, under-expressed or not expressed at all.

The invention further relates to the use of a fusion protein of the invention, the nucleic acid molecule of the invention, or the vector of the invention for increasing or inducing meiotic DNA double strand/double stranded breaks in a plant.

The invention also relates to the use of a fusion protein of the invention, the nucleic acid molecule of the invention, or the vector of the invention for increasing or inducing meiotic DNA double strand/double stranded breaks in a plant, wherein the fusion protein of the invention is capable of inducing meiotic double strand/double stranded breaks in formerly cold regions in said plant by targeting the fusion protein of the present invention to a formerly cold region and inducing meiotic DNA double strand/double stranded breaks at said region. The fusion protein of the present invention can be designed to recognize any suitable region of a plant or plant cell. In the context of the present invention the fusion protein of the present invention preferably targets cold regions within a plant cell or plant. Exemplarily, endogenous cold regions may be targeted by the fusion protein of the present invention.

In the context of the invention, the term "cold regions" or "cold spots" refers to regions in the genome of an organism that have lower recombination rates than would have been expected on the basis of the genome average recombination rate. Mutatis mutandis applies for "hot spot regions" or "hot spots", which are regions in the genome of an organism that have higher recombination rates than would have been expected on the basis of the genome average recombination rate. In addition, an interval is a hot (or cold) spot if its recombination intensity is greater (or smaller) than the surrounding regions even if the overall recombination rate is not significantly different from genome average (see Petes T.D., 2001, Nature Reviews Genetics, 2(5), pp.360-369; Arnheim N., et al., 2003, American journal of human genetics, 73(1), pp.5-16; Arnheim, N., et al., 2007, Annual review of genetics, 41, pp.369-399 for detailed description).

Exemplarily, but not limiting, the below details how a skilled person is able to identify recombination hot and cold spots with statistical significance:
(i) Based on comparison of recombination events to genome average. Observed recombination events per interval are expressed as events per Mega-base (Mb). The expected number of events, which assumes an average genome-wide distribution is obtained by calculating the number of recombination events per Mega-base. To determine recombination hot and cold spots the observed number of recombination events per interval (i.e. between two genetic markers) is divided by the expected number of events. Recombination hotspots are identified as having e.g. 2-fold more events than expected, based on the genome-wide average. Observed recombination events per interval are compared to the genome wide average using a Mann-Whitney U test (explained in detail in Esberg A., et al., 2011, PLoS ONE, 6(9), p.e25211).
(ii) Based on comparison of recombination rates between different (adjacent) intervals. The recombination rate between two adjacent markers is estimated from the number of recombinants. If Nr (respectively N) is the number of recombinant (respectively all) individuals, the recombination rate r is estimated as Nr/N. The corresponding 95% confidence interval is given by 1.96 s where s is the standard error satisfying s^2 = r (1 - r) / N. The recombination rate per base pair (and the associated confidence interval) is obtained by dividing by the number of base pairs. Finally, the mean recombination rate per base pair on a chromosome arm is calculated using a weight for each interval, which is simply its length in base pairs. Intervals belonging to heterochromatic regions are excluded from the calculation. Intervals are defined as hot or cold if their 95% confidence intervals do not contain the mean calculated for that arm (explained in detail in Giraut, L. et al., 2011, PLoS Genetics, 7(11), p.e1002354).

Any plant cell capable of undergoing/performing meiosis may be used in the present invention so long as it remains viable after being transformed with a fusion protein of the present invention. As disclosed herein, a broad range of plant types may be modified to incorporate a fusion protein of the present disclosure. Suitable plants that may be modified include both monocotyledonous (monocot) plants and dicotyledonous (dicot) plants.

Preferably, in the context of the present invention, the plant may be a crop plant or the plant cell may be a crop plant cell. In this context, the crop plant may be corn (maize/Zea *mays*), wheat, barley, soybean, cotton, vegetables or fruits.

Accordingly, examples of suitable plants include, species of the family *Gramineae,* including *Sorghum bicolor* and *Zea mays;* species of the genera: *Cucurbita, Rosa, Vitis, Juglans, Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersicon, Nicotiana, Solanum, Petunia, Digitalis, Majorana, Ciahorium, Helianthus, Lactuca, Bromus, Asparagus, Antirrhinum, Heterocallis, Nemesis, Pelargonium, Panieum, Pennisetum, Ranunculus, Senecio, Salpiglossis, Cucumis, Brouvaalia, Glycine, Pisum, Phaseolus, Lolium, Oryza, Avena, Hordeum, Secale,* and *Triticum.*

In the context of the present invention plant cells may include those from corn (*Zea mays*), canola (*Brassica napus, Brassica rapa* ssp.), *Brassica* species useful as sources of seed oil, alfalfa (Medicago sativa), rice (Oryza sativa), rye (*Secale cereale*)*,* sorghum (*Sorghum bicolor, Sorghum vulgare*), millet (e.g., pearl millet (*Pennisetum glaucum*), proso millet (Panieum miliaceum), foxtail millet (*Setaria italica*), finger millet (*Eleusine coracana*)), sunflower (*Helianthus annuus*), safflower (*Carthamus tinctorius*), wheat (*Triticum aestivum*), duckweed (*Lemna*), soybean (*Glycine max*), tobacco (*Nicotiana tabacum*), potato (*Solanum tuberosum*), peanuts (*Arachis hypogaea*), cotton (*Gossypium barbadense, Gossypium hirsutum*), sweet potato (*Ipomoea batatus*), cassava (*Manihot esculenta*)*,* coffee (*Coffea* spp.), coconut (*Cocos nucijra*), pineapple (*Ananas comosus*), citrus trees (*Citrus* spp.), cocoa (*Theobroma cacao*), tea (*Camellia sinensis*), banana (*Musa* spp.), avocado (*Persea americana*)*,* fig (*Ficus casica*), guava (*Psidium guajava*)*,* mango (*Mangifera indica*)*,* olive (*Olea europaea*), papaya (*Carica papaya*), cashew (*Anacardium occidentale*)*,* macadamia (*Macadamia* spp.), almond (*Prunus amygdalus*)*,* sugar beets (*Beta vulgaris*), sugarcane (*Saccharum* spp.), oats, barley, vegetables, ornamentals, and conifers.

Examples of suitable vegetables plants include, without limitation, tomatoes (*Lycopersicon esculentum*), lettuce (e.g., *Lactuca sativa*)*,* green beans (*Phaseolus vulgaris*), lima beans (*Phaseolus limensis*), peas (*Lathyrus* spp.), and members of the genus Cucumis such as cucumber (C. *sativus*)*,* cantaloupe (C. *cantalupensis*)*,* and musk melon (C. *melo*)*.*

Examples of suitable conifer plants include, without limitation, loblolly pine (*Pinus taeda*)*,* slash pine (*Pinus elliotii*)*,* ponderosa pine (*Pinus ponderosa*), lodgepole pine (*Pinus contorta*)*,* Monterey pine (*Pinus radiata*)*,* Douglas-fir (*Pseudotsuga menziesii*)*,* Western hemlock (*Isuga canadensis*)*,* Sitka spruce (*Picea glauca),* redwood (*Sequoia sempervirens*)*,* silver fir (*Abies amabilis*)*,* balsam fir (*Abies balsamea*)*,* Western red cedar (*Thuja plicata*)*,* and Alaska yellow-cedar (*Chamaecyparis nootkatensis*)*.*

Examples of suitable leguminous plants include, without limitation, guar, locust bean, fenugreek, soybean, garden beans, cowpea, mungbean, lima bean, fava bean, lentils, chickpea, peanuts (*Arachis* sp.), crown vetch (*Vicia* sp.), hairy vetch, adzuki bean, lupine (*Lupinus* sp.), trifolium, common bean (*Phaseolus* sp.), field bean (*Pisum* sp.), clover (*Melilotus* sp.) Lotus, trefoil, lens, and false indigo. Examples of suitable forage and turf grass include, without limitation, alfalfa (*Medicago* ssp.), orchard grass, tall fescue, perennial ryegrass, creeping bent grass, and redtop. Examples of suitable crop plants and model plants include, without limitation, *Arabidopsis,* corn, rice, alfalfa, sunflower, canola, soybean, cotton, peanut, sorghum, wheat, tobacco, and lemna.

Within the *Brassicacea* family, the crop plant may be selected from the group consisting of Brassica rapa, Brassica nigra, Brassica oleracea, Brassica juncea, Brassica napus and Brassica carinata.

The present invention also pertains to a kit for performing the methods described above, containing a nucleic acid molecule or a vector encoding a fusion protein of the present invention. In the context of the invention, said fusion protein is capable of inducing double strand breaks in formerly cold regions.

In this kit, the SPO11 polypeptide is preferably encoded by a SPO11 nucleic acid molecule. In the context of the present invention, the SPO11 gene is from *Arabidopsis thaliana.* In the context of the present invention, if the SPO11 gene is from *Arabidopsis thaliana* it may then be the AtSPO11-1 gene (SEQ ID NO:8) or the AtSPO11-2 gene (SEQ ID NO:10) or a gene comprising a nucleic acid molecule that is at least 20%, preferably at least 30%, more preferably at least 40%, even more preferably at least 50%, even more preferably at least 60%, even more preferably at least 70%, even more preferably at least 80%, even more preferably at least 90% or even more preferably at least 95% identical to the nucleic acid sequence as depicted in SEQ ID NOs: 8 or 10 which is capable of initiating meiotic recombination. Alternatively a functional ortholog of AtSPO11-1 or AtSPO11-2 which is capable of initiating meiotic DNA double strand/double stranded breaks might be used.

The kit of the invention can further comprise a nucleic acid molecule carrying a target sequence recognized by said zinc finger domain. This target sequence is comprised in any kind of binary vector suitable for *Agrobacterium-mediated* transformation, and mediates insertion this target sequence in the genome of the host cell.

In this specification, a number of documents including patent applications are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

**The Figures show**

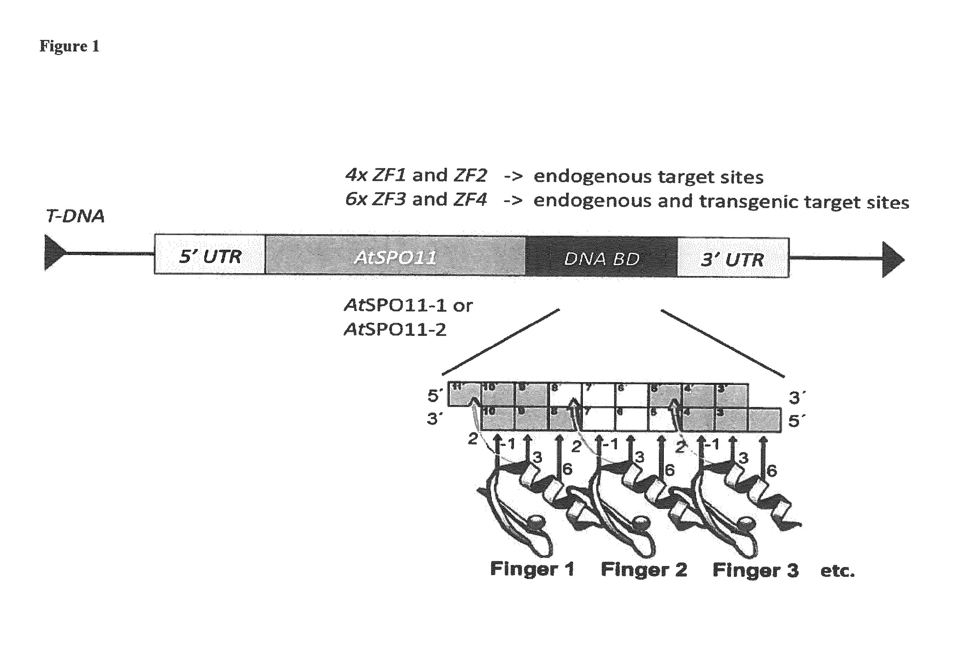

**Figure 1****: Generation of a Targeted Meiosis-Specific SPO11-PZF Nuclease**
   Genomic clones of AtSPO11-1 or AtSPO11-2 were generated, including putative 5' and 3' regulatory regions. DNA binding domains in part optimized for *Arabidopsis* codon usage were introduced at the C-terminus. This strategy anticipates that expression of the SPO11 proteins (proficient in introducing a DNA double strand break during meiosis) occurs at physiological levels.
   DNA binding domains consist of several Cys2-His2 zinc finger modules (depicted as Finger 1, Finger 2, etc.). Each zinc finger module spans 30 amino acids and contains a single amphipathic recognition helix stabilized by zinc ligation to two beta-strands. Sequence specific recognition is provided by contact of amino acid at positions -1, 3, and 6 relative to the starting point of the R-helix to a consecutive stretch of 3 bp within a DNA double helix. The number of zinc finger modules determines the length of the zinc finger binding site and thereby the specificity of the DNA binding domain.

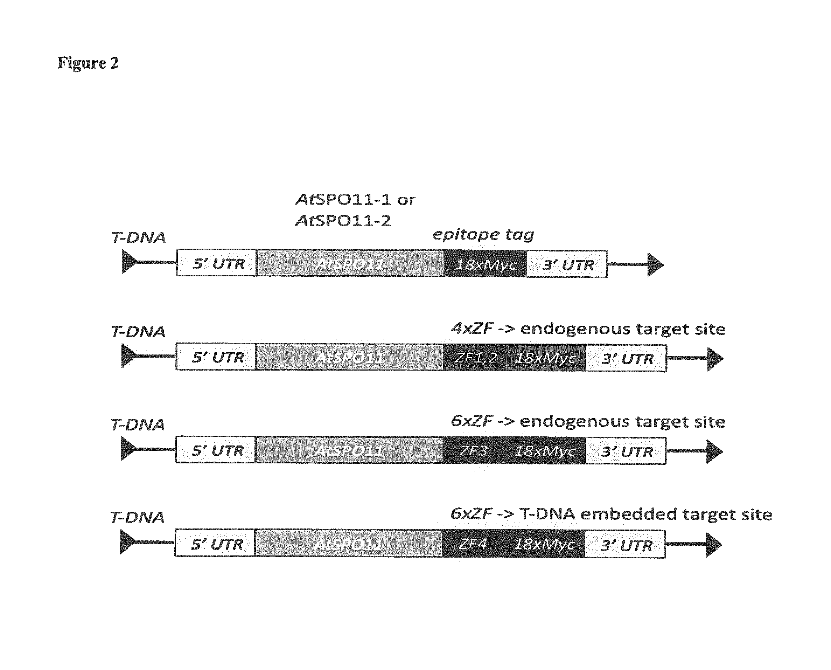

**Figure 2****: Overview of *AtS*PO11-PZF Fusion Proteins**
   ZF1 and ZF2 were designed using the Barbas Lab online tool and resemble four-finger arrays that target the endogenous *CSR1 locus* (CHLORSULFURON/IMIDAZOLINONE RESISTANT 1, AT3G48560.1, TAIR ID 2114524, nucleotides 1911 - 1922 relative to translational start codon ATG) within the *Arabidopsis thaliana* genome. Construction was done by the modular assembly technique where individual single finger molecules are pre-validated for binding to a respective 3 bp target sequence. The modular assembly approach represents a standard technique that is well accepted by the field and described in high detail in Wright, 2006 (Wright, D.A. et al., 2006, Nature protocols, 1(3), pp. 1637-1652). Further experimental details are provided in Example 5. Subsequently, individual modules were assembled and the full-length array was anticipated to bind the respective CSR1 target sequence. Additionally, six-finger constructs ZF3 and ZF4 were likewise constructed via modular assembly and pre-validated to bind their respective target sequences in a chromatin embedded context. Further experimental details are provided in Example 5. ZF3 targets the endogenous PINOID locus, AT2G34650.1, TAIR ID 4515108265, nucleotides 241 - 258 relative to translational start codon ATG within the *Arabidopsis* genome, while ZF4 targets a site within the hygromycin resistance gene aphIV of e.g. the binary vector pGreenII-0179, or other binary vectors containing the hygromycin resistance gene, that is present in many transgene insertion lines as plant selectable marker. Additionally, all constructs were fused to a C-terminal 18xMyc epitope to enable analysis of protein expression and DNA binding via western blotting and chromatin immunoprecipitation.

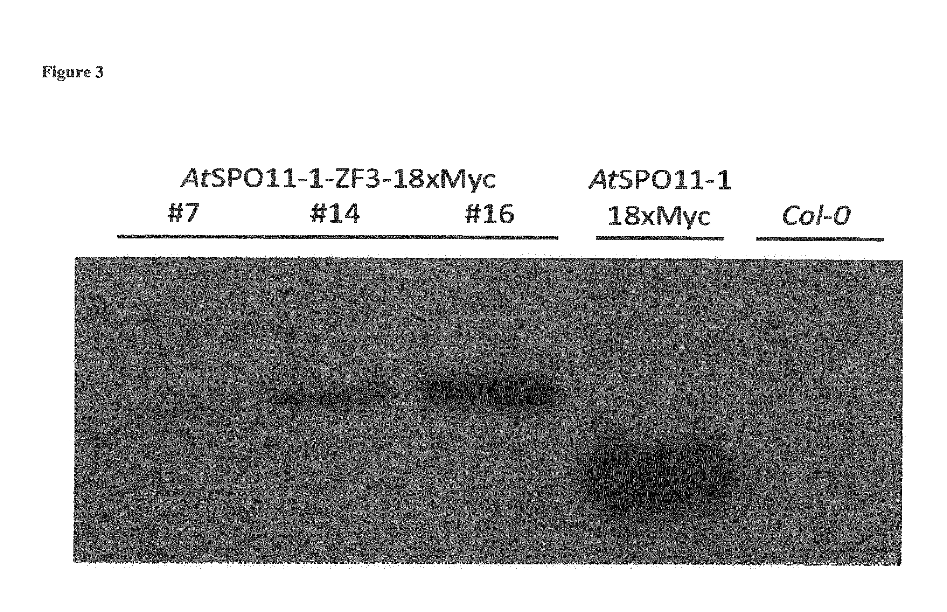

**Figure 3****: *At*SPO11-1-PZF Fusion Proteins Are Expressed in Meiosis**
   Three independent *Arabidopsis* lines harboring single locus T-DNA insertions of *At*SPO11-1-ZF3-18xMyc constructs were analyzed for fusion protein expression. Protein extracts from meiotic tissue were prepared. Immunoprecipitation (IP) using monoclonal mouse anti-Myc (clone 9E11) was performed to enrich for *At*SPO11-1-ZF3-18xMyc. Meiotic tissue from lines expressing *At*SPO11-1-18xMyc and WT tissue from ecotype *Col-0* were analyzed as controls. *At*SPO11-1-ZF3-18xMyc insertion lines #7, #14 and #16 display detectable but different levels of fusion protein expression, indicating that PZF and Myc-epitope tags do not hinder expression of the respective fusion proteins. Further experimental details are provided in Example 7.

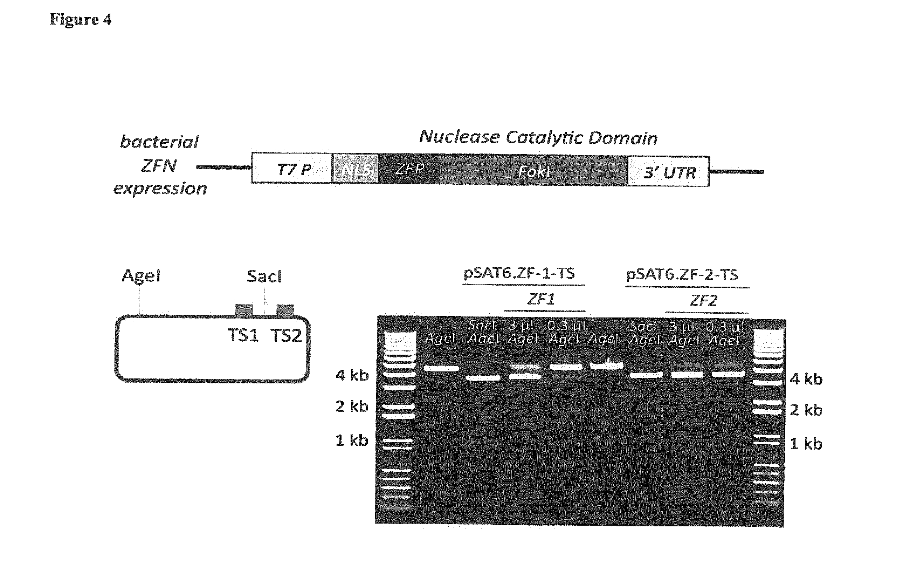

**Figure 4****: *De novo*-assembled PZF Arrays ZF1 and ZF2 Display DNA Binding Activity *in vitro***
   N-terminal fusions of both four-finger arrays to *Fok*I catalytic domains were generated and expressed in *E*. *coli.* Target site plasmids contain two ZF1 or ZF2 target sites that flank a Sacl restriction site. Cleavage with *Age*I and Sacl results in release of a 1 kb fragment. *Age*I linearized plasmids were incubated with crude lysates of Zinc-finger-*Fok*I fusions. This created a similar digestion pattern as seen in the *Age*I / *Sac*I double digest, suggesting that both arrays promote site-specific DNA cleavage *in vitro.*

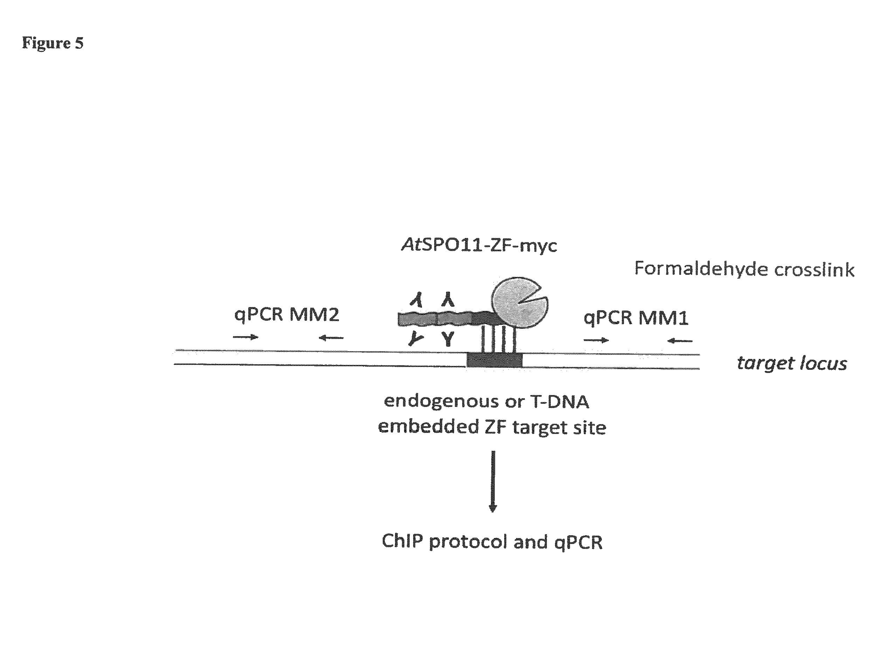

**Figure 5****: Analysis of *At*SPO11-1-PZF Target Binding Activity *in planta***
   Meiotic tissue from *Arabidopsis* lines expressing *At*SPO11-1-ZF1-18xMyc or *At*SPO11-1-ZF3-18xMyc fusions was subjected to formaldehyde cross-linking and anti-Myc Chromatin IP (ChIP). Plant lines expressing Myc-tagged SPO11-1 in absence of the PZF array were used as control. Immunoprecipitated chromatin was sheared by sonication to approximately 500bp length and qPCR analysis of target-flanking DNA was performed to quantify DNA binding levels at the target locus normalized to an unrelated genomic region.
**Figure 6****: Quantification of AtSPO11-1-PZF Target Binding Activity**
   Protein/DNA complexes (consisting of the AtSPO11-1-ZF-myc fusion covalently crosslinked to bound DNA sequences) were precipitated via chromatin IP. After immunoprecipitation the protein (inkl. SPO11) was digested and the remaining DNA was quantified by qPCR. Enrichment of target locus sequences were analyzed in both AtSPO11-1-ZF-18xMyc and AtSPO11-1-18xMyc lines and normalized to an off-target locus ACTIN2 using the delta Ct quantification method (Livak, K.J. & Schmittgen, T.D., 2001, Methods (San Diego, Calif), 25(4), pp.402-408). Further experimental details are provided in Example 8.
   Panels A, B and C depict individual experiments and were performed using plant cell suspension culture (A and B) or meiotic tissue from *Arabidopsis* lines expressing an AtSPO11-1-ZF1-18xMyc fusion (A), or an AtSPO11-1-ZF3-18xMyc fusion (B and C). Tissue was subjected to formaldehyde cross-linking and anti-Myc Chromatin IP (ChIP). Plant lines expressing Myc-tagged SPO11-1 in absence of the PZF array were used as control. Immunoprecipitated chromatin was sheared by sonication to approximately 500bp length and qPCR analysis of target-flanking DNA was performed to quantify DNA binding levels at the target locus (T) normalized to an unrelated genomic region (ACTIN2, A). Enrichment of target locus flanking sequences was analyzed by RealTime qPCR. Two parallel amplification reactions were performed to amplify a 200 bp sequence (within a 300 bp window) upstream (MM1) and downstream (MM2) of the respective zinc finger target sequences.
   **(A)** *At*SPO11-1-ZF1 Does Not Display Enhanced Target Binding Activity *in planta*:
      MM1 and MM2 depict two parallel qPCR amplification reactions upstream (MM1) and downstream (MM2) of the zinc finger target sequence. Y-axis depicts the ratio of fluorescence levels at regions directly upstream (MM1) and downstream (MM2) of the ZF target relative to background signals obtained by amplification of the ACTIN2 control locus. In other words, the y-axis depicts fold enrichment of depicted proteins at the target locus relative to protein levels at an endogenous off-target locus normalized using the ddCt method. qPCR analysis demonstrates that *At*SPO11-1-ZF1 is not enriched at the endogenous target locus.
   **(B)** AtSPO11-1-ZF3 Displays Enhanced Target Binding Activity *in planta:* Data on *At*SPO11-ZF3 target binding activity were obtained by analyzing *At*SPO11-1-ZF3 binding levels in transgenic cell suspension culture (CSC) by ChIP as depicted in Example 8. Myc-tagged SPO11 in absence of the ZF3 array was used as control. qPCR analysis was performed at regions upstream (MM1) and downstream (MM2) of the ZF3 target and suggests that *At*SPO11-1-ZF3 is enriched 2.5 to 3-fold in cell suspension culture at the target locus.
   **(C)** AtSPO11-1-ZF3 Displays Enhanced Target Binding Activity *in planta:* Data on *At*SPO11-ZF3 target binding activity in transgenic CSC were corroborated by analyzing *At*SPO11-1-ZF3 binding levels in formaldehyde fixed meiotic plant tissue. ChIP and qPCR analysis were performed as described above. Three transgenic lines *At*SPO11-1-ZF3 #7, #14, #16 were pooled for analysis. Myc-tagged SPO11 in absence of the ZF3 array was used as control. qPCR analysis suggests that *At*SPO11-1-ZF3 is enriched at the target locus in both somatic and meiotic tissue with elevated binding levels of 2.5 to 3-fold (Panel B, in CSC) and about 11-fold (Panel C, in meiotic tissue) compared to non-targeted *At*SPO11-1-18xMyc.
**Figure 7****: Detection of Locus-Specific Recombination Rates *in planta* via Fluorescent Pollen Tetrad Analysis**
   A pollen marker system (Francis K.E. et al., 2007, PNAS, 104(10), pp.3913-3918) provided the basis for fluorescent pollen tetrad analysis. A series of autofluorescent Proteins (AFPs) of different color under control of a pollen-specific LAT52 promoter were randomly inserted into the *Arabidopsis* genome. Lines were generated in the *quartet* mutant background. As a consequence the Fluorescence-Tagged Lines (FTL) give rise to plants that form fluorescent pollen grains tetrads that can be addressed by microscopy, comparable to tetrad analysis in yeast. Appropriate lines harboring a single FTL insertion of different color in close vicinity of the PZF target locus were used for tester interval generation.

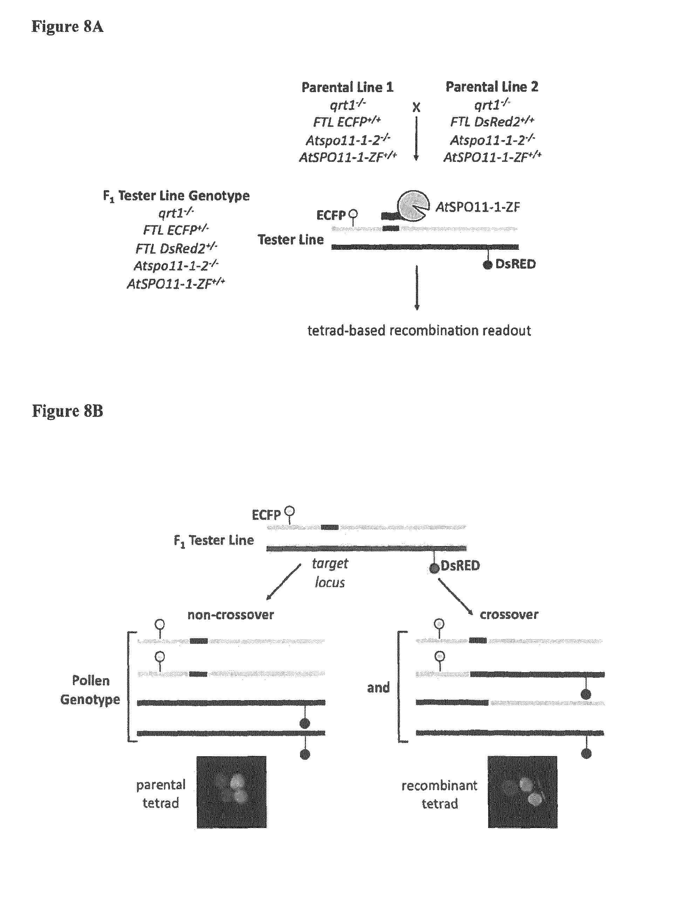

**Figure 8****: Detection of Locus-Specific Recombination Rates *in planta* via Fluorescent Pollen Tetrad Analysis**
   **(A)** FTL lines of different color flanking respective PZF target loci were selected and crossed to *At*SPO11-1-PZF lines. Isogenic plant lines were generated by propagation and selection. Parental lines harbor the *AtSPO11-1-PZF* transgene, the endogenous *Atspo11*-*1*-*2* mutation, the *quartet* mutation and the FTL transgene in homozygous configuration and differ only in the color of the expressed pollen-specific autofluorescent protein. Parental lines were crossed to generate the heteroallelic F1 tester line, harboring both FTL insertions *in trans* in a heterozygous state.
   **(B)** F1 tester line plants harbor both FTL insertions *in trans* in a heterozygous state. Plants were grown until flowering occurred. Within the flowers of these plants male gamete precursors undergo meiosis. Gametes of different fluorescence configuration are formed dependent on absence or occurrence of a meiotic recombination event between the FTL markers. Recombination events result in *trans* to *cis* pollen marker exchange and the formation of dual colored and non-colored pollen grains. Recombination readout is performed by high-throughput analysis of fluorescent marker exchange.

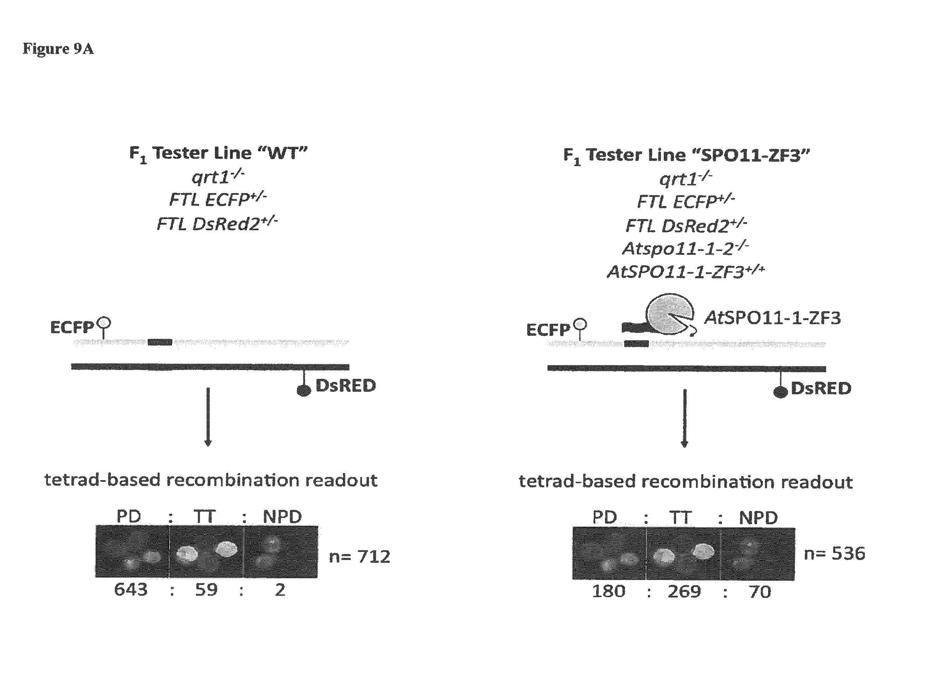

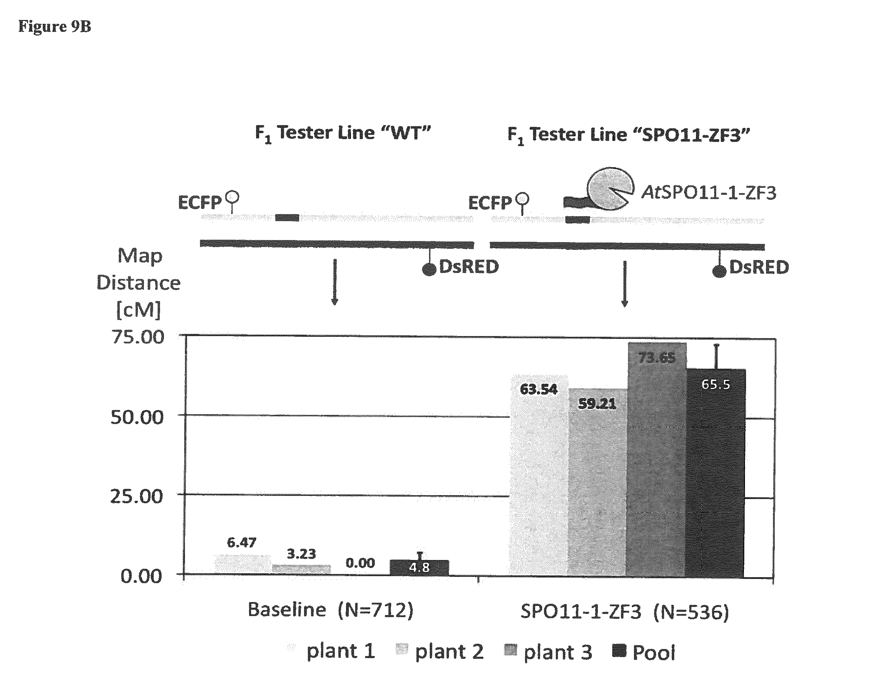

**Figure 9****: *At*SPO11-1-ZF3 Stimulates Locus-Specific Meiotic Recombination**
   **(A)** FTL lines harboring DsRed and YFP auto-fluorescent protein constructs were used to construct the ZF3 flanking tester line interval. Fluorescence microscopy was used to acquire single-channel DsRed and YFP images of pollen tetrads. CFP channels images were acquired to facilitate detection of non-fluorescent pollen grain. Three-channel overlay images were generated and scored for non-recombinant (single-colored) and recombinant (dual-colored) pollen tetrad fluorescence signal patterns. Preliminary distribution data on Parental Ditype (no cross-over, PD), Tetratype (one-strand cross-over, TT) and Non-Parental Ditype (two-strand cross-over, NPD) fluorescence patterns suggest strong elevation of recombinant tetrads in presence of the *At*SPO11-1-ZF3 fusion protein (SPO11-ZF3, right) compared to its absence (WT, left).
   **(B)** Frequencies of PD, TT and NPD fluorescence patterns were used to calculate genetic distances (Perkins D.D., 1949, Genetics, 34(5), pp.607-626; Ma C. & Mortimer R.K., 1983, Molecular and Cellular Biology, 3(10), pp.1886-1887) and thereby recombination rates in absence (WT, left) or presence (SPO11-ZF3, right) of the *At*SPO11-1-ZF3 fusion protein. FTL lines are located 1.3652 Mb upstream and 0.0841 Mb downstream of the ZF3 target site. Assuming 4.6 cM/Mb average genome-wide genetic distance in *Arabidopsis* (Drouaud J., et al., 2006, Genome research, 16(1), pp.106-114), a baseline recombination rate of 6.67 cM is expected within the tester interval. Data demonstrate strongly elevated recombination rates in presence of *At*SPO11-1-ZF3.

### The following Examples illustrate the invention:

Illustratively, as proof of the concept, the following examples demonstrate the usefulness of SPO11-fusion proteins to increase meiotic recombination in plants.

Plasmid construction describes generation of AtSPO11-1 genomic clones tagged with a zinc-finger array domain, but can be likewise applied to cloning of AtSPO11-2 and SPO11 homologs from other plant species as well. Furthermore it is described how SPO11-fusion protein coding sequences, embedded in an appropriate plant binary vector can be transformed into plants and how subsequently a tester plant with markers flanking the DSB target locus can be generated. It is anticipated that targeting either AtSPO11-1 or AtSPO11-2 is sufficient to redirect the meiotic DNA double strand break (DSB) machinery to a selected genomic locus and to locally promote DSB formation there. This targeted enhancement of meiotic DSBs leads to elevated meiotic recombination at the given genomic locus.

### Example 1: Construction of genomic AtSPO11-1 construct (plasmid pCB302/AtSPO11-1-WT)

AtSPO11-1 putative promoter and 5'-UTR sequences and the full-length genomic sequence were amplified from the genomic locus of *Arabidopsis thaliana* Ecotype *Columbia-0* (The European *Arabidopsis* Stock Centre, Seed Stock ID N1093, The *Arabidopsis* Information Resource (TAIR) genome annotation TAIR10) using KOD HiFi DNA Polymerase (Novagen) using primers gAtSPOdn (5'- AAA ACT GCA GAA TGT CGT CGG CGG ACA ACA CG -3; SEQ ID NO:43) and gATSPO_STOP_up (5'- CGC GGA TCC TCA CCC GGG AGG AGA GCT TAC TTC ACG ACG AAT C -3'; SEQ ID NO:44) and cloned into pCR2.1-TOPO (Invitrogen) resulting in pCR2.1/Fragment1. The AtSPO11-1 3'-UTR sequence was amplified using primers gATSPO_STOP_dn (5'- TCA ACC CGG GTG AAG ATC TGC TTC ATA CGA GAT AAC TAG TCT CC -3'; SEQ ID NO:45) and gAtSPOup (5'- CGC GGA TCC GCT GAA GCT GAA GTT GCC ACG -3'; SEQ ID NO:46) and cloned, resulting in pCR2.1/Fragment2. Primers gATSPO-STOP-up and gATSPO_STOP_dn were modified to introduce a unique *Sma*I blunt end restriction site (CCCGGG) coding for amino acids Proline and Glycine upstream of the AtSPO11-1 translational stop codon. PCR products were verified by Sanger sequencing prior to subcloning. Fragment1 (Promoter, 5'-UTR, AtSPO11-1 coding region; 3765 bp) was mobilized with *Pst*I and *Bam*HI and ligated into the binary vector pCB302 (Xiang C., et al., Plant molecular biology, 40(4), 1999, 711-717), resulting in pCB302/Fragment1 (SEQ ID NO:67). Fragment2 (3'-UTR; 446 bp) was mobilized with Smal and *Bam*HI and ligated into pCB302/Fragment1 resulting in pCB302/AtSPO11-1-WT (SEQ ID NO:68).

### Example 2: Construction of a c-Myc tagged AtSPO11-1 construct (plasmid pCB302/AtSPO11-1-18xMyc)

A 12-amino acid epitope tag, derived from the human oncogene c-myc (Evan G.I., et al., Molecular and Cellular Biology, 1985, 5(12), pp. 3610-3616) was fused to a genomic AtSPO11-1 construct to facilitate biochemical manipulation and cytological detection. An 18xMyc array was previously generated by PCR amplification using primers c-myc_new_dn (5'- ATC CAT GGC TCC CGG GTC CGG TTC TGC TGC TAG TGG -3; SEQ ID NO:47) and c-myc_new_up (5'- ATA GAT CTT TAG ATA TCT CGG TTC AAG TCT TCT TCT GAG AT -3'; SEQ ID NO:48) cloned into pCR2.1-TA (Invitrogen) and verified by Sanger sequencing. The 18xMyc Fragment (735 bp) was mobilized with *Sma*I and *Eco*RV and ligated into pCB302/AtSPO11-1-WT (SEQ ID NO:68) linearized with *Sma*I resulting in pCB302/AtSPO11-1-18xMyc (SEQ ID NO:69).

### Example 3: Construction of a genomic AtSPO11-1-GAL4BD construct (plasmid pCB302/AtSPO11-1-GAL4BD)

The GAL4BD sequence *(Arabidopsis* codon-usage-optimized, amino acids 1-147, Haseloff and Hodge, Patent no. US 6,255,558. 2001) was amplified from *Arabidopsis thaliana* H-GFP-2 genomic DNA (Haseloff and Hodge, Patent no. US 6,255,558. 2001) using primers GAL4_new_dn (5'- ATC CAT GGC TCC CGG GAT GAA GCT CCT GTC CTC CAT CG - 3'; SEQ ID NO:49) and Gal4_new_up (5'- ATG GAT CCT TAG ATA TCC GAG ACG GTC AAC TGG CGC TG -3'; SEQ ID NO:50) and cloned into pCR2.1-TOPO. Sanger sequencing revealed an endogenous G to A transition at nucleotide position 82, causing a glutamic acid to lysine substitution. The GAL4BD point mutation was removed from pCR2.1/GAL4BD by bridging PCR. Reactions were carried out using primers GAL4_new_dn (SEQ ID NO:49) and GAL4_2_mut_up (5'- TTC GGC TTC TCC TTG GAG CAC TTG -3', PCR 1, 96 bp fragment) (SEQ ID NO:51) and GAL4_2_mut_dn (5'- AAG TGC TCC AAG GAG AAG CCG AAG -3'; (SEQ ID NO:52)) and GAL4_new_up (PCR 2, 401 bp; (SEQ ID NO:50)). Products of both reactions (PCR 1 and 2) were excised from a 1.2% agarose gel and extracted using the E.Z.N.A Gel extraction kit (OMEGA Bio-Tek). Equal amounts (m/v) of eluted DNA were used in combination as annealed template in a third PCR reaction (PCR 3) using primers GAL4_new_dn (SEQ ID NO:49) and GAL4_new_up (SEQ ID NO:50). The corrected 474 bp GAL4BD fragment was again cloned into pCR2.1-TOPO resulting in pCR2.1/GAL4BD (SEQ ID NO:70) and the removal of the G to A transition confirmed by Sanger sequencing. GAL4BD (447 bp) was mobilized with *Sma*I and EcoRV and ligated into pCB302/AtSPO11-1-WT (SEQ ID NO:68) linearized with Smal resulting in pCB302/AtSPO11-1-GAL4BD (SEQ ID NO:71).

### Example 4: Construction of a transgenic GAL4BD target site construct (plasmid pGreenII-0179/5xUAS-short)

Tandem copies of UAS GAL4 binding sites are commonly used in GAL4-mediated enhancer trap systems and anticipated to attract AtSPO11-1-GAL4BD fusion proteins. An evaluation study conducted in *Arabidopsis* identified 5 tandem repeats of UAS 17-mers to be most effective (Engineer, C.B. et al., BMC Plant Biology 5(1), 2005, p. 9). Plant line H-GFP-2 (Haseloff and Hodge, Patent no. US 6,255,558. 2001) harbors an enhancer system composed of 5 UAS tandem repeats. Primers 5xUAS-forw (5'- TAT GGT ACC GAT TAC GCC AAG CTT GCA TG -3'; SEQ ID NO:53) and 5xUAS-rev (5'- TTG GCC ATG GAA CAG GTA GTT T -3'; SEQ ID NO:54) were used to amplify 5 tandem UAS, a minimal 35S promoter and an ER signal) and the 505 bp fragment was cloned into pCR2.1-TOPO resulting in pCR2.1/5xUAS (SEQ ID NO:73). The whole 5xUAS fragment (318 bp) was mobilized with Hindlll and EcoRI and ligated into pGreenll-0179 resulting in pGreenII-0179/5xUAS-long (SEQ ID NO:73). Co-amplified sequences of 122 bp were eliminated by Xbal digestion and religation, resulting in pGreenII-0179/5xUAS-short (SEQ ID NO:74) and sequence verification was performed by Sanger sequencing.

### Example 5: Construction of genomic AtSPO11-1-ZF constructs and AtSPO11-1-ZF-18xMyc constructs (plasmids pCB302/AtSPO11-1-ZF and pCB302/AtSPO11-1-ZF-18xMyc)

C-terminal fusions of AtSPO11-1-WT and AtSPO11-1-18xMyc genomic clones to zinc finger array DNA binding domains were generated to target AtSPO11-1 to unique sites within the genome of *Arabidopsis thaliana.* Zinc finger arrays were constructed by modular assembly (detailed description in Mani, M. et al., Biochemical and biophysical research communications, 335(2), 2005, 447-457.; Wright, D.A. et al., Nature protocols, 1(3), 2006, 1637-1652). Within this approach, zinc finger binding prediction was based on both experimental and computational data. Initial binding capacities of single zinc finger modules were identified with an ELISA based system (described for example by Segal, D.J. et al., 2003, Biochemistry, 42(7), pp. 2137-2148). Biotinylated hairpins of doule stranded (ds) DNA stretches containing the 3 bp zinc finger target were immobilized and incubated with serial dilutions of zinc finger protein fused to an epitope tag. Binding of the zinc finger protein to the respective target was detected by alkaline phophatase conjugated antibodies. Based on these ELISA data candidate interactors with a given triplet were deposited in publicly available databases. These databases provide amino acid sequences of R-helices that bind to a provided target sequence. Segal and coworkers randomly chose 80 three-finger proteins from the large set of available ZF proteins and could demonstrate that all of them did indeed bind to their predicted 9 bp target sequences in vitro. If these modules bind to their target in a chromatin embedded context was determined separately (see Example 8).

ZF3 and ZF4 were previously used in another context. ZF3 was used in the prior art (Neuteboom, L.W. et al., 2006, Biochemical and biophysical research communications, 339(1), pp. 263-270) to target a viral VP16 transactivation domain to a repressed MEL1 reporter gene in budding yeast. In addition ZF3 (also termed PTF1) was used in the prior art (de Pater, S. et al., 2009, Plant biotechnology journal, 7(8), pp. 821-835) to target a FokI nuclease domain together with another ZF array to a T-DNA construct within *Arabidopsis.* Binding of ZF4 to its target is not previously shown but personally communicated by the donor (Bert van der Zaal, Institute of Biology Leiden, Faculty of Science, Universiteit Leiden, The Netherlands).

Four-finger arrays ZF1 (target site 5' GGT CCT GGT GTA 3'; SEQ ID NO:55) and ZF2 (target site 5' GGT ACA GAT GCG 3'; SEQ ID NO:56) target the *Arabidopsis CSR1 locus* (CHLORSULFURON/IMIDAZOLINONE RESISTANT 1, AT3G48560.1, TAIR ID 2114524, nucleotides 1911 - 1922 (ZF1) and nucleotides 625 - 636 (ZF2) relative to translational start codon ATG). These modules were designed using the Barbas Lab online tool (http://www.scripps.edu/barbas/zfdesign/zfdesignhome.php). Two six-finger arrays ZF3 (target site 5' GAT GTC GCC GGC GCC GAT 3'; SEQ ID NO:57) and ZF4 (target site 5' GTC GGA GAC GCT GTC GAA 3'; SEQ ID NO:58) target an endogenous site within the *Arabidopsis* genome (ZF3, PINOID locus, AT2G34650.1, TAIR ID 4515108265, nucleotides 241 - 258 relative to translational start codon ATG) or a transgenic insertion (ZF4, aphIV, hygromycin phospotransferase plant selectable marker on pGreenII-0179 T-DNA, SEQ ID NO:6 nucleotides 1377 - 1394).

ZF1 and ZF2 sequences were amplified from templates pET28/CSR1-ZFN-1 (SEQ ID NO:75) and pET28/CSR1-ZFN-2 (SEQ ID NO:76) (provided by Tzvi Tzfira, formerly Department of Molecular, Cellular and Developmental Biology, University of Michigan, Ann Arbor, MI, USA) using high fidelity Ex Taq DNA Polymerase (TaKaRa Bio Inc.) and primers 4xZFN FW1 (5'- ATT ACC CGG GGC TGG TGG AGG TGA AAA ACC TTA CAA GTG TCC TGA ATG T -3'; SEQ ID NO:59) and 4xZFN REV1 (5'- TAT AGA TAT CGC CCG TAT GCG TCC TCT GAT -3'; SEQ ID NO:60) to introduce a 5' *Sma*I site, followed by a codon-usage optimized 1x Ala, 3x Gly linker and cloned into pCR2.1-TOPO resulting in pCR2.1/CSR1-ZF1 (SEQ ID NO:77) and pCR2.1/CSR1-ZF2 (SEQ ID NO:78). Cloned ZF1 and ZF2 sequences were verified by SANGER sequencing. ZF1 or ZF2 (354 bp) were mobilized with *Sma*I and *Eco*RV and ligated into pCB302/AtSPO11-1-WT (SEQ ID NO:68) or pCB302/AtSPO11-1-18xMyc (SEQ ID NO:69) linearized with Smal resulting in pCB302/AtSPO11-1-ZF1 (SEQ ID NO:79), pCB302/AtSPO11-1-ZF2 (SEQ ID NO:80), pCB302/AtSPO11-1-ZF1-18xMyc (SEQ ID NO:81) and pCB302/AtSPO11-1-ZF2-18xMyc (SEQ ID NO:82).

ZF3 and ZF4 sequences (573 bp) were mobilized from templates pSKNN-SgaI/PTF1-6F (SEQ ID NO:83) and pSKNN-SgaI/HPT-6F (SEQ ID NO:84) (provided by Bert van der Zaal, Institute of Biology Leiden, Faculty of Science, Universiteit Leiden, The Netherlands) with *Eco*RV and Smal and ligated into pCB302/AtSPO11-1-WT (SEQ ID NO:68) resulting in pCB302/AtSPO11-1-ZF3 (SEQ ID NO:85) and pCB302/AtSPO11-1-ZF4 (SEQ ID NO:15). The 18xMyc Fragment (735 bp) was mobilized with *Sma*I and *Eco*RV and ligated into pCB302/AtSPO11-1-ZF3 (SEQ ID NO:85) and pCB302/AtSPO11-1-ZF4 (SEQ ID NO:15) linearized with *Sma*I resulting in pCB302/AtSPO11-1-ZF3-18xMyc (SEQ ID NO:16) and pCB302/AtSPO11-1-ZF4-18xMyc (SEQ ID NO:17).

### Example 6: Generation of Arabidopsis thaliana AtSPO11-1 transgenic lines

Binary vector pCB302 (Xiang, C. et al., 1999, Plant molecular biology, 40(4), pp. 711-717) or pGreenII-0179 (hftp://www.pgreen.ac.uk/JIT/pG0179.htm) based constructs were introduced into *Agrobacterium tumefaciens* strains GV3101::pMP90 (Koncz, C. & Schell, J., 1986, MGG Molecular & General Genetics, 204(3), pp. 383-396) or C58C1::pSoup (Hellens, R.P. et al., 2000, Plant molecular biology, 42(6), pp.819-832), in case of pCB302 by electroporation and in case of pGreenII-0179 by heat shock. An ethyl methanesulfonate (EMS) mutagenesis-derived mutant allele *Atspo11-1-2* generated within the Col-0 ecotype (Grelon M. et al., EMBO J., 2001; 20(3): 589-600) was previously characterized, and confirmed to be an AtSPO11-1 null allele. Homozygous *Atspo11-1-2* mutant plants display a typical severe asynaptic meiotic phenotype, as well as significantly reduced seed production (Grelon M. et al., 2001, EMBO J., 20(3), pp.589-600; Hartung, F. & Puchta, H., 2000, Nucleic Acids Research,, 28(7), pp.1548-1554; Hartung, F. & Puchta, H., 2001, Gene, 271(1), pp.81-86) due to lack of DNA double strand/double stranded break (DSB) formation and random segregation of chromosomes in meiosis I. To determine the presence of the mutation, offspring of *Atspo11-2* heterozygous mutant plants was analyzed by PCR. A 954 bp amplicon from the *Atspo11-1-2* locus was amplified from genomic DNA with primers spo11-1-2-MG52 (5'- GGA TCG GGC CTA AAA GCC AAC G-3'; SEQ ID NO:61) and spo11-1-2-MG96 (5'- CTT TGA ATG CTG ATG GAT GCA TGT AGT AG -3'; SEQ ID NO:62). Presence of the *Atspo11-1-2* allele generated a cleaved amplified polymorphic sequence (CAPS) marker by introducing a Vspl restriction site and resulting in formation of 471 bp and 484 bp amplicon fragments after PCR product digest. Heterozygous *Atspo11-1-2* plants were identified and transformed with previously described pCB302 constructs (genomic AtSPO11-1 fusions) using a modified floral dip method (Clough, S.J. & Bent, A.F., The Plant journal : for cell and molecular biology, 1998, 16(6), pp.735-743). Ecotype Col-0 plants were analogously transformed with the pGreenll-0179 based 5xUAS GAL4BD target site construct. Successfully transformed T0 plants were selected by BASTA herbizide (pCB302 mediated), or Hygromycin antibiotic (pGreenII-0179 mediated) resistance. For each transformed AtSPO11-1 fusion protein several individual resistant transformants were analyzed for presence of the *Atspo11-1-2* mutant allele and propagated. In particular, the mutant spo11-1-2 status was determined by CAPS PCR as described above. Propagation was performed as follows. Plants were grown under 16h light cycles until flowering occured. Shoots with green fruit bodies were bagged (put into pergamin bags) until seeds were released. Seeds were separated from dry plant tissue by sieving and planted again. Transgene segregation analysis was performed in generation T1. Single-locus transgene integration was identified by analysis of the resistance phenotype (i.e. a segregation pattern of about 75% herbizide resistant vs. 25% sensitive plants; transgene insertion at two, three or more non-linked loci would lead to an elevated pattern of herbizide resistance).

The meaning of "elevated" in this regard is explained in the following. A single locus insertion will result in an offspring of 25% WT plants (i.e. herbicide sensitive), 50% heterozygous plants and 25% homozygous plants (75% in total, i.e. herbicide resistant). A transgene insertion at two loci A and B will lead to a more complex segregation pattern of different homozygous and heterozygous configurations and theoretically result in 93,7% heribicide resistant and 6,3% herbicide sensitive plants. Since pathogen mediated plant death might be misinterpreted as "sensitive" phenotype it is well accepted within the field that herbicide resistance rates above 80% indicate multiple transgene integration events.

Single locus insertion lines were further propagated to yield plants harboring AtSPO11-1 fusions and the *Atspo11-1-2* mutation (or a single 5xUAS insertion) in homozygous state.

### Example 7: Detection of AtSPO11-1 Fusion Protein Expression

Due to lack of functional AtSPO11 antibodies, 18xMyc based constructs were used for detection of fusion protein expression. Tissue enriched for meiotic cells (i.e. gamete precursor cells within the diploid flower primordia; referred to as "meiotic tissue") from *Arabidopsis thaliana* homozygous transgenic lines was harvested (unopened floral buds were collected on liquid nitrogen) and subsequently homogenized. Homogenization was performed by mechanical force by performing the steps (a) or (b) described below:
(a) grinding of floral buds under liquid nitrogen; or
(b) disruption of floral buds within extraction buffer.

Afterwards, protein extracts in 2x extraction buffer (100 mM Tris-Cl, pH 7.5; 20% Glycerol (v/v); 0.2% NP-40 (v/v); 2 mM EDTA, pH 8.0; 1 mM DTT, 2x Complete PI Cocktail (Mini Kit, Roche); 10 µg/mL Pepstatin) were prepared. In particular, tissue and extraction buffer were mixed. Homogenates of tissue and extraction buffer were separated by centrifugation at 13.000 RPM. The supernatant contained the plant protein extract (including the meiotically active proteins) and was used for further experimental procedures.

Samples were cleared by centrifugation and supplemented with 150 mM NaCl. 18xMyc fusion proteins were immunoprecipitated (mouse anti-Myc AB, clone 9E11), separated by SDS-PAGE and transferred to a PVDF-membrane (Immobilon-P, Millipore). Detection was performed using rabbit anti-Myc (Gramsch, #CM-100) and goat anti-Rabbit IgG (H+L, life technologies, #65-6120), peroxidase conjugated antibodies.

The 9E11 antibody is derived from a mouse hybridoma cell line that expresses the respective antibody and was produced and affinity-purified in-house. This antibody is commercially available from e.g., Santa Cruz Biotechnology Inc, and Dallas Texas, sc-47694.

### Example 8: Detection of AtSPO11-1-ZF fusion protein DNA binding activity

Due to lack of functional AtSPO11 antibodies, 18xMyc based constructs were used for detection of fusion protein DNA binding activity. Binding to target locus sequences was analyzed in both AtSPO11-1-ZF-18xMyc and AtSPO11-1-18xMyc lines. About 4g of buds from *Arabidopsis thaliana* homozygous transgenic lines was harvested and protein DNA interactions were cross-linked by treatment with 1% formaldehyde. Excess of cross-linking agent was quenched by addition of Glycine. The sample was homogenized in modified 2x extraction buffer (100 mM Tris-Cl, pH 7.5; 20 % Glycerol (v/v); 0.2 % NP-40 (v/v); 2 mM EDTA, pH 8.0; 1 mM DTT, 2x Complete PI Cocktail (Mini Kit, Roche); 16 µg/mL Pepstatin; 2 mM PMSF). Chromatin was sonicated in an ice bath to about 500 bp in length using an ultrasonic probe (Bandelin SONOPULS HD 2070, Bandelin electronic GmbH, Berlin, Germany) at the following settings: 4 cycles of 20 seconds each, 37% power output, continous duty cycle).

Afterwards, samples were cleared by centrifugation supplemented with 150 mM NaCl. Chromatin was pre-cleared by incubation with Protein G coated magnetic nanoparticles (Adembead Protein G, 200 nm size, Ademtech). Chromatin immunoprecipitation of 18xMyc fusion protein / DNA complexes was performed (mouse anti-Myc AB, clone 9E11). Beads were washed once in each Low Salt (150 mM NaCl; 0.2 % SDS; 0.5 % Triton X-100; 2 mM EDTA; 20 mM Tris-HCl pH 8), High Salt (500 mM NaCl; 0.2 % SDS; 0.5 % Triton X-100; 2 mM EDTA; 20 mM Tris-HCl pH 8), LiCl buffer (0.25 M LiCl; 0.5 % NP-40; 0.5 % Na-deoxycholate; 1 mM EDTA; 10 mM Tris-HCl pH 8) and twice in 1x TE buffer (10 mM Tris-HCl pH 8; 1 mM EDTA). Reversal of cross-links was performed in TES elution buffer (50 mM Tris-HCl, pH 8.0; 10 mM EDTA; 1% SDS; 200 mM NaCl) by incubation at 65°C 3h to overnight. Chromatin was treated with RNase A and Proteinase K. DNA was purified by Phenol / Chloroform / Isoamylalcohol extraction and EtOH / NaCl / Glycogen precipitation.

Enrichment of target locus flanking sequences was analyzed by RealTime PCR mediated amplification of approx. 200 bp sequences within 300 bp windows upstream and downstream of respective zinc finger target sequences. Two parallel amplification reactions were performed. A 179 bp amplicon upstream of the PTF1-6F target locus was amplified with primers PTF1-6F RT up 1 fwd (5'- AAA TCA CCA CCG GAG CAA TA -3'; SEQ ID NO:63) and PTF1-6F RT up 1 rev (5'- TTT GCG ATG AAA GTT GTG GA -3'; SEQ ID NO:64). A 174 bp amplicon downstream of the PTF1-6F target locus was amplified with primers PTF1-6F RT down 1 fwd (5'- GCG AAA ATC TCG GAA GGT TA -3'; SEQ ID NO:65) and PTF1-6F RT down 1 rev (5'- AGC AGC GGA ACA GAG AGT TG -3'; SEQ ID NO:66). Quantitative Real Time PCR amplifications were performed using the iQ™ SYBR® Green Supermix (BIO-RAD #170-8880) on an iCycler iQ™ Real-Time PCR Detection System (BIO-RAD #170-8740) equipped with iQ™5 Optical System Software v2.1 (BIO-RAD #170-9753) according to the manufacturers recommendations.

The readout of qPCR is fluorescence intensity, which is mediated by a fluorescent dye that intercalates into the PCR amplification product and which levels are periodically monitored. Per single experiment DNA sequences upstream and downstream of the ZF target are amplified. In parallel DNA sequences at a locus where AtSPO11-ZF-myc is not anticipated to specifically bind (the ACTIN2 gene) are amplified as control. Fluorescence levels at the target were corrected by fluorescence levels at the ACTIN2 control using the delta Ct quantification method (Livak, K.J. & Schmittgen, T.D., 2001, Methods (San Diego, Calif), 25(4), pp.402-408).

### Example 9: Targeted stimulation of meiotic recombination in Arabidopsis thaliana by AtSPO11-1-ZF fusion proteins

### (A) AtSPO11-1-ZF-18xMyc fusion proteins are expressed in meiosis

A T-DNA encoding the fusion protein comprising the full length AtSPO11-1 and the zinc finger ZF3 DNA binding domain at its C-terminus was introduced into *Arabidopsis thaliana Atspo11-1-2* heterozygous mutant plants via *Agrobacterium tumefaciens* mediated transformation, as described in Example 6. Single locus insertion lines were identified and plants homozygous for the *Atspo11-1-2* mutant allele and AtSPO11-1-ZF3-18xMyc transgene were derived via propagation and selection, as described in Example 6. Three independently transformed lines containing the AtSPO11-1-ZF3-18xMyc construct were analyzed for fusion protein expression. Meiotic tissue from lines expressing AtSPO11-1-18xMyc (93 kDa signal) and WT tissue from ecotype Col-0 were analyzed as controls, as described in Example 7. AtSPO11-1-ZF3-18xMyc insertion lines #7, #14 and #16 display well-detectable but different levels of fusion protein expression, indicating that PZF and Myc-epitope tags do not hinder expression of the respective fusion proteins (Figure 3). The 18x c-Myc tagged AtSPO11-1 positive control can be detected as 93 kDa signal, the 192 amino acid ZF3 DNA binding domain confers a calculated shift of about 21 kDa, resulting in a 114 kDa signal for AtSPO11-1-ZF3-18xMyc fusion proteins. Similar results were obtained for AtSPO11-1-ZF1-18xMyc and AtSPO11-1-ZF2-18xMyc insertion lines.

### (B) AtSPO11-1-ZF fusion proteins complement the fertility defect of the Atspo11-1-2 mutant line

Homozygous *Atspo11-1-2* mutant plants exhibit an overall morphology similar to wild type, except for seed production. *Atspo11-1-2* mutants produced very reduced amounts of seeds (Grelon M. et al., 2001, EMBO J., 20(3), pp.589-600) due to random segregation of chromosomes caused by absence of meiotic recombination initiation.

*Atspo11-1* mutant plants do produce few seeds, since chromosomes segregate randomly during meiosis within these plants due to lack of DSB formation. Plants harboring a functional SPO11-1-ZF or SPO11-1-ZF-myc transgene in an endogenous *Atspo11-1* mutant background show again wild type levels of seed formation if the transgene is capable of introducing wild type levels of DSB formation.

The ability of various AtSPO11-1 fusion proteins to complement the fertility defect of an *Atspo11-1-2* plant line has been examined. AtSPO11-1 fusions to DNA binding domains ZF1 to ZF4, GAL4 and 18xMyc display similar fertility levels as wild type Col-0 plants. Similar results were obtained for constructs harboring ZF1 to ZF4 and 18xMyc domains in addition (Figure 2). These results demonstrate that AtSPO11-1-fusions are functional and do not confer any adverse effects. Since fertility levels are comparable to wild type it is concluded that AtSPO11-1 fusion proteins induce DSB formation on all five *Arabidopsis* chromosomes and therefore likely also mediates DSB formation at natural sites.

### (C) Modules ZF1 to ZF3 promote DNA binding at target sites

N-terminal fusions of ZF1 and ZF2 to Fokl catalytic domains were expressed in E. coli from plasmids pET28/CSR1-ZFN-1 (SEQ ID NO:75) and pET28/CSR1-ZFN-2 (SEQ ID NO:76) (provided by Tsvi Tzfira, formerly Department of Molecular, Cellular and Developmental Biology, University of Michigan, Ann Arbor, MI, USA). Target site plasmids pSAT6/ZF-1-TS (SEQ ID NO:18) or pSAT6/ZF-2-TS (SEQ ID NO:86) contain two ZF1 or ZF2 target sites that flank a Sacl restriction site. *Age*I linearized plasmids were incubated with crude lysates of ZF-*Fok*I fusions in analogy to (Tovkach, A., et al., 2009, The Plant Journal, 57(4), pp.747-757). This created a similar digestion pattern as seen in an *Age*I / *Sac*I double digest, suggesting that both ZF1-*Fok*I and ZF2-*Fok*I promote site-specific DNA binding and subsequent cleavage *in vitro* (Figure 4).

ZF3 was successfully used in the prior art (termed PTF1-6F) to target a viral VP16 transactivation domain to a repressed MEL1 reporter gene in budding yeast (Neuteboom L.W. et al., 2006, Biochemical and biophysical research communications, 339(1), pp.263-270) and to target a FokI nuclease fusion together with another PZF array E2C (Beerli, R.R. et al., 1998, Proceedings of the National Academy of Sciences of the United States of America, 95(25), pp.14628-14633.) to a T-DNA reporter construct in *Arabidopsis* (de Pater S. et al., 2009, Plant biotechnology journal, 7(8), pp.821-835). The E2C protein is based on E2C(Sp1) as studied by Beerli (1998), loc. cit. This demonstrates that ZF3 is proficient to specifically bind a chromatin embedded site in the context of a higher eukaryotic organism.

### (D) Differential DNA binding activity of modules ZF1 and ZF3 to target sites in planta

To analyze AtSPO11-1-ZF target binding activity *in planta,* meiotic tissue from *Arabidopsis* lines expressing AtSPO11-1-ZF1-18xMyc or AtSPO11-1-ZF3-18xMyc was analyzed by ChIP post formaldehyde cross-linking (see Example 8 for detailed explanation). Plant lines expressing Myc-tagged SPO11-1 in absence of the ZF arrays were used as control. AtSPO11-1-ZF1-18xMyc did not display target-binding activity *in planta* (Figure 6(A)). AtSPO11-1-ZF3-18xMyc showed 2.5- to 3-fold enrichment over AtSPO11-1-18xMyc at the target locus in transgenic cell suspension culture (Figure 6(B)) and 11-fold enrichment in meiotic tissue (Figure 6(C)).

Herein, the term "transgenic cell suspension culture" refers to a meristematic suspension cell culture (i.e. non-differentiated, non-meiotic *Arabidopsis* cells dervided from either root or shoot meristems) that is transiently transformed with a pCB302/AtSPO11-ZF-Myc construct. The term "meiotic tissue" refers to generative tissue that contains meiotically active cells (i.e. unopened floral buds).

### (E) AtSPO11-1-ZF3 but not AtSPO11-1-ZF1 stimulates targeted meiotic recombination

Detection of locus-specific recombination rates *in planta* was analyzed via "Fluorescent Pollen Tetrad Analysis" (Francis K.E. et al., 2007, PNAS, 104(10), pp.3913-3918; Berchowitz, L.E. & Copenhaver, G.P., 2008, Nature protocols, 3(1), pp.41-50). "Fluorescence Tagged Lines" FTL 1268 (DsRed2, Chromosome 3, Position 17242947) and FTL 2201 (AmCyan, Chromosome 3, Position 18319139) were separately introgressed¹ into plants carrying an AtSPO11-1-ZF1 single locus insertion (Figure 7).
¹ The term "introgressed" can be replaced with the term "introduced" for the sake of clarity. Introduction of the said FTL markers into the plant genome of AtSPO11-1-ZF transgenic lines occured by the standard technique called crossing (e.g. pollen from a FTL plant is transferred to a stigma of an emasculated flower of an AtSPO11-1-ZF plant). The progeny of this crossing harbors both the FTL marker and the AtSPO11-1ZF transgene. FTL lines have been generated wtihin the COPENHAVER lab and are available to the academic community free of charge upon request. Details of FTL line preparation are found within Francis K.E. et al., 2007, PNAS, 104(10), pp.3913-3918; and Berchowitz, L.E. & Copenhaver, G.P., 2008, Nature protocols, 3(1), pp.41-50.

Similarly, FTL lines 1524 (YFP, Chromosome 2, Position 13226013) and FTL 965 (DsRed2, Chromosome 2, Position 14675407) were introduced into plants carrying an AtSPO11-1-ZF3 single locus insertion. Plants were propagated to yield individuals homozygous for the AtPSO11-ZF transgene, the endogenous *Atspo11-1-2* mutation, the *quartet* mutation and the FTL transgene.

Homozygous parental individuals were crossed to generate tester plants, homozygous for all features mentioned above apart from the heterozygous FTL insertions, flanking the target sites (Figure 8(A)).

In detail the following crossings were performed:
AtSPO11-1-ZF1 ; FTL1268 x AtSPO11-1-ZF1 ; FTL 2201
AtSPO11-1-ZF3 ; FTL 1524 x AtSPO11-1-ZF3 ; FTL 965

Additionally, FTL line crossings 1268 x 2201 and 1524 x 965 in absence of an AtSPO11-1 transgene were performed to analyze basic recombination rates at the respective genetic intervals. F1 plants were grown under controlled environmental conditions (22°C, 16 h light, 8 h dark) and pollen tetrads were analyzed by fluorescence microscopy. Single-channel DsRed CFP and YFP images were acquired (the third channel was used to facilitate detection of non- fluorescent pollen grains). Distribution data on Parental Ditype (no cross- over, PD), Tetratype (one-strand cross-over, TT) and Non-Parental Ditype (two-strand cross-over, NPD) fluorescence patterns was obtained from three-channel overlays. Frequencies of PD, TT and NPD fluorescence patterns were used to calculate genetic distances (Perkins D.D., 1949, Genetics, 34(5), pp.607-626; Ma C. & Mortimer R.K., 1983, Molecular and Cellular Biology, 3(10), pp.1886-1887) and thereby recombination rates in absence or presence of the respective AtSPO11-1-ZF fusion proteins (Figure 8(B)).

AtSPO11-1-ZF1 did mildly stimulate locus-specific meiotic recombination. Previous analysis did not demonstrate significant enrichment of AtSPO11-1-ZF1 binding levels at the respective target locus (see Figure 6(A)). In line with this observation only mild stimulation of locus-specific recombination at the ZF1 target locus was observed. Baseline map distance within the given interval was 4.83 cM (close to the calculated 4.95 cM based on a genome wide average of 4.6 cM/Mb (Drouaud J. et al., 2006, Genome research, 16(1), pp.106-114)). Some individual AtSPO11-1-ZF1 plants displayed elevated recombination frequencies, ranging from 6.07 to 6.77cM. Analysis of pooled recombination rates between WT and AtSPO11-1-ZF1 rendered these elevations statistically not significant.

AtSPO11-1-ZF3 fusions displayed strong stimulation of locus- specific recombination rates. FTL lines are located 1.3652 Mb upstream and 0.0841 Mb downstream of the ZF3 target site. A baseline recombination rate of 6.67 cM is expected within this tester interval (based on a genome wide average of 4.6 cM/Mb (Drouaud J. et al., 2006, Genome research, 16(1), pp.106-114). Recombination data on FTL baseline and AtSPO11-1-ZF3 tester lines was obtained in a double-blinded manner (different persons performed image acquisition and recombination data readout and had no knowledge about the respective plant genotypes). Frequencies of fluorescence patterns were significantly different in absence (PD:TT:NDP=643:59:2; 5.04 cM; n=704) and presence of an AtSPO11-1-ZF3 transgene (PD:TT:NPD=180:269:70; 66.38 cM, n=536). Thereby recombination data suggests about 10-fold elevated recombination rates in presence of AtSPO11-1-ZF3 (Figures 9(A) and 9(B)).

### Example 10: Targeted stimulation of meiotic recombination in Arabidopsis thaliana using AtSPO11-1-GAL4BD or AtSPO11-1-ZF4 fusion proteins

Construction and testing of individual zinc finger arrays that successfully direct an *Arabidopsis* SPO11 protein to an endogenous target is time consuming and labour intensive. An alternative strategy comprises the generation of AtSPO11-1 operably linked to a GAL4 DNA binding domain (GAL4BD) or to a zinc finger array targeting a hygromycin resistance gene (ZF4). Both target features are combined on plasmid pGreenII-0179/SxUAS-short. Several *Arabidopsis thaliana* ecotype Col-0 plant lines were generated harboring a pGreenII-0179 based 5xUAS/GAL4BD target site construct as single locus insertion at known positions within the *Arabidopsis* genome.

Fusions encoding the GAL4BD or the zinc finger ZF4 DNA binding domain with the C-terminus of the full-length AtSPO11-1 protein were generated in *Atspo11-1-2* homozygous mutant plants as described above. These fusion proteins display similar fertility levels as wild type Col-0 plants. These results demonstrate that AtSPO11-1-GAL4BD and AtSPO11-1-ZF fusions are functional and likely promote DSB formation at natural sites. Detection of locus-specific recombination rates *in planta* can be analyzed via FTL analysis as described above. Different to the previously outlined strategy, a mapped 5xUAS / HYG T-DNA insertion and appropriate FTL lines flanking the target locus need to be introgressed into plants expressing AtSPO11-1-GAL4BD or AtSPO11-ZF4 single locus insertions. Two 5xUAS lines #1 (Chromosome 2, Position 16363755) and 5xUAS line #19 (Chromosome 3, Position 17966362) were selected due to availability of flanking FTL lines:
5xUAS line #1
   FTL 1268 (DsRed2, Chromosome 3, Position 17242947)
   FTL 2201 (AmCyan, Chromosome 3, Position 18319139)
   Interval = 1076192 bp = 4.95 cM
5xUAS line #19
   FTL 965 (DsRed2, Chromosome 2, Position 14675407)
   FTL 2913 (CFP, Chromosome 2, Position 16387996)
   Interval = 1713589 bp = 7.88 cM

Plants are propagated to yield individuals homozygous for the AtSPO11-GAL4BD or AtSPO11-1ZF4 transgene, the 5x UAS/HYG target construct, the endogenous *Atspo11-1-*2 mutation, the *quartet* mutation and the FTL transgene. Homozygous parental individuals are crossed to generate tester lines. Analysis of recombination and calculation of genetic distances is performed as described above.

Alternatively, the stimulation of meiotic recombination is determined by analysis of microsatellite markers between different *Arabidopsis* ecotypes Columbia (Col-0) and Landsberg erecta (Ler) upstream and downstream of the 5xUAS/HYG target sites. The advantage is that not only the target site can be monitored for recombination rates, but also all other regions of the genome. The drawback is that recombination rate analysis via microsatellite markers is more labour intensive. Suitable CAPS or INDEL (insertion/deletion) markers have been identified through public resources (*Arabidopsis* Mapping Platform, National Laboratory for Protein Engineering and Plant Genetic Engineering, College of Life Sciences, Peking University, Beijing 100871, China, http://amp.genomics.org.cn or MSAT Database, Institut Jean-Pierre Bourgin, INRA Versailles-Grignon, 78026 Versailles Cedex, France, http://www7.inra.fr/vast/msat.php). To obtain tester lines, the AtSPO11-ZF transgene, the endogenous *Atspo11-1-2* mutation and the 5xUAS/HYG target site are introgressed² into the Ler ecotype. A plant with all above features in homozygous configuration (including all Ler specific microsatellite markers) is crossed to a Col-0 plant with all features in homozygous configuration (including all Col-0 specific microsatellite markers) to yield a tester line for recombination assays. Offspring plants are tested for events of recombination between flanking microsatellite markers, identified by presence of a heterozygous and a homozygous INDEL marker within the same plant.
² See footnote 1, *supra.*

### Example 11: Targeted stimulation of meiotic recombination in crop plants

SPO11 protein homologs are responsible for meiotic DSB formation in all higher eukaryotes from fungi to mammals. Zinc finger DNA binding domains of sufficient length allow targeting of programmed DSB formation to nearly any desired locus (with open chromatin configuration) in the genome of an organism. Any SPO11 protein of a higher plant core DSB complex can be used following the above outlined strategy. The SPO11-ZF strategy is open to all crop plant species that are amenable to transformation and with sufficient sequence information at hand to design zinc finger proteins. SPO11-ZF can be seen as an extremely efficient enhancer of conventional breeding strategies and reduce numbers of crosses needed to combine beneficial traits in regions of low genetic distance.

## Claims

1. A fusion protein comprising a zinc finger domain which is operably linked via its N-terminus to the C-terminus of a SPO11 polypeptide, wherein the zinc finger domain comprises six or more zinc finger modules.

2. The fusion protein of claim 1, wherein the zinc finger domain recognizes triplets selected from the group consisting of GNN, ANN, CNN or TNN.

3. The fusion protein of claim 2, wherein the zinc finger domain comprises a sequence as depicted in any one of SEQ ID NOs: 19 to 26.

4. The fusion protein according to any one of claims 1 to 3, wherein the SPO11 polypeptide is from *Arabidopsis thaliana.*

5. The fusion protein of claim 4, wherein the SPO11 polypeptide from *Arabidopsis thaliana* is selected from the group consisting of AtSPO11-1 (SEQ ID NO: 7), AtSPO11-2 (SEQ ID NO: 9) or a functional fragment thereof which is capable of initiating meiotic recombination.

6. A nucleic acid molecule encoding the fusion protein according to any one of claims 1 to 5.

7. The nucleic acid molecule of claim 6, wherein the fusion protein is expressed under the control of a meiosis specific promoter.

8. The nucleic acid molecule of claim 6 or claim 7, wherein the SPO11 polypeptide is encoded by a genomic DNA.

9. A vector containing the nucleic acid molecule of any one of claims 6 to 8 capable of expressing said nucleic acid molecule in a eukaryotic host cell.

10. A host cell comprising the nucleic acid molecule of any one of claims 6 to claim 8, or the vector of claim 9.

11. A method for producing the fusion protein according to any one of claims 1 to 5, comprising the steps of expressing a nucleic acid molecule of any one of claims 6 to 8 in a eukaryotic host cell and recovering the fusion protein, from said cell or the cell culture supernatant.

12. A fusion protein obtained by the method of claim 11.

13. The host cell of claim 10 or the method of claim 11, wherein the eukaryotic host cell is a plant cell.

14. A method for increasing recombination between two defined loci of two homologous chromosomes in a meiotic plant cell comprising the steps of:
(i) introducing into said cell the nucleic acid molecule of any one of claims 6 to 8, or the vector of claim 9 encoding the fusion protein as defined in any one of claims 1 to 5, which induces double-stranded breaks in DNA during meiosis, and
(ii) having the cell perform a meiosis in which the recombination between two defined loci of two homologous chromosomes in said plant cell is increased.

15. A method for generating variants of a plant comprising the steps of:
(i) introducing into a cell of a plant the nucleic acid molecule of any one of claims 6 to 8, or the vector of claim 9 encoding the fusion protein as defined in any one of claims 1 to 5, which induces meiotic double-stranded breaks in DNA;
(ii) having said cell perform a meiosis, so that meiotic recombination occurs between homologous chromosomes of said cell, at a higher rate and/or at different loci than in natural meiosis of said plant; and
(iii) generating variants of said plant containing the cell of step (ii).

16. The method of claim 15 further comprising a step of performing a genetic and/or phenotypic analysis for the presence of the meiotic recombination in said variants.

17. The method of claim 15 or claim 16 further comprising a step of crossing with wild-type plants and selecting for plants in which the nucleic acid of any one of claims 6 to 8 or the vector of claim 9 is absent in the cells of said variants.

18. Use of a fusion protein of any one of claims 1 to 5, the nucleic acid molecule of any one of claims 6 to 8, or the vector of claim 9 for increasing meiotic DNA double strand breaks in a plant.

19. The use of claim 18, wherein said fusion protein is capable of inducing meiotic double-strand breaks in formerly cold regions in said plant.

20. The host cell of claim 13, the method of any one of claims 14 to 17, or the use of claims 18 or 19, wherein the plant is a crop plant.

21. The host cell, the method, or the use of claim 20, wherein the crop plant is a plant belonging to a family selected from the group consisting of *Brassicaceae.*


SEQUENCE LISTING

<110> University of Vienna

<120> Means and Methods for Accelerated Breeding By Inducing Targeted
Stimulation of Meiotic Recombination

<130> X3133 EP S3

<160> 86

<170> BiSSAP 1.2

<210> 1
<211> 573
<212> DNA
<213> Artificial Sequence

<220>
<221> source
<222> 1..573
<223> /mol_type="unassigned DNA"
      /note="Nucleotide sequence encoding PTF1-6F"
      /organism="Artificial Sequence"

<400> 1
atcgatctcg aggcccaggc ggccctcgaa cccaccggcg agaagcctta tgcctgtcct      60

gagtgcggga agtcttttag cacctcgggt aatctcgtcc gtcaccaacg tacgcatacc     120

ggcgagaagc cttacgcttg cccggagtgt ggcaagtcat tcagcgactg ccgtgatctc     180

gcaaggcatc aacgcaccca taccggcgag aaaccgtacg cgtgccctga atgcggtaaa     240

agcttctctg acccaggtca cctcgtccgc catcagagga cccataccgg cgagaagcct     300

tacgcttgcc cggagtgtgg caagtcattc agcgactgcc gtgatctcgc aaggcatcaa     360

cgcacccata ccggcgagaa accgtacgca tgccctgagt gtggcaagtc cttctccgat     420

ccgggagcac tcgttcggca tcaacgcacc cataccggcg agaagcctta tgcctgtcct     480

gagtgcggga agtcttttag cacctcgggt aatctcgtcc gtcaccaacg tacgcatacc     540

ggcgagctgg gtggcggttc cggagaaaag ccc                                  573


<210> 2
<211> 183
<212> PRT
<213> Artificial Sequence

<220>
<223> Amino acid sequence of zinc finger array PTF1-6F

<400> 2
Leu Glu Pro Thr Gly Glu Lys Pro Tyr Ala Cys Pro Glu Cys Gly Lys
1               5                   10                  15
Ser Phe Ser Thr Ser Gly Asn Leu Val Arg His Gln Arg Thr His Thr
            20                  25                  30
Gly Glu Lys Pro Tyr Ala Cys Pro Glu Cys Gly Lys Ser Phe Ser Asp
        35                  40                  45
Cys Arg Asp Leu Ala Arg His Gln Arg Thr His Thr Gly Glu Lys Pro

50                  55                  60
Tyr Ala Cys Pro Glu Cys Gly Lys Ser Phe Ser Asp Pro Gly His Leu
65                  70                  75                  80
Val Arg His Gln Arg Thr His Thr Gly Glu Lys Pro Tyr Ala Cys Pro
                85                  90                  95
Glu Cys Gly Lys Ser Phe Ser Asp Cys Arg Asp Leu Ala Arg His Gln
            100                 105                 110
Arg Thr His Thr Gly Glu Lys Pro Tyr Ala Cys Pro Glu Cys Gly Lys
        115                 120                 125
Ser Phe Ser Asp Pro Gly Ala Leu Val Arg His Gln Arg Thr His Thr
    130                 135                 140
Gly Glu Lys Pro Tyr Ala Cys Pro Glu Cys Gly Lys Ser Phe Ser Thr
145                 150                 155                 160
Ser Gly Asn Leu Val Arg His Gln Arg Thr His Thr Gly Glu Leu Gly
                165                 170                 175
Gly Gly Ser Gly Glu Lys Pro
            180

<210> 3
<211> 573
<212> DNA
<213> Artificial Sequence

<220>
<221> source
<222> 1..573
<223> /mol_type="unassigned DNA"
      /note="Nucleotide sequence encoding HPT-6F"
      /organism="Artificial Sequence"

<400> 3
atcgatctcg aggcccaggc ggccctcgaa cccaccggcg agaaaccgta cgcttgccct      60

gaatgcggaa aaagcttctc tcagtccagc aacctcgtga ggcatcagag gacccatacc     120

ggcgagaaac cgtacgcatg ccctgagtgt ggcaagtcct tctccgatcc gggagcactc     180

gttcggcatc aacgcaccca taccggcgag aaaccgtatg cgtgccccga atgcgggaaa     240

tccttttcga ccagcggtga actcgtgcgt caccagcgta cgcataccgg cgagaagccc     300

tacgcatgcc cggaatgcgg aaaatccttc tccgatcccg gtaacttggt tcggcatcag     360

cgcacccata ccggcgagaa accatacgcc tgtcccgagt gcggtaaaag ctttagtcag     420

cgtgcccatc ttgaacgcca ccagaggacc cataccggcg agaaaccgta cgcatgccct     480

gagtgtggca agtccttctc cgatccggga gcactcgttc ggcatcaacg cacccatacc     540

ggcgagctgg gtggcggttc cggagaaaag ccc                                  573


<210> 4
<211> 183
<212> PRT
<213> Artificial Sequence

<220>
<223> Amino acid sequence of zinc finger array HPT-6F

<400> 4
Leu Glu Pro Thr Gly Glu Lys Pro Tyr Ala Cys Pro Glu Cys Gly Lys
1               5                   10                  15

Ser Phe Ser Gln Ser Ser Asn Leu Val Arg His Gln Arg Thr His Thr
            20                  25                  30
Gly Glu Lys Pro Tyr Ala Cys Pro Glu Cys Gly Lys Ser Phe Ser Asp
        35                  40                  45
Pro Gly Ala Leu Val Arg His Gln Arg Thr His Thr Gly Glu Lys Pro
    50                  55                  60
Tyr Ala Cys Pro Glu Cys Gly Lys Ser Phe Ser Thr Ser Gly Glu Leu
65                  70                  75                  80
Val Arg His Gln Arg Thr His Thr Gly Glu Lys Pro Tyr Ala Cys Pro
                85                  90                  95
Glu Cys Gly Lys Ser Phe Ser Asp Pro Gly Asn Leu Val Arg His Gln
            100                 105                 110
Arg Thr His Thr Gly Glu Lys Pro Tyr Ala Cys Pro Glu Cys Gly Lys
        115                 120                 125
Ser Phe Ser Gln Arg Ala His Leu Glu Arg His Gln Arg Thr His Thr
    130                 135                 140
Gly Glu Lys Pro Tyr Ala Cys Pro Glu Cys Gly Lys Ser Phe Ser Asp
145                 150                 155                 160
Pro Gly Ala Leu Val Arg His Gln Arg Thr His Thr Gly Glu Leu Gly
                165                 170                 175
Gly Gly Ser Gly Glu Lys Pro
            180

<210> 5
<211> 2029
<212> DNA
<213> Arabidopsis thaliana

<220>
<221> source
<222> 1..2029
<223> /mol_type="unassigned DNA"
      /note="Nucleotide sequence of the PINOID gene incl. regulatory
      regions"
      /organism="Arabidopsis thaliana"

<400> 5
aaaactccaa cacttctcct tcttccatct ctctcaaact tctgaaaatc ttcttcttct      60

tctcaatcat aaaccctaaa tcttcatctc ttcagatcag aaactaatgt ccattcaaag     120

acaccctgtc ctgtttcatc tctgtcatta gtcttctgtt ttcagattcc aatttttttt     180

cttgaattat cctcttagcc atttcttgat ttaacttcga ttttcccggc gatgttacga     240

gaatcagacg gtgagatgag tttaggaaca acaaactcac cgataagcag cggaacagag     300

agttgcagca gtttcagccg gttatcattc gacgcgccgc cgtcaactat ccccgaagaa     360

gaaagcttcc tttctctcaa acctcaccga tcctcagatt tcgcttacgc agagatccga     420

agacgaaaaa aacaaggcct aaccttccga gattttcgcc tcatgcgtcg tatcggcgcc     480

ggcgacatcg gaacagttta cttatgccgt ctagccggag acgaagaaga gagccggagc     540

tcgtattttg cgatgaaagt tgtggataaa gaagctcttg cgttgaagaa gaagatgcat     600

agagcagaga tggagaaaac gattttgaaa atgcttgacc atccattttt gccgactctt     660

tacgctgagt ttgaagcctc acatttctct tgcatcgtta tggaatattg ctccggtggt     720

gatttacact ctctccgtca tagacaacct caccggcgat tctccctctc ttccgccagg     780

taaaaaatat caaattttat tgaataattt aatattatgg acaaagtcag attttttttc 840

aaaaaaaaaa aattgtgaaa aaagattcat catcatcaat gtatatatat attttatagt 900

tacatgcatt gactctgttc acatttgtta tcttgttctg caagaacaga cctgttctta 960

tcatgtcggt cttttccagt tctttgaatt gttatcaaag agtctttttc agcccatcac 1020

aatttataaa cgtcaataat tatgatttta ttagctaatg agtatttatt ttgtttttgg 1080

ttacagattt tatgccgccg aagttctagt ggcgttagaa tatctacaca tgttgggtat 1140

catctacaga gatctgaagc ctgaaaatat cttagttaga tccgacggtc acattatgct 1200

ctctgacttt gacctctctc tatgctccga ctcaatcgca gccgttgaat cttcctcgtc 1260

ttcgccggag aatcaacaac tccgttcacc gcgacgattc actcgtctcg ctagactttt 1320

ccaacgagtc ttgcggtcta aaaaggttca gactttagaa ccaacccgtc tctttgttgc 1380

tgaaccggtt actgcccggt ccggttcgtt cgttggtacg catgaatacg tggcaccaga 1440

agttgcttca ggtggatcac atggtaatgc cgttgactgg tgggcctttg gagtgtttct 1500

ctacgagatg atatatggca agactccgtt cgttgcgccg actaatgacg tcattctccg 1560

taacattgtg aaaagacagt tgagtttccc gactgattcg ccggcgacta tgtttgagct 1620

tcatgcgcgg aatttgattt ccgggttgct taacaaagat ccgactaaaa gacttgggtc 1680

acggcgaggt gcggcggagg ttaaagtgca tccttttttc aaaggtctaa actttgcgct 1740

cattcgtacg cttactccgc cggagattcc ttcttccgtc gtcaagaagc cgatgaaatc 1800

ggcgacgttt agtggtagaa gtagtaacaa accagcggcg ttcgattact tttgaacgtt 1860

ttctacggtc gagattaggg ccacgtgtta gtctgtttta gctgtacata ttccctcgaa 1920

gctgtccttt ttttcgttct ttaattttta aaatttcgtt aatctgactt ataaattaaa 1980

aatttaggtg acttttcata caatttgtac aacaaacgga ttaatctaa 2029

<210> 6
<211> 2649
<212> DNA
<213> Artificial Sequence

<220>
<221> source
<222> 1..2649
<223> /mol_type="unassigned DNA"
/note="Nucleotide sequence of pGreenII 0179 binary vector T-DNA
region"
/organism="Artificial Sequence"

<400> 6
agatcttggc aggatatatt gtggtgtaac gttatcgatc tggattttag tactggattt 60

tggttttagg aattagaaat tttattgata gaagtatttt acaaatacaa atacatacta 120

agggtttctt atatgctcaa cacatgagcg aaaccctata agaaccctaa tttcccttat 180

cgggaaacta ctcacacatt atttatggag aaaatagaga gagatagatt tgtagagaga 240

gactggtgat ttcagcgtac cgaattaatt ctcccgcgac cagccgagcg agcttagcga 300

actgtggacg agaactgtgc caccaagcgt aaggccgttc tctcgcattt gccttgctag 360

gctcgcgcga gttgctggct gaggcgttct cgaaatcagc tcttgttcgg tcggcatcta 420

ctctattcct ttgccctcgg acgagtgctg gggcgtcggt ttccactatc ggcgagtact 480

tctacacagc catcggtcca gacggccgcg cttctgcggg cgatttgtgt acgcccgaca 540

gtcccggctc cggatcggac gattgcgtcg catcgaccct gcgcccaagc tgcatcatcg 600

aaattgccgt caaccaagct ctgatagagt tggtcaagac caatgcggag catatacgcc 660

cggaggcgtg gcgatcctgc aagctccgga tgcctccgct cgaagtagcg cgtctgctgc 720

tccatacaag ccaaccacgg cctccagaag aagatgttgg cgacctcgta ttgggaatcc 780

ccgaacatcg cctcgctcca gtcaatgacc gctgttatgc ggccattgtc cgtcaggaca 840

ttgttggagc cgaaatccgc gtgcacgagg tgccggactt cggggcagtc ctcggcccaa 900

agcatcagct catcgagagc ctgcgcgacg gacgcactga cggtgtcgtc catcacagtt 960

tgccagtgat acacatgggg atcagcaatc gcgcatatga aatcacgcca tgtagtgtat 1020

tgaccgattc cttgcggtcc gaatgggccg aacccgctcg tctggctaag atcggccgca 1080

gcgatcgcat ccatagcctc cgcgaccggc tgaagaacag cgggcagttc ggtttcaggc 1140

aggtcttgca acgtgacacc ctgtgcacgg cgggagatgc aataggtcag gctctcgctg 1200

aactccccaa tgtcaagcac ttccggaatc gggagcgcgg ccgatgcaaa gtgccgataa 1260

acataacgat ctttgtagaa accatcggcg cagctattta cccgcaggac atatccacgc 1320

cctcctacat cgaagctgaa agcacgagat tcttcgccct ccgagagctg catcaggtcg 1380

gagacgctgt cgaacttttc gatcagaaac ttctcgacag acgtcgcggt gagttcaggc 1440

tttttcatag ggatcagctt gggctgtcct ctccaaatga aatgaacttc cttatataga 1500

ggaagggtct tgcgaaggat agtgggattg tgcgtcatcc cttacgtcag tggagatgtc 1560

acatcaatcc acttgctttg aagacgtggt tggaacgtct tctttttcca cgatgctcct 1620

cgtgggtggg ggtccatctt tgggaccact gtcggcagag gcatcttgaa tgatagcctt 1680

tcctttatcg caatgatggc atttgtagga gccaccttcc ttttctactg tcctttcgat 1740

gaagtgacag atagctgggc aatggaatcc gaggaggttt cccgaaatta ccctttgttg 1800

aaaagtctca atagcccttt ggtcttctga gactgtatct ttgacatttt tggagtaggg 1860

gtacgataac attaacgttt acaatttcgc gccattcgcc attcaggctg cgcaactgtt 1920

gggaagggcg atcggtgcgg gcctcttcgc tattacgcca gctggcgaaa gggggatgtg 1980

ctgcaaggcg attaagttgg gtaacgccag ggttttccca gtcacgacgt tgtaaaacga 2040

cggccagtga attgtaatac gactcactat agggcgaatt gggtaccggg ccccccctcg 2100

aggtcgacgg tatcgataag cttgatatcg aattcctgca gcccggggga tccactagtt 2160

ctagagcggc cgccaccgcg gtggagctcc agcttttgtt ccctttagtg agggttaatt 2220

ccgagcttgg cgtaatcatg gtcatagctg tttcctgtgt gaaattgtta tccgctcaca 2280

attccacaca acatacgagc cggaaghcat aaagtgtaaa gcctggggtg cctaatgagt 2340

gagctaactc acattaattg cgttgcgctc actgcccgct ttccagtcgg gaaacctgtc 2400

gtgccagctg cattaatgaa tcggccaacg cgcggggaga ggcggtttgc gtattgggcg 2460

ctcttccgct tcctcgctca ctgactcgct gcgctcggtc gttcggctgc ggcgagcggt 2520

atcagctcac tcaaaggcgg taatacggtt atccacagaa tcaggggata acgcaggaaa 2580

gaacatgaag gccttgacag gatatattgg cgggtaaact aagtcgctgt atgtgtttgt 2640

ttgagatct 2649

<210> 7
<211> 362
<212> PRT
<213> Arabidopsis thaliana

<220>
<223> Amino acid sequence of SPO11-1

<400> 7
Met Glu Gly Lys Phe Ala Ile Ser Glu Ser Thr Asn Leu Leu Gln Arg
1               5                   10                  15
Ile Lys Asp Phe Thr Gln Ser Val Val Val Asp Leu Ala Glu Gly Arg
            20                  25                  30
Ser Pro Lys Ile Ser Ile Asn Gln Phe Arg Asn Tyr Cys Met Asn Pro
        35                  40                  45
Glu Ala Asp Cys Leu Cys Ser Ser Asp Lys Pro Lys Gly Gln Glu Ile
    50                  55                  60
Phe Thr Leu Lys Lys Glu Pro Gln Thr Tyr Arg Ile Asp Met Leu Leu
65                  70                  75                  80
Arg Val Leu Leu Ile Val Gln Gln Leu Leu Gln Glu Asn Arg His Ala
                85                  90                  95
Ser Lys Arg Asp Ile Tyr Tyr Met His Pro Ser Ala Phe Lys Ala Gln
            100                 105                 110
Ser Ile Val Asp Arg Ala Ile Gly Asp Ile Cys Ile Leu Phe Gln Cys
        115                 120                 125
Ser Arg Tyr Asn Leu Asn Val Val Ser Val Gly Asn Gly Leu Val Met
    130                 135                 140
Gly Trp Leu Lys Phe Arg Glu Ala Gly Arg Lys Phe Asp Cys Leu Asn
145                 150                 155                 160
Ser Leu Asn Thr Ala Tyr Pro Val Pro Val Leu Val Glu Glu Val Glu
                165                 170                 175
Asp Ile Val Ser Leu Ala Glu Tyr Ile Leu Val Val Glu Lys Glu Thr
            180                 185                 190
Val Phe Gln Arg Leu Ala Asn Asp Met Phe Cys Lys Thr Asn Arg Cys
        195                 200                 205
Ile Val Ile Thr Gly Arg Gly Tyr Pro Asp Val Ser Thr Arg Arg Phe
    210                 215                 220
Leu Arg Leu Leu Met Glu Lys Leu His Leu Pro Val His Cys Leu Val
225                 230                 235                 240
Asp Cys Asp Pro Tyr Gly Phe Glu Ile Leu Ala Thr Tyr Arg Phe Gly
                245                 250                 255

Ser Met Gln Met Ala Tyr Asp Ile Glu Ser Leu Arg Ala Pro Asp Met
            260                 265                 270
Lys Trp Leu Gly Ala Phe Pro Ser Asp Ser Glu Val Tyr Ser Val Pro
        275                 280                 285
Lys Gln Cys Leu Leu Pro Leu Thr Glu Glu Asp Lys Lys Arg Thr Glu
    290                 295                 300
Ala Met Leu Leu Arg Cys Tyr Leu Lys Arg Glu Met Pro Gln Trp Arg
305                 310                 315                 320
Leu Glu Leu Glu Thr Met Leu Lys Arg Gly Val Lys Phe Glu Ile Glu
                325                 330                 335
Ala Leu Ser Val His Ser Leu Ser Phe Leu Ser Glu Val Tyr Ile Pro
            340                 345                 350
Ser Lys Ile Arg Arg Glu Val Ser Ser Pro
        355                 360

<210> 8
<211> 1640
<212> DNA
<213> Arabidopsis thaliana

<220>
<221> source
<222> 1..1640
<223> /mol_type="unassigned DNA"
      /note="Nucleotide sequence of SPO11-1"
      /organism="Arabidopsis thaliana"

<400> 8
gtctacaaaa attcgtcgac cattgcattt ttcagttttg aaactcgaaa gagatggagg      60

gaaaattcgc tatttcagaa tcgactaatt tgcttcaaag gatcaaagat tttactcaat     120

cggtcgttgt agatctggct gaaggaagat ctccaaaaat ttccatcaat caattcagga     180

actattgcat gaatccagaa gctgattgct tatgcagctc tgataaacca aagggtcagg     240

aaattttcac tcttaagaag gaaccacaaa cctacagaat cgatatgttg ctaagagtat     300

tgttgatagt ccaacaactt ctgcaagaaa atagacatgc ttcgaaaaga gatatctact     360

acatgcatcc atcagctttc aaagcacaat ccattgtgga ccgtgcaatt ggtgatatat     420

gcattctttt tcagtgtagt cggtacaact tgaatgtggt ttctgttgga aacgggttgg     480

tgatgggttg gttaaaattt agggaagctg gaaggaaatt tgattgttta aacagcttga     540

acactgcata tcccgttcct gttcttgtag aggaagtcga agatattgtt agcttagctg     600

agtacatact ggttgtggaa aaagaaacag tattccagcg tttagcaaat gacatgtttt     660

gcaagacgaa ccgctgcatt gttatcacag gaagaggata tccagatgtc tcaacaagaa     720

ggttcttgcg actcctgatg gagaagttgc atctacctgt gcattgtcta gtcgactgtg     780

atccatatgg ctttgagatc ctagccacat accgttttgg ctccatgcaa atggcctatg     840

atattgaatc attacgagca ccagatatga aatggttggg agcttttcct tcagactctg     900

aggtatatag tgttcccaaa cagtgtcttt tgccactgac agaagaagat aagaaaagaa     960

ctgaagcaat gttgcttaga tgctacctga agcgtgaaat gccacaatgg aggttggaac    1020

ttgaaacgat gttgaaaaga ggggtcaagt ttgagatcga agcactctca gttcactcac    1080

tgtccttttt gtcagaggta tatattcctt ctaagattcg tcgtgaagta agctctcctt 1140

gagcttcata cgagataact agtctccaac tcaaggtaat gctcattgtt ttttttatga 1200

ctctttctaa gctttttagt gcaaaccaaa tgcattggta gaatggacaa atgaacatag 1260

ttttaatgga aaaatcataa atatacagat tataaaaaat aattctcagt ttttaatttt 1320

taataataca aagtacattc actaatgact aatgagtaat aagtttgaaa ttttgaataa 1380

tacaaaagta atttagggtt catttgcttt atattgttac caaacctaat ttgaagtatt 1440

aaagccgttc ccctgactaa aaacctttga acactccgat ccaaaaaaaa gaagagaaaa 1500

acctttgcat acagctaaaa acgatgtcgt ctctctctcg ctttctcctt cgtggcaact 1560

tcagcttcag cacccacacg aatcgccgct tcttctccgc cgttaccgct gctgcagcca 1620

caccctcgcc acccaaacgt 1640

<210> 9
<211> 383
<212> PRT
<213> Arabidopsis thaliana

<220>
<223> Amino acid sequence of SPO11-2

<400> 9
Met Glu Glu Ser Ser Gly Leu Ser Ser Met Lys Phe Phe Ser Asp Gln
1               5                   10                  15
His Leu Ser Tyr Ala Asp Ile Leu Leu Pro His Glu Ala Arg Ala Arg
            20                  25                  30
Ile Glu Val Ser Val Leu Asn Leu Leu Arg Ile Leu Asn Ser Pro Asp
        35                  40                  45
Pro Ala Ile Ser Asp Leu Ser Leu Ile Asn Arg Lys Arg Ser Asn Ser
    50                  55                  60
Cys Ile Asn Lys Gly Ile Leu Thr Asp Val Ser Tyr Ile Phe Leu Ser
65                  70                  75                  80
Thr Ser Phe Thr Lys Ser Ser Leu Thr Asn Ala Lys Thr Ala Lys Ala
                85                  90                  95
Phe Val Arg Val Trp Lys Val Met Glu Ile Cys Phe Gln Ile Leu Leu
            100                 105                 110
Gln Glu Lys Arg Val Thr Gln Arg Glu Leu Phe Tyr Lys Leu Leu Cys
        115                 120                 125
Asp Ser Pro Asp Tyr Phe Ser Ser Gln Ile Glu Val Asn Arg Ser Val
    130                 135                 140
Gln Asp Val Val Ala Leu Leu Arg Cys Ser Arg Tyr Ser Leu Gly Ile
145                 150                 155                 160
Met Ala Ser Ser Arg Gly Leu Val Ala Gly Arg Leu Phe Leu Gln Glu
                165                 170                 175
Pro Gly Lys Glu Ala Val Asp Cys Ser Ala Cys Gly Ser Ser Gly Phe
            180                 185                 190
Ala Ile Thr Gly Asp Leu Asn Leu Leu Asp Asn Thr Ile Met Arg Thr
        195                 200                 205
Asp Ala Arg Tyr Ile Ile Ile Val Glu Lys His Ala Ile Phe His Arg
    210                 215                 220
Leu Val Glu Asp Arg Val Phe Asn His Ile Pro Cys Val Phe Ile Thr
225                 230                 235                 240
Ala Lys Gly Tyr Pro Asp Ile Ala Thr Arg Phe Phe Leu His Arg Met
                245                 250                 255

Ser Thr Thr Phe Pro Asp Leu Pro Ile Leu Val Leu Val Asp Trp Asn
            260                 265                 270
Pro Ala Gly Leu Ala Ile Leu Cys Thr Phe Lys Phe Gly Ser Ile Gly
        275                 280                 285
Met Gly Leu Glu Ala Tyr Arg Tyr Ala Cys Asn Val Lys Trp Ile Gly
    290                 295                 300
Leu Arg Gly Asp Asp Leu Asn Leu Ile Pro Glu Glu Ser Leu Val Pro
305                 310                 315                 320
Leu Lys Pro Lys Asp Ser Gln Ile Ala Lys Ser Leu Leu Ser Ser Lys
                325                 330                 335
Ile Leu Gln Glu Asn Tyr Ile Glu Glu Leu Ser Leu Met Val Gln Thr
            340                 345                 350
Gly Lys Arg Ala Glu Ile Glu Ala Leu Tyr Cys His Gly Tyr Asn Tyr
        355                 360                 365
Leu Gly Lys Tyr Ile Ala Thr Lys Ile Val Gln Gly Lys Tyr Ile
    370                 375                 380

<210> 10
<211> 1655
<212> DNA
<213> Arabidopsis thaliana

<220>
<221> source
<222> 1..1655
<223> /mol_type="unassigned DNA"
      /note="Nucleotide sequence of SPO11-2"
      /organism="Arabidopsis thaliana"

<400> 10
ggaatcggaa aaaaaatctc aaaatggagg aaagttcagg actatcatcg atgaagttct      60

tctccgatca acacctctct tacgctgaca ttcttcttcc tcacgaagct cgtgctagaa     120

tcgaagtttc tgttctcaat ctcctccgaa ttttgaactc cccagatcca gctatctccg     180

atctctctct gatcaatagg aagagaagca atagttgtat aaacaaaggg atactcacag     240

atgtttcata tatattcctc tctacttcgt ttactaagag ttcattgacg aatgctaaaa     300

cagctaaagc ttttgttcgt gtgtggaaag ttatggaaat atgctttcag attctgcttc     360

aagagaaacg agtcacacaa agagagctct tctataagtt gctttgtgat tcacctgatt     420

acttttcatc tcagattgaa gttaacagaa gtgtccaaga tgtggtagca cttctacgtt     480

gtagtagata cagtcttggt attatggctt caagcagagg ccttgttgct ggaaggttat     540

ttctacaaga accaggtaag gaagctgtag attgttcggc ctgtggttct tcaggttttg     600

ctataactgg agacttgaat ttgctagaca ataccatcat gagaactgat gctcgttata     660

tcattattgt ggaaaagcat gcgatctttc atcggctcgt ggaagatcgt gtgttcaatc     720

acattccttg cgtgttcatc acagcgaaag ggtatccgga tattgccaca aggtttttcc     780

tccaccggat gagcacaact tttcctgatc tgccaattct tgttctagtt gattggaatc     840

cagctgggtt agctatacta tgcaccttca agtttggaag catagggatg ggacttgaag     900

catacagata cgcttgcaat gtgaagtgga ttggtctccg aggagatgat ctgaatctga     960

taccagaaga gtctttggtt cccttaaagc caaaagattc acagattgct aagagcttat    1020

tgtcctccaa aatattgcag gaaaactaca tagaggagtt gtcactgatg gttcaaactg 1080

gtaaaagagc ggaaattgaa gctctctatt gtcatggtta taattatctc ggtaaatata 1140

tagctaccaa gatcgtgcaa ggcaaataca tataaactta aaacaccaac atgtaaggtc 1200

ttccaccaat aaagatgcgt ttgctttata ttggttccaa atataagaga gtaacttatt 1260

agggtgaagt tttatgcaaa ctctaataga atgtaaaaat agattacaat caaatcaaaa 1320

ttttccaaac tgaagtatga tctttgttta catatggcat tgtgatgatt tagtaacaaa 1380

acttgtccta atctccactt tcctatgcag gtataatgat aattccaacc aaaaattctc 1440

gctcacttct tgcttgatac aagactaatc ttctttccct ccacaacaaa cttgccactt 1500

cgctccagct gcaacaagga cgttagaaca atatcagtaa gaatgacaaa ctttggaact 1560

ttgtgtagaa tatgagaatc ccactttaaa atgattatgt ttactaactt cactatcaca 1620

cttctttaat ctcaaatata atgaatttca tacgc 1655

<210> 11
<211> 8
<212> PRT
<213> Artificial Sequence

<220>
<223> Linker

<400> 11
Leu Glu Pro Thr Gly Glu Lys Pro
1               5

<210> 12
<211> 16
<212> PRT
<213> Artificial Sequence

<220>
<223> Linker

<400> 12
Ile Asp Leu Glu Ala Gln Ala Ala Leu Glu Pro Thr Gly Glu Lys Pro
1               5                   10                  15

<210> 13
<211> 4201
<212> DNA
<213> Artificial Sequence

<220>
<221> source
<222> 1..4201
<223> /mol_type="unassigned DNA"
/note="Nucleotide sequence of AtSPO11-1 genomic sequence (PS lab
clone 49DI"
/organism="Artificial Sequence"

<400> 13
aatgtcgtcg gcggacaaca cgaagacatt ctctttgata gcttcaccaa acttgactaa 60
gtatgagacc tctctgttct taattcctta agtatttccg ttaattagtt gttccttttt 120
taatactaga gaaaataaaa ttacaaaatg taatattttc acgaggaata aaatcttgct 180
taagtttatg cttctatatt acttatttta atcttatgtt tctgtttagg cgcgaggctt 240
cttttcttta ggtatctctc attagtttat acgttacaag attacacatt gcattgatat 300
atgattttac tttcaaaaaa aatttaaatg ttgaacgacc ttaaaatccg tgcgctttac 360
ttttaaatct ttccgaatga aacccaacca ccgacgcgac agcttttcat ttgtttacta 420
attaaacgga acaaagcaat ttaaatgtta tcagtgttgt gttttagtta gaaatactat 480
aaataataca agatgcctct tttgttttag aacttttttg gtatcatgaa ttcgttatcg 540
gataaaatac gttaaagcta agtagtatat tcttagatta tatataaatt aggtgagcaa 600
tctatcatag agaagaaatg attaaaataa gtgtatggca taatagattc gaacattttg 660
gtatgttcct cacgtgtccg agcataaagc ttatgaacaa atctcgacca ctagaagtct 720
agaactaatt ctaatctagt ttaccataca cattaattat ttcaaattta gtgcacatga 780
ttattttata ttggcatcac taaaatgtct ccaagtctga ataaatcgat ttatattggt 840
ttgattgtta tcatgaatct ttttcacaat cactttgcct agtttcggat cgggcctaaa 900
agccaacggc ccaacgtgtt taaaaacaca tgcgttttta aaatgacacg tcagttacgt 960
atcgggccta aatgccaatg gcccaacgtc atggaaagta ggaactaaac gacacgcatg 1020
tttaaattcg aaggttttaa cggcaagctt ttcgcaccca tccctttctt tcccgtctac 1080
aaaaattcgt cgaccattgc atttttcagt tttgaaactc gaaagagatg gagggaaaat 1140
tcgctatttc agaatcgact aatttgcttc aaaggatcaa aggtatcaga aaatcccaaa 1200
attttccgaa tcgtattgag taatttgtcc tctaatctat gtcgaaattt ctagtcgatg 1260
acttcgctgg ctcttccgtt gttcatcact atgtttcggt tttttgagca gattttactc 1320
aatcggtcgt tgtagatctg gctgaaggaa gatctccaaa aatttccatc aatcaattca 1380
ggaactattg catgaatcca gaagctgatt ggtttgtctc tcttcgatct cgattattca 1440
attttgtttt cactttcttt gattgaagaa tcagacgtgt ttggtctttg gttcgagaca 1500
tttttgaagg atttaagcac aacgtcaacg agtattgaaa actaagacct catgaattta 1560
ccattttatt ctgctgtcta tagcttatgc agctctgata aaccaaaggg tcaggaaatt 1620
ttcactctta agaaggaacc acaaacctac agaatcggtg agtcaggttt cagataattc 1680
tacaagtaca ttagtgttat cttaatatag catcacattg cggtgttttt actttcagat 1740
atgttgctaa gagtattgtt gatagtccaa caacttctgc aagaaaatag acatgcttcg 1800
aaaagagata tctactacat gcatccatca gctttcaaag gtgaatttgg aagcaacgta 1860

taatgtttta gttatgtttt tcttctaaac gaatacgtat tgagattttt atacggatat 1920

tagttatgca tccctctttg ttgtgttctt ggccttctag taacactgtt ttcgtctact 1980

tcacagcaca atccattgtg gaccgtgcaa ttggtgatat atgcattctt tttcagtgta 2040

gtcggtacaa cttgaatgtg gttagtactt taatactttc accttcaatt ttccttctga 2100

agagttatta taagctgcga ttgaagatgt gattactttt tcattcatgt gatattttgc 2160

aggtttctgt tggaaacggg tgggtgctct tgataacgag tgcaatgctt ctatcttgag 2220

gcatgtcctt attttaagat ccacctcatt attcttttgt gttgtaggtt ggtgatgggt 2280

tggttaaaat ttagggaagc tggaaggaaa tttgattgtt taaacagctt gaacactgta 2340

agaatttgaa cataagattc actttttata ttggcatcag gaagtataac tctgcaagtg 2400

atttcatttc aggcatatcc cgttcctgtt cttgtagagg aagtcgaagg tacatatatt 2460

tgcaatcatt ttaagtgatg ctttatgctc tttagagaag tcaaatcctt atctcagctg 2520

atatcaggaa gtataagatt cactatctat ggtggcatca ggaactataa ttggatgtca 2580

ttttcctgct tgcagatatt gttagcttag ctgagtacat actggttgtg gaaaaagaaa 2640

caggtaagaa agacctctta tttatgtagt aactaggaag atggttttct ttcttcatga 2700

ctaaatcgct ttcatccatg gtttatagta ttccagcgtt tagcaaatga catgttttgc 2760

aagacgaacc gctgcattgt tatcacagta agaaatctgc ttcattctta ctgatttcag 2820

ttttgactag ttttcatttc cattttatgt ttgacatcaa agttacgatt gtttttggca 2880

gggaagagga tatccagatg tctcaacaag aaggtacata acttaatccc aggttcctgg 2940

aatactattt tatcagacat tatcttctat tgattaatat atcagcgaat aacacagatt 3000

ctttccaggt tcttgcgact cctgatggag aagttgcatc tacctgtgca ttgtctagtc 3060

gactgtgatc catatggctt tgagatccta gccacatacc gttttggctc catggttaga 3120

ttctgttaca aaatatagat gactgaactc ggaaaatttc accgcttgca ttgtttttgt 3180

tgatagtgat aatgtgatat ggcactgacc catttcattt tgaaccattt tccttatgtc 3240

acttgttttc tttctgtaag caaatggcct atgatattga atcattacga gcaccagata 3300

tgaaatggtt gggagctttt ccttcagact ctgaggtata tagtgttccc aaacagtgtc 3360

ttttgccact gacagaagaa ggtaagagat cattaaacac cttatcgcct taaatcttta 3420

gattttccta tttttcactg cttccttttt tgagattgta acaacttccc cggacagata 3480

agaaaagaac tgaagcaatg ttgcttagat gctacctgaa gcgtgaaatg ccacaatgga 3540

ggtatgaaaa acttccctga cttttttata tttacatttc aaccaaatga caaaaactag 3600

tcttgatcaa aacgattttg gtcgtctaag gttggaactt gaaacgatgt tgaaaagagg 3660

ggtcaagttt gagatcgaag cactctcagt tcactcactg tcctttttgt cagaggtata 3720

tattccttct aagattcgtc gtgaagtaag ctctcctccc gggtgaagat ctgcttcata 3780

cgagataact agtctccaac tcaaggtaat gctcattgtt ttttttatga ctctttctaa 3840

gctttttagt gcaaaccaaa tgcattggta gaatggacaa atgaacatag ttttaatgga 3900

aaaatcataa atatacagat tataaaaaat aattctcagt ttttaatttt taataataca 3960

aagtacattc actaatgact aatgagtaat aagtttgaaa ttttgaataa tacaaaagta 4020

atttagggtt catttgcttt atattgttac caaacctaat ttgaagtatt aaagccgttc 4080

ccctgactaa aaacctttga acactccgat ccaaaaaaaa gaagagaaaa acctttgcat 4140

acagctaaaa acgatgtcgt ctctctctcg ctttctcctt cgtggcaact tcagcttcag 4200

c 4201

<210> 14
<211> 9446
<212> DNA
<213> Artificial Sequence

<220>
<221> source
<222> 1..9446
<223> /mol_type="unassigned DNA"
      /note="Nucleotide sequence of AtSPO11-2 genomic sequence (PS lab
      clone 4BE)"
      /organism="Artificial Sequence"

<400> 14
   agaaaaggtt tatccgacca cttgtgtttc tgtgcatcct ccaaacccca aaatgtaata 60
   ccatttttca cgtaatcatt atgaactaag gttaatcatt tcattttaaa gtagatagta 120
   tatatgcgga ataatatgtg taaaacagat ttgatgtatt aaatcgttca tattttgcga 180
   ggacataatc ttggtcagat actcagatca aacatgctat tgctttaatc aaacaaaggg 240
   ctttggcaac ccataaaaaa aaagcttttg agatccataa tatgacccca aacattttct 300
   aaattcaaaa ctggtccagt aaatgcctaa atgtgatcgt gttttttgtt cttttttaaa 360
   tagaagaagg ttcggggtca aatctcgtaa tccactcaaa cccgaaatca caacatttat 420
   tattagtttt gttccaagca tcattcaaac caacaacttc taacctttgc ttaaaacctt 480
   tttaccagag ctaactacac ttggtttaaa tgtgatcgtt cacctaaaaa aaaaacaaat 540
   aatccacgag agagggacaa ttagacgaat caaattaaag caaacataga tcaatgtctc 600
   aataaaccta aagcatgtat gttatgagat ttattctata catttgtctc tttggagatt 660
   gagggtcaat ggagagatta aagcataatt aatgtattac accactaatt aacattcaat 720
   aattacagat cagaaactct tgagaggaac tgaagttgcc taaggagagg gctaagagtc 780
   tccatgagct tctcacaagg atgattatga acaccttcgt aggttgttac gacaacgttt 840
   ggatcttttg ccagtctttg cacttgtttc ttcacgttgc atgtgtggta tgtacatcta 900
   taatagctcc tgcaaatatt attttgtata aaaatgtgaa tagtaaatta atccatatag 960
   gtgatgtatg tgtgttatcg atgttaagtt ttttggtata gatcttatgg aacaaagttc 1020
   atattaatca cgacttcacg aggtataatt ggtaataatt tcagataata gagattgtaa 1080
   tttaaggtat tgattaagta agtacatgca ttttgaaaat ttacaactaa ttttttaagt 1140
   aaaatttggg tttattagtt tggttctacg aaaacatcat ccaatgtaaa ctatatatac 1200
   atatgggctg gacgaacagc aacaaccaaa ccatattcgt ttcattatca cctatggatt 1260
   catcgatata tgatttattc gactaaaacc aaaccaaaat atgttgtcat agcaacaaca 1320
   gtcaagcgag caagatttca tctgttatcg tttcctagca acaacgaaat aaaaggccca 1380
   aattgttctt tttaggttgt aaatgtgata tattgatcaa aatttggggg tacatcaatg 1440
   ttacttaaaa acacaatatt tttggttata tataccaatt atattttgga ttatataagt 1500
   cagggattgg tttggataaa ccacaaaatg aacaccccca attccaagaa acaagcaata 1560
   atgtaatgca accggactaa taacctaaat tgatgacttt ggtcgtttag gttgttaaac 1620
   taatgtttcc aaaatataca cgtgaccaag gatgtgtgtg tatgttatag atatgcttag 1680
   gtcaatttcg atatctactt taacgttgtc atatatattt ttaaacacac tagtaaatgt 1740
   atgggacgca ataaaataaa attaaaagaa cctgggatga gcattgttct tgacagattt 1800
   ctgaccgtac tttcgccaac gataaccatc atcaagaaca tcatcatcac tccttgtatg 1860
   aaaagctatc ctctgcattg ccaaagttct cttccctttt cctttgttgc aattcttatt 1920
   atgatcacta ttattgctat tattactcac taatcctccc atctctcctc cacctcctcg 1980
   aaagttctcc agctgagcag atagatgata acctgctgga gtacaagaag aagaagatgg 2040
   acctacatcg cccgaaaaga ttaacgaagg agccatcatc attaatgttt tgtcatctaa 2100
   ggaagaaaaa ggattgttgt tttcgccttc ttctagggtt aacataggat ttgtgttgtc 2160
   aaccccttcc attgattatg aagctttcac tatcttgcta gatctctctc tctctctctt 2220
   actatatata tgtgtgtgtt tatgtaaata tatgtttgca taagaggaag aaggagaaga 2280
   ggagaagaaa ggttataagg atatttgtag gaaagaaaga agaagaatga gttaaagggt 2340
   ataatattga agccaactat attcacctaa cttttctgtt caactgccta agtctttcct 2400
   ccttttccta gctatatatc tctttacaac gtttttggat ctatatatta tatagaatca 2460
   tatattaata tatatccaaa aatgcatgta catgtcaaat gtacttcaac ccatattttg 2520
   aaatctaaat agtaaatatt aatctttaaa aaaacaaaaa ttgacatcga tggtatatat 2580
   attgagtggt cttatacatt ttgagaatag tttgtagaat aaattgatta gcttatttaa 2640
   atttatatat ttcggcacca attcgtaata tgtattgatg tttttagaat atgtaaattg 2700
   taacgattgt tttaacttca tgtggaaaaa ctactcacat gtttcatata aaccgatcaa 2760
   aatctagtta tatttatggt caaagttctt gttgatgttt tcccatgaca aagggaatgt 2820
   tcggtaaagg cttaacttgc ttataaattt acatgtaaca agtagtaatt aatttgcttt 2880
   tatttgcatt tcaaacgtgt ggttaaatat agtgtaccaa ctccatacac acactcgtga 2940
   gcctgaaatg cacgtctggt cggtttgaga actagctaaa atttagagat tgttttagat 3000
   cactaaattt atatcatcct tgtcgttgtt tatagatatt tatatgcaca ataatggatt 3060
   aataatattc acattttgtc aaaaaaaaat ataaaaaaaa ataatttata taacgaatta 3120
   atacgataag attttttttt ctcgatacca taagaattct gcgtatttgt tccattgtcc 3180
   aatgatattt ccaccacgta atatggacta tatatagtgg ttaatttgaa catatcattg 3240
   atcttttaac tctaagtatg agacatttat atgattaaag tgggatattt ttatgaattt 3300
   cgcatccccc ttcatttcgt tttcgcaaag tttcattttg aaatttcgtt cgtccaacca 3360
   tacatcccct actaagttat actaccgata atgaaactta tagaaatcat gaagtcgcgt 3420
   aaaatgttgg gcattaaata cggataaatg gacttaagca aattattgtg gttgtggttg 3480
   ttaagaatct ctttgaccgc tcgaataatt atacatccaa atgtaattaa cctatgcaaa 3540
   ctcatgcatg cacgtggaag cttctcattc tcataaacca taaaattatg tacaaatgta 3600
   aatcaaatgt tagtggatcc attttcttaa aatatatttt ttcttttata atttttattt 3660
   agttgatgtt ttaagagaat acacacttaa taaattattt aatttttgtt tactttaaac 3720
   taaaatatca tttattattt aataaaccaa tagaaaaatt atctgtggaa taaaaatcat 3780
   caaaaccaat gaaaatataa taaattttgc attaaaattt taaaatatca tatatttaaa 3840
   aacaaaaata aaagcttaga acatcactta taaaaaacaa agagagtata taaaattgaa 3900
   ctttccgatt caacttattg tgtaaattaa atatctacaa aaatcaattg attaccacat 3960
   tgaaatatag ttattgatag cttaaaacta tacaaccgca aaacaattac aattttggct 4020
   tgtagttagg ctaaattttt ggcttatata taaaattaaa aaataaaata aaatatatga 4080
   cacaattagt tccacatgag aaaaaaaatc atgaactgca tgccggtttc ttataaacaa 4140
   aacggaagac agtgcaaatc tcaaatcccg aaacttgacg cgcgcctctc tgtctctctc 4200
   aatcaaattc aaatcaaaat ttcaaaaaag cggaatcgga aaaaaaatct caaaatggag 4260
   gaaagttcag gactatcatc gatgaagttc ttctccgatc aacacctctc ttacgctgac 4320
   attcttcttc ctcacgaagt aagtttcgct tccaattttg aatttatcga atcactagtc 4380
   ttcttcactg tcgaatctcg tagttctgac tgaaatcgaa gctcgttgtt acaggctcgt 4440
   gctagaatcg aagtttctgt tctcaatctc ctccgaattt tgaactcccc agatccagct 4500
   atctccgatc tctctctggt actacttaat ctcgtgctta agtccgtttc ttagatcgcg 4560
   ttatagcttt ctcgaattcg tgattttctg ggtttttgaa gacaattgaa atgtatcgtt 4620
   tgaacagatc aataggaaga gaagcaatag ttgtataaac aaagggatac tcacagatgt 4680
   ttcatatata ttcctctcta cttcgtttac taagagttca ttgacgaatg ctaaaacagc 4740
   taaagctttt gttcgtggta attgctcaat gtctctctct gtgatcttca cccaaatctt 4800
   tgattagaat ggtatgagaa aatggttgtg attttattga aacagtgtgg aaagttatgg 4860
   aaatatgctt tcagattctg cttcaagaga aacgagtcac acaaagagag ctcttctata 4920
   agttgctttg tgattcacct gattactttt catctcagat tgaagttaac agaagtgtcc 4980
   aaggtcaatc taaagcttaa ttgcatttgg attaaagagt ttttgagctg attttttggt 5040
   cgtgctttgg tttagcagat gtggtagcac ttctacgttg tagtagatac agtcttggta 5100
   ttatggcttc aagcagaggc cttgttgctg gaaggttatt tctacaagtg agttattact 5160
   tgcttctagc aatccttatg tagctaacat tgtgatctct gaccatttct ttactttaca 5220
   ctcaggaacc aggtaaggaa gctgtagatt gttcggcctg tggttcttca ggttttgcta 5280
   taactggaga cttgaatttg ctagacaata ccatcatgag aactgatgct cgttatatca 5340
   ttattgtgga aaaggtagct tcttgatgta atcaagcaca tatgagagat gtcgtaattg 5400
   tctgtaactc actcctagaa tctgttttta gcatgcgatc tttcatcggc tcgtggaaga 5460
   tcgtgtgttc aatcacattc cttgcgtgtt catcacagcg aaagggtatc cggatattgc 5520
   cacaaggtca attcttcaac tcagttacaa aaccatattg caatgtagtc agtggtatta 5580
   agtagcttgg aagttaaatc ttttaggttt ttcctccacc ggatgagcac aacttttcct 5640
   gatctgccaa ttcttgttct agttgattgg taagcataaa taaacatctt cctctttgtt 5700
   aacataaaac tttagtgctc atatgttcat aaatgagagg tcaggaatcc agctgggtta 5760
   gctatactat gcaccttcaa gtttggaagc atagggatgg gacttgaagc atacagatac 5820
   ggtaaagact tagctgtttg caccaaataa tctccaagtc tctacaatga taatttctgc 5880
   atcctcttta gcttgcaatg tgaagtggat tggtctccga ggagatgatc tgaatctgat 5940
   accagaagag tctttggttc ccttaaagcc aaaagattca cagattgcta agagcttatt 6000
   gtcctccaaa atattgcagg taaccaacta tacattatac attataagca gagatcagat 6060
   gtaatggtca agccgaagca attctaagat tcgaatctat aatctatcat ttatacccta 6120
   attgattttg acatttgctt ctgaccagga aaactacata gaggagttgt cactgatggt 6180
   tcaaactggt aaaagagcgg aaattgaagc tctctattgt catggttata attatctcgg 6240
   taaatatata gctaccaaga tcgtgcaagg caaatacata cccgggtaac acttaaaaca 6300
   ccaacatgta aggtcttcca ccaataaaga tgcgtttgct ttatattggt tccaaatata 6360
   agagagtaac ttattagggt gaagttttat gcaaactcta atagaatgta aaaatagatt 6420
   acaatcaaat caaaattttc caaactgaag tatgatcttt gtttacatat ggcattgtga 6480
   tgatttagta acaaaacttg tcctaatctc cactttccta tgcaggtata atgataattc 6540
   caaccaaaaa ttctcgctca cttcttgctt gatacaagac taatcttctt tccctccaca 6600
   acaaacttgc cacttcgctc cagctgcaac aaggacgtta gaacaatatc agtaagaatg 6660
   acaaactttg gaactttgtg tagaatatga gaatcccact ttaaaatgat tatgtttact 6720
   aacttcacta tcacacttct ttaatctcaa atataatgaa tttcatacgc ttaatgtaga 6780
   ttctatacga agatttggca taaagattag ctaagattgc attaccttaa gtttcaagta 6840
   agcaatagca tcttttcttg aagaaaactc tgaaagaact gatggtgctt gttcatcgat 6900
   cagagatttt agctgtttca gcttcatgaa tccaccttta ccagcatcct aagaatgttt 6960
   ttgcacatat acatataagt cgatatgagt ggtgggatgt tttagaagaa tacaatgaca 7020
   gttcaaaata aattgacaac gaaccttttt aacaatctgc ttgcacaggt tctttaactt 7080
   gattttgggt tcaatgattt catcgtgctt ccttttcgcg ggaagagaac tttcaattac 7140
   agagtccttg tcatcatcat ctgcttcact ctcgtcttca tcttcctctt cgtcttcttc 7200
   gtcatcatca tcatcatcat catcatcatc atcactgtta tcaaaagagg ttttcttccc 7260
   ttcgtaacga acaccagcta ttttctttgg ccggtctttg atacccagtc cttgttttcc 7320
   cgctgtggct ttatcctaca gacattccaa aacgtcaata tattattaga tcccaacctc 7380
   ttttccaaga acagcctgca aagaacatac ctgaaccatg ttgtaaagat tctcttgatc 7440
   attctcacta aacatttttc tctcgctagc ttttaatttc tttttgcccg acttggcccc 7500
   cagaagacca cccgagacaa acccggattt aaatccccac cagttcgaag aaggttcttc 7560
   aattttctgt tctgttcagt ccaaaagatc catacatttt cagttcaaaa aaaagagcag 7620
   aaagtcacaa tttcaagatg caaggacaaa ctaatcacct ttaatactag catcttggtc 7680
   ctcagatata atttcaatat ccatagaatc ggctatgtca caaactgcag gagaaggatc 7740
   ctcagttcgc ttaacctaca aaacagtaaa ataacgtgcc taagtcggac tatcactttc 7800
   ataatgagat ttgtttacat aatggtgata gcagagaaaa ttctaacaac atgcacaagt 7860
   tcacagtaca tgtagaagtt atgatccatt ctaatcaaca tgaatcttga tcatgagcgg 7920
   ataaaagaca ggacagaact gttttgaaag gaagcttacc agtattcctt caagatcttt 7980
   agacgagtat gaattgacaa gcttcccttt ctctctacgt ttataccttc cttgtggacg 8040
   agtgacttta gcaacggtct tcaacttggc aggctcagat ttgacagcat catcttcact 8100
   ttcatcttcc ttatctgaat ctatcgtatg ccacataaaa caacaatcat taggataaat 8160
   aagcttctct aagtcaagaa agaactcaac ttaataagaa aaacttatac catcatcatt 8220
   cttgctagtc ttggtaggag cagcttgctg caaataacac acacacatac acaaaaagat 8280
   tgaaattatt tcaaatattt caaagagcat agttaaaacg aaagatgaac acagccatta 8340
   ccactttcaa tttcttgaga atgttatcga actgagttgt atcaaatgcc catggatttg 8400
   gcttatcaag accaacacct gctccaacat tttaaaaaaa ttcagaccta taacataaac 8460
   gatagatgca tatacaccac acaaatatac aaaaggctta aagtagctct atcttgattc 8520
   cacaacttca cattcaccaa attacataaa ctaaactatg taaacagaca tttcctccta 8580
   aaaaccgaaa ataatgttgt tgttttttag tgtaagtgaa aagtagctct attaaagttt 8640
   cctccaatct aaattccacc gagaaaaatt caaatcacta gtttccatta aggagaagaa 8700
   gaagaagaaa ctaaccagat gtatcttgtt tgttcgtaac tctcacataa cctttgatac 8760
   cttgcttatc tttaccaagt ccttctcctt cttcccaacc ctaaaaaaat caccaatcca 8820
   aaaaattagg tcttctcctt tataaaatct ctacacacat taacatcatt acaggaaaat 8880
   tcactcacca tcgatttcat aagcttgaaa gcggcggaat ccttacatat tccgacgtac 8940
   ttaaccggag cttccggcgc cgccatcttc tcttctaatt ttccggcgat tgaatggaga 9000
   caatgcgtcg ccgtttttca aaagcggtgc ggaagagaag agtgggcgag gaagtttaga 9060
   gaaactggaa cagaaaacct aaagcgaaga agtgatgagt aaaactcctc tacacgtggt 9120
   ctttagtaaa cttcagtgga tatgtaacat gatcgacggt cgagagttta tcatatttta 9180
   aatgggcttt aggaaggaaa ggcccaataa agaaatgaat atttccacca cagaattatg 9240
   aattgagtaa accagaaaat cgaagaagaa cggcaatggc tacttcttct actcagcttc 9300
   tactgtcttc ttcttctttg tttcactctc aaattaccaa aaagccattc cttctcccgg 9360
   cgacgaagat cggcgtttgg agaccgaaga agtctctctc gttatcatgt cgtccttcag 9420
   cctcggtttc agctgcttct tccgcc 9446

<210> 15
<211> 4786
<212> DNA
<213> Artificial Sequence

<220>
<221> source
<222> 1..4786
<223> /mol_type="unassigned DNA"
      /note="Fragment of Plasmid pCB302/AtSPO11-1-ZF4"
      /organism="Artificial Sequence"

<400> 15
ggatccgctg aagctgaagt tgccacgaag gagaaagcga gagagagacg acatcgtttt 60

tagctgtatg caaaggtttt tctcttcttt tttttggatc ggagtgttca aaggttttta 120

gtcaggggaa cggctttaat acttcaaatt aggtttggta acaatataaa gcaaatgaac 180

cctaaattac ttttgtatta ttcaaaattt caaacttatt actcattagt cattagtgaa 240

tgtactttgt attattaaaa attaaaaact gagaattatt ttttataatc tgtatattta 300

tgatttttcc attaaaacta tgttcatttg tccattctac caatgcattt ggtttgcact 360

aaaaagctta gaaagagtca taaaaaaaac aatgagcatt accttgagtt ggagactagt 420

tatctcgtat gaagcagatc ttcacccggg cttttctccg gaaccgccac ccagctcgcc 480

ggtatgggtg cgttgatgcc gaacgagtgc tcccggatcg gagaaggact tgccacactc 540
agggcatgcg tacggtttct cgccggtatg ggtcctctgg tggcgttcaa gatgggcacg 600
ctgactaaag cttttaccgc actcgggaca ggcgtatggt ttctcgccgg tatgggtgcg 660
ctgatgccga accaagttac cgggatcgga gaaggatttt ccgcattccg ggcatgcgta 720
gggcttctcg ccggtatgcg tacgctggtg acgcacgagt tcaccgctgg tcgaaaagga 780
tttcccgcat tcggggcacg catacggttt ctcgccggta tgggtgcgtt gatgccgaac 840
gagtgctccc ggatcggaga aggacttgcc acactcaggg catgcgtacg gtttctcgcc 900
ggtatgggtc ctctgatgcc tcacgaggtt gctggactga gagaagcttt ttccgcattc 960
agggcaagcg tacggtttct cgccggtggg ttcgaggccc gcctgggcct cgagatcgat 1020
gggaggagag cttacttcac gacgaatctt agaaggaata tatacctctg acaaaaagga 1080
cagtgagtga actgagagtg cttcgatctc aaacttgacc cctcttttca acatcgtttc 1140
aagttccaac cttagacgac caaaatcgtt ttgatcaaga ctagtttttg tcatttggtt 1200
gaaatgtaaa tataaaaaag tcagggaagt ttttcatacc tccattgtgg catttcacgc 1260
ttcaggtagc atctaagcaa cattgcttca gttcttttct tatctgtccg gggaagttgt 1320
tacaatctca aaaaaggaag cagtgaaaaa taggaaaatc taaagattta aggcgataag 1380
gtgtttaatg atctcttacc ttcttctgtc agtggcaaaa gacactgttt gggaacacta 1440
tatacctcag agtctgaagg aaaagctccc aaccatttca tatctggtgc tcgtaatgat 1500
tcaatatcat aggccatttg cttacagaaa gaaaacaagt gacataagga aaatggttca 1560
aaatgaaatg ggtcagtgcc atatcacatt atcactatca acaaaaacaa tgcaagcggt 1620
gaaattttcc gagttcagtc atctatattt tgtaacagaa tctaaccatg gagccaaaac 1680
ggtatgtggc taggatctca aagccatatg gatcacagtc gactagacaa tgcacaggta 1740
gatgcaactt ctccatcagg agtcgcaaga acctggaaag aatctgtgtt attcgctgat 1800
atattaatca atagaagata atgtctgata aaatagtatt ccaggaacct gggattaagt 1860
tatgtacctt cttgttgaga catctggata tcctcttccc tgccaaaaac aatcgtaact 1920
ttgatgtcaa acataaaatg gaaatgaaaa ctagtcaaaa ctgaaatcag taagaatgaa 1980
gcagatttct tactgtgata acaatgcagc ggttcgtctt gcaaaacatg tcatttgcta 2040
aacgctggaa tactataaac catggatgaa agcgatttag tcatgaagaa agaaaaccat 2100
cttcctagtt actacataaa taagaggtct ttcttacctg tttctttttc cacaaccagt 2160
atgtactcag ctaagctaac aatatctgca agcaggaaaa tgacatccaa ttatagttcc 2220
tgatgccacc atagatagtg aatcttatac ttcctgatat cagctgagat aaggatttga 2280
cttctctaaa gagcataaag catcacttaa aatgattgca aatatatgta ccttcgactt 2340
cctctacaag aacaggaacg ggatatgcct gaaatgaaat cacttgcaga gttatacttc 2400

ctgatgccaa tataaaaagt gaatcttatg ttcaaattct tacagtgttc aagctgttta 2460

aacaatcaaa tttccttcca gcttccctaa attttaacca acccatcacc aacctacaac 2520

acaaaagaat aatgaggtgg atcttaaaat aaggacatgc ctcaagatag aagcattgca 2580

ctcgttatca agagcaccca cccgtttcca acagaaacct gcaaaatatc acatgaatga 2640

aaaagtaatc acatcttcaa tcgcagctta taataactct tcagaaggaa aattgaaggt 2700

gaaagtatta aagtactaac cacattcaag ttgtaccgac tacactgaaa aagaatgcat 2760

atatcaccaa ttgcacggtc cacaatggat tgtgctgtga agtagacgaa aacagtgtta 2820

ctagaaggcc aagaacacaa caaagaggga tgcataacta atatccgtat aaaaatctca 2880

atacgtattc gtttagaaga aaaacataac taaaacatta tacgttgctt ccaaattcac 2940

ctttgaaagc tgatggatgc atgtagtaga tatctctttt cgaagcatgt ctattttctt 3000

gcagaagttg ttggactatc aacaatactc ttagcaacat atctgaaagt aaaaacaccg 3060

caatgtgatg ctatattaag ataacactaa tgtacttgta gaattatctg aaacctgact 3120

caccgattct gtaggtttgt ggttccttct taagagtgaa aatttcctga ccctttggtt 3180

tatcagagct gcataagcta tagacagcag aataaaatgg taaattcatg aggtcttagt 3240

tttcaatact cgttgacgtt gtgcttaaat ccttcaaaaa tgtctcgaac caaagaccaa 3300

acacgtctga ttcttcaatc aaagaaagtg aaaacaaaat tgaataatcg agatcgaaga 3360

gagacaaacc aatcagcttc tggattcatg caatagttcc tgaattgatt gatggaaatt 3420

tttggagatc ttccttcagc cagatctaca acgaccgatt gagtaaaatc tgctcaaaaa 3480

accgaaacat agtgatgaac aacggaagag ccagcgaagt catcgactag aaatttcgac 3540

atagattaga ggacaaatta ctcaatacga ttcggaaaat tttgggattt tctgatacct 3600

ttgatccttt gaagcaaatt agtcgattct gaaatagcga attttccctc catctctttc 3660

gagtttcaaa actgaaaaat gcaatggtcg acgaattttt gtagacggga agaaaaggga 3720

tgggtgcgaa aagcttgccg ttaaaacctt cgaatttaaa catgcgtgtc gtttagttcc 3780

tactttccat gacgttgggc cattggcatt taggcccgat acgtaactga cgtgtcattt 3840

taaaaacgca tgtgttttta aacacgttgg gccgttggct tttaggcccg atccgaaact 3900

aggcaaagtg attgtgaaaa agattcatga taacaatcaa accaatataa atcgatttat 3960

tcagacttgg agacatttta gtgatgccaa tataaaataa tcatgtgcac taaatttgaa 4020

ataattaatg tgtatggtaa actagattag aattagttct agacttctag tggtcgagat 4080

ttgttcataa gctttatgct cggacacgtg aggaacatac caaaatgttc gaatctatta 4140

tgccatacac ttattttaat catttcttct ctatgataga ttgctcacct aatttatata 4200

taatctaaga atatactact tagctttaac gtattttatc cgataacgaa ttcatgatac 4260

caaaaaagtt ctaaaacaaa agaggcatct tgtattattt atagtatttc taactaaaac 4320

acaacactga taacatttaa attgctttgt tccgtttaat tagtaaacaa atgaaaagct 4380

gtcgcgtcgg tggttgggtt tcattcggaa agatttaaaa gtaaagcgca cggattttaa 4440

ggtcgttcaa catttaaatt ttttttgaaa gtaaaatcat atatcaatgc aatgtgtaat 4500

cttgtaacgt ataaactaat gagagatacc taaagaaaag aagcctcgcg cctaaacaga 4560

aacataagat taaaataagt aatatagaag cataaactta agcaagattt tattcctcgt 4620

gaaaatatta cattttgtaa ttttattttc tctagtatta aaaaaggaac aactaattaa 4680

cggaaatact taaggaatta agaacagaga ggtctcatac ttagtcaagt ttggtgaagc 4740

tatcaaagag aatgtcttcg tgttgtccgc cgacgacatt ctgcag 4786

<210> 16
<211> 5521
<212> DNA
<213> Artificial Sequence

<220>
<221> source
<222> 1..5521
<223> /mol_type="unassigned DNA"
      /note="Fragment of Plasmid pCB302/AtSPO11-1-ZF3-18xMyc"
      /organism="Artificial Sequence"

<400> 16
ggatccgctg aagctgaagt tgccacgaag gagaaagcga gagagagacg acatcgtttt 60

tagctgtatg caaaggtttt tctcttcttt tttttggatc ggagtgttca aaggttttta 120

gtcaggggaa cggctttaat acttcaaatt aggtttggta acaatataaa gcaaatgaac 180

cctaaattac ttttgtatta ttcaaaattt caaacttatt actcattagt cattagtgaa 240

tgtactttgt attattaaaa attaaaaact gagaattatt ttttataatc tgtatattta 300

tgatttttcc attaaaacta tgttcatttg tccattctac caatgcattt ggtttgcact 360

aaaaagctta gaaagagtca taaaaaaaac aatgagcatt accttgagtt ggagactagt 420

tatctcgtat gaagcagatc ttcacccatc tccgttcaag tcttcttctg agattaattt 480

ttgttcaccg ttcaagtctt cctcggagat tagcttttgt tcaccgttca aatcttcttc 540

agaaatcaac ttttgttcac ctctagagga tccgttcaag tcttcttctg agattaattt 600

ttgttcaccg ttcaagtctt cttctgagat taatttttgt tcaccgttca aatcttcttc 660

agaaatcaac ttttgttcac cactagtgga tccgttcaag tcttcttctg agattaattt 720

ttgttcaccg ttcaagtctt cctcggagat tagcttttgt tcaccgttca aatcttcttc 780

agaaatcaac ttttgttcac cactagtgga tccgttcaag tcttcttctg agattaattt 840

ttgttcaccg ttcaagtctt cctcggagat tagcttttgt tcaccgttca aatcttcttc 900

agaaatcaac ttttgttcac ctctagagga tccgttcaag tcttcttctg agattaattt 960

ttgttcaccg ttcaagtctt cctcggagat tagcttttgt tcaccgttca aatcttcttc 1020

agaaatcaac ttttgttcac cactagtgga tccgttcaag tcttcttctg agattaattt 1080

ttgttcaccg ttcaagtctt cctcggagat tagcttttgt tcaccgttca aatcttcttc 1140

agaaatcaac ttttgttcac cactagcagc agaaccggac ccgggctttt ctccggaacc 1200

gccacccagc tcgccggtat gcgtacgttg gtgacggacg agattacccg aggtgctaaa 1260

agacttcccg cactcaggac aggcataagg cttctcgccg gtatgggtgc gttgatgccg 1320

aacgagtgct cccggatcgg agaaggactt gccacactca gggcatgcgt acggtttctc 1380

gccggtatgg gtgcgttgat gccttgcgag atcacggcag tcgctgaatg acttgccaca 1440

ctccgggcaa gcgtaaggct tctcgccggt atgggtcctc tgatggcgga cgaggtgacc 1500

tgggtcagag aagcttttac cgcattcagg gcacgcgtac ggtttctcgc cggtatgggt 1560

gcgttgatgc cttgcgagat cacggcagtc gctgaatgac ttgccacact ccgggcaagc 1620

gtaaggcttc tcgccggtat gcgtacgttg gtgacggacg agattacccg aggtgctaaa 1680

agacttcccg cactcaggac aggcataagg cttctcgccg gtgggttcga gggccgcctg 1740

ggcctcgaga tcgatgggag gagagcttac ttcacgacga atcttagaag gaatatatac 1800

ctctgacaaa aaggacagtg agtgaactga gagtgcttcg atctcaaact tgacccctct 1860

tttcaacatc gtttcaagtt ccaaccttag acgaccaaaa tcgttttgat caagactagt 1920

ttttgtcatt tggttgaaat gtaaatataa aaaagtcagg gaagtttttc atacctccat 1980

tgtggcattt cacgcttcag gtagcatcta agcaacattg cttcagttct tttcttatct 2040

gtccggggaa gttgttacaa tctcaaaaaa ggaagcagtg aaaaatagga aaatctaaag 2100

atttaaggcg ataaggtgtt taatgatctc ttaccttctt ctgtcagtgg caaaagacac 2160

tgtttgggaa cactatatac ctcagagtct gaaggaaaag ctcccaacca tttcatatct 2220

ggtgctcgta atgattcaat atcataggcc atttgcttac agaaagaaaa caagtgacat 2280

aaggaaaatg gttcaaaatg aaatgggtca gtgccatatc acattatcac tatcaacaaa 2340

aacaatgcaa gcggtgaaat tttccgagtt cagtcatcta tattttgtaa cagaatctaa 2400

ccatggagcc aaaacggtat gtggctagga tctcaaagcc atatggatca cagtcgacta 2460

gacaatgcac aggtagatgc aacttctcca tcaggagtcg caagaacctg gaaagaatct 2520

gtgttattcg ctgatatatt aatcaataga agataatgtc tgataaaata gtattccagg 2580

aacctgggat taagttatgt accttcttgt tgagacatct ggatatcctc ttccctgcca 2640

aaaacaatcg taactttgat gtcaaacata aaatggaaat gaaaactagt caaaactgaa 2700

atcagtaaga atgaagcaga tttcttactg tgataacaat gcagcggttc gtcttgcaaa 2760

acatgtcatt tgctaaacgc tggaatacta taaaccatgg atgaaagcga tttagtcatg 2820

aagaaagaaa accatcttcc tagttactac ataaataaga ggtctttctt acctgtttct 2880

ttttccacaa ccagtatgta ctcagctaag ctaacaatat ctgcaagcag gaaaatgaca 2940

tccaattata gttcctgatg ccaccataga tagtgaatct tatacttcct gatatcagct 3000

gagataagga tttgacttct ctaaagagca taaagcatca cttaaaatga ttgcaaatat 3060

atgtaccttc gacttcctct acaagaacag gaacgggata tgcctgaaat gaaatcactt 3120

gcagagttat acttcctgat gccaatataa aaagtgaatc ttatgttcaa attcttacag 3180

tgttcaagct gtttaaacaa tcaaatttcc ttccagcttc cctaaatttt aaccaaccca 3240

tcaccaacct acaacacaaa agaataatga ggtggatctt aaaataagga catgcctcaa 3300

gatagaagca ttgcactcgt tatcaagagc acccacccgt ttccaacaga aacctgcaaa 3360

atatcacatg aatgaaaaag taatcacatc ttcaatcgca gcttataata actcttcaga 3420

aggaaaattg aaggtgaaag tattaaagta ctaaccacat tcaagttgta ccgactacac 3480

tgaaaaagaa tgcatatatc accaattgca cggtccacaa tggattgtgc tgtgaagtag 3540

acgaaaacag tgttactaga aggccaagaa cacaacaaag agggatgcat aactaatatc 3600

cgtataaaaa tctcaatacg tattcgttta gaagaaaaac ataactaaaa cattatacgt 3660

tgcttccaaa ttcacctttg aaagctgatg gatgcatgta gtagatatct cttttcgaag 3720

catgtctatt ttcttgcaga agttgttgga ctatcaacaa tactcttagc aacatatctg 3780

aaagtaaaaa caccgcaatg tgatgctata ttaagataac actaatgtac ttgtagaatt 3840

atctgaaacc tgactcaccg attctgtagg tttgtggttc cttcttaaga gtgaaatttt 3900

cctgaccctt tggtttatca gagctgcata agctatagac agcagaataa aatggtaaat 3960

tcatgaggtc ttagttttca atactcgttg acgttgtgct taaatccttc aaaaatgtct 4020

cgaaccaaag accaaacacg tctgattctt caatcaaaga aagtgaaaac aaaattgaat 4080

aatcgagatc gaagagagac aaaccaatca gcttctggat tcatgcaata gttcctgaat 4140

tgattgatgg aaatttttgg agatcttcct tcagccagat ctacaacgac cgattgagta 4200

aaatctgctc aaaaaaccga aacatagtga tgaacaacgg aagagccagc gaagtcatcg 4260

actagaaatt tcgacataga ttagaggaca aattactcaa tacgattcgg aaaattttgg 4320

gattttctga tacctttgat cctttgaagc aaattagtcg attctgaaat agcgaatttt 4380

ccctccatct ctttcgagtt tcaaaactga aaatgcaatt ggtcgacgaa tttttgtaga 4440

cgggaagaaa agggatgggt gcgaaaagct tgccgttaaa accttcgaat ttaaacatgc 4500

gtgtcgttta gttcctactt tccatgacgt tgggccattg gcatttaggc ccgatacgta 4560

actgacgtgt cattttaaaa acgcatgtgt ttttaaacac gttgggccgt tggcttttag 4620

gcccgatccg aaactaggca aagtgattgt gaaaaagatt catgataaca atcaaaccaa 4680

tataaatcga tttattcaga cttggagaca ttttagtgat gccaatataa aataatcatg 4740

tgcactaaat ttgaaataat taatgtgtat ggtaaactag attagaatta gttctagact 4800

tctagtggtc gagatttgtt cataagcttt atgctcggac acgtgaggaa cataccaaaa 4860

tgttcgaatc tattatgcca tacacttatt ttaatcattt cttctctatg atagattgct 4920

cacctaattt atatataatc taagaatata ctacttagct ttaacgtatt ttatccgata 4980

acgaattcat gataccaaaa aagttctaaa acaaaagagg catcttgtat tatttatagt 5040

atttctaact aaaacacaac actgataaca tttaaattgc tttgttccgt ttaattagta 5100

aacaaatgaa aagctgtcgc gtcggtggtt gggtttcatt cggaaagatt taaaagtaaa 5160

gcgcacggat tttaaggtcg ttcaacattt aaattttttt tgaaagtaaa atcatatatc 5220

aatgcaatgt gtaatcttgt aacgtataaa ctaatgagag atacctaaag aaaagaagcc 5280

tcgcgcctaa acagaaacat aagattaaaa taagtaatat agaagcataa acttaagcaa 5340

gattttattc ctcgtgaaaa tattacattt tgtaatttta ttttctctag tattaaaaaa 5400

ggaacaacta attaacggaa atacttaagg aattaagaac agagaggtct catacttagt 5460

caagtttggt gaagctatca aagagaatgt cttcgtgttg tccgccgacg acattctgca 5520

g 5521

<210> 17
<211> 5521
<212> DNA
<213> Artificial Sequence

<220>
<221> source
<222> 1..5521
<223> /mol_type="unassigned DNA"
      /note="Fragment of Plasmid pCB302/AtSPO11-1-ZF4-18xMyc"
      /organism="Artificial Sequence"

<400> 17
ggatccgctg aagctgaagt tgccacgaag gagaaagcga gagagagacg acatcgtttt 60

tagctgtatg caaaggtttt tctcttcttt tttttggatc ggagtgttca aaggttttta 120

gtcaggggaa cggctttaat acttcaaatt aggtttggta acaatataaa gcaaatgaac 180

cctaaattac ttttgtatta ttcaaaattt caaacttatt actcattagt cattagtgaa 240

tgtactttgt attattaaaa attaaaaact gagaattatt ttttataatc tgtatattta 300

tgatttttcc attaaaacta tgttcatttg tccattctac caatgcattt ggtttgcact 360

aaaaagctta gaaagagtca taaaaaaaac aatgagcatt accttgagtt ggagactagt 420

tatctcgtat gaagcagatc ttcacccatc tccgttcaag tcttcttctg agattaattt 480

ttgttcaccg ttcaagtctt cctcggagat tagcttttgt tcaccgttca aatcttcttc 540

agaaatcaac ttttgttcac ctctagagga tccgttcaag tcttcttctg agattaattt 600

ttgttcaccg ttcaagtctt cttctgagat taatttttgt tcaccgttca aatcttcttc 660

agaaatcaac ttttgttcac cactagtgga tccgttcaag tcttcttctg agattaattt 720

ttgttcaccg ttcaagtctt cctcggagat tagcttttgt tcaccgttca aatcttcttc 780

agaaatcaac ttttgttcac cactagtgga tccgttcaag tcttcttctg agattaattt 840

ttgttcaccg ttcaagtctt cctcggagat tagcttttgt tcaccgttca aatcttcttc 900

agaaatcaac ttttgttcac ctctagagga tccgttcaag tcttcttctg agattaattt 960

ttgttcaccg ttcaagtctt cctcggagat tagcttttgt tcaccgttca aatcttcttc 1020

agaaatcaac ttttgttcac cactagtgga tccgttcaag tcttcttctg agattaattt 1080

ttgttcaccg ttcaagtctt cctcggagat tagcttttgt tcaccgttca aatcttcttc 1140

agaaatcaac ttttgttcac cactagcagc agaaccggac ccgggctttt ctccggaacc 1200

gccacccagc tcgccggtat gggtgcgttg atgccgaacg agtgctcccg gatcggagaa 1260

ggacttgcca cactcagggc atgcgtacgg tttctcgccg gtatgggtcc tctggtggcg 1320

ttcaagatgg gcacgctgac taaagctttt accgcactcg ggacaggcgt atggtttctc 1380

gccggtatgg gtgcgctgat gccgaaccaa gttaccggga tcggagaagg attttccgca 1440

ttccgggcat gcgtagggct tctcgccggt atgcgtacgc tggtgacgca cgagttcacc 1500

gctggtcgaa aaggatttcc cgcattcggg gcacgcatac ggtttctcgc cggtatgggt 1560

gcgttgatgc cgaacgagtg ctcccggatc ggagaaggac ttgccacact cagggcatgc 1620

gtacggtttc tcgccggtat gggtcctctg atgcctcacg aggttgctgg actgagagaa 1680

gctttttccg cattcagggc aagcgtacgg tttctcgccg gtgggttcga gggccgcctg 1740

ggcctcgaga tcgatgggag gagagcttac ttcacgacga atcttagaag gaatatatac 1800

ctctgacaaa aaggacagtg agtgaactga gagtgcttcg atctcaaact tgacccctct 1860

tttcaacatc gtttcaagtt ccaaccttag acgaccaaaa tcgttttgat caagactagt 1920

ttttgtcatt tggttgaaat gtaaatataa aaaagtcagg gaagtttttc atacctccat 1980

tgtggcattt cacgcttcag gtagcatcta agcaacattg cttcagttct tttcttatct 2040

gtccggggaa gttgttacaa tctcaaaaaa ggaagcagtg aaaaatagga aaatctaaag 2100

atttaaggcg ataaggtgtt taatgatctc ttaccttctt ctgtcagtgg caaaagacac 2160

tgtttgggaa cactatatac ctcagagtct gaaggaaaag ctcccaacca tttcatatct 2220

ggtgctcgta atgattcaat atcataggcc atttgcttac agaaagaaaa caagtgacat 2280

aaggaaaatg gttcaaaatg aaatgggtca gtgccatatc acattatcac tatcaacaaa 2340

aacaatgcaa gcggtgaaat tttccgagtt cagtcatcta tattttgtaa cagaatctaa 2400

ccatggagcc aaaacggtat gtggctagga tctcaaagcc atatggatca cagtcgacta 2460

gacaatgcac aggtagatgc aacttctcca tcaggagtcg caagaacctg gaaagaatct 2520

gtgttattcg ctgatatatt aatcaataga agataatgtc tgataaaata gtattccagg 2580

aacctgggat taagttatgt accttcttgt tgagacatct ggatatcctc ttccctgcca 2640

aaaacaatcg taactttgat gtcaaacata aaatggaaat gaaaactagt caaaactgaa 2700

atcagtaaga atgaagcaga tttcttactg tgataacaat gcagcggttc gtcttgcaaa 2760

acatgtcatt tgctaaacgc tggaatacta taaaccatgg atgaaagcga tttagtcatg 2820

aagaaagaaa accatcttcc tagttactac ataaataaga ggtctttctt acctgtttct 2880

ttttccacaa ccagtatgta ctcagctaag ctaacaatat ctgcaagcag gaaaatgaca 2940

tccaattata gttcctgatg ccaccataga tagtgaatct tatacttcct gatatcagct 3000

gagataagga tttgacttct ctaaagagca taaagcatca cttaaaatga ttgcaaatat 3060

atgtaccttc gacttcctct acaagaacag gaacgggata tgcctgaaat gaaatcactt 3120

gcagagttat acttcctgat gccaatataa aaagtgaatc ttatgttcaa attcttacag 3180

tgttcaagct gtttaaacaa tcaaatttcc ttccagcttc cctaaatttt aaccaaccca 3240

tcaccaacct acaacacaaa agaataatga ggtggatctt aaaataagga catgcctcaa 3300

gatagaagca ttgcactcgt tatcaagagc acccacccgt ttccaacaga aacctgcaaa 3360

atatcacatg aatgaaaaag taatcacatc ttcaatcgca gcttataata actcttcaga 3420

aggaaaattg aaggtgaaag tattaaagta ctaaccacat tcaagttgta ccgactacac 3480

tgaaaaagaa tgcatatatc accaattgca cggtccacaa tggattgtgc tgtgaagtag 3540

acgaaaacag tgttactaga aggccaagaa cacaacaaag agggatgcat aactaatatc 3600

cgtataaaaa tctcaatacg tattcgttta gaagaaaaac ataactaaaa cattatacgt 3660

tgcttccaaa ttcacctttg aaagctgatg gatgcatgta gtagatatct cttttcgaag 3720

catgtctatt ttcttgcaga agttgttgga ctatcaacaa tactcttagc aacatatctg 3780

aaagtaaaaa caccgcaatg tgatgctata ttaagataac actaatgtac ttgtagaatt 3840

atctgaaacc tgactcaccg attctgtagg tttgtggttc cttcttaaga gtgaaaattt 3900

cctgaccctt tggtttatca gagctgcata agctatagac agcagaataa aatggtaaat 3960

tcatgaggtc ttagttttca atactcgttg acgttgtgct taaatccttc aaaaatgtct 4020

cgaaccaaag accaaacacg tctgattctt caatcaaaga aagtgaaaac aaaattgaat 4080

aatcgagatc gaagagagac aaaccaatca gcttctggat tcatgcaata gttcctgaat 4140

tgattgatgg aaatttttgg agatcttcct tcagccagat ctacaacgac cgattgagta 4200

aaatctgctc aaaaaaccga aacatagtga tgaacaacgg aagagccagc gaagtcatcg 4260

actagaaatt tcgacataga ttagaggaca aattactcaa tacgattcgg aaaattttgg 4320

gattttctga tacctttgat cctttgaagc aaattagtcg attctgaaat agcgaatttt 4380

ccctccatct ctttcgagtt tcaaaactga aaaatgcaat ggtcgacgaa tttttgtaga 4440

cgggaagaaa agggatgggt gcgaaaagct tgccgttaaa accttcgaat ttaaacatgc 4500

gtgtcgttta gttcctactt tccatgacgt tgggccattg gcatttaggc ccgatacgta 4560

actgacgtgt cattttaaaa acgcatgtgt ttttaaacac gttgggccgt tggcttttag 4620

gcccgatccg aaactaggca aagtgattgt gaaaaagatt catgataaca atcaaaccaa 4680

tataaatcga tttattcaga cttggagaca ttttagtgat gccaatataa aataatcatg 4740

tgcactaaat ttgaaataat taatgtgtat ggtaaactag attagaatta gttctagact 4800

tctagtggtc gagatttgtt cataagcttt atgctcggac acgtgaggaa cataccaaaa 4860

tgttcgaatc tattatgcca tacacttatt ttaatcattt cttctctatg atagattgct 4920

cacctaattt atatataatc taagaatata ctacttagct ttaacgtatt ttatccgata 4980

acgaattcat gataccaaaa aagttctaaa acaaaagagg catcttgtat tatttatagt 5040

atttctaact aaaacacaac actgataaca tttaaattgc tttgttccgt ttaattagta 5100

aacaaatgaa aagctgtcgc gtcggtggtt gggtttcatt cggaaagatt taaaagtaaa 5160

gcgcacggat tttaaggtcg ttcaacattt aaattttttt tgaaagtaaa atcatatatc 5220

aatgcaatgt gtaatcttgt aacgtataaa ctaatgagag atacctaaag aaaagaagcc 5280

tcgcgcctaa acagaaacat aagattaaaa taagtaatat agaagcataa acttaagcaa 5340

gattttattc ctcgtgaaaa tattacattt tgtaatttta ttttctctag tattaaaaaa 5400

ggaacaacta attaacggaa atacttaagg aattaagaac agagaggtct catacttagt 5460

caagtttggt gaagctatca aagagaatgt cttcgtgttg tccgccgacg acattctgca 5520

g 5521

<210> 18
<211> 4488
<212> DNA
<213> Artificial Sequence

<220>
<221> source
<222> 1..4488
<223> /mol_type="unassigned DNA"
      /note="Plasmid pSAT6/ZF-1-TS"
      /organism="Artificial Sequence"

<400> 18
tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcaggcgcc 240

attcgccatt caggctgcgc aactgttggg aagggcgatc ggtgcgggcc tcttcgctat 300

tacgccagct ggcgaaaggg ggatgtgctg caaggcgatt aagttgggta acgccagggt 360
tttcccagtc acgacgttgt aaaacgacgg ccagtgccac ccataatacc cataatagct 420
gtttgccaac cggtcaacat gtggagcacg acacacttgt ctactccaaa aatatcaaag 480
atacagtctc agaagaccaa agggcaattg agacttttca acaaagggta atatccggaa 540
acctcctcgg attccattgc ccagctatct gtcactttat tgtgaagata gtggaaaagg 600
aaggtggctc ctacaaatgc catcattgcg ataaaggaaa ggccatcgtt gaagatgcct 660
ctgccgacag tggtcccaaa gatggacccc cacccacgag gagcatcgtg gaaaaagaag 720
acgttccaac cacgtcttca aagcaagtgg attgatgtga taacatggtg gagcacgaca 780
cacttgtcta ctccaaaaat atcaaagata cagtctcaga agaccaaagg gcaattgaga 840
cttttcaaca aagggtaata tccggaaacc tcctcggatt ccattgccca gctatctgtc 900
actttattgt gaagatagtg gaaaaggaag gtggctccta caaatgccat cattgcgata 960
aaggaaaggc catcgttgaa gatgcctctg ccgacagtgg tcccaaagat ggacccccac 1020
ccacgaggag catcgtggaa aaagaagacg ttccaaccac gtcttcaaag caagtggatt 1080
gatgtgatat ctccactgac gtaagggatg acgcacaatc ccactatcct tcgcaagacc 1140
cttcctctat ataaggaagt tcatttcatt tggagaggac gtcgagagtt ctcaacacaa 1200
catatacaaa acaaacgaat ctcaagcaat caagcattct acttctattg cagcaattta 1260
aatcatttct tttaaagcaa aagcaatttt ctgaaaattt tcaccattta cgaacgatag 1320
ccatggtgcc aaaaaagaag agaaaggtag aagacccctc tcgagtacac caggaccgag 1380
ctcggtcctg gtgtaactag tcaaaagtga actggaggag aagaaatctg aacttcgtca 1440
taaattgaaa tatgtgcctc atgaatatat tgaattaatt gaaattgcca gaaattccac 1500
tcaggataga attcttgaaa tgaaggtaat ggaatttttt atgaaagttt atggatatag 1560
aggtaaacat ttgggtggat caaggaaacc ggacggagca atttatactg tcggatctcc 1620
tattgattac ggtgtgatcg tggatactaa agcttatagc ggaggttata atctgccaat 1680
tggccaagca gatgaaatgc aacgatatgt cgaagaaaat caaacacgaa acaaacatat 1740
caaccctaat gaatggtgga aagtctatcc atcttctgta acggaattta agtttttatt 1800
tgtgagtggt cactttaaag gaaactacaa agctcagctt acacgattaa atcatatcac 1860
taattgtaat ggagctgttc ttagtgtaga agagctttta attggtggag aaatgattaa 1920
agccggcaca ttaaccttag aggaagtgag acggaaattt aataacggcg agataaactt 1980
tggatcctaa agatccacct agtctagagt ccgcaaaaat caccagtctc tctctacaaa 2040
tctatctctc tctatttttc tccagaataa tgtgtgagta gttcccagat aagggaatta 2100
gggttcttat agggtttcgc tcatgtgttg agcatataag aaacccttag tatgtatttg 2160

tatttgtaaa atacttctat caataaaatt tctaattcct aaaaccaaaa tccagtgacg 2220

cggccgcacc cataataccc ataatagctg tttgccagta atcatggtca tagctgtttc 2280

ctgtgtgaaa ttgttatccg ctcacaattc cacacaacat acgagccgga agcataaagt 2340

gtaaagcctg gggtgcctaa tgagtgagct aactcacatt aattgcgttg cgctcactgc 2400

ccgctttcca gtcgggaaac ctgtcgtgcc agctgcatta atgaatcggc caacgcgcgg 2460

ggagaggcgg tttgcgtatt gggcgctctt ccgcttcctc gctcactgac tcgctgcgct 2520

cggtcgttcg gctgcggcga gcggtatcag ctcactcaaa ggcggtaata cggttatcca 2580

cagaatcagg ggataacgca ggaaagaaca tgtgagcaaa aggccagcaa aaggccagga 2640

accgtaaaaa ggccgcgttg ctggcgtttt tccataggct ccgcccccct gacgagcatc 2700

acaaaaatcg acgctcaagt cagaggtggc gaaacccgac aggactataa agataccagg 2760

cgtttccccc tggaagctcc ctcgtgcgct ctcctgttcc gaccctgccg cttaccggat 2820

acctgtccgc ctttctccct tcgggaagcg tggcgctttc tcaatgctca cgctgtaggt 2880

atctcagttc ggtgtaggtc gttcgctcca agctgggctg tgtgcacgaa ccccccgttc 2940

agcccgaccg ctgcgcctta tccggtaact atcgtcttga gtccaacccg gtaagacacg 3000

acttatcgcc actggcagca gccactggta acaggattag cagagcgagg tatgtaggcg 3060

gtgctacaga gttcttgaag tggtggccta actacggcta cactagaagg acagtatttg 3120

gtatctgcgc tctgctgaag ccagttacct tcggaaaaag agttggtagc tcttgatccg 3180

gcaaacaaac caccgctggt agcggtggtt tttttgtttg caagcagcag attacgcgca 3240

gaaaaaaagg atctcaagaa gatcctttga tcttttctac ggggtctgac gctcagtgga 3300

acgaaaactc acgttaaggg attttggtca tgagattatc aaaaaggatc ttcacctaga 3360

tccttttaaa ttaaaaatga agttttaaat caatctaaag tatatatgag taaacttggt 3420

ctgacagtta ccaatgctta atcagtgagg cacctatctc agcgatctgt ctatttcgtt 3480

catccatagt tgcctgactc cccgtcgtgt agataactac gatacgggag ggcttaccat 3540

ctggccccag tgctgcaatg ataccgcgag acccacgctc accggctcca gatttatcag 3600

caataaacca gccagccgga agggccgagc gcagaagtgg tcctgcaact ttatccgcct 3660

ccatccagtc tattaattgt tgccgggaag ctagagtaag tagttcgcca gttaatagtt 3720

tgcgcaacgt tgttgccatt gctacaggca tcgtggtgtc acgctcgtcg tttggtatgg 3780

cttcattcag ctccggttcc caacgatcaa ggcgagttac atgatccccc atgttgtgca 3840

aaaaagcggt tagctccttc ggtcctccga tcgttgtcag aagtaagttg gccgcagtgt 3900

tatcactcat ggttatggca gcactgcata attctcttac tgtcatgcca tccgtaagat 3960

gcttttctgt gactggtgag tactcaacca agtcattctg agaatagtgt atgcggcgac 4020

cgagttgctc ttgcccggcg tcaatacggg ataataccgc gccacatagc agaactttaa 4080

aagtgctcat cattggaaaa cgttcttcgg ggcgaaaact ctcaaggatc ttaccgctgt 4140

tgagatccag ttcgatgtaa cccactcgtg cacccaactg atcttcagca tcttttactt 4200

tcaccagcgt ttctgggtga gcaaaaacag gaaggcaaaa tgccgcaaaa aagggaataa 4260

gggcgacacg gaaatgttga atactcatac tcttcctttt tcaatattat tgaagcattt 4320

atcagggtta ttgtctcatg agcggataca tatttgaatg tatttagaaa aataaacaaa 4380

taggggttcc gcgcacattt ccccgaaaag tgccacctga cgtctaagaa accattatta 4440

tcatgacatt aacctataaa aataggcgta tcacgaggcc ctttcgtc 4488

<210> 19
<211> 7
<212> PRT
<213> Artificial Sequence

<220>
<223> Zinc finger modules

<400> 19
Thr Ser Gly Asn Leu Val Arg
1               5

<210> 20
<211> 7
<212> PRT
<213> Artificial Sequence

<220>
<223> Zinc finger modules

<400> 20
Asp Cys Arg Asp Leu Ala Arg
1               5

<210> 21
<211> 7
<212> PRT
<213> Artificial Sequence

<220>
<223> Zinc finger modules

<400> 21
Asp Pro Gly His Leu Val Arg
1               5

<210> 22
<211> 7
<212> PRT
<213> Artificial Sequence

<220>
<223> Zinc finger modules

<400> 22
Asp Pro Gly Ala Leu Val Arg
1               5

<210> 23
<211> 7
<212> PRT
<213> Artificial Sequence

<220>
<223> Zinc finger modules

<400> 23
Thr Ser Gly Asn Leu Val Arg
1               5

<210> 24
<211> 7
<212> PRT
<213> Artificial Sequence

<220>
<223> Zinc finger modules

<400> 24
Thr Ser Gly Glu Leu Val Arg
1               5

<210> 25
<211> 7
<212> PRT
<213> Artificial Sequence

<220>
<223> Zinc finger modules

<400> 25
Asp Pro Gly Asn Leu Val Arg
1               5

<210> 26
<211> 7
<212> PRT
<213> Artificial Sequence

<220>
<223> Zinc finger modules

<400> 26
Gln Arg Ala His Leu Glu Arg
1               5

<210> 27
<211> 8
<212> PRT
<213> Artificial Sequence

<220>
<223> amino acid sequence of FLAG tag

<400> 27
Asp Tyr Lys Asp Asp Asp Asp Lys
1               5

<210> 28
<211> 10

<212> PRT
<213> Artificial Sequence

<220>
<223> amino acid sequence of Myc tag

<400> 28
Glu Gln Lys Leu Ile Ser Glu Glu Asp Leu
1               5                   10

<210> 29
<211> 14
<212> PRT
<213> Artificial Sequence

<220>
<223> amino acid sequence of V5 tag

<400> 29
Gly Lys Pro Ile Pro Asn Pro Leu Leu Gly Leu Asp Ser Thr
1               5                   10

<210> 30
<211> 6
<212> PRT
<213> Artificial Sequence

<220>
<223> amino acid sequence of HQ tag

<400> 30
His Gln His Gln His Gln
1               5

<210> 31
<211> 9
<212> PRT
<213> Artificial Sequence

<220>
<223> amino acid sequence of HA tag

<400> 31
Tyr Pro Tyr Asp Val Pro Asp Tyr Ala
1               5

<210> 32
<211> 6
<212> PRT
<213> Artificial Sequence

<220>
<223> amino acid sequence of AU1 tag

<400> 32
Asp Thr Tyr Arg Tyr Ile
1               5

<210> 33
<211> 11
<212> PRT
<213> Artificial Sequence

<220>
<223> amino acid sequence of T7 tag

<400> 33
Met Ala Ser Met Thr Gly Gly Gln Gln Met Gly
1               5                   10

<210> 34
<211> 11
<212> PRT
<213> Artificial Sequence

<220>
<223> amino acid sequence of VSV-G tag

<400> 34
Tyr Thr Asp Ile Glu Met Asn Arg Leu Gly Lys
1               5                   10

<210> 35
<211> 5
<212> PRT
<213> Artificial Sequence

<220>
<223> amino acid sequence of DDDDK tag

<400> 35
Asp Asp Asp Asp Lys
1               5

<210> 36
<211> 16
<212> PRT
<213> Artificial Sequence

<220>
<223> amino acid sequence of S tag

<400> 36
Lys Glu Thr Ala Ala Ala Lys Phe Glu Arg Gln His Ile Asp Ser Cys
1               5                   10                  15

<210> 37
<211> 12
<212> PRT
<213> Artificial Sequence

<220>
<223> amino acid sequence of CruzTag09

<400> 37
Met Lys Ala Glu Phe Arg Arg Gln Glu Ser Asp Arg
1               5                   10

<210> 38
<211> 12
<212> PRT
<213> Artificial Sequence

<220>

<223> amino acid sequence of CruzTag22

<400> 38
Met Arg Asp Ala Leu Asp Arg Leu Asp Arg Leu Ala
1               5                   10

<210> 39
<211> 12
<212> PRT
<213> Artificial Sequence

<220>
<223> amino acid sequence of CruzTag41

<400> 39
Met Lys Asp Gly Glu Glu Tyr Ser Arg Ala Phe Arg
1               5                   10

<210> 40
<211> 9
<212> PRT
<213> Artificial Sequence

<220>
<223> amino acid sequence of Glu-Glu tag

<400> 40
Glu Glu Glu Glu Tyr Met Pro Met Glu
1               5

<210> 41
<211> 12
<212> PRT
<213> Artificial Sequence

<220>
<223> amino acid sequence of Ha.11 tag

<400> 41
Cys Thr Pro Thr Asp Val Pro Asp Tyr Ala Ser Leu
1               5                   10

<210> 42
<211> 6
<212> PRT
<213> Artificial Sequence

<220>
<223> amino acid sequence of KT3 tag

<400> 42
Pro Pro Glu Pro Glu Thr
1               5

<210> 43
<211> 32
<212> DNA
<213> Artificial Sequence

<220>
<221> source
<222> 1..32
<223> /mol_type="unassigned DNA"

/note="Primer gAtSPOdn"
      /organism="Artificial Sequence"

<400> 43
aaaactgcag aatgtcgtcg gcggacaaca cg                                    32


<210> 44
<211> 43
<212> DNA
<213> Artificial Sequence

<220>
<221> source
<222> 1..43
<223> /mol_type="unassigned DNA"
      /note="Primer gAtSPOup"
      /organism="Artificial Sequence"

<400> 44
cgcggatcct cacccgggag gagagcttac ttcacgacga atc                        43


<210> 45
<211> 44
<212> DNA
<213> Artificial Sequence

<220>
<221> source
<222> 1..44
<223> /mol_type="unassigned DNA"
      /note="Primer gATSPO_STOP_dn"
      /organism="Artificial Sequence"

<400> 45
tcaacccggg tgaagatctg cttcatacga gataactagt ctcc                       44


<210> 46
<211> 30
<212> DNA
<213> Artificial Sequence

<220>
<221> source
<222> 1..30
<223> /mol_type="unassigned DNA"
      /note="Primer gAtSPOup"
      /organism="Artificial Sequence"

<400> 46
cgcggatccg ctgaagctga agttgccacg                                       30


<210> 47
<211> 36
<212> DNA
<213> Artificial Sequence

<220>
<221> source
<222> 1..36

<223> /mol_type="unassigned DNA"
      /note="Primer c-myc_new_dn"
      /organism="Artificial Sequence"

<400> 47
atccatggct cccgggtccg gttctgctgc tagtgg                                36


<210> 48
<211> 41
<212> DNA
<213> Artificial Sequence

<220>
<221> source
<222> 1..41
<223> /mol_type="unassigned DNA"
      /note="Primer c-myc_new_up"
      /organism="Artificial Sequence"

<400> 48
atagatcttt agatatctcg gttcaagtct tcttctgaga t                          41


<210> 49
<211> 38
<212> DNA
<213> Artificial Sequence

<220>
<221> source
<222> 1..38
<223> /mol_type="unassigned DNA"
      /note="Primer GAL4_new_dn"
      /organism="Artificial Sequence"

<400> 49
atccatggct cccgggatga agctcctgtc ctccatcg                              38


<210> 50
<211> 38
<212> DNA
<213> Artificial Sequence

<220>
<221> source
<222> 1..38
<223> /mol_type="unassigned DNA"
      /note="Primer Gal4_new_up"
      /organism="Artificial Sequence"

<400> 50
atggatcctt agatatccga gacggtcaac tggcgctg                              38


<210> 51
<211> 24
<212> DNA
<213> Artificial Sequence

<220>
<221> source

<222> 1..24
<223> /mol_type="unassigned DNA"
      /note="Primer GAL4_2_mut_up"
      /organism="Artificial Sequence"

<400> 51
ttcggcttct ccttggagca cttg                                              24


<210> 52
<211> 24
<212> DNA
<213> Artificial Sequence

<220>
<221> source
<222> 1..24
<223> /mol_type="unassigned DNA"
      /note="Primer GAL4_2_mut_dn"
      /organism="Artificial Sequence"

<400> 52
aagtgctcca aggagaagcc gaag                                              24


<210> 53
<211> 29
<212> DNA
<213> Artificial Sequence

<220>
<221> source
<222> 1..29
<223> /mol_type="unassigned DNA"
      /note="Primer 5xUAS-forw"
      /organism="Artificial Sequence"

<400> 53
tatggtaccg attacgccaa gcttgcatg                                         29


<210> 54
<211> 22
<212> DNA
<213> Artificial Sequence

<220>
<221> source
<222> 1..22
<223> /mol_type="unassigned DNA"
      /note="Primer 5xUAS-rev"
      /organism="Artificial Sequence"

<400> 54
ttggccatgg aacaggtagt tt                                                22


<210> 55
<211> 12
<212> DNA
<213> Artificial Sequence

<220>

<221> source
<222> 1..12
<223> /mol_type="unassigned DNA"
      /note="Four zinger arrays ZF1, target site"
      /organism="Artificial Sequence"

<400> 55
ggtcctggtg ta                                                          12


<210> 56
<211> 12
<212> DNA
<213> Artificial Sequence

<220>
<221> source
<222> 1..12
<223> /mol_type="unassigned DNA"
      /note="Four zinger arrays ZF2 target site"
      /organism="Artificial Sequence"

<400> 56
ggtacagatg cg                                                          12


<210> 57
<211> 18
<212> DNA
<213> Artificial Sequence

<220>
<221> source
<222> 1..18
<223> /mol_type="unassigned DNA"
      /note="Six-finger arrays ZF3 target site"
      /organism="Artificial Sequence"

<400> 57
gatgtcgccg gcgccgat                                                    18


<210> 58
<211> 18
<212> DNA
<213> Artificial Sequence

<220>
<221> source
<222> 1..18
<223> /mol_type="unassigned DNA"
      /note="Six-finger arrays ZF4 target site"
      /organism="Artificial Sequence"

<400> 58
gtcggagacg ctgtcgaa                                                    18


<210> 59
<211> 49
<212> DNA
<213> Artificial Sequence

<220>
<221> source
<222> 1..49
<223> /mol_type="unassigned DNA"
      /note="Primer 4xZFN FW1"
      /organism="Artificial Sequence"

<400> 59
attacccggg gctggtggag gtgaaaaacc ttacaagtgt cctgaatgt                49


<210> 60
<211> 30
<212> DNA
<213> Artificial Sequence

<220>
<221> source
<222> 1..30
<223> /mol_type="unassigned DNA"
      /note="Primer 4xZFN REV1"
      /organism="Artificial Sequence"

<400> 60
tatagatatc gcccgtatgc gtcctctgat                                     30


<210> 61
<211> 22
<212> DNA
<213> Artificial Sequence

<220>
<221> source
<222> 1..22
<223> /mol_type="unassigned DNA"
      /note="Primer spo11-1-2-MG52"
      /organism="Artificial Sequence"

<400> 61
ggatcgggcc taaaagccaa cg                                             22


<210> 62
<211> 29
<212> DNA
<213> Artificial Sequence

<220>
<221> source
<222> 1..29
<223> /mol_type="unassigned DNA"
      /note="Primer spo11-1-2-MG96"
      /organism="Artificial Sequence"

<400> 62
ctttgaatgc tgatggatgc atgtagtag                                      29


<210> 63
<211> 20
<212> DNA
<213> Artificial Sequence

<220>
<221> source
<222> 1..20
<223> /mol_type="unassigned DNA"
      /note="Primer PTF1-6F RT up 1 fwd"
      /organism="Artificial Sequence"

<400> 63
aaatcaccac cggagcaata                                                    20


<210> 64
<211> 20
<212> DNA
<213> Artificial Sequence

<220>
<221> source
<222> 1..20
<223> /mol_type="unassigned DNA"
      /note="Primer PTF1-6F RT up 1 rev"
      /organism="Artificial Sequence"

<400> 64
tttgcgatga aagttgtgga                                                    20


<210> 65
<211> 20
<212> DNA
<213> Artificial Sequence

<220>
<221> source
<222> 1..20
<223> /mol_type="unassigned DNA"
      /note="Primer PTF1-6F RT down 1 fwd"
      /organism="Artificial Sequence"

<400> 65
gcgaaaatct cggaaggtta                                                    20


<210> 66
<211> 20
<212> DNA
<213> Artificial Sequence

<220>
<221> source
<222> 1..20
<223> /mol_type="unassigned DNA"
      /note="Primer PTF1-6F RT down 1 rev"
      /organism="Artificial Sequence"

<400> 66
agcagcggaa cagagagttg                                                    20


<210> 67
<211> 3778
<212> DNA

<213> Artificial Sequence

<220>
<221> source
<222> 1..3778
<223> /mol_type="unassigned DNA"
      /note="Fragment of Plasmid pCB302/Fragment1"
      /organism="Artificial Sequence"

<400> 67
ggatcctcac ccgggaggag agcttacttc acgacgaatc ttagaaggaa tatatacctc      60

tgacaaaaag gacagtgagt gaactgagag tgcttcgatc tcaaacttga cccctctttt     120

caacatcgtt tcaagttcca accttagacg accaaaatcg ttttgatcaa gactagtttt     180

tgtcatttgg ttgaaatgta aatataaaaa agtcagggaa gtttttcata cctccattgt     240

ggcatttcac gcttcaggta gcatctaagc aacattgctt cagttctttt cttatctgtc     300

cggggaagtt gttacaatct caaaaaagga agcagtgaaa aataggaaaa tctaaagatt     360

taaggcgata aggtgtttaa tgatctctta ccttcttctg tcagtggcaa aagacactgt     420

ttgggaacac tatatacctc agagtctgaa ggaaaagctc ccaaccattt catatctggt     480

gctcgtaatg attcaatatc ataggccatt tgcttacaga aagaaaacaa gtgacataag     540

gaaaatggtt caaaatgaaa tgggtcagtg ccatatcaca ttatcactat caacaaaaac     600

aatgcaagcg gtgaaatttt ccgagttcag tcatctatat tttgtaacag aatctaacca     660

tggagccaaa acggtatgtg gctaggatct caaagccata tggatcacag tcgactagac     720

aatgcacagg tagatgcaac ttctccatca ggagtcgcaa gaacctggaa agaatctgtg     780

ttattcgctg atatattaat caatagaaga taatgtctga taaaatagta ttccaggaac     840

ctgggattaa gttatgtacc ttcttgttga gacatctgga tatcctcttc cctgccaaaa     900

acaatcgtaa ctttgatgtc aaacataaaa tggaaatgaa aactagtcaa aactgaaatc     960

agtaagaatg aagcagattt cttactgtga taacaatgca gcggttcgtc ttgcaaaaca    1020

tgtcatttgc taaacgctgg aatactataa accatggatg aaagcgattt agtcatgaag    1080

aaagaaaacc atcttcctag ttactacata aataagaggt ctttcttacc tgtttctttt    1140

tccacaacca gtatgtactc agctaagcta acaatatctg caagcaggaa aatgacatcc    1200

aattatagtt cctgatgcca ccatagatag tgaatcttat acttcctgat atcagctgag    1260

ataaggattt gacttctcta aagagcataa agcatcactt aaaatgattg caaatatatg    1320

taccttcgac ttcctctaca agaacaggaa cgggatatgc ctgaaatgaa atcacttgca    1380

gagttatact tcctgatgcc aatataaaaa gtgaatctta tgttcaaatt cttacagtgt    1440

tcaagctgtt taaacaatca aatttccttc cagcttccct aaattttaac caacccatca    1500

ccaacctaca acacaaaaga ataatgaggt ggatcttaaa ataaggacat gcctcaagat    1560

agaagcattg cactcgttat caagagcacc cacccgtttc caacagaaac ctgcaaaata    1620

tcacatgaat gaaaaagtaa tcacatcttc aatcgcagct tataataact cttcagaagg 1680

aaaattgaag gtgaaagtat taaagtacta accacattca agttgtaccg actacactga 1740

aaaagaatgc atatatcacc aattgcacgg tccacaatgg attgtgctgt gaagtagacg 1800

aaaacagtgt tactagaagg ccaagaacac aacaaagagg gatgcataac taatatccgt 1860

ataaaaatct caatacgtat tcgtttagaa gaaaaacata actaaaacat tatacgttgc 1920

ttccaaattc acctttgaaa gctgatggat gcatgtagta gatatctctt ttcgaagcat 1980

gtctattttc ttgcagaagt tgttggacta tcaacaatac tcttagcaac atatctgaaa 2040

gtaaaaacac cgcaatgtga tgctatatta agataacact aatgtacttg tagaattatc 2100

tgaaacctga ctcaccgatt ctgtaggttt gtggttcctt cttaagagtg aaaatttcct 2160

gaccctttgg tttatcagag ctgcataagc tatagacagc agaataaaat ggtaaattca 2220

tgaggtctta gttttcaata ctcgttgacg ttgtgcttaa atccttcaaa aatgtctcga 2280

accaaagacc aaacacgtct gattcttcaa tcaaagaaag tgaaaacaaa attgaataat 2340

cgagatcgaa gagagacaaa ccaatcagct tctggattca tgcaatagtt cctgaattga 2400

ttgatggaaa tttttggaga tcttccttca gccagatcta caacgaccga ttgagtaaaa 2460

tctgctcaaa aaaccgaaac atagtgatga acaacggaag agccagcgaa gtcatcgact 2520

agaaatttcg acatagatta gaggacaaat tactcaatac gattcggaaa attttgggat 2580

tttctgatac ctttgatcct ttgaagcaaa ttagtcgatt ctgaaatagc gaattttccc 2640

tccatctctt tcgagtttca aaactgaaaa atgcaatggt cgacgaattt ttgtagacgg 2700

gaagaaaagg gatgggtgcg aaaagcttgc cgttaaaacc ttcgaattta aacatgcgtg 2760

tcgtttagtt cctactttcc atgacgttgg gccattggca tttaggcccg atacgtaact 2820

gacgtgtcat tttaaaaacg catgtgtttt taaacacgtt gggccgttgg cttttaggcc 2880

cgatccgaaa ctaggcaaag tgattgtgaa aaagattcat gataacaatc aaaccaatat 2940

aaatcgattt attcagactt ggagacattt tagtgatgcc aatataaaat aatcatgtgc 3000

actaaatttg aaataattaa tgtgtatggt aaactagatt agaattagtt ctagacttct 3060

agtggtcgag atttgttcat aagctttatg ctcggacacg tgaggaacat accaaaatgt 3120

tcgaatctat tatgccatac acttatttta atcatttctt ctctatgata gattgctcac 3180

ctaatttata tataatctaa gaatatacta cttagcttta acgtatttta tccgataacg 3240

aattcatgat accaaaaaag ttctaaaaca aaagaggcat cttgtattat ttatagtatt 3300

tctaactaaa acacaacact gataacattt aaattgcttt gttccgttta attagtaaac 3360

aaatgaaaag ctgtcgcgtc ggtggttggg tttcattcgg aaagatttaa aagtaaagcg 3420

cacggatttt aaggtcgttc aacatttaaa tttttttgga aagtaaaatc atatatcaat 3480

gcaatgtgta atcttgtaac gtataaacta atgagagata cctaaagaaa agaagcctcg 3540

cgcctaaaca gaaacataag attaaaataa gtaatataga agcataaact taagcaagat 3600

tttattcctc gtgaaaatat tacattttgt aattttattt tctctagtat taaaaaagga 3660

acaactaatt aacggaaata cttaaggaat taagaacaga gaggtctcat acttagtcaa 3720

gtttggtgaa gctatcaaag agaatgtctt cgtgttgtcc gccgacgaca ttctgcag 3778

<210> 68
<211> 4227
<212> DNA
<213> Artificial Sequence

<220>
<221> source
<222> 1..4227
<223> /mol_type="unassigned DNA"
      /note="Fragment of Plasmid pCB302/AtSPO11-1-WT"
      /organism="Artificial Sequence"

<400> 68
   ggatccgctg aagctgaagt tgccacgaag gagaaagcga gagagagacg acatcgtttt 60
   tagctgtatg caaaggtttt tctcttcttt tttttggatc ggagtgttca aaggttttta 120
   gtcaggggaa cggctttaat acttcaaatt aggtttggta acaatataaa gcaaatgaac 180
   cctaaattac ttttgtatta ttcaaaattt caaacttatt actcattagt cattagtgaa 240
   tgtactttgt attattaaaa attaaaaact gagaattatt ttttataatc tgtatattta 300
   tgatttttcc attaaaacta tgttcatttg tccattctac caatgcattt ggtttgcact 360
   aaaaagctta gaaagagtca taaaaaaaac aatgagcatt accttgagtt ggagactagt 420
   tatctcgtat gaagcagatc ttcacccggg aggagagctt acttcacgac gaatcttaga 480
   aggaatatat acctctgaca aaaaggacag tgagtgaact gagagtgctt cgatctcaaa 540
   cttgacccct cttttcaaca tcgtttcaag ttccaacctt agacgaccaa aatcgttttg 600
   atcaagacta gtttttgtca tttggttgaa atgtaaatat aaaaaagtca gggaagtttt 660
   tcatacctcc attgtggcat ttcacgcttc aggtagcatc taagcaacat tgcttcagtt 720
   cttttcttat ctgtccgggg aagttgttac aatctcaaaa aaggaagcag tgaaaaatag 780
   gaaaatctaa agatttaagg cgataaggtg tttaatgatc tcttaccttc ttctgtcagt 840
   ggcaaaagac actgtttggg aacactatat acctcagagt ctgaaggaaa agctcccaac 900
   catttcatat ctggtgctcg taatgattca atatcatagg ccatttgctt acagaaagaa 960
   aacaagtgac ataaggaaaa tggttcaaaa tgaaatgggt cagtgccata tcacattatc 1020
   actatcaaca aaaacaatgc aagcggtgaa attttccgag ttcagtcatc tatattttgt 1080
   aacagaatct aaccatggag ccaaaacggt atgtggctag gatctcaaag ccatatggat 1140
   cacagtcgac tagacaatgc acaggtagat gcaacttctc catcaggagt cgcaagaacc 1200
   tggaaagaat ctgtgttatt cgctgatata ttaatcaata gaagataatg tctgataaaa 1260
   tagtattcca ggaacctggg attaagttat gtaccttctt gttgagacat ctggatatcc 1320
   tcttccctgc caaaaacaat cgtaactttg atgtcaaaca taaaatggaa atgaaaacta 1380
   gtcaaaactg aaatcagtaa gaatgaagca gatttcttac tgtgataaca atgcagcggt 1440
   tcgtcttgca aaacatgtca tttgctaaac gctggaatac tataaaccat ggatgaaagc 1500
   gatttagtca tgaagaaaga aaaccatctt cctagttact acataaataa gaggtctttc 1560
   ttacctgttt ctttttccac aaccagtatg tactcagcta agctaacaat atctgcaagc 1620
   aggaaaatga catccaatta tagttcctga tgccaccata gatagtgaat cttatacttc 1680
   ctgatatcag ctgagataag gatttgactt ctctaaagag cataaagcat cacttaaaat 1740
   gattgcaaat atatgtacct tcgacttcct ctacaagaac aggaacggga tatgcctgaa 1800
   atgaaatcac ttgcagagtt atacttcctg atgccaatat aaaaagtgaa tcttatgttc 1860
   aaattcttac agtgttcaag ctgtttaaac aatcaaattt ccttccagct tccctaaatt 1920
   ttaaccaacc catcaccaac ctacaacaca aaagaataat gaggtggatc ttaaaataag 1980
   gacatgcctc aagatagaag cattgcactc gttatcaaga gcacccaccc gtttccaaca 2040
   gaaacctgca aaatatcaca tgaatgaaaa agtaatcaca tcttcaatcg cagcttataa 2100
   taactcttca gaaggaaaat tgaaggtgaa agtattaaag tactaaccac attcaagttg 2160
   taccgactac actgaaaaag aatgcatata tcaccaattg cacggtccac aatggattgt 2220
   gctgtgaagt agacgaaaac agtgttacta gaaggccaag aacacaacaa agagggatgc 2280
   ataactaata tccgtataaa aatctcaata cgtattcgtt tagaagaaaa acataactaa 2340
   aacattatac gttgcttcca aattcacctt tgaaagctga tggatgcatg tagtagatat 2400
   ctcttttcga agcatgtcta ttttcttgca gaagttgttg gactatcaac aatactctta 2460
   gcaacatatc tgaaagtaaa aacaccgcaa tgtgatgcta tattaagata acactaatgt 2520
   acttgtagaa ttatctgaaa cctgactcac cgattctgta ggtttgtggt tccttcttaa 2580
   gagtgaaaat ttcctgaccc tttggtttat cagagctgca taagctatag acagcagaat 2640
   aaaatggtaa attcatgagg tcttagtttt caatactcgt tgacgttgtg cttaaatcct 2700
   tcaaaaatgt ctcgaaccaa agaccaaaca cgtctgattc ttcaatcaaa gaaagtgaaa 2760
   acaaaattga ataatcgaga tcgaagagag acaaaccaat cagcttctgg attcatgcaa 2820
   tagttcctga attgattgat ggaaattttt ggagatcttc cttcagccag atctacaacg 2880
   accgattgag taaaatctgc tcaaaaaacc gaaacatagt gatgaacaac ggaagagcca 2940
   gcgaagtcat cgactagaaa tttcgacata gattagagga caaattactc aatacgattc 3000
   ggaaaatttt gggattttct gatacctttg atcctttgaa gcaaattagt cgattctgaa 3060
   atagcgaatt ttccctccat ctctttcgag tttcaaaact gaaaaatgca atggtcgacg 3120
   aatttttgta gacgggaaga aaagggatgg gtgcgaaaag cttgccgtta aaaccttcga 3180
   atttaaacat gcgtgtcgtt tagttcctac tttccatgac gttgggccat tggcatttag 3240
   gcccgatacg taactgacgt gtcattttaa aaacgcatgt gtttttaaac acgttgggcc 3300
   gttggctttt aggcccgatc cgaaactagg caaagtgatt gtgaaaaaga ttcatgataa 3360
   caatcaaacc aatataaatc gatttattca gacttggaga cattttagtg atgccaatat 3420
   aaaataatca tgtgcactaa atttgaaata attaatgtgt atggtaaact agattagaat 3480
   tagttctaga cttctagtgg tcgagatttg ttcataagct ttatgctcgg acacgtgagg 3540
   aacataccaa aatgttcgaa tctattatgc catacactta ttttaatcat ttcttctcta 3600
   tgatagattg ctcacctaat ttatatataa tctaagaata tactacttag ctttaacgta 3660
   ttttatccga taacgaattc atgataccaa aaaagttcta aaacaaaaga ggcatcttgt 3720
   attatttata gtatttctaa ctaaaacaca acactgataa catttaaatt gctttgttcc 3780
   gtttaattag taaacaaatg aaaagctgtc gcgtcggtgg ttgggtttca ttcggaaaga 3840
   tttaaaagta aagcgcacgg attttaaggt cgttcaacat ttaaattttt tttgaaagta 3900
   aaatcatata tcaatgcaat gtgtaatctt gtaacgtata aactaatgag agatacctaa 3960
   agaaaagaag cctcgcgcct aaacagaaac ataagattaa aataagtaat atagaagcat 4020
   aaacttaagc aagattttat tcctcgtgaa aatattacat tttgtaattt tattttctct 4080
   agtattaaaa aaggaacaac taattaacgg aaatacttaa ggaattaaga acagagaggt 4140
   ctcatactta gtcaagtttg gtgaagctat caaagagaat gtcttcgtgt tgtccgccga 4200
   cgacattctg cagatcagtc cactaga 4227

<210> 69
<211> 4948
<212> DNA
<213> Artificial Sequence

<220>
<221> source
<222> 1..4948
<223> /mol_type="unassigned DNA"
/note="Fragment of Plasmid pCB302/AtSPO11-1-18xMyc"
/organism="Artificial Sequence"

<400> 69
   ggatccgctg aagctgaagt tgccacgaag gagaaagcga gagagagacg acatcgtttt 60
   tagctgtatg caaaggtttt tctcttcttt tttttggatc ggagtgttca aaggttttta 120
   gtcaggggaa cggctttaat acttcaaatt aggtttggta acaatataaa gcaaatgaac 180
   cctaaattac ttttgtatta ttcaaaattt caaacttatt actcattagt cattagtgaa 240
   tgtactttgt attattaaaa attaaaaact gagaattatt ttttataatc tgtatattta 300
   tgatttttcc attaaaacta tgttcatttg tccattctac caatgcattt ggtttgcact 360
   aaaaagctta gaaagagtca taaaaaaaac aatgagcatt accttgagtt ggagactagt 420
   tatctcgtat gaagcagatc ttcacccatc tccgttcaag tcttcttctg agattaattt 480
   ttgttcaccg ttcaagtctt cctcggagat tagcttttgt tcaccgttca aatcttcttc 540
   agaaatcaac ttttgttcac ctctagagga tccgttcaag tcttcttctg agattaattt 600
   ttgttcaccg ttcaagtctt cttctgagat taatttttgt tcaccgttca aatcttcttc 660
   agaaatcaac ttttgttcac cactagtgga tccgttcaag tcttcttctg agattaattt 720
   ttgttcaccg ttcaagtctt cctcggagat tagcttttgt tcaccgttca aatcttcttc 780
   agaaatcaac ttttgttcac cactagtgga tccgttcaag tcttcttctg agattaattt 840
   ttgttcaccg ttcaagtctt cctcggagat tagcttttgt tcaccgttca aatcttcttc 900
   agaaatcaac ttttgttcac ctctagagga tccgttcaag tcttcttctg agattaattt 960
   ttgttcaccg ttcaagtctt cctcggagat tagcttttgt tcaccgttca aatcttcttc 1020
   agaaatcaac ttttgttcac cactagtgga tccgttcaag tcttcttctg agattaattt 1080
   ttgttcaccg ttcaagtctt cctcggagat tagcttttgt tcaccgttca aatcttcttc 1140
   agaaatcaac ttttgttcac cactagcagc agaaccggac ccgggaggag agcttacttc 1200
   acgacgaatc ttagaaggaa tatatacctc tgacaaaaag gacagtgagt gaactgagag 1260
   tgcttcgatc tcaaacttga cccctctttt caacatcgtt tcaagttcca accttagacg 1320
   accaaaatcg ttttgatcaa gactagtttt tgtcatttgg ttgaaatgta aatataaaaa 1380
   agtcagggaa gtttttcata cctccattgt ggcatttcac gcttcaggta gcatctaagc 1440
   aacattgctt cagttctttt cttatctgtc cggggaagtt gttacaatct caaaaaagga 1500
   agcagtgaaa aataggaaaa tctaaagatt taaggcgata aggtgtttaa tgatctctta 1560
   ccttcttctg tcagtggcaa aagacactgt ttgggaacac tatatacctc agagtctgaa 1620
   ggaaaagctc ccaaccattt catatctggt gctcgtaatg attcaatatc ataggccatt 1680
   tgcttacaga aagaaaacaa gtgacataag gaaaatggtt caaaatgaaa tgggtcagtg 1740
   ccatatcaca ttatcactat caacaaaaac aatgcaagcg gtgaaatttt ccgagttcag 1800
   tcatctatat tttgtaacag aatctaacca tggagccaaa acggtatgtg gctaggatct 1860
   caaagccata tggatcacag tcgactagac aatgcacagg tagatgcaac ttctccatca 1920
   ggagtcgcaa gaacctggaa agaatctgtg ttattcgctg atatattaat caatagaaga 1980
   taatgtctga taaaatagta ttccaggaac ctgggattaa gttatgtacc ttcttgttga 2040
   gacatctgga tatcctcttc cctgccaaaa acaatcgtaa ctttgatgtc aaacataaaa 2100
   tggaaatgaa aactagtcaa aactgaaatc agtaagaatg aagcagattt cttactgtga 2160
   taacaatgca gcggttcgtc ttgcaaaaca tgtcatttgc taaacgctgg aatactataa 2220
   accatggatg aaagcgattt agtcatgaag aaagaaaacc atcttcctag ttactacata 2280
   aataagaggt ctttcttacc tgtttctttt tccacaacca gtatgtactc agctaagcta 2340
   acaatatctg caagcaggaa aatgacatcc aattatagtt cctgatgcca ccatagatag 2400
   tgaatcttat acttcctgat atcagctgag ataaggattt gacttctcta aagagcataa 2460
   agcatcactt aaaatgattg caaatatatg taccttcgac ttcctctaca agaacaggaa 2520
   cgggatatgc ctgaaatgaa atcacttgca gagttatact tcctgatgcc aatataaaaa 2580
   gtgaatctta tgttcaaatt cttacagtgt tcaagctgtt taaacaatca aatttccttc 2640
   cagcttccct aaattttaac caacccatca ccaacctaca acacaaaaga ataatgaggt 2700
   ggatcttaaa ataaggacat gcctcaagat agaagcattg cactcgttat caagagcacc 2760
   cacccgtttc caacagaaac ctgcaaaata tcacatgaat gaaaaagtaa tcacatcttc 2820
   aatcgcagct tataataact cttcagaagg aaaattgaag gtgaaagtat taaagtacta 2880
   accacattca agttgtaccg actacactga aaaagaatgc atatatcacc aattgcacgg 2940
   tccacaatgg attgtgctgt gaagtagacg aaaacagtgt tactagaagg ccaagaacac 3000
   aacaaagagg gatgcataac taatatccgt ataaaaatct caatacgtat tcgtttagaa 3060
   gaaaaacata actaaaacat tatacgttgc ttccaaattc acctttgaaa gctgatggat 3120
   gcatgtagta gatatctctt ttcgaagcat gtctattttc ttgcagaagt tgttggacta 3180
   tcaacaatac tcttagcaac atatctgaaa gtaaaaacac cgcaatgtga tgctatatta 3240
   agataacact aatgtacttg tagaattatc tgaaacctga ctcaccgatt ctgtaggttt 3300
   gtggttcctt cttaagagtg aaaatttcct gaccctttgg tttatcagag ctgcataagc 3360
   tatagacagc agaataaaat ggtaaattca tgaggtctta gttttcaata ctcgttgacg 3420
   ttgtgcttaa atccttcaaa aatgtctcga accaaagacc aaacacgtct gattcttcaa 3480
   tcaaagaaag tgaaaacaaa attgaataat cgagatcgaa gagagacaaa ccaatcagct 3540
   tctggattca tgcaatagtt cctgaattga ttgatggaaa tttttggaga tcttccttca 3600
   gccagatcta caacgaccga ttgagtaaaa tctgctcaaa aaaccgaaac atagtgatga 3660
   acaacggaag agccagcgaa gtcatcgact agaaatttcg acatagatta gaggacaaat 3720
   tactcaatac gattcggaaa attttgggat tttctgatac ctttgatcct ttgaagcaaa 3780
   ttagtcgatt ctgaaatagc gaattttccc tccatctctt tcgagtttca aaactgaaaa 3840
   atgcaatggt cgacgaattt ttgtagacgg gaagaaaagg gatgggtgcg aaaagcttgc 3900
   cgttaaaacc ttcgaattta aacatgcgtg tcgtttagtt cctactttcc atgacgttgg 3960
   gccattggca tttaggcccg atacgtaact gacgtgtcat tttaaaaacg catgtgtttt 4020
   taaacacgtt gggccgttgg cttttaggcc cgatccgaaa ctaggcaaag tgattgtgaa 4080
   aaagattcat gataacaatc aaaccaatat aaatcgattt attcagactt ggagacattt 4140
   tagtgatgcc aatataaaat aatcatgtgc actaaatttg aaataattaa tgtgtatggt 4200
   aaactagatt agaattagtt ctagacttct agtggtcgag atttgttcat aagctttatg 4260
   ctcggacacg tgaggaacat accaaaatgt tcgaatctat tatgccatac acttatttta 4320
   atcatttctt ctctatgata gattgctcac ctaatttata tataatctaa gaatatacta 4380
   cttagcttta acgtatttta tccgataacg aattcatgat accaaaaaag ttctaaaaca 4440
   aaagaggcat cttgtattat ttatagtatt tctaactaaa acacaacact gataacattt 4500
   aaattgcttt gttccgttta attagtaaac aaatgaaaag ctgtcgcgtc ggtggttggg 4560
   tttcattcgg aaagatttaa aagtaaagcg cacggatttt aaggtcgttc aacatttaaa 4620
   ttttttttga aagtaaaatc atatatcaat gcaatgtgta atcttgtaac gtataaacta 4680
   atgagagata cctaaagaaa agaagcctcg cgcctaaaca gaaacataag attaaaataa 4740
   gtaatataga agcataaact taagcaagat tttattcctc gtgaaaatat tacattttgt 4800
   aattttattt tctctagtat taaaaaagga acaactaatt aacggaaata cttaaggaat 4860
   taagaacaga gaggtctcat acttagtcaa gtttggtgaa gctatcaaag agaatgtctt 4920
   cgtgttgtcc gccgacgaca ttctgcag 4948

<210> 70
<211> 4405
<212> DNA
<213> Artificial Sequence

<220>
<221> source
<222> 1..4405
<223> /mol_type="unassigned DNA"
 /note="Plasmid pCR2.1/GAL4BD"
 /organism="Artificial Sequence"

<400> 70
agcgcccaat acgcaaaccg cctctccccg cgcgttggcc gattcattaa tgcagctggc 60

acgacaggtt tcccgactgg aaagcgggca gtgagcgcaa cgcaattaat gtgagttagc 120

tcactcatta ggcaccccag gctttacact ttatgcttcc ggctcgtatg ttgtgtggaa 180

ttgtgagcgg ataacaattt cacacaggaa acagctatga ccatgattac gccaagcttg 240

gtaccgagct cggatccact agtaacggcc gccagtgtgc tggaattcgc ccttatggat 300

ccttagatat ccgagacggt caactggcgc tggcccttgt tgctgctctc ctccgaggag 360

gaggtcgcgc tgatgcggtg ctggcgcagg gtgaggggca tgtcggtctc cacggaggcc 420

aggcggtcgg tgacggcgtc tttgttcacg ttgtcctgga cgaagaggcc ggtgagcagg 480

gctttgatgt cctggaggga gtccattttc aggatcatgt cgaggtcctc tcgagggaag 540

atcaggagga agagctgctc caggcgctcc aggcgggact ccacttcggt gaggtgggcg 600
cgggtcagcg gggagcgctt ggttttggga gagtagcgac actcccagtt gttcttcaga 660
cacttggcgc acttcggctt ctccttggag cacttgagct tcttgaggcg gcagatgtcg 720
caggcctgct cgatggagga caggagcttc atcccgggag ccatggataa gggcgaattc 780
tgcagatatc catcacactg gcggccgctc gagcatgcat ctagagggcc caattcgccc 840
tatagtgagt cgtattacaa ttcactggcc gtcgttttac aacgtcgtga ctgggaaaac 900
cctggcgtta cccaacttaa tcgccttgca gcacatcccc ctttcgccag ctggcgtaat 960
agcgaagagg cccgcaccga tcgcccttcc caacagttgc gcagcctgaa tggcgaatgg 1020
acgcgccctg tagcggcgca ttaagcgcgg cgggtgtggt ggttacgcgc agcgtgaccg 1080
ctacacttgc cagcgcccta gcgcccgctc ctttcgcttt cttcccttcc tttctcgcca 1140
cgttcgccgg ctttccccgt caagctctaa atcgggggct ccctttaggg ttccgattta 1200
gtgctttacg gcacctcgac cccaaaaaac ttgattaggg tgatggttca cgtagtgggc 1260
catcgccctg atagacggtt tttcgccctt tgacgttgga gtccacgttc tttaatagtg 1320
gactcttgtt ccaaactgga acaacactca accctatctc ggtctattct tttgatttat 1380
aagggatttt gccgatttcg gcctattggt taaaaaatga gctgatttaa caaaaattta 1440
acgcgaattt taacaaaatt cagggcgcaa gggctgctaa aggaagcgga acacgtagaa 1500
agccagtccg cagaaacggt gctgaccccg gatgaatgtc agctactggg ctatctggac 1560
aagggaaaac gcaagcgcaa agagaaagca ggtagcttgc agtgggctta catggcgata 1620
gctagactgg gcggttttat ggacagcaag cgaaccggaa ttgccagctg gggcgccctc 1680
tggtaaggtt gggaagccct gcaaagtaaa ctggatggct ttcttgccgc caaggatctg 1740
atggcgcagg ggatcaagat ctgatcaaga gacaggatga ggatcgtttc gcatgattga 1800
acaagatgga ttgcacgcag gttctccggc cgcttgggtg gagaggctat tcggctatga 1860
ctgggcacaa cagacaatcg gctgctctga tgccgccgtg ttccggctgt cagcgcaggg 1920
gcgcccggtt ctttttgtca agaccgacct gtccggtgcc ctgaatgaac tgcaggacga 1980
ggcagcgcgg ctatcgtggc tggccacgac gggcgttcct tgcgcagctg tgctcgacgt 2040
tgtcactgaa gcgggaaggg actggctgct attgggcgaa gtgccggggc aggatctcct 2100
gtcatcccac cttgctcctg ccgagaaagt atccatcatg gctgatgcaa tgcggcggct 2160
gcatacgctt gatccggcta cctgcccatt cgaccaccaa gcgaaacatc gcatcgagcg 2220
agcacgtact cggatggaag ccggtcttgt cgatcaggat gatctggacg aagagcatca 2280
ggggctcgcg ccagccgaac tgttcgccag gctcaaggcg cgcatgcccg acggcgagga 2340
tctcgtcgtg acccatggcg atgcctgctt gccgaatatc atggtggaaa atggccgctt 2400
ttctggattc atcgactgtg gccggctggg tgtggcggac cgctatcagg acatagcgtt 2460

ggctacccgt gatattgctg aagagcttgg cggcgaatgg gctgaccgct tcctcgtgct 2520

ttacggtatc gccgctcccg attcgcagcg catcgccttc tatcgccttc ttgacgagtt 2580

cttctgaatt gaaaaaggaa gagtatgagt attcaacatt tccgtgtcgc ccttattccc 2640

ttttttgcgg cattttgcct tcctgttttt gctcacccag aaacgctggt gaaagtaaaa 2700

gatgctgaag atcagttggg tgcacgagtg ggttacatcg aactggatct caacagcggt 2760

aagatccttg agagttttcg ccccgaagaa cgttttccaa tgatgagcac ttttaaagtt 2820

ctgctatgtg gcgcggtatt atcccgtatt gacgccgggc aagagcaact cggtcgccgc 2880

atacactatt ctcagaatga cttggttgag tactcaccag tcacagaaaa gcatcttacg 2940

gatggcatga cagtaagaga attatgcagt gctgccataa ccatgagtga taacactgcg 3000

gccaacttac ttctgacaac gatcggagga ccgaaggagc taaccgcttt tttgcacaac 3060

atgggggatc atgtaactcg ccttgatcgt tgggaaccgg agctgaatga agccatacca 3120

aacgacgagc gtgacaccac gatgcctgta gcaatggcaa caacgttgcg caaactatta 3180

actggcgaac tacttactct agcttcccgg caacaattaa tagactggat ggaggcggat 3240

aaagttgcag gaccacttct gcgctcggcc cttccggctg gctggtttat tgctgataaa 3300

tctggagccg gtgagcgtgg gtctcgcggt atcattgcag cactggggcc agatggtaag 3360

ccctcccgta tcgtagttat ctacacgacg gggagtcagg caactatgga tgaacgaaat 3420

agacagatcg ctgagatagg tgcctcactg attaagcatt ggtaactgtc agaccaagtt 3480

tactcatata tactttagat tgatttaaaa cttcattttt aatttaaaag gatctaggtg 3540

aagatccttt ttgataatct catgaccaaa atcccttaac gtgagttttc gttccactga 3600

gcgtcagacc ccgtagaaaa gatcaaagga tcttcttgag atcctttttt tctgcgcgta 3660

atctgctgct tgcaaacaaa aaaaccaccg ctaccagcgg tggtttgttt gccggatcaa 3720

gagctaccaa ctctttttcc gaaggtaact ggcttcagca gagcgcagat accaaatact 3780

gttcttctag tgtagccgta gttaggccac cacttcaaga actctgtagc accgcctaca 3840

tacctcgctc tgctaatcct gttaccagtg gctgctgcca gtggcgataa gtcgtgtctt 3900

accgggttgg actcaagacg atagttaccg gataaggcgc agcggtcggg ctgaacgggg 3960

ggttcgtgca cacagcccag cttggagcga acgacctaca ccgaactgag atacctacag 4020

cgtgagctat gagaaagcgc cacgcttccc gaagggagaa aggcggacag gtatccggta 4080

agcggcaggg tcggaacagg agagcgcacg agggagcttc cagggggaaa cgcctggtat 4140

ctttatagtc ctgtcgggtt tcgccacctc tgacttgagc gtcgattttt gtgatgctcg 4200

tcaggggggc ggagcctatg gaaaaacgcc agcaacgcgg cctttttacg gttcctggcc 4260

ttttgctggc cttttgctca catgttcttt cctgcgttat cccctgattc tgtggataac 4320

cgtattaccg cctttgagtg agctgatacc gctcgccgca gccgaacgac cgagcgcagc 4380

gagtcagtga gcgaggaagc ggaag 4405

<210> 71
<211> 4660
<212> DNA
<213> Artificial Sequence

<220>
<221> source
<222> 1..4660
<223> /mol_type="unassigned DNA"
/note="Fragment of Plasmid pCB302/AtSPO11-1-GAL4BD"
/organism="Artificial Sequence"

<400> 71
ggatccgctg aagctgaagt tgccacgaag gagaaagcga gagagagacg acatcgtttt 60

tagctgtatg caaaggtttt tctcttcttt tttttggatc ggagtgttca aaggttttta 120

gtcaggggaa cggctttaat acttcaaatt aggtttggta acaatataaa gcaaatgaac 180

cctaaattac ttttgtatta ttcaaaattt caaacttatt actcattagt cattagtgaa 240

tgtactttgt attattaaaa attaaaaact gagaattatt ttttataatc tgtatattta 300

tgatttttcc attaaaacta tgttcatttg tccattctac caatgcattt ggtttgcact 360

aaaaagctta gaaagagtca taaaaaaaac aatgagcatt accttgagtt ggagactagt 420

tatctcgtat gaagcagatc ttcacccatc cgagacggtc aactggcgct ggcccttgtt 480

gctgctctcc tccgaggagg aggtcgcgct gatgcggtgc tggcgcaggg tgaggggcat 540

gtcggtctcc acggaggcca ggcggtcggt gacggcgtct ttgttcacgt tgtcctggac 600

gaagaggccg gtgagcaggg ctttgatgtc ctggagggag tccattttca ggatcatgtc 660

gaggtcctct cgagggaaga tcaggaggaa gagctgctcc aggcgctcca ggcgggactc 720

cacttcggtg aggtgggcgc gggtcagcgg ggagcgcttg gttttgggag agtagcgaca 780

ctcccagttg ttcttcagac acttggcgca cttcggcttc tccttggagc acttgagctt 840

cttgaggcgg cagatgtcgc aggcctgctc gatggaggac aggagcttca tcccgggagg 900

agagcttact tcacgacgaa tcttagaagg aatatatacc tctgacaaaa aggacagtga 960

gtgaactgag agtgcttcga tctcaaactt gacccctctt ttcaacatcg tttcaagttc 1020

caaccttaga cgaccaaaat cgttttgatc aagactagtt tttgtcattt ggttgaaatg 1080

taaatataaa aaagtcaggg aagtttttca tacctccatt gtggcatttc acgcttcagg 1140

tagcatctaa gcaacattgc ttcagttctt ttcttatctg tccggggaag ttgttacaat 1200

ctcaaaaaag gaagcagtga aaaataggaa aatctaaaga tttaaggcga taaggtgttt 1260

aatgatctct taccttcttc tgtcagtggc aaaagacact gtttgggaac actatatacc 1320

tcagagtctg aaggaaaagc tcccaaccat ttcatatctg gtgctcgtaa tgattcaata 1380

tcataggcca tttgcttaca gaaagaaaac aagtgacata aggaaaatgg ttcaaaatga 1440

aatgggtcag tgccatatca cattatcact atcaacaaaa acaatgcaag cggtgaaatt 1500

ttccgagttc agtcatctat attttgtaac agaatctaac catggagcca aaacggtatg 1560

tggctaggat ctcaaagcca tatggatcac agtcgactag acaatgcaca ggtagatgca 1620

acttctccat caggagtcgc aagaacctgg aaagaatctg tgttattcgc tgatatatta 1680

atcaatagaa gataatgtct gataaaatag tattccagga acctgggatt aagttatgta 1740

ccttcttgtt gagacatctg gatatcctct tccctgccaa aaacaatcgt aactttgatg 1800

tcaaacataa aatggaaatg aaaactagtc aaaactgaaa tcagtaagaa tgaagcagat 1860

ttcttactgt gataacaatg cagcggttcg tcttgcaaaa catgtcattt gctaaacgct 1920

ggaatactat aaaccatgga tgaaagcgat ttagtcatga agaaagaaaa ccatcttcct 1980

agttactaca taaataagag gtctttctta cctgtttctt tttccacaac cagtatgtac 2040

tcagctaagc taacaatatc tgcaagcagg aaaatgacat ccaattatag ttcctgatgc 2100

caccatagat agtgaatctt atacttcctg atatcagctg agataaggat ttgacttctc 2160

taaagagcat aaagcatcac ttaaaatgat tgcaaatata tgtaccttcg acttcctcta 2220

caagaacagg aacgggatat gcctgaaatg aaatcacttg cagagttata cttcctgatg 2280

ccaatataaa aagtgaatct tatgttcaaa ttcttacagt gttcaagctg tttaaacaat 2340

caaatttcct tccagcttcc ctaaatttta accaacccat caccaaccta caacacaaaa 2400

gaataatgag gtggatctta aaataaggac atgcctcaag atagaagcat tgcactcgtt 2460

atcaagagca cccacccgtt tccaacagaa acctgcaaaa tatcacatga atgaaaaagt 2520

aatcacatct tcaatcgcag cttataataa ctcttcagaa ggaaaattga aggtgaaagt 2580

attaaagtac taaccacatt caagttgtac cgactacact gaaaaagaat gcatatatca 2640

ccaattgcac ggtccacaat ggattgtgct gtgaagtaga cgaaaacagt gttactagaa 2700

ggccaagaac acaacaaaga gggatgcata actaatatcc gtataaaaat ctcaatacgt 2760

attcgtttag aagaaaaaca taactaaaac attatacgtt gcttccaaat tcacctttga 2820

aagctgatgg atgcatgtag tagatatctc ttttcgaagc atgtctattt tcttgcagaa 2880

gttgttggac tatcaacaat actcttagca acatatctga aagtaaaaac accgcaatgt 2940

gatgctatat taagataaca ctaatgtact tgtagaatta tctgaaacct gactcaccga 3000

ttctgtaggt ttgtggttcc ttcttaagag tgaaaatttc ctgacccttt ggtttatcag 3060

agctgcataa gctatagaca gcagaataaa atggtaaatt catgaggtct tagttttcaa 3120

tactcgttga cgttgtgctt aaatccttca aaaatgtctc gaaccaaaga ccaaacacgt 3180

ctgattcttc aatcaaagaa agtgaaaaca aaattgaata atcgagatcg aagagagaca 3240

aaccaatcag cttctggatt catgcaatag ttcctgaatt gattgatgga aatttttgga 3300

gatcttcctt cagccagatc tacaacgacc gattgagtaa aatctgctca aaaaaccgaa 3360

acatagtgat gaacaacgga agagccagcg aagtcatcga ctagaaattt cgacatagat 3420

tagaggacaa attactcaat acgattcgga aaattttggg attttctgat acctttgatc 3480

ctttgaagca aattagtcga ttctgaaata gcgaattttc cctccatctc tttcgagttt 3540

caaaactgaa aaatgcaatg gtcgacgaat ttttgtagac gggaagaaaa gggatgggtg 3600

cgaaaagctt gccgttaaaa ccttcgaatt taaacatgcg tgtcgtttag ttcctacttt 3660

ccatgacgtt gggccattgg catttaggcc cgatacgtaa ctgacgtgtc attttaaaaa 3720

cgcatgtgtt tttaaacacg ttgggccgtt ggcttttagg cccgatccga aactaggcaa 3780

agtgattgtg aaaaagattc atgataacaa tcaaaccaat ataaatcgat ttattcagac 3840

ttggagacat tttagtgatg ccaatataaa ataatcatgt gcactaaatt tgaaataatt 3900

aatgtgtatg gtaaactaga ttagaattag ttctagactt ctagtggtcg agatttgttc 3960

ataagcttta tgctcggaca cgtgaggaac ataccaaaat gttcgaatct attatgccat 4020

acacttattt taatcatttc ttctctatga tagattgctc acctaattta tatataatct 4080

aagaatatac tacttagctt taacgtattt tatccgataa cgaattcatg ataccaaaaa 4140

agttctaaaa caaaagaggc atcttgtatt atttatagta tttctaacta aaacacaaca 4200

ctgataacat ttaaattgct ttgttccgtt taattagtaa acaaatgaaa agctgtcgcg 4260

tcggtggttg ggtttcattc ggaaagattt aaaagtaaag cgcacggatt ttaaggtcgt 4320

tcaacattta aatttttttt gaaagtaaaa tcatatatca atgcaatgtg taatcttgta 4380

acgtataaac taatgagaga tacctaaaga aaagaagcct cgcgcctaaa cagaaacata 4440

agattaaaat aagtaatata gaagcataaa cttaagcaag attttattcc tcgtgaaaat 4500

attacatttt gtaattttat tttctctagt attaaaaaag gaacaactaa ttaacggaaa 4560

tacttaagga attaagaaca gagaggtctc atacttagtc aagtttggtg aagctatcaa 4620

agagaatgtc ttcgtgttgt ccgccgacga cattctgcag 4660

<210> 72
<211> 4438
<212> DNA
<213> Artificial Sequence

<220>
<221> source
<222> 1..4438
<223> /mol_type="unassigned DNA"
/note="Plasmid pCR2.1/5xUAS"
/organism="Artificial Sequence"

<400> 72
ggtaccgatt acgccaagct tgcatgcctg caggtcggag tactgtcctc cgagcggagt 60

actgtcctcc gagcggagta ctgtcctccg agcggagtac tgtcctccga gcggagtact 120

gtcctccgag cggagactct agaagctact ccacgtccat aagggacaca tcacaatccc 180

actatccttc gcaagaccct tcctctatat aaggaagttc atttcatttg gagaggacga 240

cctgcaggtc gacggatcca aggagatata acaatgaaga ctaatctttt tctctttctc 300

atcttttcac ttctcctatc attatcctcg gccgaattca gtaaaggaga agaacttttc 360

actggagttg tcccaattct tgttgaatta gatggtgatg ttaatgggca caaattttct 420

gtcagtggag agggtgaagg tgatgcaaca tacggaaaac ttacccttaa atttatttgc 480

actactggaa aactacctgt tccatggaag ggcgaattct gcagatatcc atcacactgg 540

cggccgctcg agcatgcatc tagagggccc aattcgccct atagtgagtc gtattacaat 600

tcactggccg tcgttttaca acgtcgtgac tgggaaaacc ctggcgttac ccaacttaat 660

cgccttgcag cacatccccc tttcgccagc tggcgtaata gcgaagaggc ccgcaccgat 720

cgcccttccc aacagttgcg cagcctgaat ggcgaatgga cgcgccctgt agcggcgcat 780

taagcgcggc gggtgtggtg gttacgcgca gcgtgaccgc tacacttgcc agcgccctag 840

cgcccgctcc tttcgctttc ttcccttcct ttctcgccac gttcgccggc tttccccgtc 900

aagctctaaa tcgggggctc cctttagggt tccgatttag tgctttacgg cacctcgacc 960

ccaaaaaact tgattagggt gatggttcac gtagtgggcc atcgccctga tagacggttt 1020

ttcgcccttt gacgttggag tccacgttct ttaatagtgg actcttgttc caaactggaa 1080

caacactcaa ccctatctcg gtctattctt ttgatttata agggattttg ccgatttcgg 1140

cctattggtt aaaaaatgag ctgatttaac aaaaatttaa cgcgaatttt aacaaaattc 1200

agggcgcaag ggctgctaaa ggaagcggaa cacgtagaaa gccagtccgc agaaacggtg 1260

ctgaccccgg atgaatgtca gctactgggc tatctggaca agggaaaacg caagcgcaaa 1320

gagaaagcag gtagcttgca gtgggcttac atggcgatag ctagactggg cggttttatg 1380

gacagcaagc gaaccggaat tgccagctgg ggcgccctct ggtaaggttg ggaagccctg 1440

caaagtaaac tggatggctt tcttgccgcc aaggatctga tggcgcaggg gatcaagatc 1500

tgatcaagag acaggatgag gatcgtttcg catgattgaa caagatggat tgcacgcagg 1560

ttctccggcc gcttgggtgg agaggctatt cggctatgac tgggcacaac agacaatcgg 1620

ctgctctgat gccgccgtgt tccggctgtc agcgcagggg cgcccggttc tttttgtcaa 1680

gaccgacctg tccggtgccc tgaatgaact gcaggacgag gcagcgcggc tatcgtggct 1740

ggccacgacg ggcgttcctt gcgcagctgt gctcgacgtt gtcactgaag cgggaaggga 1800

ctggctgcta ttgggcgaag tgccggggca ggatctcctg tcatcccacc ttgctcctgc 1860

cgagaaagta tccatcatgg ctgatgcaat gcggcggctg catacgcttg atccggctac 1920

ctgcccattc gaccaccaag cgaaacatcg catcgagcga gcacgtactc ggatggaagc 1980

cggtcttgtc gatcaggatg atctggacga agagcatcag gggctcgcgc cagccgaact 2040

gttcgccagg ctcaaggcgc gcatgcccga cggcgaggat ctcgtcgtga cccatggcga 2100

tgcctgcttg ccgaatatca tggtggaaaa tggccgcttt tctggattca tcgactgtgg 2160

ccggctgggt gtggcggacc gctatcagga catagcgttg gctacccgtg atattgctga 2220

agagcttggc ggcgaatggg ctgaccgctt cctcgtgctt tacggtatcg ccgctcccga 2280

ttcgcagcgc atcgccttct atcgccttct tgacgagttc ttctgaattg aaaaaggaag 2340

agtatgagta ttcaacattt ccgtgtcgcc cttattccct tttttgcggc attttgcctt 2400

cctgtttttg ctcacccaga aacgctggtg aaagtaaaag atgctgaaga tcagttgggt 2460

gcacgagtgg gttacatcga actggatctc aacagcggta agatccttga gagttttcgc 2520

cccgaagaac gttttccaat gatgagcact tttaaagttc tgctatgtgg cgcggtatta 2580

tcccgtattg acgccgggca agagcaactc ggtcgccgca tacactattc tcagaatgac 2640

ttggttgagt actcaccagt cacagaaaag catcttacgg atggcatgac agtaagagaa 2700

ttatgcagtg ctgccataac catgagtgat aacactgcgg ccaacttact tctgacaacg 2760

atcggaggac cgaaggagct aaccgctttt ttgcacaaca tgggggatca tgtaactcgc 2820

cttgatcgtt gggaaccgga gctgaatgaa gccataccaa acgacgagcg tgacaccacg 2880

atgcctgtag caatggcaac aacgttgcgc aaactattaa ctggcgaact acttactcta 2940

gcttcccggc aacaattaat agactggatg gaggcggata aagttgcagg accacttctg 3000

cgctcggccc ttccggctgg ctggtttatt gctgataaat ctggagccgg tgagcgtggg 3060

tctcgcggta tcattgcagc actggggcca gatggtaagc cctcccgtat cgtagttatc 3120

tacacgacgg ggagtcaggc aactatggat gaacgaaata gacagatcgc tgagataggt 3180

gcctcactga ttaagcattg gtaactgtca gaccaagttt actcatatat actttagatt 3240

gatttaaaac ttcattttta atttaaaagg atctaggtga agatcctttt tgataatctc 3300

atgaccaaaa tcccttaacg tgagttttcg ttccactgag cgtcagaccc cgtagaaaag 3360

atcaaaggat cttcttgaga tccttttttt ctgcgcgtaa tctgctgctt gcaaacaaaa 3420

aaaccaccgc taccagcggt ggtttgtttg ccggatcaag agctaccaac tctttttccg 3480

aaggtaactg gcttcagcag agcgcagata ccaaatactg ttcttctagt gtagccgtag 3540

ttaggccacc acttcaagaa ctctgtagca ccgcctacat acctcgctct gctaatcctg 3600

ttaccagtgg ctgctgccag tggcgataag tcgtgtctta ccgggttgga ctcaagacga 3660

tagttaccgg ataaggcgca gcggtcgggc tgaacggggg gttcgtgcac acagcccagc 3720

ttggagcgaa cgacctacac cgaactgaga tacctacagc gtgagctatg agaaagcgcc 3780

acgcttcccg aagggagaaa ggcggacagg tatccggtaa gcggcagggt cggaacagga 3840

gagcgcacga gggagcttcc agggggaaac gcctggtatc tttatagtcc tgtcgggttt 3900

cgccacctct gacttgagcg tcgatttttg tgatgctcgt caggggggcg gagcctatgg 3960

aaaaacgcca gcaacgcggc ctttttacgg ttcctggcct tttgctggcc ttttgctcac 4020

atgttctttc ctgcgttatc ccctgattct gtggataacc gtattaccgc ctttgagtga 4080

gctgataccg ctcgccgcag ccgaacgacc gagcgcagcg agtcagtgag cgaggaagcg 4140

gaagagcgcc caatacgcaa accgcctctc cccgcgcgtt ggccgattca ttaatgcagc 4200

tggcacgaca ggtttcccga ctggaaagcg ggcagtgagc gcaacgcaat taatgtgagt 4260

tagctcactc attaggcacc ccaggcttta cactttatgc ttccggctcg tatgttgtgt 4320

ggaattgtga gcggataaca atttcacaca ggaaacagct atgaccatga ttacgccaag 4380

cttggtaccg agctcggatc cactagtaac ggccgccagt gtgctggaat tcgccctt 4438

<210> 73
<211> 5444
<212> DNA
<213> Artificial Sequence

<220>
<221> source
<222> 1..5444
<223> /mol_type="unassigned DNA"
      /note="Plasmid pGreenII-0179/5xUAS-long"
      /organism="Artificial Sequence"

<400> 73
tttttatccc cggaagcctg tggatagagg gtagttatcc acgtgaaacc gctaatgccc 60

cgcaaagcct tgattcacgg ggctttccgg cccgctccaa aaactatcca cgtgaaatcg 120

ctaatcaggg tacgtgaaat cgctaatcgg agtacgtgaa atcgctaata aggtcacgtg 180

aaatcgctaa tcaaaaaggc acgtgagaac gctaatagcc ctttcagatc aacagcttgc 240

aaacacccct cgctccggca agtagttaca gcaagtagta tgttcaatta gcttttcaat 300

tatgaatata tatatcaatt attggtcgcc cttggcttgt ggacaatgcg ctacgcgcac 360

cggctccgcc cgtggacaac cgcaagcggt tgcccaccgt cgagcgccag cgcctttgcc 420

cacaacccgg cggccggccg caacagatcg ttttataaat tttttttttt gaaaaagaaa 480

aagcccgaaa ggcggcaacc tctcgggctt ctggatttcc gatccccgga attagagatc 540

ttggcaggat atattgtggt gtaacgttat cgatctggat tttagtactg gattttggtt 600

ttaggaatta gaaattttat tgatagaagt attttacaaa tacaaataca tactaagggt 660

ttcttatatg ctcaacacat gagcgaaacc ctataagaac cctaatttcc cttatcggga 720

aactactcac acattattta tggagaaaat agagagagat agatttgtag agagagactg 780

gtgatttcag cgtaccgaat taattctccc gcgaccagcc gagcgagctt agcgaactgt 840

ggacgagaac tgtgccacca agcgtaaggc cgttctctcg catttgcctt gctaggctcg 900

cgcgagttgc tggctgaggc gttctcgaaa tcagctcttg ttcggtcggc atctactcta 960

ttcctttgcc ctcggacgag tgctggggcg tcggtttcca ctatcggcga gtacttctac 1020

acagccatcg gtccagacgg ccgcgcttct gcgggcgatt tgtgtacgcc cgacagtccc 1080

ggctccggat cggacgattg cgtcgcatcg accctgcgcc caagctgcat catcgaaatt 1140

gccgtcaacc aagctctgat agagttggtc aagaccaatg cggagcatat acgcccggag 1200

gcgtggcgat cctgcaagct ccggatgcct ccgctcgaag tagcgcgtct gctgctccat 1260

acaagccaac cacggcctcc agaagaagat gttggcgacc tcgtattggg aatccccgaa 1320

catcgcctcg ctccagtcaa tgaccgctgt tatgcggcca ttgtccgtca ggacattgtt 1380

ggagccgaaa tccgcgtgca cgaggtgccg gacttcgggg cagtcctcgg cccaaagcat 1440

cagctcatcg agagcctgcg cgacggacgc actgacggtg tcgtccatca cagtttgcca 1500

gtgatacaca tggggatcag caatcgcgca tatgaaatca cgccatgtag tgtattgacc 1560

gattccttgc ggtccgaatg ggccgaaccc gctcgtctgg ctaagatcgg ccgcagcgat 1620

cgcatccata gcctccgcga ccggctgaag aacagcgggc agttcggttt caggcaggtc 1680

ttgcaacgtg acaccctgtg cacggcggga gatgcaatag gtcaggctct cgctgaactc 1740

cccaatgtca agcacttccg gaatcgggag cgcggccgat gcaaagtgcc gataaacata 1800

acgatctttg tagaaaccat cggcgcagct atttacccgc aggacatatc cacgccctcc 1860

tacatcgaag ctgaaagcac gagattcttc gccctccgag agctgcatca ggtcggagac 1920

gctgtcgaac ttttcgatca gaaacttctc gacagacgtc gcggtgagtt caggcttttt 1980

catagggatc agcttgggct gtcctctcca aatgaaatga acttccttat atagaggaag 2040

ggtcttgcga aggatagtgg gattgtgcgt catcccttac gtcagtggag atgtcacatc 2100

aatccacttg ctttgaagac gtggttggaa cgtcttcttt ttccacgatg ctcctcgtgg 2160

gtgggggtcc atctttggga ccactgtcgg cagaggcatc ttgaatgata gcctttcctt 2220

tatcgcaatg atggcatttg taggagccac cttccttttc tactgtcctt tcgatgaagt 2280

gacagatagc tgggcaatgg aatccgagga ggtttcccga aattaccctt tgttgaaaag 2340

tctcaatagc cctttggtct tctgagactg tatctttgac atttttggag taggggtacg 2400

ataacattaa cgtttacaat ttcgcgccat tcgccattca ggctgcgcaa ctgttgggaa 2460

gggcgatcgg tgcgggcctc ttcgctatta cgccagctgg cgaaaggggg atgtgctgca 2520

aggcgattaa gttgggtaac gccagggttt tcccagtcac gacgttgtaa aacgacggcc 2580

agtgaattgt aatacgactc actatagggc gaattgggta ccgggccccc cctcgaggtc 2640

gacggtatcg ataagcttgc atgcctgcag gtcggagtac tgtcctccga gcggagtact 2700

gtcctccgag cggagtactg tcctccgagc ggagtactgt cctccgagcg gagtactgtc 2760

ctccgagcgg agactctaga agctactcca cgtccataag ggacacatca caatcccact 2820

atccttcgca agacccttcc tctatataag gaagttcatt tcatttggag aggacgacct 2880

gcaggtcgac ggatccaagg agatataaca atgaagacta atctttttct ctttctcatc 2940

ttttcacttc tcctatcatt atcctcggcc gaattcctgc agcccggggg atccactagt 3000

tctagagcgg ccgccaccgc ggtggagctc cagcttttgt tccctttagt gagggttaat 3060

tccgagcttg gcgtaatcat ggtcatagct gtttcctgtg tgaaattgtt atccgctcac 3120

aattccacac aacatacgag ccggaaghca taaagtgtaa agcctggggt gcctaatgag 3180

tgagctaact cacattaatt gcgttgcgct cactgcccgc tttccagtcg ggaaacctgt 3240

cgtgccagct gcattaatga atcggccaac gcgcggggag aggcggtttg cgtattgggc 3300

gctcttccgc ttcctcgctc actgactcgc tgcgctcggt cgttcggctg cggcgagcgg 3360

tatcagctca ctcaaaggcg gtaatacggt tatccacaga atcaggggat aacgcaggaa 3420

agaacatgaa ggccttgaca ggatatattg gcgggtaaac taagtcgctg tatgtgtttg 3480

tttgagatct catgtgagca aaaggccagc aaaaggccag gaaccgtaaa aaggccgcgt 3540

tgctggcgtt tttccatagg ctccgccccc ctgacgagca tcacaaaaat cgacgctcaa 3600

gtcagaggtg gcgaaacccg acaggactat aaagatacca ggcgtttccc cctggaagct 3660

ccctcgtgcg ctctcctgtt ccgaccctgc cgcttaccgg atacctgtcc gcctttctcc 3720

cttcgggaag cgtggcgctt tctcatagct cacgctgtag gtatctcagt tcggtgtagg 3780

tcgttcgctc caagctgggc tgtgtgcacg aaccccccgt tcagcccgac cgctgcgcct 3840

tatccggtaa ctatcgtctt gagtccaacc cggtaagaca cgacttatcg ccactggcag 3900

cagccactgg taacaggatt agcagagcga ggtatgtagg cggtgctaca gagttcttga 3960

agtggtggcc taactacggc tacactagaa gaacagtatt tggtatctgc gctctgctga 4020

agccagttac cttcggaaga agagttggta gctcttgatc cggcaaacaa accaccgctg 4080

gtagcggtgg tttttttgtt tgcaagcagc agattacgcg cagaaaaaaa ggatctcaag 4140

aagatccttt gatcttttct acggggtctg acgctcagtg gaacgaaaac tcacgttaag 4200

ggattttggt catgagatta tcaaaaagga tcttcaccta gatcctttta aattaaaaat 4260

gaagttttaa atcaatctaa agtatatatg tgtaacattg gtctagtgat tagaaaaact 4320

catcgagcat caaatgaaac tgcaatttat tcatatcagg attatcaata ccatattttt 4380

gaaaaagccg tttctgtaat gaaggagaaa actcaccgag gcagttccat aggatggcaa 4440

gatcctggta tcggtctgcg attccgactc gtccaacatc aatacaacct attaatttcc 4500

cctcgtcaaa aataaggtta tcaagtgaga aatcaccatg agtgacgact gaatccggtg 4560

agaatggcaa aagtttatgc atttctttcc agacttgttc aacaggccag ccattacgct 4620

cgtcatcaaa atcactcgca tcaaccaaac cgttattcat tcgtgattgc gcctgagcga 4680

gacgaaatac gcgatcgctg ttaaaaggac aattacaaac aggaatcgaa tgcaaccggc 4740

gcaggaacac tgccagcgca tcaacaatat tttcacctga atcaggatat tcttctaata 4800

cctggaatgc tgttttccct gggatcgcag tggtgagtaa ccatgcatca tcaggagtac 4860

ggataaaatg cttgatggtc ggaagaggca taaattccgt cagccagttt agtctgacca 4920

tctcatctgt aacaacattg gcaacgctac ctttgccatg tttcagaaac aactctggcg 4980

catcgggctt cccatacaat cggtagattg tcgcacctga ttgcccgaca ttatcgcgag 5040

cccatttata cccatataaa tcagcatcca tgttggaatt taatcgcggc cttgagcaag 5100

acgtttcccg ttgaatatgg ctcataacac cccttgtatt actgtttatg taagcagaca 5160

gttttattgt tcatgatgat atatttttat cttgtgcaat gtaacatcag agattttgag 5220

acacaacgtg gctttgttga ataaatcgaa cttttgctga gttgaaggat cagatcacgc 5280

atcttcccga caacgcagac cgttccgtgg caaagcaaaa gttcaaaatc accaactggt 5340

ccacctacaa caaagctctc atcaaccgtg gctccctcac tttctggctg gatgatgggg 5400

cgattcaggc gatccccatc caacagcccg ccgtcgagcg ggct 5444

<210> 74
<211> 5220
<212> DNA
<213> Artificial Sequence

<220>
<221> source
<222> 1..5220
<223> /mol_type="unassigned DNA"
/note="Plasmid pGreenII-0179/5xUAS-short"
/organism="Artificial Sequence"

<400> 74
tttttatccc cggaagcctg tggatagagg gtagttatcc acgtgaaacc gctaatgccc 60

cgcaaagcct tgattcacgg ggctttccgg cccgctccaa aaactatcca cgtgaaatcg 120

ctaatcaggg tacgtgaaat cgctaatcgg agtacgtgaa atcgctaata aggtcacgtg 180

aaatcgctaa tcaaaaaggc acgtgagaac gctaatagcc ctttcagatc aacagcttgc 240

aaacacccct cgctccggca agtagttaca gcaagtagta tgttcaatta gcttttcaat 300

tatgaatata tatatcaatt attggtcgcc cttggcttgt ggacaatgcg ctacgcgcac 360

cggctccgcc cgtggacaac cgcaagcggt tgcccaccgt cgagcgccag cgcctttgcc 420

cacaacccgg cggccggccg caacagatcg ttttataaat tttttttttt gaaaaagaaa 480

aagcccgaaa ggcggcaacc tctcgggctt ctggatttcc gatccccgga attagagatc 540

ttggcaggat atattgtggt gtaacgttat cgatctggat tttagtactg gattttggtt 600

ttaggaatta gaaattttat tgatagaagt attttacaaa tacaaataca tactaagggt 660

ttcttatatg ctcaacacat gagcgaaacc ctataagaac cctaatttcc cttatcggga 720

aactactcac acattattta tggagaaaat agagagagat agatttgtag agagagactg 780

gtgatttcag cgtaccgaat taattctccc gcgaccagcc gagcgagctt agcgaactgt 840

ggacgagaac tgtgccacca agcgtaaggc cgttctctcg catttgcctt gctaggctcg 900

cgcgagttgc tggctgaggc gttctcgaaa tcagctcttg ttcggtcggc atctactcta 960

ttcctttgcc ctcggacgag tgctggggcg tcggtttcca ctatcggcga gtacttctac 1020

acagccatcg gtccagacgg ccgcgcttct gcgggcgatt tgtgtacgcc cgacagtccc 1080

ggctccggat cggacgattg cgtcgcatcg accctgcgcc caagctgcat catcgaaatt 1140

gccgtcaacc aagctctgat agagttggtc aagaccaatg cggagcatat acgcccggag 1200

gcgtggcgat cctgcaagct ccggatgcct ccgctcgaag tagcgcgtct gctgctccat 1260

acaagccaac cacggcctcc agaagaagat gttggcgacc tcgtattggg aatccccgaa 1320

catcgcctcg ctccagtcaa tgaccgctgt tatgcggcca ttgtccgtca ggacattgtt 1380

ggagccgaaa tccgcgtgca cgaggtgccg gacttcgggg cagtcctcgg cccaaagcat 1440

cagctcatcg agagcctgcg cgacggacgc actgacggtg tcgtccatca cagtttgcca 1500

gtgatacaca tggggatcag caatcgcgca tatgaaatca cgccatgtag tgtattgacc 1560

gattccttgc ggtccgaatg ggccgaaccc gctcgtctgg ctaagatcgg ccgcagcgat 1620

cgcatccata gcctccgcga ccggctgaag aacagcgggc agttcggttt caggcaggtc 1680

ttgcaacgtg acaccctgtg cacggcggga gatgcaatag gtcaggctct cgctgaactc 1740

cccaatgtca agcacttccg gaatcgggag cgcggccgat gcaaagtgcc gataaacata 1800

acgatctttg tagaaaccat cggcgcagct atttacccgc aggacatatc cacgccctcc 1860

tacatcgaag ctgaaagcac gagattcttc gccctccgag agctgcatca ggtcggagac 1920

gctgtcgaac ttttcgatca gaaacttctc gacagacgtc gcggtgagtt caggcttttt 1980

catagggggg gatcagcttg ggctgtcctc tccaaatgaa atgaacttcc ttatatagag 2040

gaagggtctt gcgaaggata gtgggattgt gcgtcatccc ttacgtcagt ggagatgtca 2100

catcaatcca cttgctttga agacgtggtt ggaacgtctt ctttttccac gatgctcctc 2160

gtgggtgggg gtccatcttt gggaccactg tcggcagagg catcttgaat gatagccttt 2220

cctttatcgc aatgatggca tttgtaggag ccaccttcct tttctactgt cctttcgatg 2280

aagtgacaga tagctgggca atggaatccg aggaggtttc ccgaaattac cctttgttga 2340

aaagtctcaa tagccctttg gtcttctgag actgtatctt tgacattttt ggagtagggg 2400

tacgataaca ttaacgctta caatttccat tcgccattca ggctgcgcaa ctgttgggaa 2460

gggcgatcgg tgcgggcctc ttcgctatta cgccagctgg cgaaaggggg atgtgctgca 2520

aggcgattaa gttgggtaac gccagggttt tcccagtcac gacgttgtaa aacgacggcc 2580

agtgagcgcg cgtaatacga ctcactatag ggcgaattgg gtaccgggcc ccccctcgag 2640

gtcgacggta tcgataagct tgcatgcctg caggtcggag tactgtcctc cgagcggagt 2700

actgtcctcc gagcggagta ctgtcctccg agcggagtac tgtcctccga gcggagtact 2760

gtcctccgag cggagactct agagcggccg ccaccgcggt ggagctccag cttttgttcc 2820

ctttagtgag ggttaattgc gcgcttggcg taatcatggt catagctgtt tcctgtgtga 2880

aattgttatc cgctcacaat tccacacaac atacgagccg gaagcataaa gtgtaaagcc 2940

tggggtgcct aatgagtgag ctaactcaca ttaattgcgt tgcgctcact gcccgctttc 3000

cagtcgggaa acctgtcgtg ccagctgcat taatgaatcg gccaacgcgc ggggagaggc 3060

ggtttgcgta ttgggcgctc ttccgcttcc tcgctcactg actcgctgcg ctcggtcgtt 3120

cggctgcggc gagcggtatc agctcactca aaggcggtaa tacggttatc cacagaatca 3180

ggggataacg caggaaagaa catgaaggcc ttgacaggat atattggcgg gtaaactaag 3240

tcgctgtatg tgtttgtttg agatctcatg tgagcaaaag gccagcaaaa ggccaggaac 3300

cgtaaaaagg ccgcgttgct ggcgtttttc cataggctcc gcccccctga cgagcatcac 3360

aaaaatcgac gctcaagtca gaggtggcga aacccgacag gactataaag ataccaggcg 3420

tttccccctg gaagctccct cgtgcgctct cctgttccga ccctgccgct taccggatac 3480

ctgtccgcct ttctcccttc gggaagcgtg gcgctttctc atagctcacg ctgtaggtat 3540

ctcagttcgg tgtaggtcgt tcgctccaag ctgggctgtg tgcacgaacc ccccgttcag 3600

cccgaccgct gcgccttatc cggtaactat cgtcttgagt ccaacccggt aagacacgac 3660

ttatcgccac tggcagcagc cactggtaac aggattagca gagcgaggta tgtaggcggt 3720

gctacagagt tcttgaagtg gtggcctaac tacggctaca ctagaagaac agtatttggt 3780

atctgcgctc tgctgaagcc agttaccttc ggaagaagag ttggtagctc ttgatccggc 3840

aaacaaacca ccgctggtag cggtggtttt tttgtttgca agcagcagat tacgcgcaga 3900

aaaaaaggat ctcaagaaga tcctttgatc ttttctacgg ggtctgacgc tcagtggaac 3960

gaaaactcac gttaagggat tttggtcatg agattatcaa aaaggatctt cacctagatc 4020

cttttaaatt aaaaatgaag ttttaaatca atctaaagta tatatgtgta acattggtct 4080

agtgattaga aaaactcatc gagcatcaaa tgaaactgca atttattcat atcaggatta 4140

tcaataccat atttttgaaa aagccgtttc tgtaatgaag gagaaaactc accgaggcag 4200

ttccatagga tggcaagatc ctggtatcgg tctgcgattc cgactcgtcc aacatcaata 4260

caacctatta atttcccctc gtcaaaaata aggttatcaa gtgagaaatc accatgagtg 4320

acgactgaat ccggtgagaa tggcaaaagt ttatgcattt ctttccagac ttgttcaaca 4380

ggccagccat tacgctcgtc atcaaaatca ctcgcatcaa ccaaaccgtt attcattcgt 4440

gattgcgcct gagcgagacg aaatacgcga tcgctgttaa aaggacaatt acaaacagga 4500

atcgaatgca accggcgcag gaacactgcc agcgcatcaa caatattttc acctgaatca 4560

ggatattctt ctaatacctg gaatgctgtt ttccctggga tcgcagtggt gagtaaccat 4620

gcatcatcag gagtacggat aaaatgcttg atggtcggaa gaggcataaa ttccgtcagc 4680

cagtttagtc tgaccatctc atctgtaaca acattggcaa cgctaccttt gccatgtttc 4740

agaaacaact ctggcgcatc gggcttccca tacaatcggt agattgtcgc acctgattgc 4800

ccgacattat cgcgagccca tttataccca tataaatcag catccatgtt ggaatttaat 4860

cgcggccttg agcaagacgt ttcccgttga atatggctca taacacccct tgtattactg 4920

tttatgtaag cagacagttt tattgttcat gatgatatat ttttatcttg tgcaatgtaa 4980

catcagagat tttgagacac aacgtggctt tgttgaataa atcgaacttt tgctgagttg 5040

aaggatcaga tcacgcatct tcccgacaac gcagaccgtt ccgtggcaaa gcaaaagttc 5100

aaaatcacca actggtccac ctacaacaaa gctctcatca accgtggctc cctcactttc 5160

tggctggatg atggggcgat tcaggcgatc cccatccaac agcccgccgt cgagcgggct 5220

<210> 75
<211> 6208
<212> DNA
<213> Artificial Sequence

<220>
<221> source
<222> 1..6208
<223> /mol_type="unassigned DNA"
      /note="Plasmid pET28/CSR1-ZFN-1"
      /organism="Artificial Sequence"

<400> 75
tcgagatctc gatcccgcga aattaatacg actcactata ggggaattgt gagcggataa 60

caattcccct ctagaaataa ttttgtttaa ctttaagaag gagatatacc atggtgccaa 120

aaaagaagag aaaggtagaa gacccctctc gagctgaaaa accttacaag tgtcctgaat 180

gtggaaagtc tttttctcag tcttcttctc ttgttcgtca ccagcgaaca cacacaggtg 240

agaagccata taaatgccca gaatgtggta aatcattcag tacttctgga catcttgttc 300

gtcaccaacg gacccacacc ggggagaagc catttaaatg ccctgagtgc gggaagagtt 360

tttcaactaa aaactctctt accgaacatc aacgtactca tactggagag aagccattta 420

aatgccctga atgcggcaag agcttcagca cttctggaca tcttgttcgt catcagagga 480

cgcatacggg cggactagtc aaaagtgaac tggaggagaa gaaatctgaa cttcgtcata 540

aattgaaata tgtgcctcat gaatatattg aattaattga aattgccaga aattccactc 600

aggatagaat tcttgaaatg aaggtaatgg aattttttat gaaagtttat ggatatagag 660

gtaaacattt gggtggatca aggaaaccgg acggagcaat ttatactgtc ggatctccta 720

ttgattacgg tgtgatcgtg gatactaaag cttatagcgg aggttataat ctgccaattg 780

gccaagcaga tgaaatgcaa cgatatgtcg aagaaaatca aacacgaaac aaacatatca 840

accctaatga atggtggaaa gtctatccat cttctgtaac ggaatttaag tttttatttg 900

tgagtggtca ctttaaagga aactacaaag ctcagcttac acgattaaat catatcacta 960

attgtaatgg agctgttctt agtgtagaag agcttttaat tggtggagaa atgattaaag 1020

ccggcacatt aaccttagag gaagtgagac ggaaatttaa taacggcgag ataaactttg 1080

gatccctcga gcaccaccac caccaccact gagatccggc tgctaacaaa gcccgaaagg 1140

aagctgagtt ggctgctgcc accgctgagc aataactagc ataacccctt ggggcctcta 1200

aacgggtctt gaggggtttt ttgctgaaag gaggaactat atccggattg gcgaatggga 1260

cgcgccctgt agcggcgcat taagcgcggc gggtgtggtg gttacgcgca gcgtgaccgc 1320

tacacttgcc agcgccctag cgcccgctcc tttcgctttc ttcccttcct ttctcgccac 1380

gttcgccggc tttccccgtc aagctctaaa tcgggggctc cctttagggt tccgatttag 1440

tgctttacgg cacctcgacc ccaaaaaact tgattagggt gatggttcac gtagtgggcc 1500

atcgccctga tagacggttt ttcgcccttt gacgttggag tccacgttct ttaatagtgg 1560

actcttgttc caaactggaa caacactcaa ccctatctcg gtctattctt ttgatttata 1620

agggattttg ccgatttcgg cctattggtt aaaaaatgag ctgatttaac aaaaatttaa 1680

cgcgaatttt aacaaaatat taacgtttac aatttcaggt ggcacttttc ggggaaatgt 1740

gcgcggaacc cctatttgtt tatttttcta aatacattca aatatgtatc cgctcatgaa 1800

ttaattctta gaaaaactca tcgagcatca aatgaaactg caatttattc atatcaggat 1860

tatcaatacc atatttttga aaaagccgtt tctgtaatga aggagaaaac tcaccgaggc 1920

agttccatag gatggcaaga tcctggtatc ggtctgcgat tccgactcgt ccaacatcaa 1980

tacaacctat taatttcccc tcgtcaaaaa taaggttatc aagtgagaaa tcaccatgag 2040

tgacgactga atccggtgag aatggcaaaa gtttatgcat ttctttccag acttgttcaa 2100

caggccagcc attacgctcg tcatcaaaat cactcgcatc aaccaaaccg ttattcattc 2160

gtgattgcgc ctgagcgaga cgaaatacgc gatcgctgtt aaaaggacaa ttacaaacag 2220

gaatcgaatg caaccggcgc aggaacactg ccagcgcatc aacaatattt tcacctgaat 2280

caggatattc ttctaatacc tggaatgctg ttttcccggg gatcgcagtg gtgagtaacc 2340

atgcatcatc aggagtacgg ataaaatgct tgatggtcgg aagaggcata aattccgtca 2400

gccagtttag tctgaccatc tcatctgtaa catcattggc aacgctacct ttgccatgtt 2460

tcagaaacaa ctctggcgca tcgggcttcc catacaatcg atagattgtc gcacctgatt 2520

gcccgacatt atcgcgagcc catttatacc catataaatc agcatccatg ttggaattta 2580

atcgcggcct agagcaagac gtttcccgtt gaatatggct cataacaccc cttgtattac 2640

tgtttatgta agcagacagt tttattgttc atgaccaaaa tcccttaacg tgagttttcg 2700

ttccactgag cgtcagaccc cgtagaaaag atcaaaggat cttcttgaga tccttttttt 2760

ctgcgcgtaa tctgctgctt gcaaacaaaa aaaccaccgc taccagcggt ggtttgtttg 2820

ccggatcaag agctaccaac tctttttccg aaggtaactg gcttcagcag agcgcagata 2880

ccaaatactg tccttctagt gtagccgtag ttaggccacc acttcaagaa ctctgtagca 2940

ccgcctacat acctcgctct gctaatcctg ttaccagtgg ctgctgccag tggcgataag 3000

tcgtgtctta ccgggttgga ctcaagacga tagttaccgg ataaggcgca gcggtcgggc 3060

tgaacggggg gttcgtgcac acagcccagc ttggagcgaa cgacctacac cgaactgaga 3120

tacctacagc gtgagctatg agaaagcgcc acgcttcccg aagggagaaa ggcggacagg 3180

tatccggtaa gcggcagggt cggaacagga gagcgcacga gggagcttcc agggggaaac 3240

gcctggtatc tttatagtcc tgtcgggttt cgccacctct gacttgagcg tcgatttttg 3300

tgatgctcgt caggggggcg gagcctatgg aaaaacgcca gcaacgcggc ctttttacgg 3360

ttcctggcct tttgctggcc ttttgctcac atgttctttc ctgcgttatc ccctgattct 3420

gtggataacc gtattaccgc ctttgagtga gctgataccg ctcgccgcag ccgaacgacc 3480

gagcgcagcg agtcagtgag cgaggaagcg gaagagcgcc tgatgcggta ttttctcctt 3540

acgcatctgt gcggtatttc acaccgcata tatggtgcac tctcagtaca atctgctctg 3600

atgccgcata gttaagccag tatacactcc gctatcgcta cgtgactggg tcatggctgc 3660

gccccgacac ccgccaacac ccgctgacgc gccctgacgg gcttgtctgc tcccggcatc 3720

cgcttacaga caagctgtga ccgtctccgg gagctgcatg tgtcagaggt tttcaccgtc 3780

atcaccgaaa cgcgcgaggc agctgcggta aagctcatca gcgtggtcgt gaagcgattc 3840

acagatgtct gcctgttcat ccgcgtccag ctcgttgagt ttctccagaa gcgttaatgt 3900

ctggcttctg ataaagcggg ccatgttaag ggcggttttt tcctgtttgg tcactgatgc 3960

ctccgtgtaa gggggatttc tgttcatggg ggtaatgata ccgatgaaac gagagaggat 4020

gctcacgata cgggttactg atgatgaaca tgcccggtta ctggaacgtt gtgagggtaa 4080

acaactggcg gtatggatgc ggcgggacca gagaaaaatc actcagggtc aatgccagcg 4140

cttcgttaat acagatgtag gtgttccaca gggtagccag cagcatcctg cgatgcagat 4200

ccggaacata atggtgcagg gcgctgactt ccgcgtttcc agactttacg aaacacggaa 4260

accgaagacc attcatgttg ttgctcaggt cgcagacgtt ttgcagcagc agtcgcttca 4320

cgttcgctcg cgtatcggtg attcattctg ctaaccagta aggcaacccc gccagcctag 4380

ccgggtcctc aacgacagga gcacgatcat gcgcacccgt ggggccgcca tgccggcgat 4440

aatggcctgc ttctcgccga aacgtttggt ggcgggacca gtgacgaagg cttgagcgag 4500

ggcgtgcaag attccgaata ccgcaagcga caggccgatc atcgtcgcgc tccagcgaaa 4560

gcggtcctcg ccgaaaatga cccagagcgc tgccggcacc tgtcctacga gttgcatgat 4620

aaagaagaca gtcataagtg cggcgacgat agtcatgccc cgcgcccacc ggaaggagct 4680

gactgggttg aaggctctca agggcatcgg tcgagatccc ggtgcctaat gagtgagcta 4740

acttacatta attgcgttgc gctcactgcc cgctttccag tcgggaaacc tgtcgtgcca 4800

gctgcattaa tgaatcggcc aacgcgcggg gagaggcggt ttgcgtattg ggcgccaggg 4860

tggtttttct tttcaccagt gagacgggca acagctgatt gcccttcacc gcctggccct 4920

gagagagttg cagcaagcgg tccacgctgg tttgccccag caggcgaaaa tcctgtttga 4980

tggtggttaa cggcgggata taacatgagc tgtcttcggt atcgtcgtat cccactaccg 5040

agatatccgc accaacgcgc agcccggact cggtaatggc gcgcattgcg cccagcgcca 5100

tctgatcgtt ggcaaccagc atcgcagtgg gaacgatgcc ctcattcagc atttgcatgg 5160

tttgttgaaa accggacatg gcactccagt cgccttcccg ttccgctatc ggctgaattt 5220

gattgcgagt gagatattta tgccagccag ccagacgcag acgcgccgag acagaactta 5280

atgggcccgc taacagcgcg atttgctggt gacccaatgc gaccagatgc tccacgccca 5340

gtcgcgtacc gtcttcatgg gagaaaataa tactgttgat gggtgtctgg tcagagacat 5400

caagaaataa cgccggaaca ttagtgcagg cagcttccac agcaatggca tcctggtcat 5460

ccagcggata gttaatgatc agcccactga cgcgttgcgc gagaagattg tgcaccgccg 5520

ctttacaggc ttcgacgccg cttcgttcta ccatcgacac caccacgctg gcacccagtt 5580

gatcggcgcg agatttaatc gccgcgacaa tttgcgacgg cgcgtgcagg gccagactgg 5640

aggtggcaac gccaatcagc aacgactgtt tgcccgccag ttgttgtgcc acgcggttgg 5700

gaatgtaatt cagctccgcc atcgccgctt ccactttttc ccgcgttttc gcagaaacgt 5760

ggctggcctg gttcaccacg cgggaaacgg tctgataaga gacaccggca tactctgcga 5820

catcgtataa cgttactggt ttcacattca ccaccctgaa ttgactctct tccgggcgct 5880

atcatgccat accgcgaaag gttttgcgcc attcgatggt gtccgggatc tcgacgctct 5940

cccttatgcg actcctgcat taggaagcag cccagtagta ggttgaggcc gttgagcacc 6000

gccgccgcaa ggaatggtgc atgcaaggag atggcgccca acagtccccc ggccacgggg 6060

cctgccacca tacccacgcc gaaacaagcg ctcatgagcc cgaagtggcg agcccgatct 6120

tccccatcgg tgatgtcggc gatataggcg ccagcaaccg cacctgtggc gccggtgatg 6180

ccggccacga tgcgtccggc gtagagga 6208

<210> 76
<211> 6208

<212> DNA
<213> Artificial Sequence

<220>
<221> source
<222> 1..6208
<223> /mol_type="unassigned DNA"
      /note="Plasmid pET28/CSR1-ZFN-2"
      /organism="Artificial Sequence"

<400> 76
tcgagatctc gatcccgcga aattaatacg actcactata ggggaattgt gagcggataa      60

caattcccct ctagaaataa ttttgtttaa ctttaagaag gagatatacc atggtgccaa     120

aaaagaagag aaaggtagaa gacccctctc gagctgaaaa accttacaag tgtcctgaat     180

gtggaaagtc tttttctcgt tctgatgatc ttgttcgtca ccagcgaaca cacacaggtg     240

agaagccata taaatgccca gaatgtggta aatcattcag tacttctgga aatttggttc     300

gtcaccaacg gacccacacc ggggagaagc catttaaatg ccctgagtgc gggaagagtt     360

tttcaagccc ggcggatctg acccgccatc aacgtactca tactggagag aagccattta     420

aatgccctga atgcggcaag agcttcagca cttctggaca tcttgttcgt catcagagga     480

cgcatacggg cggactagtc aaaagtgaac tggaggagaa gaaatctgaa cttcgtcata     540

aattgaaata tgtgcctcat gaatatattg aattaattga aattgccaga aattccactc     600

aggatagaat tcttgaaatg aaggtaatgg aattttttat gaaagtttat ggatatagag     660

gtaaacattt gggtggatca aggaaaccgg acggagcaat ttatactgtc ggatctccta     720

ttgattacgg tgtgatcgtg gatactaaag cttatagcgg aggttataat ctgccaattg     780

gccaagcaga tgaaatgcaa cgatatgtcg aagaaaatca aacacgaaac aaacatatca     840

accctaatga atggtggaaa gtctatccat cttctgtaac ggaatttaag tttttatttg     900

tgagtggtca ctttaaagga aactacaaag ctcagcttac acgattaaat catatcacta     960

attgtaatgg agctgttctt agtgtagaag agcttttaat tggtggagaa atgattaaag    1020

ccggcacatt aaccttagag gaagtgagac ggaaatttaa taacggcgag ataaactttg    1080

gatccctcga gcaccaccac caccaccact gagatccggc tgctaacaaa gcccgaaagg    1140

aagctgagtt ggctgctgcc accgctgagc aataactagc ataacccctt ggggcctcta    1200

aacgggtctt gaggggtttt ttgctgaaag gaggaactat atccggattg gcgaatggga    1260

cgcgccctgt agcggcgcat taagcgcggc gggtgtggtg gttacgcgca gcgtgaccgc    1320

tacacttgcc agcgccctag cgcccgctcc tttcgctttc ttcccttcct ttctcgccac    1380

gttcgccggc tttccccgtc aagctctaaa tcgggggctc cctttagggt tccgatttag    1440

tgctttacgg cacctcgacc ccaaaaaact tgattagggt gatggttcac gtagtgggcc    1500

atcgccctga tagacggttt ttcgcccttt gacgttggag tccacgttct ttaatagtgg    1560

actcttgttc caaactggaa caacactcaa ccctatctcg gtctattctt ttgatttata 1620

agggattttg ccgatttcgg cctattggtt aaaaaatgag ctgatttaac aaaaatttaa 1680

cgcgaatttt aacaaaatat taacgtttac aatttcaggt ggcacttttc ggggaaatgt 1740

gcgcggaacc cctatttgtt tatttttcta aatacattca aatatgtatc cgctcatgaa 1800

ttaattctta gaaaaactca tcgagcatca aatgaaactg caatttattc atatcaggat 1860

tatcaatacc atatttttga aaaagccgtt tctgtaatga aggagaaaac tcaccgaggc 1920

agttccatag gatggcaaga tcctggtatc ggtctgcgat tccgactcgt ccaacatcaa 1980

tacaacctat taatttcccc tcgtcaaaaa taaggttatc aagtgagaaa tcaccatgag 2040

tgacgactga atccggtgag aatggcaaaa gtttatgcat ttctttccag acttgttcaa 2100

caggccagcc attacgctcg tcatcaaaat cactcgcatc aaccaaaccg ttattcattc 2160

gtgattgcgc ctgagcgaga cgaaatacgc gatcgctgtt aaaaggacaa ttacaaacag 2220

gaatcgaatg caaccggcgc aggaacactg ccagcgcatc aacaatattt tcacctgaat 2280

caggatattc ttctaatacc tggaatgctg ttttcccggg gatcgcagtg gtgagtaacc 2340

atgcatcatc aggagtacgg ataaaatgct tgatggtcgg aagaggcata aattccgtca 2400

gccagtttag tctgaccatc tcatctgtaa catcattggc aacgctacct ttgccatgtt 2460

tcagaaacaa ctctggcgca tcgggcttcc catacaatcg atagattgtc gcacctgatt 2520

gcccgacatt atcgcgagcc catttatacc catataaatc agcatccatg ttggaattta 2580

atcgcggcct agagcaagac gtttcccgtt gaatatggct cataacaccc cttgtattac 2640

tgtttatgta agcagacagt tttattgttc atgaccaaaa tcccttaacg tgagttttcg 2700

ttccactgag cgtcagaccc cgtagaaaag atcaaaggat cttcttgaga tccttttttt 2760

ctgcgcgtaa tctgctgctt gcaaacaaaa aaaccaccgc taccagcggt ggtttgtttg 2820

ccggatcaag agctaccaac tctttttccg aaggtaactg gcttcagcag agcgcagata 2880

ccaaatactg tccttctagt gtagccgtag ttaggccacc acttcaagaa ctctgtagca 2940

ccgcctacat acctcgctct gctaatcctg ttaccagtgg ctgctgccag tggcgataag 3000

tcgtgtctta ccgggttgga ctcaagacga tagttaccgg ataaggcgca gcggtcgggc 3060

tgaacggggg gttcgtgcac acagcccagc ttggagcgaa cgacctacac cgaactgaga 3120

tacctacagc gtgagctatg agaaagcgcc acgcttcccg aagggagaaa ggcggacagg 3180

tatccggtaa gcggcagggt cggaacagga gagcgcacga gggagcttcc agggggaaac 3240

gcctggtatc tttatagtcc tgtcgggttt cgccacctct gacttgagcg tcgatttttg 3300

tgatgctcgt caggggggcg gagcctatgg aaaaacgcca gcaacgcggc ctttttacgg 3360

ttcctggcct tttgctggcc ttttgctcac atgttctttc ctgcgttatc ccctgattct 3420

gtggataacc gtattaccgc ctttgagtga gctgataccg ctcgccgcag ccgaacgacc 3480

gagcgcagcg agtcagtgag cgaggaagcg gaagagcgcc tgatgcggta ttttctcctt 3540

acgcatctgt gcggtatttc acaccgcata tatggtgcac tctcagtaca atctgctctg 3600

atgccgcata gttaagccag tatacactcc gctatcgcta cgtgactggg tcatggctgc 3660

gccccgacac ccgccaacac ccgctgacgc gccctgacgg gcttgtctgc tcccggcatc 3720

cgcttacaga caagctgtga ccgtctccgg gagctgcatg tgtcagaggt tttcaccgtc 3780

atcaccgaaa cgcgcgaggc agctgcggta aagctcatca gcgtggtcgt gaagcgattc 3840

acagatgtct gcctgttcat ccgcgtccag ctcgttgagt ttctccagaa gcgttaatgt 3900

ctggcttctg ataaagcggg ccatgttaag ggcggttttt tcctgtttgg tcactgatgc 3960

ctccgtgtaa gggggatttc tgttcatggg ggtaatgata ccgatgaaac gagagaggat 4020

gctcacgata cgggttactg atgatgaaca tgcccggtta ctggaacgtt gtgagggtaa 4080

acaactggcg gtatggatgc ggcgggacca gagaaaaatc actcagggtc aatgccagcg 4140

cttcgttaat acagatgtag gtgttccaca gggtagccag cagcatcctg cgatgcagat 4200

ccggaacata atggtgcagg gcgctgactt ccgcgtttcc agactttacg aaacacggaa 4260

accgaagacc attcatgttg ttgctcaggt cgcagacgtt ttgcagcagc agtcgcttca 4320

cgttcgctcg cgtatcggtg attcattctg ctaaccagta aggcaacccc gccagcctag 4380

ccgggtcctc aacgacagga gcacgatcat gcgcacccgt ggggccgcca tgccggcgat 4440

aatggcctgc ttctcgccga aacgtttggt ggcgggacca gtgacgaagg cttgagcgag 4500

ggcgtgcaag attccgaata ccgcaagcga caggccgatc atcgtcgcgc tccagcgaaa 4560

gcggtcctcg ccgaaaatga cccagagcgc tgccggcacc tgtcctacga gttgcatgat 4620

aaagaagaca gtcataagtg cggcgacgat agtcatgccc cgcgcccacc ggaaggagct 4680

gactgggttg aaggctctca agggcatcgg tcgagatccc ggtgcctaat gagtgagcta 4740

acttacatta attgcgttgc gctcactgcc cgctttccag tcgggaaacc tgtcgtgcca 4800

gctgcattaa tgaatcggcc aacgcgcggg gagaggcggt ttgcgtattg ggcgccaggg 4860

tggtttttct tttcaccagt gagacgggca acagctgatt gcccttcacc gcctggccct 4920

gagagagttg cagcaagcgg tccacgctgg tttgccccag caggcgaaaa tcctgtttga 4980

tggtggttaa cggcgggata taacatgagc tgtcttcggt atcgtcgtat cccactaccg 5040

agatatccgc accaacgcgc agcccggact cggtaatggc gcgcattgcg cccagcgcca 5100

tctgatcgtt ggcaaccagc atcgcagtgg gaacgatgcc ctcattcagc atttgcatgg 5160

tttgttgaaa accggacatg gcactccagt cgccttcccg ttccgctatc ggctgaattt 5220

gattgcgagt gagatattta tgccagccag ccagacgcag acgcgccgag acagaactta 5280

atgggcccgc taacagcgcg atttgctggt gacccaatgc gaccagatgc tccacgccca 5340

gtcgcgtacc gtcttcatgg gagaaaataa tactgttgat gggtgtctgg tcagagacat 5400

caagaaataa cgccggaaca ttagtgcagg cagcttccac agcaatggca tcctggtcat 5460

ccagcggata gttaatgatc agcccactga cgcgttgcgc gagaagattg tgcaccgccg 5520

ctttacaggc ttcgacgccg cttcgttcta ccatcgacac caccacgctg gcacccagtt 5580

gatcggcgcg agatttaatc gccgcgacaa tttgcgacgg cgcgtgcagg gccagactgg 5640

aggtggcaac gccaatcagc aacgactgtt tgcccgccag ttgttgtgcc acgcggttgg 5700

gaatgtaatt cagctccgcc atcgccgctt ccactttttc ccgcgttttc gcagaaacgt 5760

ggctggcctg gttcaccacg cgggaaacgg tctgataaga gacaccggca tactctgcga 5820

catcgtataa cgttactggt ttcacattca ccaccctgaa ttgactctct tccgggcgct 5880

atcatgccat accgcgaaag gttttgcgcc attcgatggt gtccgggatc tcgacgctct 5940

cccttatgcg actcctgcat taggaagcag cccagtagta ggttgaggcc gttgagcacc 6000

gccgccgcaa ggaatggtgc atgcaaggag atggcgccca acagtccccc ggccacgggg 6060

cctgccacca tacccacgcc gaaacaagcg ctcatgagcc cgaagtggcg agcccgatct 6120

tccccatcgg tgatgtcggc gatataggcg ccagcaaccg cacctgtggc gccggtgatg 6180

ccggccacga tgcgtccggc gtagagga 6208

<210> 77
<211> 4297
<212> DNA
<213> Artificial Sequence

<220>
<221> source
<222> 1..4297
<223> /mol_type="unassigned DNA"
      /note="Plasmid pCR2.1/CSR1-ZF1"
      /organism="Artificial Sequence"

<400> 77
tatagatatc gcccgtatgc gtcctctgat gacgaacaag atgtccagaa gtgctgaagc 60

tcttgccgca ttcagggcat ttaaatggct tctctccagt atgagtacgt tgatgttcgg 120

taagagagtt tttagttgaa aaactcttcc cgcactcagg gcatttaaat ggcttctccc 180

cggtgtgggt ccgttggtga cgaacaagat gtccagaagt actgaatgat ttaccacatt 240

ctgggcattt atatggcttc tcacctgtgt gtgttcgctg gtgacgaaca agagaagaag 300

actgagaaaa agactttcca cattcaggac acttgtaagg tttttcacct ccaccagccc 360

cgggtaataa gccgaattct gcagatatcc atcacactgg cggccgctcg agcatgcatc 420

tagagggccc aattcgccct atagtgagtc gtattacaat tcactggccg tcgttttaca 480

acgtcgtgac tgggaaaacc ctggcgttac ccaacttaat cgccttgcag cacatccccc 540

tttcgccagc tggcgtaata gcgaagaggc ccgcaccgat cgcccttccc aacagttgcg 600

cagcctgaat ggcgaatgga cgcgccctgt agcggcgcat taagcgcggc gggtgtggtg 660

gttacgcgca gcgtgaccgc tacacttgcc agcgccctag cgcccgctcc tttcgctttc 720

ttcccttcct ttctcgccac gttcgccggc tttccccgtc aagctctaaa tcgggggctc 780

cctttagggt tccgatttag tgctttacgg cacctcgacc ccaaaaaact tgattagggt 840

gatggttcac gtagtgggcc atcgccctga tagacggttt ttcgcccttt gacgttggag 900

tccacgttct ttaatagtgg actcttgttc caaactggaa caacactcaa ccctatctcg 960

gtctattctt ttgatttata agggattttg ccgatttcgg cctattggtt aaaaaatgag 1020

ctgatttaac aaaaatttaa cgcgaatttt aacaaaattc agggcgcaag ggctgctaaa 1080

ggaagcggaa cacgtagaaa gccagtccgc agaaacggtg ctgaccccgg atgaatgtca 1140

gctactgggc tatctggaca agggaaaacg caagcgcaaa gagaaagcag gtagcttgca 1200

gtgggcttac atggcgatag ctagactggg cggttttatg gacagcaagc gaaccggaat 1260

tgccagctgg ggcgccctct ggtaaggttg ggaagccctg caaagtaaac tggatggctt 1320

tcttgccgcc aaggatctga tggcgcaggg gatcaagatc tgatcaagag acaggatgag 1380

gatcgtttcg catgattgaa caagatggat tgcacgcagg ttctccggcc gcttgggtgg 1440

agaggctatt cggctatgac tgggcacaac agacaatcgg ctgctctgat gccgccgtgt 1500

tccggctgtc agcgcagggg cgcccggttc tttttgtcaa gaccgacctg tccggtgccc 1560

tgaatgaact gcaggacgag gcagcgcggc tatcgtggct ggccacgacg ggcgttcctt 1620

gcgcagctgt gctcgacgtt gtcactgaag cgggaaggga ctggctgcta ttgggcgaag 1680

tgccggggca ggatctcctg tcatcccacc ttgctcctgc cgagaaagta tccatcatgg 1740

ctgatgcaat gcggcggctg catacgcttg atccggctac ctgcccattc gaccaccaag 1800

cgaaacatcg catcgagcga gcacgtactc ggatggaagc cggtcttgtc gatcaggatg 1860

atctggacga agagcatcag gggctcgcgc cagccgaact gttcgccagg ctcaaggcgc 1920

gcatgcccga cggcgaggat ctcgtcgtga cccatggcga tgcctgcttg ccgaatatca 1980

tggtggaaaa tggccgcttt tctggattca tcgactgtgg ccggctgggt gtggcggacc 2040

gctatcagga catagcgttg gctacccgtg atattgctga agagcttggc ggcgaatggg 2100

ctgaccgctt cctcgtgctt tacggtatcg ccgctcccga ttcgcagcgc atcgccttct 2160

atcgccttct tgacgagttc ttctgaattg aaaaaggaag agtatgagta ttcaacattt 2220

ccgtgtcgcc cttattccct tttttgcggc attttgcctt cctgtttttg ctcacccaga 2280

aacgctggtg aaagtaaaag atgctgaaga tcagttgggt gcacgagtgg gttacatcga 2340

actggatctc aacagcggta agatccttga gagttttcgc cccgaagaac gttttccaat 2400

gatgagcact tttaaagttc tgctatgtgg cgcggtatta tcccgtattg acgccgggca 2460

agagcaactc ggtcgccgca tacactattc tcagaatgac ttggttgagt actcaccagt 2520

cacagaaaag catcttacgg atggcatgac agtaagagaa ttatgcagtg ctgccataac 2580

catgagtgat aacactgcgg ccaacttact tctgacaacg atcggaggac cgaaggagct 2640

aaccgctttt ttgcacaaca tgggggatca tgtaactcgc cttgatcgtt gggaaccgga 2700

gctgaatgaa gccataccaa acgacgagcg tgacaccacg atgcctgtag caatggcaac 2760

aacgttgcgc aaactattaa ctggcgaact acttactcta gcttcccggc aacaattaat 2820

agactggatg gaggcggata aagttgcagg accacttctg cgctcggccc ttccggctgg 2880

ctggtttatt gctgataaat ctggagccgg tgagcgtggg tctcgcggta tcattgcagc 2940

actggggcca gatggtaagc cctcccgtat cgtagttatc tacacgacgg ggagtcaggc 3000

aactatggat gaacgaaata gacagatcgc tgagataggt gcctcactga ttaagcattg 3060

gtaactgtca gaccaagttt actcatatat actttagatt gatttaaaac ttcattttta 3120

atttaaaagg atctaggtga agatcctttt tgataatctc atgaccaaaa tcccttaacg 3180

tgagttttcg ttccactgag cgtcagaccc cgtagaaaag atcaaaggat cttcttgaga 3240

tccttttttt ctgcgcgtaa tctgctgctt gcaaacaaaa aaaccaccgc taccagcggt 3300

ggtttgtttg ccggatcaag agctaccaac tctttttccg aaggtaactg gcttcagcag 3360

agcgcagata ccaaatactg ttcttctagt gtagccgtag ttaggccacc acttcaagaa 3420

ctctgtagca ccgcctacat acctcgctct gctaatcctg ttaccagtgg ctgctgccag 3480

tggcgataag tcgtgtctta ccgggttgga ctcaagacga tagttaccgg ataaggcgca 3540

gcggtcgggc tgaacggggg gttcgtgcac acagcccagc ttggagcgaa cgacctacac 3600

cgaactgaga tacctacagc gtgagctatg agaaagcgcc acgcttcccg aagggagaaa 3660

ggcggacagg tatccggtaa gcggcagggt cggaacagga gagcgcacga gggagcttcc 3720

agggggaaac gcctggtatc tttatagtcc tgtcgggttt cgccacctct gacttgagcg 3780

tcgatttttg tgatgctcgt caggggggcg gagcctatgg aaaaacgcca gcaacgcggc 3840

ctttttacgg ttcctggcct tttgctggcc ttttgctcac atgttctttc ctgcgttatc 3900

ccctgattct gtggataacc gtattaccgc ctttgagtga gctgataccg ctcgccgcag 3960

ccgaacgacc gagcgcagcg agtcagtgag cgaggaagcg gaagagcgcc caatacgcaa 4020

accgcctctc cccgcgcgtt ggccgattca ttaatgcagc tggcacgaca ggtttcccga 4080

ctggaaagcg ggcagtgagc gcaacgcaat taatgtgagt tagctcactc attaggcacc 4140

ccaggcttta cactttatgc ttccggctcg tatgttgtgt ggaattgtga gcggataaca 4200

atttcacaca ggaaacagct atgaccatga ttacgccaag cttggtaccg agctcggatc 4260

cactagtaac ggccgccagt gtgctggaat tcggctt 4297

<210> 78
<211> 4297
<212> DNA
<213> Artificial Sequence

<220>
<221> source
<222> 1..4297
<223> /mol_type="unassigned DNA"
      /note="Plasmid pCR2.1/CSR1-ZF2"
      /organism="Artificial Sequence"

<400> 78
tatagatatc gcccgtatgc gtcctctgat gacgaacaag atgtccagaa gtgctgaagc      60

tcttgccgca ttcagggcat ttaaatggct tctctccagt atgagtacgt tgatggcggg     120

tcagatccgc cgggcttgaa aaactcttcc cgcactcagg gcatttaaat ggcttctccc     180

cggtgtgggt ccgttggtga cgaaccaaat ttccagaagt actgaatgat ttaccacatt     240

ctgggcattt atatggcttc tcacctgtgt gtgttcgctg gtgacgaaca agatcatcag     300

aacgagaaaa agactttcca cattcaggac acttgtaagg tttttcacct ccaccagccc     360

cgggtaataa gccgaattct gcagatatcc atcacactgg cggccgctcg agcatgcatc     420

tagagggccc aattcgccct atagtgagtc gtattacaat tcactggccg tcgttttaca     480

acgtcgtgac tgggaaaacc ctggcgttac ccaacttaat cgccttgcag cacatccccc     540

tttcgccagc tggcgtaata gcgaagaggc ccgcaccgat cgcccttccc aacagttgcg     600

cagcctgaat ggcgaatgga cgcgccctgt agcggcgcat taagcgcggc gggtgtggtg     660

gttacgcgca gcgtgaccgc tacacttgcc agcgccctag cgcccgctcc tttcgctttc     720

ttcccttcct ttctcgccac gttcgccggc tttccccgtc aagctctaaa tcgggggctc     780

cctttagggt tccgatttag tgctttacgg cacctcgacc ccaaaaaact tgattagggt     840

gatggttcac gtagtgggcc atcgccctga tagacggttt ttcgcccttt gacgttggag     900

tccacgttct ttaatagtgg actcttgttc caaactggaa caacactcaa ccctatctcg     960

gtctattctt ttgatttata agggattttg ccgatttcgg cctattggtt aaaaaatgag    1020

ctgatttaac aaaaatttaa cgcgaatttt aacaaaattc agggcgcaag ggctgctaaa    1080

ggaagcggaa cacgtagaaa gccagtccgc agaaacggtg ctgaccccgg atgaatgtca    1140

gctactgggc tatctggaca agggaaaacg caagcgcaaa gagaaagcag gtagcttgca    1200

gtgggcttac atggcgatag ctagactggg cggttttatg gacagcaagc gaaccggaat    1260

tgccagctgg ggcgccctct ggtaaggttg ggaagccctg caaagtaaac tggatggctt    1320

tcttgccgcc aaggatctga tggcgcaggg gatcaagatc tgatcaagag acaggatgag    1380

gatcgtttcg catgattgaa caagatggat tgcacgcagg ttctccggcc gcttgggtgg    1440

agaggctatt cggctatgac tgggcacaac agacaatcgg ctgctctgat gccgccgtgt    1500

tccggctgtc agcgcagggg cgcccggttc tttttgtcaa gaccgacctg tccggtgccc 1560

tgaatgaact gcaggacgag gcagcgcggc tatcgtggct ggccacgacg ggcgttcctt 1620

gcgcagctgt gctcgacgtt gtcactgaag cgggaaggga ctggctgcta ttgggcgaag 1680

tgccggggca ggatctcctg tcatcccacc ttgctcctgc cgagaaagta tccatcatgg 1740

ctgatgcaat gcggcggctg catacgcttg atccggctac ctgcccattc gaccaccaag 1800

cgaaacatcg catcgagcga gcacgtactc ggatggaagc cggtcttgtc gatcaggatg 1860

atctggacga agagcatcag gggctcgcgc cagccgaact gttcgccagg ctcaaggcgc 1920

gcatgcccga cggcgaggat ctcgtcgtga cccatggcga tgcctgcttg ccgaatatca 1980

tggtggaaaa tggccgcttt tctggattca tcgactgtgg ccggctgggt gtggcggacc 2040

gctatcagga catagcgttg gctacccgtg atattgctga agagcttggc ggcgaatggg 2100

ctgaccgctt cctcgtgctt tacggtatcg ccgctcccga ttcgcagcgc atcgccttct 2160

atcgccttct tgacgagttc ttctgaattg aaaaaggaag agtatgagta ttcaacattt 2220

ccgtgtcgcc cttattccct tttttgcggc attttgcctt cctgtttttg ctcacccaga 2280

aacgctggtg aaagtaaaag atgctgaaga tcagttgggt gcacgagtgg gttacatcga 2340

actggatctc aacagcggta agatccttga gagttttcgc cccgaagaac gttttccaat 2400

gatgagcact tttaaagttc tgctatgtgg cgcggtatta tcccgtattg acgccgggca 2460

agagcaactc ggtcgccgca tacactattc tcagaatgac ttggttgagt actcaccagt 2520

cacagaaaag catcttacgg atggcatgac agtaagagaa ttatgcagtg ctgccataac 2580

catgagtgat aacactgcgg ccaacttact tctgacaacg atcggaggac cgaaggagct 2640

aaccgctttt ttgcacaaca tgggggatca tgtaactcgc cttgatcgtt gggaaccgga 2700

gctgaatgaa gccataccaa acgacgagcg tgacaccacg atgcctgtag caatggcaac 2760

aacgttgcgc aaactattaa ctggcgaact acttactcta gcttcccggc aacaattaat 2820

agactggatg gaggcggata aagttgcagg accacttctg cgctcggccc ttccggctgg 2880

ctggtttatt gctgataaat ctggagccgg tgagcgtggg tctcgcggta tcattgcagc 2940

actggggcca gatggtaagc cctcccgtat cgtagttatc tacacgacgg ggagtcaggc 3000

aactatggat gaacgaaata gacagatcgc tgagataggt gcctcactga ttaagcattg 3060

gtaactgtca gaccaagttt actcatatat actttagatt gatttaaaac ttcattttta 3120

atttaaaagg atctaggtga agatcctttt tgataatctc atgaccaaaa tcccttaacg 3180

tgagttttcg ttccactgag cgtcagaccc cgtagaaaag atcaaaggat cttcttgaga 3240

tccttttttt ctgcgcgtaa tctgctgctt gcaaacaaaa aaaccaccgc taccagcggt 3300

ggtttgtttg ccggatcaag agctaccaac tctttttccg aaggtaactg gcttcagcag 3360

agcgcagata ccaaatactg ttcttctagt gtagccgtag ttaggccacc acttcaagaa 3420

ctctgtagca ccgcctacat acctcgctct gctaatcctg ttaccagtgg ctgctgccag 3480

tggcgataag tcgtgtctta ccgggttgga ctcaagacga tagttaccgg ataaggcgca 3540

gcggtcgggc tgaacggggg gttcgtgcac acagcccagc ttggagcgaa cgacctacac 3600

cgaactgaga tacctacagc gtgagctatg agaaagcgcc acgcttcccg aagggagaaa 3660

ggcggacagg tatccggtaa gcggcagggt cggaacagga gagcgcacga gggagcttcc 3720

agggggaaac gcctggtatc tttatagtcc tgtcgggttt cgccacctct gacttgagcg 3780

tcgatttttg tgatgctcgt caggggggcg gagcctatgg aaaaacgcca gcaacgcggc 3840

ctttttacgg ttcctggcct tttgctggcc ttttgctcac atgttctttc ctgcgttatc 3900

ccctgattct gtggataacc gtattaccgc ctttgagtga gctgataccg ctcgccgcag 3960

ccgaacgacc gagcgcagcg agtcagtgag cgaggaagcg gaagagcgcc caatacgcaa 4020

accgcctctc cccgcgcgtt ggccgattca ttaatgcagc tggcacgaca ggtttcccga 4080

ctggaaagcg ggcagtgagc gcaacgcaat taatgtgagt tagctcactc attaggcacc 4140

ccaggcttta cactttatgc ttccggctcg tatgttgtgt ggaattgtga gcggataaca 4200

atttcacaca ggaaacagct atgaccatga ttacgccaag cttggtaccg agctcggatc 4260

cactagtaac ggccgccagt gtgctggaat tcggctt 4297

<210> 79
<211> 4567
<212> DNA
<213> Artificial Sequence

<220>
<221> source
<222> 1..4567
<223> /mol_type="unassigned DNA"
      /note="Fragment of Plasmid pCB302/AtSPO11-1-ZF1"
      /organism="Artificial Sequence"

<400> 79
ggatccgctg aagctgaagt tgccacgaag gagaaagcga gagagagacg acatcgtttt 60

tagctgtatg caaaggtttt tctcttcttt tttttggatc ggagtgttca aaggttttta 120

gtcaggggaa cggctttaat acttcaaatt aggtttggta acaatataaa gcaaatgaac 180

cctaaattac ttttgtatta ttcaaaattt caaacttatt actcattagt cattagtgaa 240

tgtactttgt attattaaaa attaaaaact gagaattatt ttttataatc tgtatattta 300

tgatttttcc attaaaacta tgttcatttg tccattctac caatgcattt ggtttgcact 360

aaaaagctta gaaagagtca taaaaaaaac aatgagcatt accttgagtt ggagactagt 420

tatctcgtat gaagcagatc ttcacccatc gcccgtatgc gtcctctgat gacgaacaag 480

atgtccagaa gtgctgaagc tcttgccgca ttcagggcat ttaaatggct tctctccagt 540

atgagtacgt tgatgttcgg taagagagtt tttagttgaa aaactcttcc cgcactcagg 600

gcatttaaat ggcttctccc cggtgtgggt ccgttggtga cgaacaagat gtccagaagt 660

actgaatgat ttaccacatt ctgggcattt atatggcttc tcacctgtgt gtgttcgctg 720

gtgacgaaca agagaagaag actgagaaaa agactttcca cattcaggac acttgtaagg 780

tttttcacct ccaccagccc cgggaggaga gcttacttca cgacgaatct tagaaggaat 840

atatacctct gacaaaaagg acagtgagtg aactgagagt gcttcgatct caaacttgac 900

ccctcttttc aacatcgttt caagttccaa ccttagacga ccaaaatcgt tttgatcaag 960

actagttttt gtcatttggt tgaaatgtaa atataaaaaa gtcagggaag tttttcatac 1020

ctccattgtg gcatttcacg cttcaggtag catctaagca acattgcttc agttcttttc 1080

ttatctgtcc ggggaagttg ttacaatctc aaaaaaggaa gcagtgaaaa ataggaaaat 1140

ctaaagattt aaggcgataa ggtgtttaat gatctcttac cttcttctgt cagtggcaaa 1200

agacactgtt tgggaacact atatacctca gagtctgaag gaaaagctcc caaccatttc 1260

atatctggtg ctcgtaatga ttcaatatca taggccattt gcttacagaa agaaaacaag 1320

tgacataagg aaaatggttc aaaatgaaat gggtcagtgc catatcacat tatcactatc 1380

aacaaaaaca atgcaagcgg tgaaattttc cgagttcagt catctatatt ttgtaacaga 1440

atctaaccat ggagccaaaa cggtatgtgg ctaggatctc aaagccatat ggatcacagt 1500

cgactagaca atgcacaggt agatgcaact tctccatcag gagtcgcaag aacctggaaa 1560

gaatctgtgt tattcgctga tatattaatc aatagaagat aatgtctgat aaaatagtat 1620

tccaggaacc tgggattaag ttatgtacct tcttgttgag acatctggat atcctcttcc 1680

ctgccaaaaa caatcgtaac tttgatgtca aacataaaat ggaaatgaaa actagtcaaa 1740

actgaaatca gtaagaatga agcagatttc ttactgtgat aacaatgcag cggttcgtct 1800

tgcaaaacat gtcatttgct aaacgctgga atactataaa ccatggatga aagcgattta 1860

gtcatgaaga aagaaaacca tcttcctagt tactacataa ataagaggtc tttcttacct 1920

gtttcttttt ccacaaccag tatgtactca gctaagctaa caatatctgc aagcaggaaa 1980

atgacatcca attatagttc ctgatgccac catagatagt gaatcttata cttcctgata 2040

tcagctgaga taaggatttg acttctctaa agagcataaa gcatcactta aaatgattgc 2100

aaatatatgt accttcgact tcctctacaa gaacaggaac gggatatgcc tgaaatgaaa 2160

tcacttgcag agttatactt cctgatgcca atataaaaag tgaatcttat gttcaaattc 2220

ttacagtgtt caagctgttt aaacaatcaa atttccttcc agcttcccta aattttaacc 2280

aacccatcac caacctacaa cacaaaagaa taatgaggtg gatcttaaaa taaggacatg 2340

cctcaagata gaagcattgc actcgttatc aagagcaccc acccgtttcc aacagaaacc 2400

tgcaaaatat cacatgaatg aaaaagtaat cacatcttca atcgcagctt ataataactc 2460

ttcagaagga aaattgaagg tgaaagtatt aaagtactaa ccacattcaa gttgtaccga 2520

ctacactgaa aaagaatgca tatatcacca attgcacggt ccacaatgga ttgtgctgtg 2580

aagtagacga aaacagtgtt actagaaggc caagaacaca acaaagaggg atgcataact 2640

aatatccgta taaaaatctc aatacgtatt cgtttagaag aaaaacataa ctaaaacatt 2700

atacgttgct tccaaattca cctttgaaag ctgatggatg catgtagtag atatctcttt 2760

tcgaagcatg tctattttct tgcagaagtt gttggactat caacaatact cttagcaaca 2820

tatctgaaag taaaaacacc gcaatgtgat gctatattaa gataacacta atgtacttgt 2880

agaattatct gaaacctgac tcaccgattc tgtaggtttg tggttccttc ttaagagtga 2940

aaatttcctg accctttggt ttatcagagc tgcataagct atagacagca gaataaaatg 3000

gtaaattcat gaggtcttag ttttcaatac tcgttgacgt tgtgcttaaa tccttcaaaa 3060

atgtctcgaa ccaaagacca aacacgtctg attcttcaat caaagaaagt gaaaacaaaa 3120

ttgaataatc gagatcgaag agagacaaac caatcagctt ctggattcat gcaatagttc 3180

ctgaattgat tgatggaaat ttttggagat cttccttcag ccagatctac aacgaccgat 3240

tgagtaaaat ctgctcaaaa aaccgaaaca tagtgatgaa caacggaaga gccagcgaag 3300

tcatcgacta gaaatttcga catagattag aggacaaatt actcaatacg attcggaaaa 3360

ttttgggatt ttctgatacc tttgatcctt tgaagcaaat tagtcgattc tgaaatagcg 3420

aattttccct ccatctcttt cgagtttcaa aactgaaaaa tgcaatggtc gacgaatttt 3480

tgtagacggg aagaaaaggg atgggtgcga aaagcttgcc gttaaaacct tcgaatttaa 3540

acatgcgtgt cgtttagttc ctactttcca tgacgttggg ccattggcat ttaggcccga 3600

tacgtaactg acgtgtcatt ttaaaaacgc atgtgttttt aaacacgttg ggccgttggc 3660

ttttaggccc gatccgaaac taggcaaagt gattgtgaaa aagattcatg ataacaatca 3720

aaccaatata aatcgattta ttcagacttg gagacatttt agtgatgcca atataaaata 3780

atcatgtgca ctaaatttga aataattaat gtgtatggta aactagatta gaattagttc 3840

tagacttcta gtggtcgaga tttgttcata agctttatgc tcggacacgt gaggaacata 3900

ccaaaatgtt cgaatctatt atgccataca cttattttaa tcatttcttc tctatgatag 3960

attgctcacc taatttatat ataatctaag aatatactac ttagctttaa cgtattttat 4020

ccgataacga attcatgata ccaaaaaagt tctaaaacaa aagaggcatc ttgtattatt 4080

tatagtattt ctaactaaaa cacaacactg ataacattta aattgctttg ttccgtttaa 4140

ttagtaaaca aatgaaaagc tgtcgcgtcg gtggttgggt ttcattcgga aagatttaaa 4200

agtaaagcgc acggatttta aggtcgttca acatttaaat tttttttgaa agtaaaatca 4260

tatatcaatg caatgtgtaa tcttgtaacg tataaactaa tgagagatac ctaaagaaaa 4320

gaagcctcgc gcctaaacag aaacataaga ttaaaataag taatatagaa gcataaactt 4380

aagcaagatt ttattcctcg tgaaaatatt acattttgta attttatttt ctctagtatt 4440

aaaaaaggaa caactaatta acggaaatac ttaaggaatt aagaacagag aggtctcata 4500

cttagtcaag tttggtgaag ctatcaaaga gaatgtcttc gtgttgtccg ccgacgacat 4560

tctgcag 4567

<210> 80
<211> 4567
<212> DNA
<213> Artificial Sequence

<220>
<221> source
<222> 1..4567
<223> /mol_type="unassigned DNA"
      /note="Fragment of Plasmid pCB302/AtSPO11-1-ZF2"
      /organism="Artificial Sequence"

<400> 80
ggatccgctg aagctgaagt tgccacgaag gagaaagcga gagagagacg acatcgtttt 60

tagctgtatg caaaggtttt tctcttcttt tttttggatc ggagtgttca aaggttttta 120

gtcaggggaa cggctttaat acttcaaatt aggtttggta acaatataaa gcaaatgaac 180

cctaaattac ttttgtatta ttcaaaattt caaacttatt actcattagt cattagtgaa 240

tgtactttgt attattaaaa attaaaaact gagaattatt ttttataatc tgtatattta 300

tgatttttcc attaaaacta tgttcatttg tccattctac caatgcattt ggtttgcact 360

aaaaagctta gaaagagtca taaaaaaaac aatgagcatt accttgagtt ggagactagt 420

tatctcgtat gaagcagatc ttcacccatc gcccgtatgc gtcctctgat gacgaacaag 480

atgtccagaa gtgctgaagc tcttgccgca ttcagggcat ttaaatggct tctctccagt 540

atgagtacgt tgatggcggg tcagatccgc cgggcttgaa aaactcttcc cgcactcagg 600

gcatttaaat ggcttctccc cggtgtgggt ccgttggtga cgaaccaaat ttccagaagt 660

actgaatgat ttaccacatt ctgggcattt atatggcttc tcacctgtgt gtgttcgctg 720

gtgacgaaca agatcatcag aacgagaaaa agactttcca cattcaggac acttgtaagg 780

tttttcacct ccaccagccc cgggaggaga gcttacttca cgacgaatct tagaaggaat 840

atatacctct gacaaaaagg acagtgagtg aactgagagt gcttcgatct caaacttgac 900

ccctcttttc aacatcgttt caagttccaa ccttagacga ccaaaatcgt tttgatcaag 960

actagttttt gtcatttggt tgaaatgtaa atataaaaaa gtcagggaag tttttcatac 1020

ctccattgtg gcatttcacg cttcaggtag catctaagca acattgcttc agttcttttc 1080

ttatctgtcc ggggaagttg ttacaatctc aaaaaaggaa gcagtgaaaa ataggaaaat 1140

ctaaagattt aaggcgataa ggtgtttaat gatctcttac cttcttctgt cagtggcaaa 1200

agacactgtt tgggaacact atatacctca gagtctgaag gaaaagctcc caaccatttc 1260

atatctggtg ctcgtaatga ttcaatatca taggccattt gcttacagaa agaaaacaag 1320

tgacataagg aaaatggttc aaaatgaaat gggtcagtgc catatcacat tatcactatc 1380

aacaaaaaca atgcaagcgg tgaaattttc cgagttcagt catctatatt ttgtaacaga 1440

atctaaccat ggagccaaaa cggtatgtgg ctaggatctc aaagccatat ggatcacagt 1500

cgactagaca atgcacaggt agatgcaact tctccatcag gagtcgcaag aacctggaaa 1560

gaatctgtgt tattcgctga tatattaatc aatagaagat aatgtctgat aaaatagtat 1620

tccaggaacc tgggattaag ttatgtacct tcttgttgag acatctggat atcctcttcc 1680

ctgccaaaaa caatcgtaac tttgatgtca aacataaaat ggaaatgaaa actagtcaaa 1740

actgaaatca gtaagaatga agcagatttc ttactgtgat aacaatgcag cggttcgtct 1800

tgcaaaacat gtcatttgct aaacgctgga atactataaa ccatggatga aagcgattta 1860

gtcatgaaga aagaaaacca tcttcctagt tactacataa ataagaggtc tttcttacct 1920

gtttcttttt ccacaaccag tatgtactca gctaagctaa caatatctgc aagcaggaaa 1980

atgacatcca attatagttc ctgatgccac catagatagt gaatcttata cttcctgata 2040

tcagctgaga taaggatttg acttctctaa agagcataaa gcatcactta aaatgattgc 2100

aaatatatgt accttcgact tcctctacaa gaacaggaac gggatatgcc tgaaatgaaa 2160

tcacttgcag agttatactt cctgatgcca atataaaaag tgaatcttat gttcaaattc 2220

ttacagtgtt caagctgttt aaacaatcaa atttccttcc agcttcccta aattttaacc 2280

aacccatcac caacctacaa cacaaaagaa taatgaggtg gatcttaaaa taaggacatg 2340

cctcaagata gaagcattgc actcgttatc aagagcaccc acccgtttcc aacagaaacc 2400

tgcaaaatat cacatgaatg aaaaagtaat cacatcttca atcgcagctt ataataactc 2460

ttcagaagga aaattgaagg tgaaagtatt aaagtactaa ccacattcaa gttgtaccga 2520

ctacactgaa aaagaatgca tatatcacca attgcacggt ccacaatgga ttgtgctgtg 2580

aagtagacga aaacagtgtt actagaaggc caagaacaca acaaagaggg atgcataact 2640

aatatccgta taaaaatctc aatacgtatt cgtttagaag aaaaacataa ctaaaacatt 2700

atacgttgct tccaaattca cctttgaaag ctgatggatg catgtagtag atatctcttt 2760

tcgaagcatg tctattttct tgcagaagtt gttggactat caacaatact cttagcaaca 2820

tatctgaaag taaaaacacc gcaatgtgat gctatattaa gataacacta atgtacttgt 2880

agaattatct gaaacctgac tcaccgattc tgtaggtttg tggttccttc ttaagagtga 2940

aaatttcctg accctttggt ttatcagagc tgcataagct atagacagca gaataaaatg 3000

gtaaattcat gaggtcttag ttttcaatac tcgttgacgt tgtgcttaaa tccttcaaaa 3060

atgtctcgaa ccaaagacca aacacgtctg attcttcaat caaagaaagt gaaaacaaaa 3120

ttgaataatc gagatcgaag agagacaaac caatcagctt ctggattcat gcaatagttc 3180

ctgaattgat tgatggaaat ttttggagat cttccttcag ccagatctac aacgaccgat 3240

tgagtaaaat ctgctcaaaa aaccgaaaca tagtgatgaa caacggaaga gccagcgaag 3300

tcatcgacta gaaatttcga catagattag aggacaaatt actcaatacg attcggaaaa 3360

ttttgggatt ttctgatacc tttgatcctt tgaagcaaat tagtcgattc tgaaatagcg 3420

aattttccct ccatctcttt cgagtttcaa aactgaaaaa tgcaatggtc gacgaatttt 3480

tgtagacggg aagaaaaggg atgggtgcga aaagcttgcc gttaaaacct tcgaatttaa 3540

acatgcgtgt cgtttagttc ctactttcca tgacgttggg ccattggcat ttaggcccga 3600

tacgtaactg acgtgtcatt ttaaaaacgc atgtgttttt aaacacgttg ggccgttggc 3660

ttttaggccc gatccgaaac taggcaaagt gattgtgaaa aagattcatg ataacaatca 3720

aaccaatata aatcgattta ttcagacttg gagacatttt agtgatgcca atataaaata 3780

atcatgtgca ctaaatttga aataattaat gtgtatggta aactagatta gaattagttc 3840

tagacttcta gtggtcgaga tttgttcata agctttatgc tcggacacgt gaggaacata 3900

ccaaaatgtt cgaatctatt atgccataca cttattttaa tcatttcttc tctatgatag 3960

attgctcacc taatttatat ataatctaag aatatactac ttagctttaa cgtattttat 4020

ccgataacga attcatgata ccaaaaaagt tctaaaacaa aagaggcatc ttgtattatt 4080

tatagtattt ctaactaaaa cacaacactg ataacattta aattgctttg ttccgtttaa 4140

ttagtaaaca aatgaaaagc tgtcgcgtcg gtggttgggt ttcattcgga aagatttaaa 4200

agtaaagcgc acggatttta aggtcgttca acatttaaat tttttttgaa agtaaaatca 4260

tatatcaatg caatgtgtaa tcttgtaacg tataaactaa tgagagatac ctaaagaaaa 4320

gaagcctcgc gcctaaacag aaacataaga ttaaaataag taatatagaa gcataaactt 4380

aagcaagatt ttattcctcg tgaaaatatt acattttgta attttatttt ctctagtatt 4440

aaaaaaggaa caactaatta acggaaatac ttaaggaatt aagaacagag aggtctcata 4500

cttagtcaag tttggtgaag ctatcaaaga gaatgtcttc gtgttgtccg ccgacgacat 4560

tctgcag 4567

<210> 81
<211> 5302
<212> DNA
<213> Artificial Sequence

<220>
<221> source
<222> 1..5302
<223> /mol_type="unassigned DNA"
/note="Fragment of Plasmid pCB302/AtSPO11-1-ZF1-18xMyc"

/organism="Artificial Sequence"

<400> 81
ggatccgctg aagctgaagt tgccacgaag gagaaagcga gagagagacg acatcgtttt 60

tagctgtatg caaaggtttt tctcttcttt tttttggatc ggagtgttca aaggttttta 120

gtcaggggaa cggctttaat acttcaaatt aggtttggta acaatataaa gcaaatgaac 180

cctaaattac ttttgtatta ttcaaaattt caaacttatt actcattagt cattagtgaa 240

tgtactttgt attattaaaa attaaaaact gagaattatt ttttataatc tgtatattta 300

tgatttttcc attaaaacta tgttcatttg tccattctac caatgcattt ggtttgcact 360

aaaaagctta gaaagagtca taaaaaaaac aatgagcatt accttgagtt ggagactagt 420

tatctcgtat gaagcagatc ttcacccatc tccgttcaag tcttcttctg agattaattt 480

ttgttcaccg ttcaagtctt cctcggagat tagcttttgt tcaccgttca aatcttcttc 540

agaaatcaac ttttgttcac ctctagagga tccgttcaag tcttcttctg agattaattt 600

ttgttcaccg ttcaagtctt cttctgagat taatttttgt tcaccgttca aatcttcttc 660

agaaatcaac ttttgttcac cactagtgga tccgttcaag tcttcttctg agattaattt 720

ttgttcaccg ttcaagtctt cctcggagat tagcttttgt tcaccgttca aatcttcttc 780

agaaatcaac ttttgttcac cactagtgga tccgttcaag tcttcttctg agattaattt 840

ttgttcaccg ttcaagtctt cctcggagat tagcttttgt tcaccgttca aatcttcttc 900

agaaatcaac ttttgttcac ctctagagga tccgttcaag tcttcttctg agattaattt 960

ttgttcaccg ttcaagtctt cctcggagat tagcttttgt tcaccgttca aatcttcttc 1020

agaaatcaac ttttgttcac cactagtgga tccgttcaag tcttcttctg agattaattt 1080

ttgttcaccg ttcaagtctt cctcggagat tagcttttgt tcaccgttca aatcttcttc 1140

agaaatcaac ttttgttcac cactagcagc agaaccggac ccatcgcccg tatgcgtcct 1200

ctgatgacga acaagatgtc cagaagtgct gaagctcttg ccgcattcag ggcatttaaa 1260

tggcttctct ccagtatgag tacgttgatg ttcggtaaga gagtttttag ttgaaaaact 1320

cttcccgcac tcagggcatt taaatggctt ctccccggtg tgggtccgtt ggtgacgaac 1380

aagatgtcca gaagtactga atgatttacc acattctggg catttatatg gcttctcacc 1440

tgtgtgtgtt cgctggtgac gaacaagaga agaagactga gaaaaagact ttccacattc 1500

aggacacttg taaggttttt cacctccacc agccccggga ggagagctta cttcacgacg 1560

aatcttagaa ggaatatata cctctgacaa aaaggacagt gagtgaactg agagtgcttc 1620

gatctcaaac ttgacccctc ttttcaacat cgtttcaagt tccaacctta gacgaccaaa 1680

atcgttttga tcaagactag tttttgtcat ttggttgaaa tgtaaatata aaaaagtcag 1740

ggaagttttt catacctcca ttgtggcatt tcacgcttca ggtagcatct aagcaacatt 1800

gcttcagttc ttttcttatc tgtccgggga agttgttaca atctcaaaaa aggaagcagt 1860
gaaaaatagg aaaatctaaa gatttaaggc gataaggtgt ttaatgatct cttaccttct 1920
tctgtcagtg gcaaaagaca ctgtttggga acactatata cctcagagtc tgaaggaaaa 1980
gctcccaacc atttcatatc tggtgctcgt aatgattcaa tatcataggc catttgctta 2040
cagaaagaaa acaagtgaca taaggaaaat ggttcaaaat gaaatgggtc agtgccatat 2100
cacattatca ctatcaacaa aaacaatgca agcggtgaaa ttttccgagt tcagtcatct 2160
atattttgta acagaatcta accatggagc caaaacggta tgtggctagg atctcaaagc 2220
catatggatc acagtcgact agacaatgca caggtagatg caacttctcc atcaggagtc 2280
gcaagaacct ggaaagaatc tgtgttattc gctgatatat taatcaatag aagataatgt 2340
ctgataaaat agtattccag gaacctggga ttaagttatg taccttcttg ttgagacatc 2400
tggatatcct cttccctgcc aaaaacaatc gtaactttga tgtcaaacat aaaatggaaa 2460
tgaaaactag tcaaaactga aatcagtaag aatgaagcag atttcttact gtgataacaa 2520
tgcagcggtt cgtcttgcaa aacatgtcat ttgctaaacg ctggaatact ataaaccatg 2580
gatgaaagcg atttagtcat gaagaaagaa aaccatcttc ctagttacta cataaataag 2640
aggtctttct tacctgtttc tttttccaca accagtatgt actcagctaa gctaacaata 2700
tctgcaagca ggaaaatgac atccaattat agttcctgat gccaccatag atagtgaatc 2760
ttatacttcc tgatatcagc tgagataagg atttgacttc tctaaagagc ataaagcatc 2820
acttaaaatg attgcaaata tatgtacctt cgacttcctc tacaagaaca ggaacgggat 2880
atgcctgaaa tgaaatcact tgcagagtta tacttcctga tgccaatata aaaagtgaat 2940
cttatgttca aattcttaca gtgttcaagc tgtttaaaca atcaaatttc cttccagctt 3000
ccctaaattt taaccaaccc atcaccaacc tacaacacaa aagaataatg aggtggatct 3060
taaaataagg acatgcctca agatagaagc attgcactcg ttatcaagag cacccacccg 3120
tttccaacag aaacctgcaa aatatcacat gaatgaaaaa gtaatcacat cttcaatcgc 3180
agcttataat aactcttcag aaggaaaatt gaaggtgaaa gtattaaagt actaaccaca 3240
ttcaagttgt accgactaca ctgaaaaaga atgcatatat caccaattgc acggtccaca 3300
atggattgtg ctgtgaagta gacgaaaaca gtgttactag aaggccaaga acacaacaaa 3360
gagggatgca taactaatat ccgtataaaa atctcaatac gtattcgttt agaagaaaaa 3420
cataactaaa acattatacg ttgcttccaa attcaccttt gaaagctgat ggatgcatgt 3480
agtagatatc tcttttcgaa gcatgtctat tttcttgcag aagttgttgg actatcaaca 3540
atactcttag caacatatct gaaagtaaaa acaccgcaat gtgatgctat attaagataa 3600
cactaatgta cttgtagaat tatctgaaac ctgactcacc gattctgtag gtttgtggtt 3660
ccttcttaag agtgaaaatt tcctgaccct ttggtttatc agagctgcat aagctataga 3720

cagcagaata aaatggtaaa ttcatgaggt cttagttttc aatactcgtt gacgttgtgc 3780

ttaaatcctt caaaaatgtc tcgaaccaaa gaccaaacac gtctgattct tcaatcaaag 3840

aaagtgaaaa caaaattgaa taatcgagat cgaagagaga caaaccaatc agcttctgga 3900

ttcatgcaat agttcctgaa ttgattgatg gaaatttttg gagatcttcc ttcagccaga 3960

tctacaacga ccgattgagt aaaatctgct caaaaaaccg aaacatagtg atgaacaacg 4020

gaagagccag cgaagtcatc gactagaaat ttcgacatag attagaggac aaattactca 4080

atacgattcg gaaaattttg ggattttctg atacctttga tcctttgaag caaattagtc 4140

gattctgaaa tagcgaattt tccctccatc tctttcgagt ttcaaaactg aaaaatgcaa 4200

tggtcgacga atttttgtag acgggaagaa aagggatggg tgcgaaaagc ttgccgttaa 4260

aaccttcgaa tttaaacatg cgtgtcgttt agttcctact ttccatgacg ttgggccatt 4320

ggcatttagg cccgatacgt aactgacgtg tcattttaaa aacgcatgtg tttttaaaca 4380

cgttgggccg ttggctttta ggcccgatcc gaaactaggc aaagtgattg tgaaaaagat 4440

tcatgataac aatcaaacca atataaatcg atttattcag acttggagac attttagtga 4500

tgccaatata aaataatcat gtgcactaaa tttgaaataa ttaatgtgta tggtaaacta 4560

gattagaatt agttctagac ttctagtggt cgagatttgt tcataagctt tatgctcgga 4620

cacgtgagga acataccaaa atgttcgaat ctattatgcc atacacttat tttaatcatt 4680

tcttctctat gatagattgc tcacctaatt tatatataat ctaagaatat actacttagc 4740

tttaacgtat tttatccgat aacgaattca tgataccaaa aaagttctaa aacaaaagag 4800

gcatcttgta ttatttatag tatttctaac taaaacacaa cactgataac atttaaattg 4860

ctttgttccg tttaattagt aaacaaatga aaagctgtcg cgtcggtggt tgggtttcat 4920

tcggaaagat ttaaaagtaa agcgcacgga ttttaaggtc gttcaacatt taaatttttt 4980

ttgaaagtaa aatcatatat caatgcaatg tgtaatcttg taacgtataa actaatgaga 5040

gatacctaaa gaaaagaagc ctcgcgccta aacagaaaca taagattaaa ataagtaata 5100

tagaagcata aacttaagca agattttatt cctcgtgaaa atattacatt ttgtaatttt 5160

attttctcta gtattaaaaa aggaacaact aattaacgga aatacttaag gaattaagaa 5220

cagagaggtc tcatacttag tcaagtttgg tgaagctatc aaagagaatg tcttcgtgtt 5280

gtccgccgac gacattctgc ag 5302

<210> 82
<211> 5302
<212> DNA
<213> Artificial Sequence

<220>
<221> source

<222> 1..5302
<223> /mol_type="unassigned DNA"
      /note="Fragment of Plasmid pCB302/AtSPO11-1-ZF2-18xMyc"
      /organism="Artificial Sequence"

<400> 82
ggatccgctg aagctgaagt tgccacgaag gagaaagcga gagagagacg acatcgtttt      60

tagctgtatg caaaggtttt tctcttcttt tttttggatc ggagtgttca aaggttttta     120

gtcaggggaa cggctttaat acttcaaatt aggtttggta acaatataaa gcaaatgaac     180

cctaaattac ttttgtatta ttcaaaattt caaacttatt actcattagt cattagtgaa     240

tgtactttgt attattaaaa attaaaaact gagaattatt ttttataatc tgtatattta     300

tgatttttcc attaaaacta tgttcatttg tccattctac caatgcattt ggtttgcact     360

aaaaagctta gaaagagtca taaaaaaaac aatgagcatt accttgagtt ggagactagt     420

tatctcgtat gaagcagatc ttcacccatc tccgttcaag tcttcttctg agattaattt     480

ttgttcaccg ttcaagtctt cctcggagat tagcttttgt tcaccgttca aatcttcttc     540

agaaatcaac ttttgttcac ctctagagga tccgttcaag tcttcttctg agattaattt     600

ttgttcaccg ttcaagtctt cttctgagat taatttttgt tcaccgttca aatcttcttc     660

agaaatcaac ttttgttcac cactagtgga tccgttcaag tcttcttctg agattaattt     720

ttgttcaccg ttcaagtctt cctcggagat tagcttttgt tcaccgttca aatcttcttc     780

agaaatcaac ttttgttcac cactagtgga tccgttcaag tcttcttctg agattaattt     840

ttgttcaccg ttcaagtctt cctcggagat tagcttttgt tcaccgttca aatcttcttc     900

agaaatcaac ttttgttcac ctctagagga tccgttcaag tcttcttctg agattaattt     960

ttgttcaccg ttcaagtctt cctcggagat tagcttttgt tcaccgttca aatcttcttc    1020

agaaatcaac ttttgttcac cactagtgga tccgttcaag tcttcttctg agattaattt    1080

ttgttcaccg ttcaagtctt cctcggagat tagcttttgt tcaccgttca aatcttcttc    1140

agaaatcaac ttttgttcac cactagcagc agaaccggac ccatcgcccg tatgcgtcct    1200

ctgatgacga acaagatgtc cagaagtgct gaagctcttg ccgcattcag ggcatttaaa    1260

tggcttctct ccagtatgag tacgttgatg gcgggtcaga tccgccgggc ttgaaaaact    1320

cttcccgcac tcagggcatt taaatggctt ctccccggtg tgggtccgtt ggtgacgaac    1380

caaatttcca gaagtactga atgatttacc acattctggg catttatatg gcttctcacc    1440

tgtgtgtgtt cgctggtgac gaacaagatc atcagaacga gaaaaagact ttccacattc    1500

aggacacttg taaggttttt cacctccacc agccccggga ggagagctta cttcacgacg    1560

aatcttagaa ggaatatata cctctgacaa aaaggacagt gagtgaactg agagtgcttc    1620

gatctcaaac ttgacccctc ttttcaacat cgtttcaagt tccaacctta gacgaccaaa    1680

atcgttttga tcaagactag tttttgtcat ttggttgaaa tgtaaatata aaaaagtcag    1740

ggaagttttt catacctcca ttgtggcatt tcacgcttca ggtagcatct aagcaacatt 1800

gcttcagttc ttttcttatc tgtccgggga agttgttaca atctcaaaaa aggaagcagt 1860

gaaaaatagg aaaatctaaa gatttaaggc gataaggtgt ttaatgatct cttaccttct 1920

tctgtcagtg gcaaaagaca ctgtttggga acactatata cctcagagtc tgaaggaaaa 1980

gctcccaacc atttcatatc tggtgctcgt aatgattcaa tatcataggc catttgctta 2040

cagaaagaaa acaagtgaca taaggaaaat ggttcaaaat gaaatgggtc agtgccatat 2100

cacattatca ctatcaacaa aaacaatgca agcggtgaaa ttttccgagt tcagtcatct 2160

atattttgta acagaatcta accatggagc caaaacggta tgtggctagg atctcaaagc 2220

catatggatc acagtcgact agacaatgca caggtagatg caacttctcc atcaggagtc 2280

gcaagaacct ggaaagaatc tgtgttattc gctgatatat taatcaatag aagataatgt 2340

ctgataaaat agtattccag gaacctggga ttaagttatg taccttcttg ttgagacatc 2400

tggatatcct cttccctgcc aaaaacaatc gtaactttga tgtcaaacat aaaatggaaa 2460

tgaaaactag tcaaaactga aatcagtaag aatgaagcag atttcttact gtgataacaa 2520

tgcagcggtt cgtcttgcaa aacatgtcat ttgctaaacg ctggaatact ataaaccatg 2580

gatgaaagcg atttagtcat gaagaaagaa aaccatcttc ctagttacta cataaataag 2640

aggtctttct tacctgtttc tttttccaca accagtatgt actcagctaa gctaacaata 2700

tctgcaagca ggaaaatgac atccaattat agttcctgat gccaccatag atagtgaatc 2760

ttatacttcc tgatatcagc tgagataagg atttgacttc tctaaagagc ataaagcatc 2820

acttaaaatg attgcaaata tatgtacctt cgacttcctc tacaagaaca ggaacgggat 2880

atgcctgaaa tgaaatcact tgcagagtta tacttcctga tgccaatata aaaagtgaat 2940

cttatgttca aattcttaca gtgttcaagc tgtttaaaca atcaaatttc cttccagctt 3000

ccctaaattt taaccaaccc atcaccaacc tacaacacaa aagaataatg aggtggatct 3060

taaaataagg acatgcctca agatagaagc attgcactcg ttatcaagag cacccacccg 3120

tttccaacag aaacctgcaa aatatcacat gaatgaaaaa gtaatcacat cttcaatcgc 3180

agcttataat aactcttcag aaggaaaatt gaaggtgaaa gtattaaagt actaaccaca 3240

ttcaagttgt accgactaca ctgaaaaaga atgcatatat caccaattgc acggtccaca 3300

atggattgtg ctgtgaagta gacgaaaaca gtgttactag aaggccaaga acacaacaaa 3360

gagggatgca taactaatat ccgtataaaa atctcaatac gtattcgttt agaagaaaaa 3420

cataactaaa acattatacg ttgcttccaa attcaccttt gaaagctgat ggatgcatgt 3480

agtagatatc tcttttcgaa gcatgtctat tttcttgcag aagttgttgg actatcaaca 3540

atactcttag caacatatct gaaagtaaaa acaccgcaat gtgatgctat attaagataa 3600

cactaatgta cttgtagaat tatctgaaac ctgactcacc gattctgtag gtttgtggtt 3660

ccttcttaag agtgaaaatt tcctgaccct ttggtttatc agagctgcat aagctataga 3720

cagcagaata aaatggtaaa ttcatgaggt cttagttttc aatactcgtt gacgttgtgc 3780

ttaaatcctt caaaaatgtc tcgaaccaaa gaccaaacac gtctgattct tcaatcaaag 3840

aaagtgaaaa caaaattgaa taatcgagat cgaagagaga caaaccaatc agcttctgga 3900

ttcatgcaat agttcctgaa ttgattgatg gaaatttttg gagatcttcc ttcagccaga 3960

tctacaacga ccgattgagt aaaatctgct caaaaaaccg aaacatagtg atgaacaacg 4020

gaagagccag cgaagtcatc gactagaaat ttcgacatag attagaggac aaattactca 4080

atacgattcg gaaaattttg ggattttctg atacctttga tcctttgaag caaattagtc 4140

gattctgaaa tagcgaattt tccctccatc tctttcgagt ttcaaaactg aaaaatgcaa 4200

tggtcgacga atttttgtag acgggaagaa aagggatggg tgcgaaaagc ttgccgttaa 4260

aaccttcgaa tttaaacatg cgtgtcgttt agttcctact ttccatgacg ttgggccatt 4320

ggcatttagg cccgatacgt aactgacgtg tcattttaaa aacgcatgtg tttttaaaca 4380

cgttgggccg ttggctttta ggcccgatcc gaaactaggc aaagtgattg tgaaaaagat 4440

tcatgataac aatcaaacca atataaatcg atttattcag acttggagac attttagtga 4500

tgccaatata aaataatcat gtgcactaaa tttgaaataa ttaatgtgta tggtaaacta 4560

gattagaatt agttctagac ttctagtggt cgagatttgt tcataagctt tatgctcgga 4620

cacgtgagga acataccaaa atgttcgaat ctattatgcc atacacttat tttaatcatt 4680

tcttctctat gatagattgc tcacctaatt tatatataat ctaagaatat actacttagc 4740

tttaacgtat tttatccgat aacgaattca tgataccaaa aaagttctaa aacaaaagag 4800

gcatcttgta ttatttatag tatttctaac taaaacacaa cactgataac atttaaattg 4860

ctttgttccg tttaattagt aaacaaatga aaagctgtcg cgtcggtggt tgggtttcat 4920

tcggaaagat ttaaaagtaa agcgcacgga ttttaaggtc gttcaacatt taaatttttt 4980

ttgaaagtaa aatcatatat caatgcaatg tgtaatcttg taacgtataa actaatgaga 5040

gatacctaaa gaaaagaagc ctcgcgccta aacagaaaca taagattaaa ataagtaata 5100

tagaagcata aacttaagca agattttatt cctcgtgaaa atattacatt ttgtaatttt 5160

attttctcta gtattaaaaa aggaacaact aattaacgga aatacttaag gaattaagaa 5220

cagagaggtc tcatacttag tcaagtttgg tgaagctatc aaagagaatg tcttcgtgtt 5280

gtccgccgac gacattctgc ag 5302

<210> 83
<211> 3589
<212> DNA
<213> Artificial Sequence

<220>
<221> source
<222> 1..3589
<223> /mol_type="unassigned DNA"
      /note="Plasmid pSKNN-SgaI/PTF1-6F"
      /organism="Artificial Sequence"

<400> 83
gtggcacttt tcggggaaat gtgcgcggaa cccctatttg tttatttttc taaatacatt      60

caaatatgta tccgctcatg agacaataac cctgataaat gcttcaataa tattgaaaaa     120

ggaagagtat gagtattcaa catttccgtg tcgcccttat tccctttttt gcggcatttt     180

gccttcctgt ttttgctcac ccagaaacgc tggtgaaagt aaaagatgct gaagatcagt     240

tgggtgcacg agtgggttac atcgaactgg atctcaacag cggtaagatc cttgagagtt     300

ttcgccccga agaacgtttt ccaatgatga gcacttttaa agttctgcta tgtggcgcgg     360

tattatcccg tattgacgcc gggcaagagc aactcggtcg ccgcatacac tattctcaga     420

atgacttggt tgagtactca ccagtcacag aaaagcatct tacggatggc atgacagtaa     480

gagaattatg cagtgctgcc ataaccatga gtgataacac tgcggccaac ttacttctga     540

caacgatcgg aggaccgaag gagctaaccg cttttttgca caacatgggg gatcatgtaa     600

ctcgccttga tcgttgggaa ccggagctga atgaagccat accaaacgac gagcgtgaca     660

ccacgatgcc tgtagcaatg gcaacaacgt tgcgcaaact attaactggc gaactactta     720

ctctagcttc ccggcaacaa ttaatagact ggatggaggc ggataaagtt gcaggaccac     780

ttctgcgctc ggcccttccg gctggctggt ttattgctga taaatctgga gccggtgagc     840

gtgggtctcg cggtatcatt gcagcactgg ggccagatgg taagccctcc cgtatcgtag     900

ttatctacac gacggggagt caggcaacta tggatgaacg aaatagacag atcgctgaga     960

taggtgcctc actgattaag cattggtaac tgtcagacca agtttactca tatatacttt    1020

agattgattt aaaacttcat ttttaattta aaaggatcta ggtgaagatc ctttttgata    1080

atctcatgac caaaatccct taacgtgagt tttcgttcca ctgagcgtca gaccccgtag    1140

aaaagatcaa aggatcttct tgagatcctt tttttctgcg cgtaatctgc tgcttgcaaa    1200

caaaaaaacc accgctacca gcggtggttt gtttgccgga tcaagagcta ccaactcttt    1260

ttccgaaggt aactggcttc agcagagcgc agataccaaa tactgtcctt ctagtgtagc    1320

cgtagttagg ccaccacttc aagaactctg tagcaccgcc tacatacctc gctctgctaa    1380

tcctgttacc agtggctgct gccagtggcg ataagtcgtg tcttaccggg ttggactcaa    1440

gacgatagtt accggataag gcgcagcggt cgggctgaac ggggggttcg tgcacacagc    1500

ccagcttgga gcgaacgacc tacaccgaac tgagatacct acagcgtgag ctatgagaaa    1560

gcgccacgct tcccgaaggg agaaaggcgg acaggtatcc ggtaagcggc agggtcggaa    1620

caggagagcg cacgagggag cttccagggg gaaacgcctg gtatctttat agtcctgtcg 1680

ggtttcgcca cctctgactt gagcgtcgat ttttgtgatg ctcgtcaggg gggcggagcc 1740

tatggaaaaa cgccagcaac gcggcctttt tacggttcct ggccttttgc tggccttttg 1800

ctcacatgtt ctttcctgcg ttatcccctg attctgtgga taaccgtatt accgcctttg 1860

agtgagctga taccgctcgc cgcagccgaa cgaccgagcg cagcgagtca gtgagcgagg 1920

aagcggaaga gcgcccaata cgcaaaccgc ctctccccgc gcgttggccg attcattaat 1980

gcagctggca cgacaggttt cccgactgga aagcgggcag tgagcgcaac gcaattaatg 2040

tgagttagct cactcattag gcaccccagg ctttacactt tatgcttccg gctcgtatgt 2100

tgtgtggaat tgtgagcgga taacaatttc acacaggaaa cagctatgac catgattacg 2160

ccaagcgcgc aattaaccct cactaaaggg aacaaaagct ggagctggcg gccgcttgag 2220

cctccgaaaa agaagcgtaa ggtcgagcta gcaggtaccg ggatcctgca ggatatcgat 2280

ctcgaggccc aggcggccct cgaacccacc ggcgagaagc cttatgcctg tcctgagtgc 2340

gggaagtctt ttagcacctc gggtaatctc gtccgtcacc aacgtacgca taccggcgag 2400

aagccttacg cttgcccgga gtgtggcaag tcattcagcg actgccgtga tctcgcaagg 2460

catcaacgca cccataccgg cgagaaaccg tacgcgtgcc ctgaatgcgg taaaagcttc 2520

tctgacccag gtcacctcgt ccgccatcag aggacccata ccggcgagaa gccttacgct 2580

tgcccggagt gtggcaagtc attcagcgac tgccgtgatc tcgcaaggca tcaacgcacc 2640

cataccggcg agaaaccgta cgcatgccct gagtgtggca agtccttctc cgatccggga 2700

gcactcgttc ggcatcaacg cacccatacc ggcgagaagc cttatgcctg tcctgagtgc 2760

gggaagtctt ttagcacctc gggtaatctc gtccgtcacc aacgtacgca taccggcgag 2820

ctgggtggcg gttccggaga aaagcccggg aagaaaacct cgggccaggc cggccaacta 2880

gtgaattcga agatctcgat gcattcgcga ggcgccgtcg acgggcggcc gcgtacccaa 2940

ttcgccctat agtgagtcgt attacgcgcg ctcactggcc gtcgttttac aacgtcgtga 3000

ctgggaaaac cctggcgtta cccaacttaa tcgccttgca gcacatcccc ctttcgccag 3060

ctggcgtaat agcgaagagg cccgcaccga tcgcccttcc caacagttgc gcagcctgaa 3120

tggcgaatgg gacgcgccct gtagcggcgc attaagcgcg gcgggtgtgg tggttacgcg 3180

cagcgtgacc gctacacttg ccagcgccct agcgcccgct cctttcgctt tcttcccttc 3240

ctttctcgcc acgttcgccg gctttccccg tcaagctcta aatcgggggc tccctttagg 3300

gttccgattt agtgctttac ggcacctcga ccccaaaaaa cttgattagg gtgatggttc 3360

acgtagtggg ccatcgccct gatagacggt ttttcgccct ttgacgttgg agtccacgtt 3420

ctttaatagt ggactcttgt tccaaactgg aacaacactc aaccctatct cggtctattc 3480

ttttgattta taagggattt tgccgatttc ggcctattgg ttaaaaaatg agctgattta 3540

acaaaaattt aacgcgaatt ttaacaaaat attaacgctt acaatttag 3589

<210> 84
<211> 3589
<212> DNA
<213> Artificial Sequence

<220>
<221> source
<222> 1..3589
<223> /mol_type="unassigned DNA"
      /note="Plasmid pSKNN-SgaI/HPT-6F"
      /organism="Artificial Sequence"

<400> 84
gtggcacttt tcggggaaat gtgcgcggaa cccctatttg tttatttttc taaatacatt 60

caaatatgta tccgctcatg agacaataac cctgataaat gcttcaataa tattgaaaaa 120

ggaagagtat gagtattcaa catttccgtg tcgcccttat tccctttttt gcggcatttt 180

gccttcctgt ttttgctcac ccagaaacgc tggtgaaagt aaaagatgct gaagatcagt 240

tgggtgcacg agtgggttac atcgaactgg atctcaacag cggtaagatc cttgagagtt 300

ttcgccccga agaacgtttt ccaatgatga gcacttttaa agttctgcta tgtggcgcgg 360

tattatcccg tattgacgcc gggcaagagc aactcggtcg ccgcatacac tattctcaga 420

atgacttggt tgagtactca ccagtcacag aaaagcatct tacggatggc atgacagtaa 480

gagaattatg cagtgctgcc ataaccatga gtgataacac tgcggccaac ttacttctga 540

caacgatcgg aggaccgaag gagctaaccg cttttttgca caacatgggg gatcatgtaa 600

ctcgccttga tcgttgggaa ccggagctga atgaagccat accaaacgac gagcgtgaca 660

ccacgatgcc tgtagcaatg gcaacaacgt tgcgcaaact attaactggc gaactactta 720

ctctagcttc ccggcaacaa ttaatagact ggatggaggc ggataaagtt gcaggaccac 780

ttctgcgctc ggcccttccg gctggctggt ttattgctga taaatctgga gccggtgagc 840

gtgggtctcg cggtatcatt gcagcactgg ggccagatgg taagccctcc cgtatcgtag 900

ttatctacac gacggggagt caggcaacta tggatgaacg aaatagacag atcgctgaga 960

taggtgcctc actgattaag cattggtaac tgtcagacca agtttactca tatatacttt 1020

agattgattt aaaacttcat ttttaattta aaaggatcta ggtgaagatc ctttttgata 1080

atctcatgac caaaatccct taacgtgagt tttcgttcca ctgagcgtca gaccccgtag 1140

aaaagatcaa aggatcttct tgagatcctt tttttctgcg cgtaatctgc tgcttgcaaa 1200

caaaaaaacc accgctacca gcggtggttt gtttgccgga tcaagagcta ccaactcttt 1260

ttccgaaggt aactggcttc agcagagcgc agataccaaa tactgtcctt ctagtgtagc 1320

cgtagttagg ccaccacttc aagaactctg tagcaccgcc tacatacctc gctctgctaa 1380

tcctgttacc agtggctgct gccagtggcg ataagtcgtg tcttaccggg ttggactcaa 1440

gacgatagtt accggataag gcgcagcggt cgggctgaac ggggggttcg tgcacacagc 1500

ccagcttgga gcgaacgacc tacaccgaac tgagatacct acagcgtgag ctatgagaaa 1560

gcgccacgct tcccgaaggg agaaaggcgg acaggtatcc ggtaagcggc agggtcggaa 1620

caggagagcg cacgagggag cttccagggg gaaacgcctg gtatctttat agtcctgtcg 1680

ggtttcgcca cctctgactt gagcgtcgat ttttgtgatg ctcgtcaggg gggcggagcc 1740

tatggaaaaa cgccagcaac gcggcctttt tacggttcct ggccttttgc tggccttttg 1800

ctcacatgtt ctttcctgcg ttatcccctg attctgtgga taaccgtatt accgcctttg 1860

agtgagctga taccgctcgc cgcagccgaa cgaccgagcg cagcgagtca gtgagcgagg 1920

aagcggaaga gcgcccaata cgcaaaccgc ctctccccgc gcgttggccg attcattaat 1980

gcagctggca cgacaggttt cccgactgga aagcgggcag tgagcgcaac gcaattaatg 2040

tgagttagct cactcattag gcaccccagg ctttacactt tatgcttccg gctcgtatgt 2100

tgtgtggaat tgtgagcgga taacaatttc acacaggaaa cagctatgac catgattacg 2160

ccaagcgcgc aattaaccct cactaaaggg aacaaaagct ggagctggcg gccgcttgag 2220

cctccgaaaa agaagcgtaa ggtcgagcta gcaggtaccg ggatcctgca ggatatcgat 2280

ctcgaggccc aggcggccct cgaacccacc ggcgagaaac cgtacgcttg ccctgaatgc 2340

ggaaaaagct tctctcagtc cagcaacctc gtgaggcatc agaggaccca taccggcgag 2400

aaaccgtacg catgccctga gtgtggcaag tccttctccg atccgggagc actcgttcgg 2460

catcaacgca cccataccgg cgagaaaccg tatgcgtgcc ccgaatgcgg gaaatccttt 2520

tcgaccagcg gtgaactcgt gcgtcaccag cgtacgcata ccggcgagaa gccctacgca 2580

tgcccggaat gcggaaaatc cttctccgat cccggtaact tggttcggca tcagcgcacc 2640

cataccggcg agaaaccata cgcctgtccc gagtgcggta aaagctttag tcagcgtgcc 2700

catcttgaac gccaccagag gacccatacc ggcgagaaac cgtacgcatg ccctgagtgt 2760

ggcaagtcct tctccgatcc gggagcactc gttcggcatc aacgcaccca taccggcgag 2820

ctgggtggcg gttccggaga aaagcccggg aagaaaacct cgggccaggc cggccaacta 2880

gtgaattcga agatctcgat gcattcgcga ggcgccgtcg acgggcggcc gcgtacccaa 2940

ttcgccctat agtgagtcgt attacgcgcg ctcactggcc gtcgttttac aacgtcgtga 3000

ctgggaaaac cctggcgtta cccaacttaa tcgccttgca gcacatcccc ctttcgccag 3060

ctggcgtaat agcgaagagg cccgcaccga tcgcccttcc caacagttgc gcagcctgaa 3120

tggcgaatgg gacgcgccct gtagcggcgc attaagcgcg gcgggtgtgg tggttacgcg 3180

cagcgtgacc gctacacttg ccagcgccct agcgcccgct cctttcgctt tcttcccttc 3240

ctttctcgcc acgttcgccg gctttccccg tcaagctcta aatcgggggc tccctttagg 3300

gttccgattt agtgctttac ggcacctcga ccccaaaaaa cttgattagg gtgatggttc 3360

acgtagtggg ccatcgccct gatagacggt ttttcgccct ttgacgttgg agtccacgtt 3420

ctttaatagt ggactcttgt tccaaactgg aacaacactc aaccctatct cggtctattc 3480

ttttgattta taagggattt tgccgatttc ggcctattgg ttaaaaaatg agctgattta 3540

acaaaaattt aacgcgaatt ttaacaaaat attaacgctt acaatttag 3589

<210> 85
<211> 4786
<212> DNA
<213> Artificial Sequence

<220>
<221> source
<222> 1..4786
<223> /mol_type="unassigned DNA"
      /note="Fragment of Plasmid pCB302/AtSPO11-1-ZF3"
      /organism="Artificial Sequence"

<400> 85
ggatccgctg aagctgaagt tgccacgaag gagaaagcga gagagagacg acatcgtttt 60

tagctgtatg caaaggtttt tctcttcttt tttttggatc ggagtgttca aaggttttta 120

gtcaggggaa cggctttaat acttcaaatt aggtttggta acaatataaa gcaaatgaac 180

cctaaattac ttttgtatta ttcaaaattt caaacttatt actcattagt cattagtgaa 240

tgtactttgt attattaaaa attaaaaact gagaattatt ttttataatc tgtatattta 300

tgatttttcc attaaaacta tgttcatttg tccattctac caatgcattt ggtttgcact 360

aaaaagctta gaaagagtca taaaaaaaac aatgagcatt accttgagtt ggagactagt 420

tatctcgtat gaagcagatc ttcacccggg cttttctccg gaaccgccac ccagctcgcc 480

ggtatgcgta cgttggtgac ggacgagatt acccgaggtg ctaaaagact tcccgcactc 540

aggacaggca taaggcttct cgccggtatg ggtgcgttga tgccgaacga gtgctcccgg 600

atcggagaag gacttgccac actcagggca tgcgtacggt ttctcgccgg tatgggtgcg 660

ttgatgcctt gcgagatcac ggcagtcgct gaatgacttg ccacactccg ggcaagcgta 720

aggcttctcg ccggtatggg tcctctgatg gcggacgagg tgacctgggt cagagaagct 780

tttaccgcat tcagggcacg cgtacggttt ctcgccggta tgggtgcgtt gatgccttgc 840

gagatcacgg cagtcgctga atgacttgcc acactccggg caagcgtaag gcttctcgcc 900

ggtatgcgta cgttggtgac ggacgagatt acccgaggtg ctaaaagact tcccgcactc 960

aggacaggca taaggcttct cgccggtggg ttcgagggcc gcctgggcct cgagatcgat 1020

gggaggagag cttacttcac gacgaatctt agaaggaata tatacctctg acaaaaagga 1080

cagtgagtga actgagagtg cttcgatctc aaacttgacc cctcttttca acatcgtttc 1140

aagttccaac cttagacgac caaaatcgtt ttgatcaaga ctagtttttg tcatttggtt 1200

gaaatgtaaa tataaaaaag tcagggaagt ttttcatacc tccattgtgg catttcacgc 1260

ttcaggtagc atctaagcaa cattgcttca gttcttttct tatctgtccg gggaagttgt 1320

tacaatctca aaaaaggaag cagtgaaaaa taggaaaatc taaagattta aggcgataag 1380

gtgtttaatg atctcttacc ttcttctgtc agtggcaaaa gacactgttt gggaacacta 1440

tatacctcag agtctgaagg aaaagctccc aaccatttca tatctggtgc tcgtaatgat 1500

tcaatatcat aggccatttg cttacagaaa gaaaacaagt gacataagga aaatggttca 1560

aaatgaaatg ggtcagtgcc atatcacatt atcactatca acaaaaacaa tgcaagcggt 1620

gaaattttcc gagttcagtc atctatattt tgtaacagaa tctaaccatg gagccaaaac 1680

ggtatgtggc taggatctca aagccatatg gatcacagtc gactagacaa tgcacaggta 1740

gatgcaactt ctccatcagg agtcgcaaga acctggaaag aatctgtgtt attcgctgat 1800

atattaatca atagaagata atgtctgata aaatagtatt ccaggaacct gggattaagt 1860

tatgtacctt cttgttgaga catctggata tcctcttccc tgccaaaaac aatcgtaact 1920

ttgatgtcaa acataaaatg gaaatgaaaa ctagtcaaaa ctgaaatcag taagaatgaa 1980

gcagatttct tactgtgata acaatgcagc ggttcgtctt gcaaaacatg tcatttgcta 2040

aacgctggaa tactataaac catggatgaa agcgatttag tcatgaagaa agaaaaccat 2100

cttcctagtt actacataaa taagaggtct ttcttacctg tttctttttc cacaaccagt 2160

atgtactcag ctaagctaac aatatctgca agcaggaaaa tgacatccaa ttatagttcc 2220

tgatgccacc atagatagtg aatcttatac ttcctgatat cagctgagat aaggatttga 2280

cttctctaaa gagcataaag catcacttaa aatgattgca aatatatgta ccttcgactt 2340

cctctacaag aacaggaacg ggatatgcct gaaatgaaat cacttgcaga gttatacttc 2400

ctgatgccaa tataaaaagt gaatcttatg ttcaaattct tacagtgttc aagctgttta 2460

aacaatcaaa tttccttcca gcttccctaa attttaacca acccatcacc aacctacaac 2520

acaaaagaat aatgaggtgg atcttaaaat aaggacatgc ctcaagatag aagcattgca 2580

ctcgttatca agagcaccca cccgtttcca acagaaacct gcaaaatatc acatgaatga 2640

aaaagtaatc acatcttcaa tcgcagctta taataactct tcagaaggaa aattgaaggt 2700

gaaagtatta aagtactaac cacattcaag ttgtaccgac tacactgaaa aagaatgcat 2760

atatcaccaa ttgcacggtc cacaatggat tgtgctgtga agtagacgaa aacagtgtta 2820

ctagaaggcc aagaacacaa caaagaggga tgcataacta atatccgtat aaaaatctca 2880

atacgtattc gtttagaaga aaaacataac taaaacatta tacgttgctt ccaaattcac 2940

ctttgaaagc tgatggatgc atgtagtaga tatctctttt cgaagcatgt ctattttctt 3000

gcagaagttg ttggactatc aacaatactc ttagcaacat atctgaaagt aaaaacaccg 3060

caatgtgatg ctatattaag ataacactaa tgtacttgta gaattatctg aaacctgact 3120

caccgattct gtaggtttgt ggttccttct taagagtgaa aatttcctga ccctttggtt 3180

tatcagagct gcataagcta tagacagcag aataaaatgg taaattcatg aggtcttagt 3240

tttcaatact cgttgacgtt gtgcttaaat ccttcaaaaa tgtctcgaac caaagaccaa 3300

acacgtctga ttcttcaatc aaagaaagtg aaaacaaaat tgaataatcg agatcgaaga 3360

gagacaaacc aatcagcttc tggattcatg caatagttcc tgaattgatt gatggaaatt 3420

tttggagatc ttccttcagc cagatctaca acgaccgatt gagtaaaatc tgctcaaaaa 3480

accgaaacat agtgatgaac aacggaagag ccagcgaagt catcgactag aaatttcgac 3540

atagattaga ggacaaatta ctcaatacga ttcggaaaat tttgggattt tctgatacct 3600

ttgatccttt gaagcaaatt agtcgattct gaaatagcga attttccctc catctctttc 3660

gagtttcaaa actgaaaaat gcaatggtcg acgaattttt gtagacggga agaaaaggga 3720

tgggtgcgaa aagcttgccg ttaaaacctt cgaatttaaa catgcgtgtc gtttagttcc 3780

tactttccat gacgttgggc cattggcatt taggcccgat acgtaactga cgtgtcattt 3840

taaaaacgca tgtgttttta aacacgttgg gccgttggct tttaggcccg atccgaaact 3900

aggcaaagtg attgtgaaaa agattcatga taacaatcaa accaatataa atcgatttat 3960

tcagacttgg agacatttta gtgatgccaa tataaaataa tcatgtgcac taaatttgaa 4020

ataattaatg tgtatggtaa actagattag aattagttct agacttctag tggtcgagat 4080

ttgttcataa gctttatgct cggacacgtg aggaacatac caaaatgttc gaatctatta 4140

tgccatacac ttattttaat catttcttct ctatgataga ttgctcacct aatttatata 4200

taatctaaga atatactact tagctttaac gtattttatc cgataacgaa ttcatgatac 4260

caaaaaagtt ctaaaacaaa agaggcatct tgtattattt atagtatttc taactaaaac 4320

acaacactga taacatttaa attgctttgt tccgtttaat tagtaaacaa atgaaaagct 4380

gtcgcgtcgg tggttgggtt tcattcggaa agatttaaaa gtaaagcgca cggattttaa 4440

ggtcgttcaa catttaaatt ttttttgaaa gtaaaatcat atatcaatgc aatgtgtaat 4500

cttgtaacgt ataaactaat gagagatacc taaagaaaag aagcctcgcg cctaaacaga 4560

aacataagat taaaataagt aatatagaag cataaactta agcaagattt tattcctcgt 4620

gaaaatatta cattttgtaa ttttattttc tctagtatta aaaaaggaac aactaattaa 4680

cggaaatact taaggaatta agaacagaga ggtctcatac ttagtcaagt ttggtgaagc 4740

tatcaaagag aatgtcttcg tgttgtccgc cgacgacatt ctgcag 4786

<210> 86
<211> 4488
<212> DNA

<213> Artificial Sequence

<220>
<221> source
<222> 1..4488
<223> /mol_type="unassigned DNA"
      /note="Plasmid pSAT6/ZF-2-TS"
      /organism="Artificial Sequence"

<400> 86
tcgcgcgttt cggtgatgac ggtgaaaacc tctgacacat gcagctcccg gagacggtca      60

cagcttgtct gtaagcggat gccgggagca gacaagcccg tcagggcgcg tcagcgggtg     120

ttggcgggtg tcggggctgg cttaactatg cggcatcaga gcagattgta ctgagagtgc     180

accatatgcg gtgtgaaata ccgcacagat gcgtaaggag aaaataccgc atcaggcgcc     240

attcgccatt caggctgcgc aactgttggg aagggcgatc ggtgcgggcc tcttcgctat     300

tacgccagct ggcgaaaggg ggatgtgctg caaggcgatt aagttgggta acgccagggt     360

tttcccagtc acgacgttgt aaaacgacgg ccagtgccac ccataatacc cataatagct     420

gtttgccaac cggtcaacat gtggagcacg acacacttgt ctactccaaa aatatcaaag     480

atacagtctc agaagaccaa agggcaattg agacttttca acaaagggta atatccggaa     540

acctcctcgg attccattgc ccagctatct gtcactttat tgtgaagata gtggaaaagg     600

aaggtggctc ctacaaatgc catcattgcg ataaaggaaa ggccatcgtt gaagatgcct     660

ctgccgacag tggtcccaaa gatggacccc cacccacgag gagcatcgtg gaaaaagaag     720

acgttccaac cacgtcttca aagcaagtgg attgatgtga taacatggtg gagcacgaca     780

cacttgtcta ctccaaaaat atcaaagata cagtctcaga agaccaaagg gcaattgaga     840

cttttcaaca aagggtaata tccggaaacc tcctcggatt ccattgccca gctatctgtc     900

actttattgt gaagatagtg gaaaaggaag gtggctccta caaatgccat cattgcgata     960

aaggaaaggc catcgttgaa gatgcctctg ccgacagtgg tcccaaagat ggacccccac    1020

ccacgaggag catcgtggaa aaagaagacg ttccaaccac gtcttcaaag caagtggatt    1080

gatgtgatat ctccactgac gtaagggatg acgcacaatc ccactatcct tcgcaagacc    1140

cttcctctat ataaggaagt tcatttcatt tggagaggac gtcgagagtt ctcaacacaa    1200

catatacaaa acaaacgaat ctcaagcaat caagcattct acttctattg cagcaattta    1260

aatcatttct tttaaagcaa aagcaatttt ctgaaatttt tcaccattta cgaacgatag    1320

ccatggtgcc aaaaaagaag agaaaggtag aagacccctc tcgagcgcat ctgtaccgag    1380

ctcggtacag atgcgactag tcaaaagtga actggaggag aagaaatctg aacttcgtca    1440

taaattgaaa tatgtgcctc atgaatatat tgaattaatt gaaattgcca gaaattccac    1500

tcaggataga attcttgaaa tgaaggtaat ggaatttttt atgaaagttt atggatatag    1560

aggtaaacat ttgggtggat caaggaaacc ggacggagca atttatactg tcggatctcc    1620

tattgattac ggtgtgatcg tggatactaa agcttatagc ggaggttata atctgccaat 1680

tggccaagca gatgaaatgc aacgatatgt cgaagaaaat caaacacgaa acaaacatat 1740

caaccctaat gaatggtgga aagtctatcc atcttctgta acggaattta agtttttatt 1800

tgtgagtggt cactttaaag gaaactacaa agctcagctt acacgattaa atcatatcac 1860

taattgtaat ggagctgttc ttagtgtaga agagctttta attggtggag aaatgattaa 1920

agccggcaca ttaaccttag aggaagtgag acggaaattt aataacggcg agataaactt 1980

tggatcctaa agatccacct agtctagagt ccgcaaaaat caccagtctc tctctacaaa 2040

tctatctctc tctatttttc tccagaataa tgtgtgagta gttcccagat aagggaatta 2100

gggttcttat agggtttcgc tcatgtgttg agcatataag aaacccttag tatgtatttg 2160

tatttgtaaa atacttctat caataaaatt tctaattcct aaaaccaaaa tccagtgacg 2220

cggccgcacc cataataccc ataatagctg tttgccagta atcatggtca tagctgtttc 2280

ctgtgtgaaa ttgttatccg ctcacaattc cacacaacat acgagccgga agcataaagt 2340

gtaaagcctg gggtgcctaa tgagtgagct aactcacatt aattgcgttg cgctcactgc 2400

ccgctttcca gtcgggaaac ctgtcgtgcc agctgcatta atgaatcggc caacgcgcgg 2460

ggagaggcgg tttgcgtatt gggcgctctt ccgcttcctc gctcactgac tcgctgcgct 2520

cggtcgttcg gctgcggcga gcggtatcag ctcactcaaa ggcggtaata cggttatcca 2580

cagaatcagg ggataacgca ggaaagaaca tgtgagcaaa aggccagcaa aaggccagga 2640

accgtaaaaa ggccgcgttg ctggcgtttt tccataggct ccgcccccct gacgagcatc 2700

acaaaaatcg acgctcaagt cagaggtggc gaaacccgac aggactataa agataccagg 2760

cgtttccccc tggaagctcc ctcgtgcgct ctcctgttcc gaccctgccg cttaccggat 2820

acctgtccgc ctttctccct tcgggaagcg tggcgctttc tcaatgctca cgctgtaggt 2880

atctcagttc ggtgtaggtc gttcgctcca agctgggctg tgtgcacgaa ccccccgttc 2940

agcccgaccg ctgcgcctta tccggtaact atcgtcttga gtccaacccg gtaagacacg 3000

acttatcgcc actggcagca gccactggta acaggattag cagagcgagg tatgtaggcg 3060

gtgctacaga gttcttgaag tggtggccta actacggcta cactagaagg acagtatttg 3120

gtatctgcgc tctgctgaag ccagttacct tcggaaaaag agttggtagc tcttgatccg 3180

gcaaacaaac caccgctggt agcggtggtt tttttgtttg caagcagcag attacgcgca 3240

gaaaaaaagg atctcaagaa gatcctttga tcttttctac ggggtctgac gctcagtgga 3300

acgaaaactc acgttaaggg attttggtca tgagattatc aaaaaggatc ttcacctaga 3360

tccttttaaa ttaaaaatga agttttaaat caatctaaag tatatatgag taaacttggt 3420

ctgacagtta ccaatgctta atcagtgagg cacctatctc agcgatctgt ctatttcgtt 3480

catccatagt tgcctgactc cccgtcgtgt agataactac gatacgggag ggcttaccat 3540

ctggccccag tgctgcaatg ataccgcgag acccacgctc accggctcca gatttatcag 3600

caataaacca gccagccgga agggccgagc gcagaagtgg tcctgcaact ttatccgcct 3660

ccatccagtc tattaattgt tgccgggaag ctagagtaag tagttcgcca gttaatagtt 3720

tgcgcaacgt tgttgccatt gctacaggca tcgtggtgtc acgctcgtcg tttggtatgg 3780

cttcattcag ctccggttcc caacgatcaa ggcgagttac atgatccccc atgttgtgca 3840

aaaaagcggt tagctccttc ggtcctccga tcgttgtcag aagtaagttg gccgcagtgt 3900

tatcactcat ggttatggca gcactgcata attctcttac tgtcatgcca tccgtaagat 3960

gcttttctgt gactggtgag tactcaacca agtcattctg agaatagtgt atgcggcgac 4020

cgagttgctc ttgcccggcg tcaatacggg ataataccgc gccacatagc agaactttaa 4080

aagtgctcat cattggaaaa cgttcttcgg ggcgaaaact ctcaaggatc ttaccgctgt 4140

tgagatccag ttcgatgtaa cccactcgtg cacccaactg atcttcagca tcttttactt 4200

tcaccagcgt ttctgggtga gcaaaaacag gaaggcaaaa tgccgcaaaa aagggaataa 4260

gggcgacacg gaaatgttga atactcatac tcttcctttt tcaatattat tgaagcattt 4320

atcagggtta ttgtctcatg agcggataca tatttgaatg tatttagaaa aataaacaaa 4380

taggggttcc gcgcacattt ccccgaaaag tgccacctga cgtctaagaa accattatta 4440

tcatgacatt aacctataaa aataggcgta tcacgaggcc ctttcgtc 4488